(19) Europäisches Patentamt
European Patent Office
Office européen des brevets

(11) EP 4 722 201 A1

(12) EUROPEAN PATENT APPLICATION
published in accordance with Art. 153(4) EPC

(43) Date of publication:
**08.04.2026 Bulletin 2026/15**

(21) Application number: **24818587.8**

(22) Date of filing: **02.06.2024**

(51) International Patent Classification (IPC):
***C07D 261/04** (2006.01)* ***C07D 413/12** (2006.01)*
***A01N 43/80** (2006.01)*

(52) Cooperative Patent Classification (CPC):
**A01N 43/80; C07D 261/04; C07D 413/12**

(86) International application number:
**PCT/CN2024/096921**

(87) International publication number:
**WO 2024/251055 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.06.2023 CN 202310650531**
**08.06.2023 CN 202310674089**

(71) Applicant: **Jiangsu Flag Chemical Industry Co., Ltd**
**Nanjing, Jiangsu 210000 (CN)**

(72) Inventors:
- **ZHANG, Pu**
  **Nanjing, Jiangsu 210000 (CN)**
- **ZHANG, Feng**
  **Nanjing, Jiangsu 210000 (CN)**
- **YE, Ruyi**
  **Nanjing, Jiangsu 210000 (CN)**
- **HUANG, Jiannan**
  **Nanjing, Jiangsu 210000 (CN)**
- **BAI, Congqiang**
  **Nanjing, Jiangsu 210000 (CN)**
- **BU, Long**
  **Nanjing, Jiangsu 210000 (CN)**
- **YAO, Kaicheng**
  **Nanjing, Jiangsu 210000 (CN)**
- **XU, Dan**
  **Nanjing, Jiangsu 210000 (CN)**
- **WU, Yaojun**
  **Nanjing, Jiangsu 210000 (CN)**

(74) Representative: **Proi World Intellectual Property GmbH**
**Obermattweg 12**
**6052 Hergiswil, Kanton Nidwalden (CH)**

(54) **3-PHENYLISOXAZOLINE-5-CARBOXAMIDE COMPOUND, AND PREPARATION METHOD THEREFOR, HERBICIDAL COMPOSITION THEREOF AND USE THEREOF**

(57) The present invention relates to the field of pesticide herbicides, and specifically relates to a 3-phenylisoxazoline-5-carboxamide compound as represented by general formula (I), a stereoisomer thereof, an agriculturally acceptable salt thereof, a preparation method therefor, an herbicidal composition and the use thereof in the field of plant protection. $X_1$, $X_2$, $X_3$, $R_1$, Z and G are as defined herein.

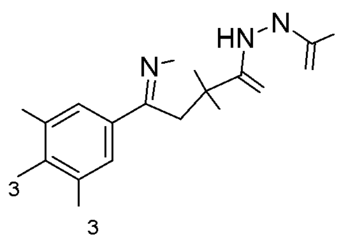

EP 4 722 201 A1

## Description

### TECHNICAL FIELD

The present invention relates to the technical field of herbicides, especially to a 3-phenylisoxazoline-5-carboxamide compound, stereoisomer thereof, agrochemically acceptable salt thereof, preparation method thereof, herbicidal composition, and use thereof.

### BACKGROUND ART

Chemical control of weed using herbicides is the most economical and effective way in weed control. However, continuously use of a single chemical herbicide or chemical herbicides having a single functional mechanism at a high dosage for a long period of time is likely to cause problems associated with evolved drug resistance and tolerance of weeds, and the development of new varieties of pesticides is the core means to solve such problems.

WO2012130798A8, WO2014048827A1, WO2019145245A1 and WO2020182723A1 each describe a 3-phenylisoxazoline-5-carboxamide compound and use thereof as a herbicide.

Later, Bayer developed the herbicide icafolin (the compound **I-31** listed in the specification of WO2018228985A1) based on this, which is a mixture of a pair of diastereoisomers:

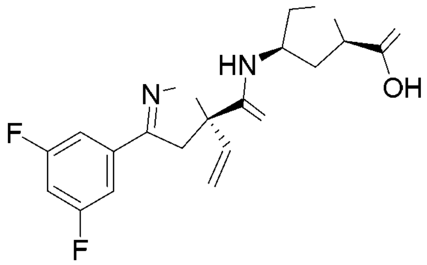

,4

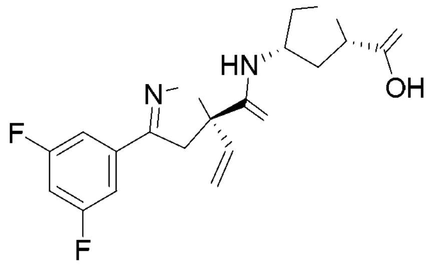

N,4N

.

Such herbicide is generally used in the form of agrochemically acceptable salts or esters, such as icafolin-methyl (the compound 1-10 listed in the specification of WO2018228985A1):

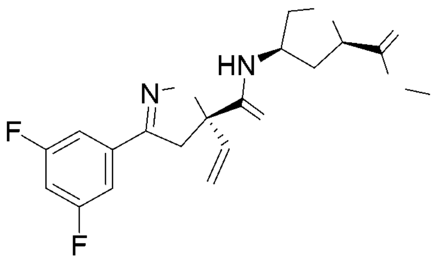

,4

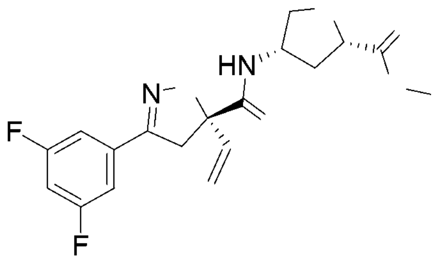

N,4N

.

WO2019145245A1 specifically discloses CK1 (the listed compound **I-011**) and CK2 (the listed compound **I-012**) in the specification:

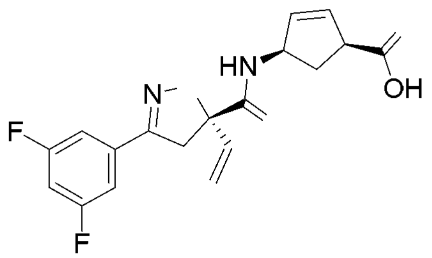

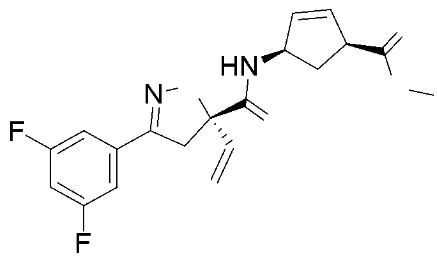

N4 N4

.

WO2019034602A1 specifically discloses CK3 (the listed compound **I-25**) and CK4 (the listed compound **I-26**) in Table 1 of the specification:

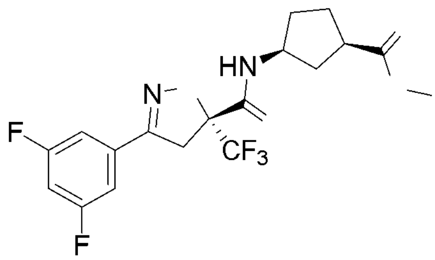

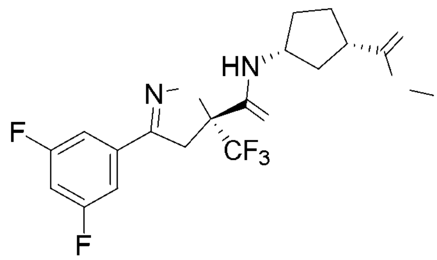

,4N N4

.

The herbicidal activity of these known compounds in the prior art, in particular at low application rates, and/or their compatibility with crop plants remain in need of improvement.

## SUMMARY OF THE INVENTION

Accordingly, it is an object of the present invention is to provide a compound having strong herbicidal activity even at relatively low application rate, sufficiently low toxicity to humans and animals, and/or high compatibility with crops, which also exhibits a broad activity spectrum for a large number of different unwanted plants.

Surprisingly, it has been found that the 3-phenylisoxazoline-5-carboxamide compound of formula (I) as defined below, stereoisomer thereof and agrochemically acceptable salt thereof exhibit excellent herbicidal activity against broad-spectrum economically important monocotyledonous and dicotyledonous annual harmful plants.

Therefore, the present invention provides a 3-phenylisoxazoline-5-carboxamide compound of formula (I), stereoisomer thereof or agriculturally acceptable salt thereof

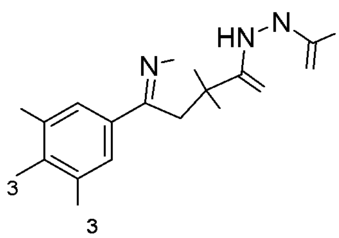

wherein

$R_1$ represents -CN or F,
or
represents $C_1$-$C_5$-alkyl, $C_3$-$C_6$-cycloalkyl, $C_2$-$C_5$-alkenyl, $C_2$-$C_5$-alkynyl or $C_1$-$C_5$-alkoxy, each of which is substituted by $m_1$ radicals selected from halogen, -CN, -OH and $C_1$-$C_5$-alkoxy;
G represents $-OR_3$ or $-NR_4R_5$;
$R_3$ represents H,

or

represents $C_1$-$C_{12}$-alkyl, $C_3$-$C_7$-cycloalkyl, $C_3$-$C_7$-cycloalkyl-$C_1$-$C_8$-alkyl, $C_2$-$C_8$-alkenyl, $C_5$-$C_6$-cycloalkenyl, -N=($C_1$-$C_6$-cycloalkyl), -N=C($C_1$-$C_5$-alkyl)$_2$, phenyl, $C_1$-$C_4$-alkyl-phenyl, aromatic heterocyclic group, $C_1$-$C_4$-alkyl-aromatic heterocyclic group or $C_2$-$C_8$-alkynyl, each of which is optionally substituted by m radicals selected from halogen, -CN, -OH, $C_1$-$C_6$-alkoxy, $C_1$-$C_6$-alkoxycarbonyl, aromatic heterocyclic group, aryl group and -S(O)$_n$$R_2$;

$R_4$ and $R_5$ independently of one another each represent H, -OH, $C_1$-$C_{12}$-alkyl, $C_1$-$C_3$-alkoxy, $C_1$-$C_6$-alkoxy-$C_1$-$C_3$-alkyl, $C_1$-$C_6$-alkoxycarbonyl-$C_1$-$C_6$-alkyl, N($C_1$-$C_3$-alkyl)$_2$ or -S(O)$_n$$R_2$,

or

$R_4$ and $R_5$ together with the nitrogen atom to which they are attached form a saturated, partially or fully unsaturated five-, six- or seven-membered ring which, in addition to the nitrogen atom, contains r carbon atoms and o oxygen atoms, and which is optionally substituted by m radicals selected from halogen, $C_1$-$C_6$-alkyl, halogenated $C_1$-$C_6$-alkyl, oxo and -$CO_2R_6$;

when Z is selected from Z-1 to Z-4, where Z-1 to Z-4 have the following meaning:

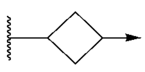 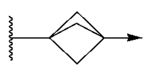 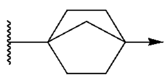 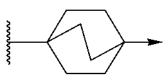

OH Z-2 Z-2

$X_3$ represents H, F, Cl, Br or I;

$X_1$ and $X_2$ independently of one another each represent H, F, Cl, Br, I, -OH, -CN, -$NO_2$, - S(O)$_n$$R_2$ or -$CO_2R_6$,

or

represent $C_1$-$C_3$-alkyl, $C_1$-$C_3$-alkoxy, $C_3$-$C_4$-cycloalkyl, $C_2$-$C_3$-alkenyl or $C_2$-$C_3$-alkynyl, each of which is substituted by m radicals selected from F, Cl, Br and I;

$R_6$ represents H,

or

represents $C_1$-$C_8$-alkyl, $C_3$-$C_6$-cycloalkyl, $C_3$-$C_8$-alkenyl or $C_3$-$C_8$-alkynyl, each of which is optionally substituted by m radicals selected from halogen, -CN and $C_1$-$C_2$-alkoxy;

when Z is selected from Z-5 to Z-7, where Z-5 to Z-7 have the following meaning:

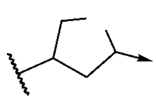 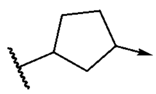 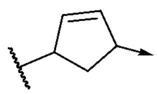

$X_3$ represents H or F;

$X_1$ represents F, Cl, Br, I, -$CH_3$, -CN, -$NO_2$, -S(O)$_n$$R_2$ or -OS(O)$_n$$R_2$;

$X_2$ represents -$CF_3$, -$CF_2H$, -$OCF_3$, -$OCF_2H$ or -$OCFH_2$;

$R_2$ represents $C_1$-$C_4$-alkyl or $C_3$-$C_4$-cycloalkyl, each of which is substituted by m radicals selected from F and Cl;

where the arrow in each case denotes a bond to the group CO-G of formula (I);

m is 0, 1, 2, 3, 4 or 5;

n is 0, 1 or 2;

o is 0, 1 or 2; and

r is 3, 4, 5 or 6.

Preferably,

$R_1$ represents $C_1$-$C_3$-alkyl, $C_3$-$C_4$-cycloalkyl, $C_2$-$C_3$-alkenyl, $C_2$-$C_3$-alkynyl or $C_1$-$C_3$-alkoxy, each of which is substituted by $m_1$ radicals selected from halogen, -CN, -OH and $C_1$-$C_2$-alkoxy;

G represents -$OR_3$ or -$NR_4R_5$;

$R_3$ represents H,

or

represents $C_1$-$C_{10}$-alkyl, $C_3$-$C_6$-cycloalkyl, $C_3$-$C_6$-cycloalkyl-$C_1$-$C_6$-alkyl, $C_2$-$C_6$-alkenyl, $C_5$-$C_6$-cycloalkenyl, -N=($C_1$-$C_5$-cycloalkyl), -N=C($C_1$-$C_3$-alkyl)$_2$, phenyl, $C_1$-$C_3$-alkylphenyl, aromatic heterocyclic group, $C_1$-$C_3$-alkyl aromatic heterocyclic group or $C_2$-$C_6$-alkynyl, each of which is substituted by $m_3$ radicals selected from F, Cl, Br, I, -CN, -OH, -S(O)$_n$$R_2$, $C_1$-$C_4$-alkoxy, aryl group and aromatic heterocyclic group;

$R_4$ and $R_5$ independently of one another each represent H, -OH, $C_1$-$C_6$-alkyl, $C_1$-$C_3$-alkoxy, $C_1$-$C_3$-alkoxy $C_1$-$C_3$-alkyl or -S(O)$_n$$R_2$,

or

$R_4$ and $R_5$ together with the nitrogen atom to which they are attached form a saturated, partially or fully unsaturated five- or six-membered ring which, in addition to the nitrogen atom, contains r carbon atoms and o oxygen atoms, and which is optionally substituted by $m_4$ radicals selected from halogen, $C_1$-$C_6$-alkyl, halogenated $C_1$-$C_6$-alkyl, oxo and $-CO_2R_6$;

$R_2$ represents $C_1$-$C_4$ alkyl;

$R_6$ represents H,

or

represents $C_1$-$C_6$-alkyl, $C_3$-$C_6$-cycloalkyl, $C_3$-$C_8$-alkenyl or $C_3$-$C_8$-alkynyl, each of which is optionally substituted by $m_5$ radicals selected from halogen, -CN and $C_1$-$C_2$-alkoxy;

$m_1$ is 0, 1, 2 or 3;

$m_3$ is 0, 1, 2, 3, 4, 5, 6, 7 or 8;

$m_4$ is 0, 1, 2, 3 or 4;

$m_5$ is 0, 1, 2 or 3;

n is 0, 1 or 2;

o is 0, 1 or 2; and

r is 3, 4 or 5.

More preferably,

$R_1$ represents $C_1$-$C_3$-alkyl, $C_2$-$C_3$-alkenyl, $C_2$-$C_3$-alkynyl or $C_1$-$C_3$-alkoxy, each of which is substituted by $m_1$ radicals selected from F, Cl and Br;

G represents $-OR_3$ or $-NR_4R_5$;

$R_3$ represents H,

or

represents $C_1$-$C_7$-alkyl, $C_3$-$C_6$-cycloalkyl, $C_3$-$C_6$-cycloalkyl-$C_1$-$C_6$-alkyl, $C_2$-$C_6$-alkenyl, $C_5$-$C_6$-cycloalkenyl, $C_2$-$C_6$-alkynyl, $C_1$-$C_3$-alkoxy-$C_1$-$C_3$-alkyl, -N=C($C_1$-$C_3$-alkyl)$_2$, phenyl, $C_1$-$C_3$-alkyl-phenyl, aromatic heterocyclic group, $C_1$-$C_3$-alkyl-aromatic heterocyclic group, phenyl-$C_1$-$C_3$-alkyl or aromatic heterocyclic group-$C_1$-$C_3$-alkyl, each of which is substituted by $m_3$ radicals selected from F, Cl, Br, I, -CN, -OH, $-OCH_3$ or $-S(O)_nR_2$;

$R_4$ and $R_5$ independently of one another each represent H, -OH, $C_1$-$C_6$-alkyl, $C_1$-$C_3$-alkoxy or $-S(O)_nR_2$,

or

$R_4$ and $R_5$ together with the nitrogen atom to which they are attached form a saturated five- or six-membered ring which, in addition to the nitrogen atom, contains four or five carbon atoms;

$R_2$ represents $C_1$-$C_3$-alkyl;

$m_1$ is 0, 1, 2 or 3;

$m_3$ is 0, 1, 2, 3, 4, 5, 6, 7 or 8; and

n is 0, 1 or 2.

Further preferably,

$R_1$ represents $-CH_3$, $-CH=CH_2$, $-CF=CH_2$, $-CF_3$, $-CF_2H$, $-CH_2F$, $-CH_2Cl$, $-CF_2CH_3$ or $-OCH_3$;

G represents $-OR_3$ or $-NR_4R_5$;

$R_3$ represents H,

or

represents $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$, $-(CH_2)_4CH_3$, $-(CH_2)_5CH_3$, $-(CH_2)_6CH_3$, $-CH_2CH=CH_2$, $-CH_2CH_2CH=CH_2$, $-CH_2CH=CHCH_3$, $-CH(CH_3)CH=CH_2$, $-CH_2C\equiv CH$, (*S*)-$CH(CH_3)C\equiv CH$, (*R*)-$CH(CH_3)C\equiv CH$, $-CH_2C\equiv CCH_3$, $-CH(CH_2CH_3)C\equiv CH$, $-CH_2CH_2S(O)_nCH_3$, $-CH_2CH_2S(O)_nCH_2CH_3$, cyclopropyl, cyclobutyl, cyclopentyl, cyclohexyl, $-CH_2$-cyclopropyl, $-CH_2$-cyclobutyl, $-CH_2$-cyclopentyl, $-CH_2$-cyclohexyl, $-CH_2CH_2CN$, $-CH_2CF_3$, $-CH_2CF_2H$, $-CH_2CH_2F$, $-CH_2CH_2Cl$, $-CH_2CH_2Br$, $-CH_2CCl_3$, $-CH_2CF_2CF_3$, $-CH_2(CF_2)_2H$, $-CH_2(CF_2)_3H$, $-CH_2(CF_2)_4H$, $-CH_2CH_2OCH_3$, $-(CH_2)_3OCH_3$, (*S*)-$CH_2CH(CH_3)OCH_3$, (*S*)-$CH(CH_3)CH_2OCH_3$, (*R*)-$CH_2CH(CH_3)OCH_3$, (*R*)-$CH(CH_3)CH_2OCH_3$, $-CH_2CH_2OCH_2CH_3$, $-N=C(CH_3)_2$, $-C_6H_5$, *p*-$CH_3$-$C_6H_4$-, *p*-F-$C_6H_4$-, *p*-Cl-$C_6H_4$-, *p*-Br-$C_6H_4$-, $-CH_2$-$C_6H_5$, *p*-$CH_3$-$C_6H_4$-$CH_2$-, *p*-F-$C_6H_4$-$CH_2$-, *p*-Cl-$C_6H_4$-$CH_2$-, *p*-Br-$C_6H_4$-$CH_2$-, *p*-CN-$C_6H_4$-$CH_2$-, *p*-$CH_3O$-$C_6H_4$-$CH_2$- or 3-pyridyl;

n is 0, 1, or 2;

or

$R_4$ and $R_5$ independently of one another each represent H, $-CH_3$, $-CH_2CH_3$, -OH, $-OCH_3$, $-OCH_2CH_3$, $-OCH(CH_3)_2$ or $-SO_2CH_3$,

or

$R_4$ and $R_5$ together with the nitrogen atom to which they are attached form a saturated five- or six-membered ring which, in addition to the nitrogen atom, contains four or five carbon atoms.

Further preferably,

Z represents Z-1;
$X_3$ represents H or F;
$X_1$ and $X_2$ independently of one another each represent H, F, Cl, Br or -CN,
or
represent $C_1$-$C_3$-alkyl or $C_1$-$C_3$-alkoxy, each of which is substituted by $m_6$ radicals selected from F, Cl and Br;
$m_6$ is 0, 1, 2, or 3.

Further preferably,

Z represents Z-1;
$X_3$ represents H or F;
$X_1$ and $X_2$ independently of one another each represent H, F, Cl, Br, $-CH_3$, -CN, $-OCH_3$, $-CF_3$, $CF_2H$, $-OCF_3$ or $-OCF_2H$.

Further preferably,

Z represents Z-5 or Z-7;
$X_3$ represents H;
$X_1$ represents F, Cl or Br; and
$X_2$ represents $-CF_3$, $-CF_2H$, $-OCF_3$ or $-OCF_2H$.

In the definitions of the compound of general formula (I) given above, the terms used are gathered and generally defined as follows.

Alkyl means saturated straight-chain or branched hydrocarbyl radicals having the number of carbon atoms specified in each case, e.g. $C_1$-$C_6$-alkyl such as methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl.

Halogen-substituted alkyl means straight-chain or branched alkyl groups where some or all of the hydrogen atoms in these groups may be replaced by halogen atoms, e.g. $C_1$-$C_2$-haloalkyl such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro, 2-difluoroethyl, 2,2-dichloro-2-fluoroethyl, 2,2,2-trichloroethyl, pentafluoroethyl and 1,1,1-trifluoroprop-2-yl.

Alkenyl means unsaturated straight-chain or branched hydrocarbyl radicals having the number of carbon atoms specified in each case and one double bond in any position, e.g. $C_2$-$C_6$-alkenyl such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

Alkynyl means straight-chain or branched hydrocarbyl radicals having the number of carbon atoms specified in each case and one triple bond in any position, e.g. $C_2$-$C_6$-alkynyl such as ethynyl, 1-propynyl, 2-propynyl (or propargyl), 1-butynyl, 2-butynyl, 3-butynyl, 1-methyl-2-propynyl, 1-pentynyl, 2-pentynyl, 3-pentynyl, 4-pentynyl, 3-methyl-1-butynyl, 1-methyl-2-butynyl, 1-methyl-3-butynyl, 2-methyl-3-butynyl, 1,1-dimethyl-2-propynyl, 1-ethyl-2-propynyl, 1-hexynyl, 2-hexynyl, 3-hexynyl, 4-hexynyl, 5-hexynyl, 3-methyl-1-pentynyl, 4-methyl-1-pentynyl, 1-methyl-2-pentynyl, 4-methyl-2-pentynyl, 1-methyl-3-pentynyl, 2-methyl-3-pentynyl, 1-methyl-4-pentynyl, 2-methyl-4-pentynyl, 3-methyl-4-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl.

Cycloalkyl means a carbocyclic saturated ring system having preferably 3-8 ring carbon atoms, for example cyclopropyl, cyclobutyl, cyclopentyl or cyclohexyl. In the case of optionally substituted cycloalkyl, cyclic systems with substituents are included, also including substituents with a double bond on the cycloalkyl radical, for example an alkylidene group such as methylidene.

In the case of optionally substituted cycloalkyl, polycyclic aliphatic systems are also included, for example bicyclo[1.1.0]butan-1-yl, bicyclo[1.1.0]butan-2-yl, bicyclo[2.1.0]pentan-1-yl, bicyclo[2.1.0]pentan-2-yl, bicyclo[2.1.0]pentan-5-yl, bicyclo[2.2.1]hept-2-yl (norbornyl), adamantan-1-yl and adamantan-2-yl.

In the case of substituted cycloalkyl, spirocyclic aliphatic systems are also included, for example spiro[2.2]pent-1-yl, spiro[2.3]hex-1-yl and spiro[2.3]hex-4-yl, 3-spiro[2.3]hex-5-yl.

Cycloalkenyl means a carbocyclic, nonaromatic, partially unsaturated ring system having preferably 4-8 carbon atoms, e.g. 1-cyclobutenyl, 2-cyclobutenyl, 1-cyclopentenyl, 2-cyclopentenyl, 3-cyclopentenyl, or 1-cyclohexenyl, 2-cyclohexenyl, 3-cyclohexenyl, 1,3-cyclohexadienyl or 1,4-cyclohexadienyl, also including substituents with a double bond on the cycloalkenyl radical, for example an alkylidene group such as methylidene. In the case of optionally substituted cycloalkenyl, the elucidations for substituted cycloalkyl apply correspondingly.

Alkoxy means saturated straight-chain or branched alkoxy radicals having the number of carbon atoms specified in each case, for example $C_1$-$C_6$-alkoxy such as methoxy, ethoxy, propoxy, 1-methylethoxy, butoxy, 1-methylpropoxy, 2-methylpropoxy, 1,1-dimethylethoxy, pentoxy, 1-methylbutoxy, 2-methylbutoxy, 3-methylbutoxy, 2,2-dimethylpropoxy, 1-ethylpropoxy, hexoxy, 1,1-dimethylpropoxy, 1,2-dimethylpropoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy. Halogen-substituted alkoxy means straight-chain or branched alkoxy radicals having the number of carbon atoms specified in each case, where some or all of the hydrogen atoms in these groups may be replaced by halogen atoms as specified above, e.g. $C_1$-$C_2$-haloalkoxy such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-1,2-difluoroethoxy, 2,2-dichloro-2-fluoroethoxy, 2,2,2-trichloroethoxy, pentafluoroethoxy and 1,1,1-trifluoroprop-2-oxy.

Aryl means phenyl which is optionally substituted by 0-5 radicals from the group consisting of fluorine, chlorine, bromine, iodine, cyano, hydroxy, ($C_1$-$C_3$)-alkyl, ($C_1$-$C_3$)-alkoxy, ($C_3$-$C_4$)-cycloalkyl, ($C_2$-$C_3$)-alkenyl and ($C_2$-$C_3$)-alkynyl.

The term "halogen" means fluorine, chlorine, bromine or iodine. If the term is used for a radical, "halogen" means a fluorine, chlorine, bromine or iodine atom.

According to the nature of the substituents and the way in which they are joined, the compounds of the formula (I) may be present as stereoisomers. If, for example, one or more asymmetrically substituted carbon atoms and/or sulfoxides are present, enantiomers and diastereomers may occur. Stereoisomers can be obtained from the mixtures obtained in the preparation by customary separation methods, for example by chromatographic separation processes. It is likewise possible to selectively prepare stereoisomers by using stereoselective reactions with use of optically active starting materials and/or auxiliaries.

The invention also relates to all stereoisomers and mixtures thereof which are encompassed by the formula (I) but not defined specifically. However, the following text will, for the sake of simplicity, always mention compounds of the formula (I), even though this is understood as meaning not only the pure compounds, but also, if appropriate, mixtures with various amounts of isomeric compounds.

According to the nature of the substituents defined above, the compounds of the formula (I) have acidic properties and can form salts, and if appropriate also internal salts or adducts with inorganic or organic bases or with metal ions. If the compounds of the formula (I) carry hydroxyl, carboxyl or other groups which induce acidic properties, these compounds can be reacted with bases to give salts. Suitable bases are, for example, hydroxides, carbonates, bicarbonates of the alkali metals and alkaline earth metals, in particular those of sodium, potassium, magnesium and calcium, furthermore ammonia, primary, secondary and tertiary amines having ($C_1$-$C_4$)-alkyl groups, mono-, di- and trialkanolamines of ($C_1$-$C_4$)-alkanols, choline and chlorocholine, and also organic amines such as trialkylamines, morpholine, piperidine or pyridine. These salts are compounds in which the acidic hydrogen is replaced by an agriculturally suitable cation, for example metal salts, especially alkali metal salts or alkaline earth metal salts, in particular sodium and potassium salts, or else ammonium salts, salts with organic amines or quaternary ammonium salts, for example with cations of the formula [NRR'R"R‴] in which R to R‴ each independently of one another represent an organic radical, in particular alkyl, aryl, aralkyl or alkylaryl. Also suitable are alkylsulfonium and alkylsulfoxonium salts, such as ($C_1$-$C_4$)-trialkylsulfonium and

($C_1$-$C_4$)-trialkylsulfoxonium salts.

The compounds of the formula (I) can form salts by addition of a suitable inorganic or organic acid, for example mineral acids, for example HCl, HBr, $H_2SO_4$, $H_3PO$ or $HNO_3$, or organic acids, for example carboxylic acids such as formic acid, acetic acid, propionic acid, oxalic acid, lactic acid or salicylic acid or sulfonic acids, for example p-toluenesulfonic acid, onto a basic group, for example amino, alkylamino, dialkylamino, piperidino, morpholino or pyridino. In such a case, these salts comprise the conjugated base of the acid as the anion.

Suitable substituents present in deprotonated form, such as, for example, sulfonic acids or carboxylic acids, may form inner salts with groups which for their part can be protonated, such as amino groups.

If a group is polysubstituted by radicals, this means that this group is substituted by one or more identical or different radicals from those mentioned.

In all the formulae specified hereinafter, the substituents and symbols have the same meaning as described in formula (I), unless defined differently. Arrows in a chemical formula denote the points at which it is joined to the rest of the molecule.

There follows a description of preferred, particularly preferred and very particularly preferred definitions of each of the individual substituents. The other substituents of the general formula (I) which are not specified hereinafter have the definition given above.

Examples of the compound of the general formula (I) are shown in the form of table below. Table 1 below lists the substituents in formula (I) defined in general terms.

Table 1 Examples of the compounds of the general formula (I)

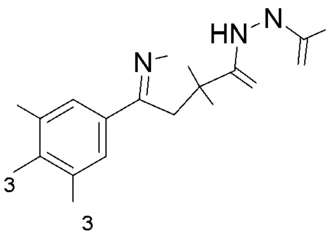

Table 1

| Example No. | $X_1$ | $X_2$ | $X_3$ | $R_1$ | Z | G | Z configuration |
|---|---|---|---|---|---|---|---|
| **I-1** | F | F | H | $-CH=CH_2$ | Z-1 | OH | cis |
| **I-2** | F | F | H | ($S$)-$CH=CH_2$ | Z-1 | OH | cis |
| **I-3** | F | F | H | $-CH=CH_2$ | Z-1 | $OCH_3$ | cis |
| **I-4** | F | F | H | ($S$)-$CH=CH_2$ | Z-1 | $OCH_3$ | cis |
| **I-5** | F | F | H | ($S$)-$CH=CH_2$ | Z-1 | $OCH_2CH_3$ | cis |
| **I-6** | F | F | H | ($S$)-$CH=CH_2$ | Z-1 | $O(CH_2)_3CH_3$ | cis |
| **I-7** | F | F | H | ($S$)-$CH=CH_2$ | Z-1 | $O(CH_2)_6CH_3$ | cis |
| **I-8** | F | F | H | $-CH=CH_2$ | Z-1 | $OCH_2CH_2SCH_3$ | cis |
| **I-9** | F | F | H | $-CH=CH_2$ | Z-1 | $OCH_2CH_2SOCH_3$ | cis |
| **I-10** | F | F | H | $-CH=CH_2$ | Z-1 | $OCH_2CH_2SO_2CH_3$ | cis |
| **I-11** | F | F | H | ($S$)-$CH=CH_2$ | Z-1 | ($R$)-$OCH(CH_3)C{\equiv}CH$ | cis |
| **I-12** | F | F | H | ($S$)-$CH=CH_2$ | Z-1 | ($S$)-$OCH(CH_3)C{\equiv}CH$ | cis |
| **I-13** | F | F | H | ($S$)-$CH=CH_2$ | Z-1 | 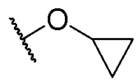 | cis |
| **I-14** | F | F | H | ($S$)-$CH=CH_2$ | Z-1 | 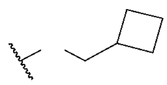 | cis |
| **I-15** | F | F | H | ($S$)-$CH=CH_2$ | Z-1 | $OCH_2CF_3$ | cis |

(continued)

| Example No. | $X_1$ | $X_2$ | $X_3$ | $R_1$ | Z | G | Z configuration |
|---|---|---|---|---|---|---|---|
| **I-16** | F | F | H | (*S*)-$CH=CH_2$ | Z-1 | $OCH_2(CF_2)_4H$ | cis |
| **I-17** | F | F | H | (*S*)-$CH=CH_2$ | Z-1 | $O(CH_2)_3OCH_3$ | cis |
| **I-18** | F | F | H | (*S*)-$CH=CH_2$ | Z-1 | (*S*)-$OCH(CH_3)CH_2OCH_3$ | cis |
| **I-19** | F | F | H | (*S*)-$CH=CH_2$ | Z-1 | (*R*)-$OCH(CH_3)CH_2OCH_3$ | cis |
| **I-20** | F | F | H | (*S*)-$CH=CH_2$ | Z-1 | $OCH_2CH_2CN$ | cis |
| **I-21** | F | F | H | (*S*)-$CH=CH_2$ | Z-1 | $NH_2$ | cis |
| **I-22** | F | F | H | (*S*)-$CH=CH_2$ | Z-1 | NHOH | cis |
| **I-23** | F | F | H | (*S*)-$CH=CH_2$ | Z-1 | $NHOCH_3$ | cis |
| **I-24** | F | F | H | (*S*)-$CH=CH_2$ | Z-1 | $NHCH_3$ | cis |
| **I-25** | F | F | H | (*S*)-$CH=CH_2$ | Z-1 | $NHSO_2CH_3$ | cis |
| **I-26** | F | F | H | (*S*)-$CH=CH_2$ | Z-1 | 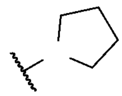 | cis |
| **I-27** | F | Cl | H | $-CH=CH_2$ | Z-1 | OH | cis |
| **I-28** | F | Cl | H | (*S*)-$CH=CH_2$ | Z-1 | OH | cis |
| **I-29** | F | Cl | H | $-CH=CH_2$ | Z-1 | $OCH_3$ | cis |
| **I-30** | F | Cl | H | (*S*)-$CH=CH_2$ | Z-1 | $OCH_3$ | cis |
| **I-31** | F | Cl | H | (*S*)-$CH=CH_2$ | Z-1 | $O(CH_2)_4CH_3$ | cis |
| **I-32** | F | Cl | H | (*S*)-$CH=CH_2$ | Z-1 | $OCH_2CH=CHCH_3$ | cis |
| **I-33** | F | Cl | H | (*S*)-$CH=CH_2$ | Z-1 | (*R*)-$OCH(CH_3)CH_2OCH_3$ | cis |
| **I-34** | F | Cl | H | (*S*)-$CH=CH_2$ | Z-1 | (*S*)-$OCH(CH_3)CH_2OCH_3$ | cis |
| **I-35** | F | Cl | H | (*S*)-$CH=CH_2$ | Z-1 | 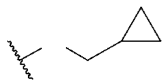 | cis |
| **I-36** | F | Cl | H | (*S*)-$CH=CH_2$ | Z-1 | $NHOCH(CH_3)_2$ | cis |
| **I-37** | Cl | Cl | H | $-CH=CH_2$ | Z-1 | OH | cis |
| **I-38** | Cl | Cl | H | (*S*)-$CH=CH_2$ | Z-1 | OH | cis |
| **I-39** | Cl | Cl | H | $-CH=CH_2$ | Z-1 | $OCH_3$ | cis |
| **I-40** | Cl | Cl | H | (*S*)-$CH=CH_2$ | Z-1 | $OCH_3$ | cis |
| **I-41** | F | CN | H | $-CH=CH_2$ | Z-1 | OH | cis |
| **I-42** | F | CN | H | (*S*)-$CH=CH_2$ | Z-1 | OH | cis |
| **I-43** | F | CN | H | $-CH=CH_2$ | Z-1 | $OCH_3$ | cis |
| **I-44** | F | CN | H | (*S*)-$CH=CH_2$ | Z-1 | $OCH_3$ | cis |
| **I-45** | Cl | CN | H | $-CH=CH_2$ | Z-1 | OH | cis |
| **I-46** | Cl | CN | H | (*S*)-$CH=CH_2$ | Z-1 | OH | cis |
| **I-47** | Cl | CN | H | $-CH=CH_2$ | Z-1 | $OCH_3$ | cis |
| **I-48** | Cl | CN | H | (*S*)-$CH=CH_2$ | Z-1 | $OCH_3$ | cis |
| **I-49** | F | $CF_3$ | H | $-CH=CH_2$ | Z-1 | OH | cis |
| **I-50** | F | $CF_3$ | H | (*S*)-$CH=CH_2$ | Z-1 | OH | cis |
| **I-51** | F | $CF_3$ | H | $-CH=CH_2$ | Z-1 | $OCH_3$ | cis |

(continued)

| Example No. | $X_1$ | $X_2$ | $X_3$ | $R_1$ | Z | G | Z configuration |
|---|---|---|---|---|---|---|---|
| **I-52** | F | $CF_3$ | H | (*S*)-CH=$CH_2$ | Z-1 | $OCH_3$ | cis |
| **I-53** | Cl | $CF_3$ | H | -CH=$CH_2$ | Z-1 | OH | cis |
| **I-54** | Cl | $CF_3$ | H | (*S*)-CH=$CH_2$ | Z-1 | OH | cis |
| **I-55** | Cl | $CF_3$ | H | -CH=$CH_2$ | Z-1 | $OCH_3$ | cis |
| **I-56** | Cl | $CF_3$ | H | (*S*)-CH=$CH_2$ | Z-1 | $OCH_3$ | cis |
| **I-57** | F | $OCF_3$ | H | -CH=$CH_2$ | Z-1 | OH | cis |
| **I-58** | F | $OCF_3$ | H | (*S*)-CH=$CH_2$ | Z-1 | OH | cis |
| **I-59** | F | $OCF_3$ | H | -CH=$CH_2$ | Z-1 | $OCH_3$ | cis |
| **I-60** | F | $OCF_3$ | H | (*S*)-CH=$CH_2$ | Z-1 | $OCH_3$ | cis |
| **I-61** | F | $OCF_3$ | H | (*S*)-CH=$CH_2$ | Z-1 | $OCH_2C{\equiv}CCH_3$ | cis |
| **I-62** | F | $OCF_3$ | H | (*S*)-CH=$CH_2$ | Z-1 | 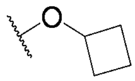 | cis |
| **I-63** | F | $OCF_3$ | H | (*S*)-CH=$CH_2$ | Z-1 | 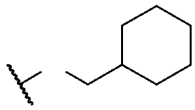 | cis |
| **I-64** | F | $OCF_3$ | H | (*S*)-CH=$CH_2$ | Z-1 | $OCH_2CF_2CF_2H$ | cis |
| **I-65** | F | $OCF_3$ | H | (*S*)-CH=$CH_2$ | Z-1 | $OCH_2CH_2OCH_2CH_3$ | cis |
| **I-66** | F | $OCF_3$ | H | (*S*)-CH=$CH_2$ | Z-1 | $N(CH_3)OCH_3$ | cis |
| **I-67** | Cl | $OCF_3$ | H | -CH=$CH_2$ | Z-1 | OH | cis |
| **I-68** | Cl | $OCF_3$ | H | (*S*)-CH=$CH_2$ | Z-1 | OH | cis |
| **I-69** | Cl | $OCF_3$ | H | -CH=$CH_2$ | Z-1 | $OCH_3$ | cis |
| **I-70** | Cl | $OCF_3$ | H | (*S*)-CH=$CH_2$ | Z-1 | $OCH_3$ | cis |
| **I-71** | F | $OCF_2H$ | H | -CH=$CH_2$ | Z-1 | OH | cis |
| **I-72** | F | $OCF_2H$ | H | (*S*)-CH=$CH_2$ | Z-1 | OH | cis |
| **I-73** | F | $OCF_2H$ | H | -CH=$CH_2$ | Z-1 | $OCH_3$ | cis |
| **I-74** | F | $OCF_2H$ | H | (*S*)-CH=$CH_2$ | Z-1 | $OCH_3$ | cis |
| **I-75** | Cl | $OCF_2H$ | H | -CH=$CH_2$ | Z-1 | OH | cis |
| **I-76** | Cl | $OCF_2H$ | H | (*S*)-CH=$CH_2$ | Z-1 | OH | cis |
| **I-77** | Cl | $OCF_2H$ | H | -CH=$CH_2$ | Z-1 | $OCH_3$ | cis |
| **I-78** | Cl | $OCF_2H$ | H | (*S*)-CH=$CH_2$ | Z-1 | $OCH_3$ | cis |
| **I-79** | F | H | H | -CH=$CH_2$ | Z-1 | OH | cis |
| **I-80** | F | H | H | (*S*)-CH=$CH_2$ | Z-1 | OH | cis |
| **I-81** | F | H | H | -CH=$CH_2$ | Z-1 | $OCH_3$ | cis |
| **I-82** | F | H | H | (*S*)-CH=$CH_2$ | Z-1 | $OCH_3$ | cis |
| **I-83** | F | $CH_3$ | H | -CH=$CH_2$ | Z-1 | OH | cis |
| **I-84** | F | $CH_3$ | H | (*S*)-CH=$CH_2$ | Z-1 | OH | cis |
| **I-85** | F | $CH_3$ | H | -CH=$CH_2$ | Z-1 | $OCH_3$ | cis |
| **I-86** | F | $CH_3$ | H | (*S*)-CH=$CH_2$ | Z-1 | $OCH_3$ | cis |
| **I-87** | F | F | F | -CH=$CH_2$ | Z-1 | OH | cis |

(continued)

| Example No. | $X_1$ | $X_2$ | $X_3$ | $R_1$ | Z | G | Z configuration |
|---|---|---|---|---|---|---|---|
| **I-88** | F | F | F | (*S*)-$CH=CH_2$ | Z-1 | OH | cis |
| **I-89** | F | F | F | $-CH=CH_2$ | Z-1 | $OCH_3$ | cis |
| **I-90** | F | F | F | (*S*)-$CH=CH_2$ | Z-1 | $OCH_3$ | cis |
| **I-91** | Cl | F | F | $-CH=CH_2$ | Z-1 | OH | cis |
| **I-92** | Cl | F | F | (*S*)-$CH=CH_2$ | Z-1 | OH | cis |
| **I-93** | Cl | F | F | $-CH=CH_2$ | Z-1 | $OCH_3$ | cis |
| **I-94** | Cl | F | F | (*S*)-$CH=CH_2$ | Z-1 | $OCH_3$ | cis |
| **I-95** | Cl | Cl | F | $-CH=CH_2$ | Z-1 | OH | cis |
| **I-96** | Cl | Cl | F | (*S*)-$CH=CH_2$ | Z-1 | OH | cis |
| **I-97** | Cl | Cl | F | $-CH=CH_2$ | Z-1 | $OCH_3$ | cis |
| **I-98** | Cl | Cl | F | (*S*)-$CH=CH_2$ | Z-1 | $OCH_3$ | cis |
| **I-99** | H | H | H | $-CH=CH_2$ | Z-1 | OH | cis |
| **I-100** | H | H | H | (*S*)-$CH=CH_2$ | Z-1 | OH | cis |
| **I-101** | H | H | H | $-CH=CH_2$ | Z-1 | $OCH_3$ | cis |
| **I-102** | H | H | H | (*S*)-$CH=CH_2$ | Z-1 | $OCH_3$ | cis |
| **I-103** | $CH_3$ | $CH_3$ | H | $-CH=CH_2$ | Z-1 | OH | cis |
| **I-104** | $CH_3$ | $CH_3$ | H | (*S*)-$CH=CH_2$ | Z-1 | OH | cis |
| **I-105** | $CH_3$ | $CH_3$ | H | $-CH=CH_2$ | Z-1 | $OCH_3$ | cis |
| **I-106** | $CH_3$ | $CH_3$ | H | (*S*)-$CH=CH_2$ | Z-1 | $OCH_3$ | cis |
| **I-107** | $OCH_3$ | $OCH_3$ | H | $-CH=CH_2$ | Z-1 | OH | cis |
| **I-108** | $OCH_3$ | $OCH_3$ | H | (*S*)-$CH=CH_2$ | Z-1 | OH | cis |
| **I-109** | $OCH_3$ | $OCH_3$ | H | $-CH=CH_2$ | Z-1 | $OCH_3$ | cis |
| **I-110** | $OCH_3$ | $OCH_3$ | H | (*S*)-$CH=CH_2$ | Z-1 | $OCH_3$ | cis |
| **I-111** | F | F | H | $CH_3$ | Z-1 | OH | cis |
| **I-112** | F | F | H | (*R*)-$CH_3$ | Z-1 | OH | cis |
| **I-113** | F | F | H | $CH_3$ | Z-1 | $OCH_3$ | cis |
| **I-114** | F | F | H | (*R*)-$CH_3$ | Z-1 | $OCH_3$ | cis |
| **I-115** | F | F | H | $CH_3$ | Z-1 | $OCH_2CH_2SCH_3$ | cis |
| **I-116** | F | F | H | $CH_3$ | Z-1 | $OCH_2CH_2SOCH_3$ | cis |
| **I-117** | F | F | H | $CH_3$ | Z-1 | $OCH_2CH_2SO_2CH_3$ | cis |
| **I-118** | F | F | H | $CH_3$ | Z-1 | $OCH_2CH_2SCH_2CH_3$ | cis |
| **I-119** | F | F | H | $CH_3$ | Z-1 | $OCH_2CH_2SOCH_2CH_3$ | cis |
| **I-120** | F | F | H | $CH_3$ | Z-1 | $OCH_2CH_2SO_2CH_2CH_3$ | cis |
| **I-121** | F | F | H | (*R*)-$CH_3$ | Z-1 | $OCH_2CH_2F$ | cis |
| **I-122** | F | F | H | (*R*)-$CH_3$ | Z-1 | $OCH_2CCl_3$ | cis |
| **I-123** | F | F | H | (*R*)-$CH_3$ | Z-1 | $OCH_2CH_2OCH_3$ | cis |
| **I-124** | F | F | H | (*R*)-$CH_3$ | Z-1 | $OCH(CH_3)CH=CH_2$ | cis |
| **I-125** | F | F | H | (*R*)-$CH_3$ | Z-1 | (*S*)-$OCH_2CH(CH_3)OCH_3$ | cis |
| **I-126** | F | F | H | (*R*)-$CH_3$ | Z-1 | (*R*)-$OCH_2CH(CH_3)OCH_3$ | cis |

(continued)

| Example No. | $X_1$ | $X_2$ | $X_3$ | $R_1$ | Z | G | Z configuration |
|---|---|---|---|---|---|---|---|
| **I-127** | F | F | H | (*R*)-$CH_3$ | Z-1 | $OCH(CH_3)_2$ | cis |
| **I-128** | F | F | H | (*R*)-$CH_3$ | Z-1 | (*R*)-$OCH(CH_3)CH_2CH_3$ | cis |
| **I-129** | F | F | H | (*R*)-$CH_3$ | Z-1 | (*S*)-$OCH(CH_3)CH_2CH_3$ | cis |
| **I-130** | F | F | H | (*R*)-$CH_3$ | Z-1 | 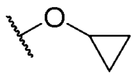 | cis |
| **I-131** | F | F | H | (*R*)-$CH_3$ | Z-1 | $NHCH_2CH_3$ | cis |
| **I-132** | F | Cl | H | $CH_3$ | Z-1 | OH | cis |
| **I-133** | F | Cl | H | (*R*)-$CH_3$ | Z-1 | OH | cis |
| **I-134** | F | Cl | H | $CH_3$ | Z-1 | $OCH_3$ | cis |
| **I-135** | F | Cl | H | (*R*)-$CH_3$ | Z-1 | $OCH_3$ | cis |
| **I-136** | F | Cl | H | (*R*)-$CH_3$ | Z-1 | $OCH(CH_3)_2$ | cis |
| **I-137** | Cl | Cl | H | $CH_3$ | Z-1 | OH | cis |
| **I-138** | Cl | Cl | H | (*R*)-$CH_3$ | Z-1 | OH | cis |
| **I-139** | Cl | Cl | H | $CH_3$ | Z-1 | $OCH_3$ | cis |
| **I-140** | Cl | Cl | H | (*R*)-$CH_3$ | Z-1 | $OCH_3$ | cis |
| **I-141** | F | CN | H | $CH_3$ | Z-1 | OH | cis |
| **I-142** | F | CN | H | (*R*)-$CH_3$ | Z-1 | OH | cis |
| **I-143** | F | CN | H | $CH_3$ | Z-1 | $OCH_3$ | cis |
| **I-144** | F | CN | H | (*R*)-$CH_3$ | Z-1 | $OCH_3$ | cis |
| **I-145** | Cl | CN | H | $CH_3$ | Z-1 | OH | cis |
| **I-146** | Cl | CN | H | (*R*)-$CH_3$ | Z-1 | OH | cis |
| **I-147** | Cl | CN | H | $CH_3$ | Z-1 | $OCH_3$ | cis |
| **I-148** | Cl | CN | H | (*R*)-$CH_3$ | Z-1 | $OCH_3$ | cis |
| **I-149** | F | $CF_3$ | H | $CH_3$ | Z-1 | OH | cis |
| **I-150** | F | $CF_3$ | H | (*R*)-$CH_3$ | Z-1 | OH | cis |
| **I-151** | F | $CF_3$ | H | $CH_3$ | Z-1 | $OCH_3$ | cis |
| **I-152** | F | $CF_3$ | H | (*R*)-$CH_3$ | Z-1 | $OCH_3$ | cis |
| **I-153** | Cl | $CF_3$ | H | $CH_3$ | Z-1 | OH | cis |
| **I-154** | Cl | $CF_3$ | H | (*R*)-$CH_3$ | Z-1 | OH | cis |
| **I-155** | Cl | $CF_3$ | H | $CH_3$ | Z-1 | $OCH_3$ | cis |
| **I-156** | Cl | $CF_3$ | H | (*R*)-$CH_3$ | Z-1 | $OCH_3$ | cis |
| **I-157** | F | $OCF_3$ | H | $CH_3$ | Z-1 | OH | cis |
| **I-158** | F | $OCF_3$ | H | (*R*)-$CH_3$ | Z-1 | OH | cis |
| **I-159** | F | $OCF_3$ | H | $CH_3$ | Z-1 | $OCH_3$ | cis |
| **I-160** | F | $OCF_3$ | H | (*R*)-$CH_3$ | Z-1 | $OCH_3$ | cis |
| **I-161** | F | $OCF_3$ | H | (*R*)-$CH_3$ | Z-1 | $OCH_2C\equiv CH$ | cis |
| **I-162** | Cl | $OCF_3$ | H | $CH_3$ | Z-1 | OH | cis |
| **I-163** | Cl | $OCF_3$ | H | (*R*)-$CH_3$ | Z-1 | OH | cis |
| **I-164** | Cl | $OCF_3$ | H | $CH_3$ | Z-1 | $OCH_3$ | cis |

(continued)

| Example No. | $X_1$ | $X_2$ | $X_3$ | $R_1$ | Z | G | Z configuration |
|---|---|---|---|---|---|---|---|
| **I-165** | Cl | $OCF_3$ | H | (*R*)-$CH_3$ | Z-1 | $OCH_3$ | cis |
| **I-166** | F | $OCF_2H$ | H | $CH_3$ | Z-1 | OH | cis |
| **I-167** | F | $OCF_2H$ | H | (*R*)-$CH_3$ | Z-1 | OH | cis |
| **I-168** | F | $OCF_2H$ | H | $CH_3$ | Z-1 | $OCH_3$ | cis |
| **I-169** | F | $OCF_2H$ | H | (*R*)-$CH_3$ | Z-1 | $OCH_3$ | cis |
| **I-170** | Cl | $OCF_2H$ | H | $CH_3$ | Z-1 | OH | cis |
| **I-171** | Cl | $OCF_2H$ | H | (*R*)-$CH_3$ | Z-1 | OH | cis |
| **I-172** | Cl | $OCF_2H$ | H | $CH_3$ | Z-1 | $OCH_3$ | cis |
| **I-173** | Cl | $OCF_2H$ | H | (*R*)-$CH_3$ | Z-1 | $OCH_3$ | cis |
| **I-174** | F | H | H | $CH_3$ | Z-1 | OH | cis |
| **I-175** | F | H | H | (*R*)-$CH_3$ | Z-1 | OH | cis |
| **I-176** | F | H | H | $CH_3$ | Z-1 | $OCH_3$ | cis |
| **I-177** | F | H | H | (*R*)-$CH_3$ | Z-1 | $OCH_3$ | cis |
| **I-178** | F | $CH_3$ | H | $CH_3$ | Z-1 | OH | cis |
| **I-179** | F | $CH_3$ | H | (*R*)-$CH_3$ | Z-1 | OH | cis |
| **I-180** | F | $CH_3$ | H | $CH_3$ | Z-1 | $OCH_3$ | cis |
| **I-181** | F | $CH_3$ | H | (*R*)-$CH_3$ | Z-1 | $OCH_3$ | cis |
| **I-182** | F | F | F | $CH_3$ | Z-1 | OH | cis |
| **I-183** | F | F | F | (*R*)-$CH_3$ | Z-1 | OH | cis |
| **I-184** | F | F | F | $CH_3$ | Z-1 | $OCH_3$ | cis |
| **I-185** | F | F | F | (*R*)-$CH_3$ | Z-1 | $OCH_3$ | cis |
| **I-186** | Cl | F | F | $CH_3$ | Z-1 | OH | cis |
| **I-187** | Cl | F | F | (*R*)-$CH_3$ | Z-1 | OH | cis |
| **I-188** | Cl | F | F | $CH_3$ | Z-1 | $OCH_3$ | cis |
| **I-189** | Cl | F | F | (*R*)-$CH_3$ | Z-1 | $OCH_3$ | cis |
| **I-190** | Cl | Cl | F | $CH_3$ | Z-1 | OH | cis |
| **I-191** | Cl | Cl | F | (*R*)-$CH_3$ | Z-1 | OH | cis |
| **I-192** | Cl | Cl | F | $CH_3$ | Z-1 | $OCH_3$ | cis |
| **I-193** | Cl | Cl | F | (*R*)-$CH_3$ | Z-1 | $OCH_3$ | cis |
| **I-194** | H | H | H | $CH_3$ | Z-1 | OH | cis |
| **I-195** | H | H | H | (*R*)-$CH_3$ | Z-1 | OH | cis |
| **I-196** | H | H | H | $CH_3$ | Z-1 | $OCH_3$ | cis |
| **I-197** | H | H | H | (*R*)-$CH_3$ | Z-1 | $OCH_3$ | cis |
| **I-198** | $CH_3$ | $CH_3$ | H | $CH_3$ | Z-1 | OH | cis |
| **I-199** | $CH_3$ | $CH_3$ | H | (*R*)-$CH_3$ | Z-1 | OH | cis |
| **I-200** | $CH_3$ | $CH_3$ | H | $CH_3$ | Z-1 | $OCH_3$ | cis |
| **I-201** | $CH_3$ | $CH_3$ | H | (*R*)-$CH_3$ | Z-1 | $OCH_3$ | cis |
| **I-202** | $OCH_3$ | $OCH_3$ | H | $CH_3$ | Z-1 | OH | cis |
| **I-203** | $OCH_3$ | $OCH_3$ | H | (*R*)-$CH_3$ | Z-1 | OH | cis |

(continued)

| Example No. | $X_1$ | $X_2$ | $X_3$ | $R_1$ | Z | G | Z configuration |
|---|---|---|---|---|---|---|---|
| **I-204** | $OCH_3$ | $OCH_3$ | H | $CH_3$ | Z-1 | $OCH_3$ | cis |
| **I-205** | $OCH_3$ | $OCH_3$ | H | (*R*)-$CH_3$ | Z-1 | $OCH_3$ | cis |
| **I-206** | F | F | H | $CF_3$ | Z-1 | OH | cis |
| **I-207** | F | F | H | (*R*)-$CF_3$ | Z-1 | OH | cis |
| **I-208** | F | F | H | $CF_3$ | Z-1 | $OCH_3$ | cis |
| **I-209** | F | F | H | (*R*)-$CF_3$ | Z-1 | $OCH_3$ | cis |
| **I-210** | F | F | H | (*R*)-$CF_3$ | Z-1 | $OCH_2CH_2CH_3$ | cis |
| **I-211** | F | F | H | (*R*)-$CF_3$ | Z-1 | $OCH_2CH(CH_3)_2$ | cis |
| **I-212** | F | F | H | (*R*)-$CF_3$ | Z-1 | 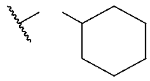 | cis |
| **I-213** | F | F | H | (*R*)-$CF_3$ | Z-1 | 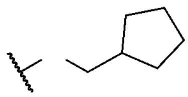 | cis |
| **I-214** | F | F | H | (*R*)-$CF_3$ | Z-1 | $OCH_2CH_2Br$ | cis |
| **I-215** | F | F | H | (*R*)-$CF_3$ | Z-1 | $OCH_2CF_2H$ | cis |
| **I-216** | F | F | H | (*R*)-$CF_3$ | Z-1 | $OCH_2CH_2CH=CH_2$ | cis |
| **I-217** | F | F | H | (*R*)-$CF_3$ | Z-1 | (*R*)-$OCH_2CH(CH_3)OCH_3$ | cis |
| **I-218** | F | F | H | (*R*)-$CF_3$ | Z-1 | (*S*)-$OCH(CH_3)CH_2OCH_3$ | cis |
| **I-219** | F | F | H | (*R*)-$CF_3$ | Z-1 | (*S*)-$OCH_2CH(CH_3)$OCH3 | cis |
| **I-220** | F | F | H | (*R*)-$CF_3$ | Z-1 | $OCH(CH_2CH_3)C\equiv CH$ | cis |
| **I-221** | F | F | H | (*R*)-$CF_3$ | Z-1 | $NHOCH_2CH_3$ | cis |
| **I-222** | F | F | H | (*R*)-$CF_3$ | Z-1 | 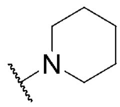 | cis |
| **I-223** | F | Cl | H | $CF_3$ | Z-1 | OH | cis |
| **I-224** | F | Cl | H | (*R*)-$CF_3$ | Z-1 | OH | cis |
| **I-225** | F | Cl | H | $CF_3$ | Z-1 | $OCH_3$ | cis |
| **I-226** | F | Cl | H | (*R*)-$CF_3$ | Z-1 | $OCH_3$ | cis |
| **I-227** | F | Cl | H | (*R*)-$CF_3$ | Z-1 | $O(CH_2)_2CH_3$ | cis |
| **I-228** | F | Cl | H | (*R*)-$CF_3$ | Z-1 | $O(CH_2)_5CH_3$ | cis |
| **I-229** | Cl | Cl | H | $CF_3$ | Z-1 | OH | cis |
| **I-230** | Cl | Cl | H | (*R*)-$CF_3$ | Z-1 | OH | cis |
| **I-231** | Cl | Cl | H | $CF_3$ | Z-1 | $OCH_3$ | cis |
| **I-232** | Cl | Cl | H | (*R*)-$CF_3$ | Z-1 | $OCH_3$ | cis |
| **I-233** | F | CN | H | $CF_3$ | Z-1 | OH | cis |
| **I-234** | F | CN | H | (*R*)-$CF_3$ | Z-1 | OH | cis |
| **I-235** | F | CN | H | $CF_3$ | Z-1 | $OCH_3$ | cis |
| **I-236** | F | CN | H | (*R*)-$CF_3$ | Z-1 | $OCH_3$ | cis |
| **I-237** | Cl | CN | H | $CF_3$ | Z-1 | OH | cis |

(continued)

| Example No. | $X_1$ | $X_2$ | $X_3$ | $R_1$ | Z | G | Z configuration |
|---|---|---|---|---|---|---|---|
| **I-238** | Cl | CN | H | (*R*)-$CF_3$ | Z-1 | OH | cis |
| **I-239** | Cl | CN | H | $CF_3$ | Z-1 | $OCH_3$ | cis |
| **I-240** | Cl | CN | H | (*R*)-$CF_3$ | Z-1 | $OCH_3$ | cis |
| **I-241** | F | $CF_3$ | H | $CF_3$ | Z-1 | OH | cis |
| **I-242** | F | $CF_3$ | H | (*R*)-$CF_3$ | Z-1 | OH | cis |
| **I-243** | F | $CF_3$ | H | $CF_3$ | Z-1 | $OCH_3$ | cis |
| **I-244** | F | $CF_3$ | H | (*R*)-$CF_3$ | Z-1 | $OCH_3$ | cis |
| **I-245** | Cl | $CF_3$ | H | $CF_3$ | Z-1 | OH | cis |
| **I-246** | Cl | $CF_3$ | H | (*R*)-$CF_3$ | Z-1 | OH | cis |
| **I-247** | Cl | $CF_3$ | H | $CF_3$ | Z-1 | $OCH_3$ | cis |
| **I-248** | Cl | $CF_3$ | H | (*R*)-$CF_3$ | Z-1 | $OCH_3$ | cis |
| **I-249** | F | $OCF_3$ | H | $CF_3$ | Z-1 | OH | cis |
| **I-250** | F | $OCF_3$ | H | (*R*)-$CF_3$ | Z-1 | OH | cis |
| **I-251** | F | $OCF_3$ | H | $CF_3$ | Z-1 | $OCH_3$ | cis |
| **I-252** | F | $OCF_3$ | H | (*R*)-$CF_3$ | Z-1 | $OCH_3$ | cis |
| **I-253** | F | $OCF_3$ | H | (*R*)-$CF_3$ | Z-1 | 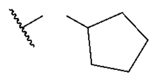 | cis |
| **I-254** | F | $OCF_3$ | H | (*R*)-$CF_3$ | Z-1 | $OCH_2CF_2CF_3$ | cis |
| **I-255** | F | $OCF_3$ | H | (*R*)-$CF_3$ | Z-1 | $OCH_2C{\equiv}CH$ | cis |
| **I-256** | Cl | $OCF_3$ | H | $CF_3$ | Z-1 | OH | cis |
| **I-257** | Cl | $OCF_3$ | H | (*R*)-$CF_3$ | Z-1 | OH | cis |
| **I-258** | Cl | $OCF_3$ | H | $CF_3$ | Z-1 | $OCH_3$ | cis |
| **I-259** | Cl | $OCF_3$ | H | (*R*)-$CF_3$ | Z-1 | $OCH_3$ | cis |
| **I-260** | F | $OCF_2H$ | H | $CF_3$ | Z-1 | OH | cis |
| **I-261** | F | $OCF_2H$ | H | (*R*)-$CF_3$ | Z-1 | OH | cis |
| **I-262** | F | $OCF_2H$ | H | $CF_3$ | Z-1 | $OCH_3$ | cis |
| **I-263** | F | $OCF_2H$ | H | (*R*)-$CF_3$ | Z-1 | $OCH_3$ | cis |
| **I-264** | Cl | $OCF_2H$ | H | $CF_3$ | Z-1 | OH | cis |
| **I-265** | Cl | $OCF_2H$ | H | (*R*)-$CF_3$ | Z-1 | OH | cis |
| **I-266** | Cl | $OCF_2H$ | H | $CF_3$ | Z-1 | $OCH_3$ | cis |
| **I-267** | Cl | $OCF_2H$ | H | (*R*)-$CF_3$ | Z-1 | $OCH_3$ | cis |
| **I-268** | F | H | H | $CF_3$ | Z-1 | OH | cis |
| **I-269** | F | H | H | (*R*)-$CF_3$ | Z-1 | OH | cis |
| **I-270** | F | H | H | $CF_3$ | Z-1 | $OCH_3$ | cis |
| **I-271** | F | H | H | (*R*)-$CF_3$ | Z-1 | $OCH_3$ | cis |
| **I-272** | F | $CH_3$ | H | $CF_3$ | Z-1 | OH | cis |
| **I-273** | F | $CH_3$ | H | (*R*)-$CF_3$ | Z-1 | OH | cis |
| **I-274** | F | $CH_3$ | H | $CF_3$ | Z-1 | $OCH_3$ | cis |
| **I-275** | F | $CH_3$ | H | (*R*)-$CF_3$ | Z-1 | $OCH_3$ | cis |

(continued)

| Example No. | $X_1$ | $X_2$ | $X_3$ | $R_1$ | Z | G | Z configuration |
|---|---|---|---|---|---|---|---|
| **I-276** | F | F | F | $CF_3$ | Z-1 | OH | cis |
| **I-277** | F | F | F | (*R*)-$CF_3$ | Z-1 | OH | cis |
| **I-278** | F | F | F | $CF_3$ | Z-1 | $OCH_3$ | cis |
| **I-279** | F | F | F | (*R*)-$CF_3$ | Z-1 | $OCH_3$ | cis |
| **I-280** | Cl | F | F | $CF_3$ | Z-1 | OH | cis |
| **I-281** | Cl | F | F | (*R*)-$CF_3$ | Z-1 | OH | cis |
| **I-282** | Cl | F | F | $CF_3$ | Z-1 | $OCH_3$ | cis |
| **I-283** | Cl | F | F | $CF_3$ | Z-1 | $OCH_3$ | cis |
| **I-284** | Cl | Cl | F | $CF_3$ | Z-1 | OH | cis |
| **I-285** | Cl | Cl | F | (*R*)-$CF_3$ | Z-1 | OH | cis |
| **I-286** | Cl | Cl | F | $CF_3$ | Z-1 | $OCH_3$ | cis |
| **I-287** | Cl | Cl | F | (*R*)-$CF_3$ | Z-1 | $OCH_3$ | cis |
| **I-288** | H | H | H | $CF_3$ | Z-1 | OH | cis |
| **I-289** | H | H | H | (*R*)-$CF_3$ | Z-1 | OH | cis |
| **I-290** | H | H | H | $CF_3$ | Z-1 | $OCH_3$ | cis |
| **I-291** | H | H | H | (*R*)-$CF_3$ | Z-1 | $OCH_3$ | cis |
| **I-292** | $CH_3$ | $CH_3$ | H | $CF_3$ | Z-1 | OH | cis |
| **I-293** | $CH_3$ | $CH_3$ | H | (*R*)-$CF_3$ | Z-1 | OH | cis |
| **I-294** | $CH_3$ | $CH_3$ | H | $CF_3$ | Z-1 | $OCH_3$ | cis |
| **I-295** | $CH_3$ | $CH_3$ | H | (*R*)-$CF_3$ | Z-1 | $OCH_3$ | cis |
| **I-296** | $OCH_3$ | $OCH_3$ | H | $CF_3$ | Z-1 | OH | cis |
| **I-297** | $OCH_3$ | $OCH_3$ | H | (*R*)-$CF_3$ | Z-1 | OH | cis |
| **I-298** | $OCH_3$ | $OCH_3$ | H | $CF_3$ | Z-1 | $OCH_3$ | cis |
| **I-299** | $OCH_3$ | $OCH_3$ | H | (*R*)-$CF_3$ | Z-1 | $OCH_3$ | cis |
| **I-300** | F | F | H | $CF_2H$ | Z-1 | OH | cis |
| **I-301** | F | F | H | (*R*)-$CF_2H$ | Z-1 | OH | cis |
| **I-302** | F | F | H | $CF_2H$ | Z-1 | $OCH_3$ | cis |
| **I-303** | F | F | H | (*R*)-$CF_2H$ | Z-1 | $OCH_3$ | cis |
| **I-304** | F | Cl | H | $CF_2H$ | Z-1 | OH | cis |
| **I-305** | F | Cl | H | (*R*)-$CF_2H$ | Z-1 | OH | cis |
| **I-306** | F | Cl | H | $CF_2H$ | Z-1 | $OCH_3$ | cis |
| **I-307** | F | Cl | H | (*R*)-$CF_2H$ | Z-1 | $OCH_3$ | cis |
| **I-308** | F | F | H | $CFH_2$ | Z-1 | OH | cis |
| **I-309** | F | F | H | (*R*)-$CFH_2$ | Z-1 | OH | cis |
| **I-310** | F | F | H | $CFH_2$ | Z-1 | $OCH_3$ | cis |
| **I-311** | F | F | H | (*R*)-$CFH_2$ | Z-1 | $OCH_3$ | cis |
| **I-312** | F | Cl | H | $CFH_2$ | Z-1 | OH | cis |
| **I-313** | F | Cl | H | (*R*)-$CFH_2$ | Z-1 | OH | cis |
| **I-314** | F | Cl | H | $CFH_2$ | Z-1 | $OCH_3$ | cis |

(continued)

| Example No. | $X_1$ | $X_2$ | $X_3$ | $R_1$ | Z | G | Z configuration |
|---|---|---|---|---|---|---|---|
| **I-315** | F | Cl | H | (*R*)-$CFH_2$ | Z-1 | $OCH_3$ | cis |
| **I-316** | Cl | Cl | H | $CFH_2$ | Z-1 | OH | cis |
| **I-317** | Cl | Cl | H | (*R*)-$CFH_2$ | Z-1 | OH | cis |
| **I-318** | Cl | Cl | H | $CFH_2$ | Z-1 | $OCH_3$ | cis |
| **I-319** | Cl | Cl | H | (*R*)-$CFH_2$ | Z-1 | $OCH_3$ | cis |
| **I-320** | F | $OCF_3$ | H | $CFH_2$ | Z-1 | OH | cis |
| **I-321** | F | $OCF_3$ | H | (*R*)-$CFH_2$ | Z-1 | OH | cis |
| **I-322** | F | $OCF_3$ | H | $CFH_2$ | Z-1 | $OCH_3$ | cis |
| **I-323** | F | $OCF_3$ | H | (*R*)-$CFH_2$ | Z-1 | $OCH_3$ | cis |
| **I-324** | F | $OCF_2H$ | H | $CFH_2$ | Z-1 | OH | cis |
| **I-325** | F | $OCF_2H$ | H | (*R*)-$CFH_2$ | Z-1 | OH | cis |
| **I-326** | F | $OCF_2H$ | H | $CFH_2$ | Z-1 | $OCH_3$ | cis |
| **I-327** | F | $OCF_2H$ | H | (*R*)-$CFH_2$ | Z-1 | $OCH_3$ | cis |
| **I-328** | Cl | $OCF_3$ | H | $CFH_2$ | Z-1 | $OCH_3$ | cis |
| **I-329** | Cl | CN | H | $CFH_2$ | Z-1 | $OCH_3$ | cis |
| **I-330** | F | $CF_3$ | H | $CFH_2$ | Z-1 | $OCH_3$ | cis |
| **I-331** | Cl | $CF_3$ | H | $CFH_2$ | Z-1 | $OCH_3$ | cis |
| **I-332** | F | F | F | $CFH_2$ | Z-1 | $OCH_3$ | cis |
| **I-333** | F | F | H | $CH_2Cl$ | Z-1 | OH | cis |
| **I-334** | F | F | H | (*R*)-$CH_2Cl$ | Z-1 | OH | cis |
| **I-335** | F | F | H | $CH_2Cl$ | Z-1 | $OCH_3$ | cis |
| **I-336** | F | F | H | (*R*)-$CH_2Cl$ | Z-1 | $OCH_3$ | cis |
| **I-337** | F | Cl | H | $CH_2Cl$ | Z-1 | OH | cis |
| **I-338** | F | Cl | H | (*R*)-$CH_2Cl$ | Z-1 | OH | cis |
| **I-339** | F | Cl | H | $CH_2Cl$ | Z-1 | $OCH_3$ | cis |
| **I-340** | F | Cl | H | (*R*)-$CH_2Cl$ | Z-1 | $OCH_3$ | cis |
| **I-341** | Cl | Cl | H | $CH_2Cl$ | Z-1 | OH | cis |
| **I-342** | Cl | Cl | H | (*R*)-$CH_2Cl$ | Z-1 | OH | cis |
| **I-343** | Cl | Cl | H | $CH_2Cl$ | Z-1 | $OCH_3$ | cis |
| **I-344** | Cl | Cl | H | (*R*)-$CH_2Cl$ | Z-1 | $OCH_3$ | cis |
| **I-345** | F | F | H | $CF_2CH_3$ | Z-1 | OH | cis |
| **I-346** | F | F | H | (*R*)-$CF_2CH_3$ | Z-1 | OH | cis |
| **I-347** | F | F | H | $CF_2CH_3$ | Z-1 | $OCH_3$ | cis |
| **I-348** | F | F | H | (*R*)-$CF_2CH_3$ | Z-1 | $OCH_3$ | cis |
| **I-349** | F | Cl | H | $CF_2CH_3$ | Z-1 | $OCH_3$ | cis |
| **I-350** | Cl | Cl | H | $CF_2CH_3$ | Z-1 | OH | cis |
| **I-351** | Cl | Cl | H | (*R*)-$CF_2CH_3$ | Z-1 | OH | cis |
| **I-352** | Cl | Cl | H | $CF_2CH_3$ | Z-1 | $OCH_3$ | cis |
| **I-353** | Cl | Cl | H | (*R*)-$CF_2CH_3$ | Z-1 | $OCH_3$ | cis |

(continued)

| Example No. | $X_1$ | $X_2$ | $X_3$ | $R_1$ | Z | G | Z configuration |
|---|---|---|---|---|---|---|---|
| **I-354** | F | F | H | $CF=CH_2$ | Z-1 | OH | cis |
| **I-355** | F | F | H | (*R*)-$CF=CH_2$ | Z-1 | $OCH_3$ | cis |
| **I-356** | F | F | H | $CF=CH_2$ | Z-1 | $OCH_3$ | cis |
| **I-357** | F | Cl | H | $CF=CH_2$ | Z-1 | $OCH_3$ | cis |
| **I-358** | Cl | Cl | H | $CF=CH_2$ | Z-1 | OH | cis |
| **I-359** | Cl | Cl | H | (*R*)-$CF=CH_2$ | Z-1 | OH | cis |
| **I-360** | Cl | Cl | H | $CF=CH_2$ | Z-1 | $OCH_3$ | cis |
| **I-361** | Cl | Cl | H | (*R*)-$CF=CH_2$ | Z-1 | $OCH_3$ | cis |
| **I-362** | F | F | H | $OCH_3$ | Z-1 | $OCH_3$ | cis |
| **I-363** | Cl | Cl | H | $OCH_3$ | Z-1 | $OCH_3$ | cis |
| **I-364** | F | F | H | $CH=CH_2$ | Z-1 | $OCH_3$ | trans |
| **I-365** | F | F | H | $-CH=CH_2$ | Z-2 | $OCH_3$ | |
| **I-366** | F | F | H | $-CH=CH_2$ | Z-3 | $OCH_3$ | |
| **I-367** | F | F | H | $-CH=CH_2$ | Z-4 | $OCH_3$ | |
| **I-368** | F | $CF_3$ | H | $CH=CH_2$ | Z-5 | $OCH_3$ | cis-(rac) |
| **I-369** | F | $OCF_3$ | H | $CH=CH_2$ | Z-5 | $OCH_3$ | cis-(rac) |
| **I-370** | F | $OCF_2H$ | H | $CH=CH_2$ | Z-5 | $OCH_3$ | cis-(rac) |
| **I-371** | Cl | $CF_3$ | H | $CH=CH_2$ | Z-5 | $OCH_3$ | cis-(rac) |
| **I-372** | Cl | $OCF_3$ | H | $CH=CH_2$ | Z-5 | $OCH_3$ | cis-(rac) |
| **I-373** | Cl | $OCF_2H$ | H | $CH=CH_2$ | Z-5 | $OCH_3$ | cis-(rac) |
| **I-374** | F | F | F | $CH=CH_2$ | Z-5 | $OCH_3$ | cis-(rac) |
| **I-375** | F | Cl | F | $CH=CH_2$ | Z-5 | $OCH_3$ | cis-(rac) |
| **I-376** | Cl | Cl | F | $CH=CH_2$ | Z-5 | $OCH_3$ | cis-(rac) |
| **I-377** | F | $CF_3$ | H | $CH_3$ | Z-5 | $OCH_3$ | cis-(rac) |
| **I-378** | F | $OCF_3$ | H | $CH_3$ | Z-5 | OH | cis-(rac) |
| **I-379** | F | $OCF_3$ | H | (*R*)-$CH_3$ | Z-5 | OH | cis-(rac) |
| **I-380** | F | $OCF_3$ | H | $CH_3$ | Z-5 | $OCH_3$ | cis-(rac) |
| **I-381** | F | $OCF_3$ | H | (*R*)-$CH_3$ | Z-5 | $OCH_3$ | cis-(rac) |
| **I-382** | F | $OCF_3$ | H | $CH_3$ | Z-5 | $OCH_2CH_2SCH_3$ | cis-(rac) |
| **I-383** | F | $OCF_3$ | H | $CH_3$ | Z-5 | $OCH_2CH_2SOCH_3$ | cis-(rac) |
| **I-384** | F | $OCF_3$ | H | $CH_3$ | Z-5 | $OCH_2CH_2SO_2CH_3$ | cis-(rac) |
| **I-385** | F | $OCF_2H$ | H | $CH_3$ | Z-5 | $OCH_3$ | cis-(rac) |
| **I-386** | Cl | $CF_3$ | H | $CH_3$ | Z-5 | $OCH_3$ | cis-(rac) |
| **I-387** | Cl | $OCF_3$ | H | $CH_3$ | Z-5 | $OCH_3$ | cis-(rac) |
| **I-388** | Cl | $OCF_2H$ | H | $CH_3$ | Z-5 | OH | cis-(rac) |
| **I-389** | Cl | $OCF_2H$ | H | (*R*)-$CH_3$ | Z-5 | OH | cis-(rac) |
| **I-390** | Cl | $OCF_2H$ | H | $CH_3$ | Z-5 | $OCH_3$ | cis-(rac) |
| **I-391** | Cl | $OCF_2H$ | H | (*R*)-$CH_3$ | Z-5 | $OCH_3$ | cis-(rac) |
| **I-392** | F | $CF_3$ | H | $CF_3$ | Z-5 | $OCH_3$ | cis-(rac) |

(continued)

| Example No. | $X_1$ | $X_2$ | $X_3$ | $R_1$ | Z | G | Z configuration |
|---|---|---|---|---|---|---|---|
| **I-393** | F | $OCF_3$ | H | $CF_3$ | Z-5 | $OCH_3$ | cis-(rac) |
| **I-394** | F | $OCF_2H$ | H | $CF_3$ | Z-5 | $OCH_3$ | cis-(rac) |
| **I-395** | Cl | $OCF_2H$ | H | $CF_3$ | Z-5 | OH | cis-(rac) |
| **I-396** | Cl | $OCF_2H$ | H | (*R*)-$CF_3$ | Z-5 | OH | cis-(rac) |
| **I-397** | Cl | $OCF_2H$ | H | $CF_3$ | Z-5 | $OCH_3$ | cis-(rac) |
| **I-398** | Cl | $OCF_2H$ | H | (*R*)-$CF_3$ | Z-5 | $OCH_3$ | cis-(rac) |
| **I-399** | Cl | $CF_3$ | H | $CF_3$ | Z-5 | $OCH_3$ | cis-(rac) |
| **I-400** | Cl | $OCF_3$ | H | $CF_3$ | Z-5 | $OCH_3$ | cis-(rac) |
| **I-401** | F | F | H | -CH=$CH_2$ | Z-6 | -OH | 1*R*,3*S*(cis) |
| **I-402** | F | F | H | (*S*)-CH=$CH_2$ | Z-6 | -OH | 1*R*,3*S*(cis) |
| **I-403** | F | F | H | -CH=$CH_2$ | Z-6 | $OCH_3$ | 1*R*,3*S*(cis) |
| **I-404** | F | F | H | (*S*)-CH=$CH_2$ | Z-6 | $OCH_3$ | 1*R*,3*S*(cis) |
| **I-405** | F | $CF_3$ | H | -CH=$CH_2$ | Z-6 | -OH | 1*R*,3*S*(cis) |
| **I-406** | F | $CF_3$ | H | -CH=$CH_2$ | Z-6 | $OCH_3$ | 1*R*,3*S*(cis) |
| **I-407** | Cl | $CF_3$ | H | -CH=$CH_2$ | Z-6 | -OH | 1*R*,3*S*(cis) |
| **I-408** | Cl | $CF_3$ | H | -CH=$CH_2$ | Z-6 | $OCH_3$ | 1*R*,3*S*(cis) |
| **I-409** | F | $OCF_3$ | H | -CH=$CH_2$ | Z-6 | -OH | 1*R*,3*S*(cis) |
| **I-410** | F | $OCF_3$ | H | -CH=$CH_2$ | Z-6 | $OCH_3$ | 1*R*,3*S*(cis) |
| **I-411** | Cl | $OCF_3$ | H | -CH=$CH_2$ | Z-6 | -OH | 1*R*,3*S*(cis) |
| **I-412** | Cl | $OCF_3$ | H | -CH=$CH_2$ | Z-6 | $OCH_3$ | 1*R*,3*S*(cis) |
| **I-413** | F | F | H | -CH=$CH_2$ | Z-6 | -OH | 1*S*,3*R*(cis) |
| **I-414** | F | F | H | (*S*)-CH=$CH_2$ | Z-6 | -OH | 1*S*,3*R*(cis) |
| **I-415** | F | F | H | -CH=$CH_2$ | Z-6 | $OCH_3$ | 1*S*,3*R*(cis) |
| **I-416** | F | F | H | (*S*)-CH=$CH_2$ | Z-6 | $OCH_3$ | 1*S*,3*R*(cis) |
| **I-417** | F | $CF_3$ | H | -CH=$CH_2$ | Z-6 | -OH | 1*S*,3*R*(cis) |
| **I-418** | F | $CF_3$ | H | -CH=$CH_2$ | Z-6 | $OCH_3$ | 1*S*,3*R*(cis) |
| **I-419** | Cl | $CF_3$ | H | -CH=$CH_2$ | Z-6 | -OH | 1*S*,3*R*(cis) |
| **I-420** | Cl | $CF_3$ | H | -CH=$CH_2$ | Z-6 | $OCH_3$ | 1*S*,3*R*(cis) |
| **I-421** | F | $OCF_3$ | H | -CH=$CH_2$ | Z-6 | -OH | 1S,3R(cis) |
| **I-422** | F | $OCF_3$ | H | -CH=$CH_2$ | Z-6 | $OCH_3$ | 1S,3R(cis) |
| **I-423** | Cl | $OCF_3$ | H | -CH=$CH_2$ | Z-6 | -OH | 1*S*,3*R*(cis) |
| **I-424** | Cl | $OCF_3$ | H | -CH=$CH_2$ | Z-6 | $OCH_3$ | 1*S*,3*R*(cis) |
| **I-425** | F | $CF_3$ | H | CH=$CH_2$ | Z-7 | OH | 1*S*,4*R*(cis) |
| **I-426** | F | $CF_3$ | H | (*S*)-CH=$CH_2$ | Z-7 | OH | 1*S*,4*R*(cis) |
| **I-427** | F | $CF_3$ | H | CH=$CH_2$ | Z-7 | $OCH_3$ | 1*S*,4R(cis) |
| **I-428** | F | $CF_3$ | H | (*S*)-CH=$CH_2$ | Z-7 | $OCH_3$ | 1*S*,4R(cis) |
| **I-429** | F | $CF_3$ | H | (*S*)-CH=$CH_2$ | Z-7 | $OCH_2CH_3$ | 1*S*,4R(cis) |
| **I-430** | F | $CF_3$ | H | (*S*)-CH=$CH_2$ | Z-7 | $O(CH_2)_3CH_3$ | 1*S*,4R(cis) |
| **I-431** | F | $CF_3$ | H | (*S*)-CH=$CH_2$ | Z-7 | $O(CH_2)_6CH_3$ | 1*S*,4R(cis) |

(continued)

| Example No. | $X_1$ | $X_2$ | $X_3$ | $R_1$ | Z | G | Z configuration |
|---|---|---|---|---|---|---|---|
| **I-432** | F | $CF_3$ | H | (*S*)-$CH=CH_2$ | Z-7 | 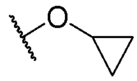 | 1*S*,4*R*(cis) |
| **I-433** | F | $CF_3$ | H | (*S*)-$CH=CH_2$ | Z-7 | 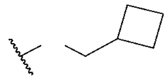 | 1*S*,4*R*(cis) |
| **I-434** | F | $CF_3$ | H | (*S*)-$CH=CH_2$ | Z-7 | $OCH_2CF_3$ | 1*S*,4*R*(cis) |
| **I-435** | F | $CF_3$ | H | (*S*)-$CH=CH_2$ | Z-7 | $OCH_2(CF_2)_4H$ | 1*S*,4*R*(cis) |
| **I-436** | F | $CF_3$ | H | (*S*)-$CH=CH_2$ | Z-7 | $O(CH_2)_3OCH_3$ | 1*S*,4*R*(cis) |
| **I-437** | F | $CF_3$ | H | (*S*)-$CH=CH_2$ | Z-7 | $OCH_2CH_2CH_3$ | 1*S*,4*R*(cis) |
| **I-438** | F | $CF_3$ | H | (*S*)-$CH=CH_2$ | Z-7 | $OCH_2CH(CH_3)_2$ | 1*S*,4*R*(cis) |
| **I-439** | F | $CF_3$ | H | (*S*)-$CH=CH_2$ | Z-7 | 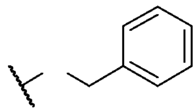 | 1*S*,4*R*(cis) |
| **I-440** | F | $CF_3$ | H | (*S*)-$CH=CH_2$ | Z-7 | 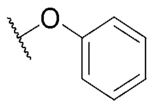 | 1*S*,4*R*(cis) |
| **I-441** | F | $CF_3$ | H | (*S*)-$CH=CH_2$ | Z-7 | $OCH_2CH_2F$ | 1*S*,4*R*(cis) |
| **I-442** | F | $CF_3$ | H | (*S*)-$CH=CH_2$ | Z-7 | $OCH_2CCl_3$ | 1*S*,4*R*(cis) |
| **I-443** | F | $CF_3$ | H | (*S*)-$CH=CH_2$ | Z-7 | $OCH_2CH_2OCH_3$ | 1*S*,4*R*(cis) |
| **I-444** | F | $CF_3$ | H | (*S*)-$CH=CH_2$ | Z-7 | $OCH(CH_3)CH=CH_2$ | 1*S*,4*R*(cis) |
| **I-445** | F | $CF_3$ | H | (*S*)-$CH=CH_2$ | Z-7 | (*S*)-$OCH_2CH(CH_3)OCH_3$ | 1*S*,4*R*(cis) |
| **I-446** | F | $CF_3$ | H | (*S*)-$CH=CH_2$ | Z-7 | $NH_2$ | 1*S*,4*R*(cis) |
| **I-447** | F | $CF_3$ | H | (*S*)-$CH=CH_2$ | Z-7 | NHOH | 1*S*,4*R*(cis) |
| **I-448** | F | $CF_3$ | H | (*S*)-$CH=CH_2$ | Z-7 | $NHOCH_3$ | 1*S*,4*R*(cis) |
| **I-449** | F | $CF_3$ | H | (*S*)-$CH=CH_2$ | Z-7 | $NHCH_3$ | 1*S*,4*R*(cis) |
| **I-450** | F | $CF_3$ | H | (*S*)-$CH=CH_2$ | Z-7 | $NHCH_2CH_3$ | 1*S*,4*R*(cis) |
| **I-451** | F | $CF_3$ | H | (*S*)-$CH=CH_2$ | Z-7 | $NHSO_2CH_3$ | 1*S*,4*R*(cis) |
| **I-452** | F | $CF_3$ | H | (*S*)-$CH=CH_2$ | Z-7 | 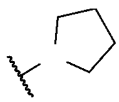 | 1*S*,4*R*(cis) |
| **I-453** | F | $OCF_3$ | H | $CH=CH_2$ | Z-7 | OH | 1*S*,4*R*(cis) |
| **I-454** | F | $OCF_3$ | H | (*S*)-$CH=CH_2$ | Z-7 | OH | 1*S*,4*R*(cis) |
| **I-455** | F | $OCF_3$ | H | $CH=CH_2$ | Z-7 | $OCH_3$ | 1*S*,4*R*(cis) |
| **I-456** | F | $OCF_3$ | H | (*S*)-$CH=CH_2$ | Z-7 | $OCH_3$ | 1*S*,4*R*(cis) |
| **I-457** | F | $OCF_3$ | H | (*S*)-$CH=CH_2$ | Z-7 | $O(CH_2)_4CH_3$ | 1*S*,4*R*(cis) |
| **I-458** | F | $OCF_3$ | H | (*S*)-$CH=CH_2$ | Z-7 | $OCH_2CH=CHCH_3$ | 1*S*,4*R*(cis) |
| **I-459** | F | $OCF_3$ | H | (*S*)-$CH=CH_2$ | Z-7 | (*R*)-$OCH(CH_3)CHzOCH_3$ | 1*S*,4*R*(cis) |
| **I-460** | F | $OCF_3$ | H | (*S*)-$CH=CH_2$ | Z-7 | 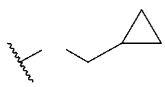 | 1*S*,4*R*(cis) |
| **I-461** | F | $OCF_3$ | H | (*S*)-$CH=CH_2$ | Z-7 | $O(CH_2)_2CH_3$ | 1*S*,4*R*(cis) |

(continued)

| Example No. | $X_1$ | $X_2$ | $X_3$ | $R_1$ | Z | G | Z configuration |
|---|---|---|---|---|---|---|---|
| **I-462** | F | $OCF_3$ | H | (*S*)-$CH=CH_2$ | Z-7 | $O(CH_2)_5CH_3$ | 1*S*,4*R*(cis) |
| **I-463** | F | $OCF_3$ | H | (*S*)-$CH=CH_2$ | Z-7 | $OCH(CH_3)_2$ | 1*S*,4*R*(cis) |
| **I-464** | F | $OCF_3$ | H | (*S*)-$CH=CH_2$ | Z-7 | 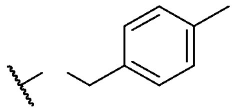 | 1*S*,4*R*(cis) |
| **I-465** | F | $OCF_3$ | H | (*S*)-$CH=CH_2$ | Z-7 | 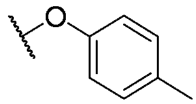 | 1*S*,4*R*(cis) |
| **I-466** | F | $OCF_3$ | H | (*S*)-$CH=CH_2$ | Z-7 | $NHOCH(CH_3)_2$ | 1*S*,4*R*(cis) |
| **I-467** | F | $OCF_3$ | H | (*S*)-$CH=CH_2$ | Z-7 | $NHOCH_2CH_3$ | 1*S*,4*R*(cis) |
| **I-468** | F | $OCF_3$ | H | (*S*)-$CH=CH_2$ | Z-7 | $NHCH_2CH_3$ | 1*S*,4*R*(cis) |
| **I-469** | F | $OCF_3$ | H | (*S*)-$CH=CH_2$ | Z-7 | 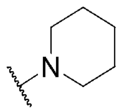 | 1*S*,4*R*(cis) |
| **I-470** | Cl | $OCF_3$ | H | (*S*)-$CH=CH_2$ | Z-7 | OH | 1*S*,4*R*(cis) |
| **I-471** | Cl | $OCF_3$ | H | (*S*)-$CH=CH_2$ | Z-7 | $OCH_3$ | 1*S*,4*R*(cis) |
| **I-472** | Cl | $OCF_3$ | H | (*S*)-$CH=CH_2$ | Z-7 | 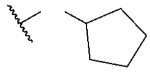 | 1*S*,4*R*(cis) |
| **I-473** | Cl | $OCF_3$ | H | (*S*)-$CH=CH_2$ | Z-7 | $OCH_2CF_2CF_3$ | 1*S*,4*R*(cis) |
| **I-474** | Cl | $OCF_3$ | H | (*S*)-$CH=CH_2$ | Z-7 | $OCH_2C{\equiv}CH$ | 1*S*,4*R*(cis) |
| **I-475** | Cl | $OCF_3$ | H | (*S*)-$CH=CH_2$ | Z-7 | 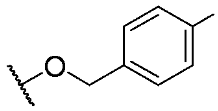 | 1*S*,4*R*(cis) |
| **I-476** | Cl | $CF_3$ | H | (*S*)-$CH=CH_2$ | Z-7 | OH | 1*S*,4*R*(cis) |
| **I-477** | Cl | $CF_3$ | H | (*S*)-$CH=CH_2$ | Z-7 | $OCH_3$ | 1*S*,4*R*(cis) |
| **I-478** | Cl | $CF_3$ | H | (*S*)-$CH=CH_2$ | Z-7 | 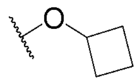 | 1*S*,4*R*(cis) |
| **I-479** | Cl | $CF_3$ | H | (*S*)-$CH=CH_2$ | Z-7 | 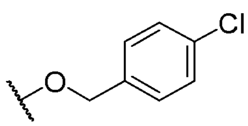 | 1*S*,4*R*(cis) |
| **I-480** | Cl | $CF_3$ | H | (*S*)-$CH=CH_2$ | Z-7 | $OCH_2CF_2CF_2H$ | 1*S*,4*R*(cis) |
| **I-481** | Cl | $CF_3$ | H | (*S*)-$CH=CH_2$ | Z-7 | $OCH_2CH_2OCH_2CH_3$ | 1*S*,4*R*(cis) |
| **I-482** | Cl | $CF_3$ | H | (*S*)-$CH=CH_2$ | Z-7 | $OCH_2C{\equiv}CCH3$ | 1*S*,4*R*(cis) |
| **I-483** | Cl | $CF_3$ | H | (*S*)-$CH=CH_2$ | Z-7 | $OCH(CH_3)_2$ | 1*S*,4*R*(cis) |
| **I-484** | Cl | $CF_3$ | H | (*S*)-$CH=CH_2$ | Z-7 | $N(CH_3)OCH_3$ | 1*S*,4*R*(cis) |
| **I-485** | Cl | $OCF_3$ | H | $CH_3$ | Z-7 | OH | 1*S*,4*R*(cis) |
| **I-486** | Cl | $OCF_3$ | H | $CH_3$ | Z-7 | $OCH_3$ | 1*S*,4*R*(cis) |
| **I-487** | Cl | $OCF_3$ | H | $CH_3$ | Z-7 | $OCH_2CH_2SCH_3$ | 1*S*,4*R*(cis) |
| **I-488** | F | $OCF_3$ | H | $CH_3$ | Z-7 | OH | 1*S*,4*R*(cis) |

(continued)

| Example No. | $X_1$ | $X_2$ | $X_3$ | $R_1$ | Z | G | Z configuration |
|---|---|---|---|---|---|---|---|
| **I-489** | F | $OCF_3$ | H | $CH_3$ | Z-7 | $OCH_3$ | 1*S*,4*R*(cis) |
| **I-490** | F | $OCF_3$ | H | $CH_3$ | Z-7 | $OCH_2CH_2SCH_3$ | 1*S*,4*R*(cis) |
| **I-491** | F | $OCF_3$ | H | $CH_3$ | Z-7 | $OCH_2CH_2SOCH_3$ | 1*S*,4*R*(cis) |
| **I-492** | F | $OCF_3$ | H | $CH_3$ | Z-7 | $OCH_2CH_2SO_2CH_3$ | 1*S*,4*R*(cis) |
| **I-493** | F | $CF_3$ | H | $CH_3$ | Z-7 | OH | 1*S*,4*R*(cis) |
| **I-494** | F | $CF_3$ | H | (*R*)-$CH_3$ | Z-7 | OH | 1*S*,4*R*(cis) |
| **I-495** | F | $CF_3$ | H | $CH_3$ | Z-7 | $OCH_3$ | 1*S*,4*R*(cis) |
| **I-496** | F | $CF_3$ | H | (*R*)-$CH_3$ | Z-7 | $OCH_3$ | 1*S*,4*R*(cis) |
| **I-497** | F | $CF_3$ | H | $CH_3$ | Z-7 | $OCH_2CH_2SCH_3$ | 1*S*,4*R*(cis) |
| **I-498** | F | $CF_3$ | H | (*R*)-$CH_3$ | Z-7 | (*S*)-$OCH(CH_3)CH_2OCH_3$ | 1*S*,4*R*(cis) |
| **I-499** | F | $CF_3$ | H | (*R*)-$CH_3$ | Z-7 | $OCH_2CH_2CN$ | 1*S*,4*R*(cis) |
| **I-500** | F | $CF_3$ | H | (*R*)-$CH_3$ | Z-7 | 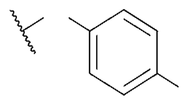 | 1*S*,4*R*(cis) |
| **I-501** | F | $CF_3$ | H | (*R*)-$CH_3$ | Z-7 | $OCH_2CH_2Br$ | 1*S*,4*R*(cis) |
| **I-502** | F | $CF_3$ | H | (*R*)-$CH_3$ | Z-7 | $OCH_2CH_2CH=CH_2$ | 1*S*,4*R*(cis) |
| **I-503** | F | $CF_3$ | H | (*R*)-$CH_3$ | Z-7 | (*R*)-$OCH_2CH(CH_3)OCH_3$ | 1*S*,4*R*(cis) |
| **I-504** | F | $OCF_2H$ | H | $CH_3$ | Z-7 | OH | 1*S*,4*R*(cis) |
| **I-505** | F | $OCF_2H$ | H | $CH_3$ | Z-7 | $OCH_3$ | 1*S*,4*R*(cis) |
| **I-506** | Cl | $OCF_2H$ | H | $CH_3$ | Z-7 | OH | 1*S*,4*R*(cis) |
| **I-507** | Cl | $OCF_2H$ | H | $CH_3$ | Z-7 | $OCH_3$ | 1S,4R(cis) |
| **I-508** | Cl | $OCF_2H$ | H | $CH_3$ | Z-7 | $OCH_2CH_2SCH_3$ | 1*S*,4*R*(cis) |
| **I-509** | Cl | $CF_3$ | H | $CH_3$ | Z-7 | OH | 1*S*,4*R*(cis) |
| **I-510** | Cl | $CF_3$ | H | (*R*)-$CH_3$ | Z-7 | OH | 1*S*,4*R*(cis) |
| **I-511** | Cl | $CF_3$ | H | $CH_3$ | Z-7 | $OCH_3$ | 1*S*,4*R*(cis) |
| **I-512** | Cl | $CF_3$ | H | (*R*)-$CH_3$ | Z-7 | $OCH_3$ | 1*S*,4*R*(cis) |
| **I-513** | Cl | $CF_3$ | H | (*R*)-$CH_3$ | Z-7 | 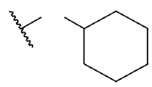 | 1*S*,4*R*(cis) |
| **I-514** | Cl | $CF_3$ | H | (*R*)-$CH_3$ | Z-7 | $OCH(CH_2CH_3)C{\equiv}CH$ | 1*S*,4*R*(cis) |
| **I-515** | Cl | $CF_3$ | H | (*R*)-$CH_3$ | Z-7 | 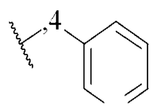 | 1*S*,4*R*(cis) |
| **I-516** | F | $CF_3$ | H | (*R*)-$CF_3$ | Z-7 | OH | 1*S*,4*R*(cis) |
| **I-517** | F | $CF_3$ | H | (*R*)-$CF_3$ | Z-7 | $OCH_3$ | 1*S*,4*R*(cis) |
| **I-518** | F | $CF_3$ | H | (*R*)-$CF_3$ | Z-7 | $OCH_2CF_2H$ | 1*S*,4*R*(cis) |
| **I-519** | F | $CF_3$ | H | (*R*)-$CF_3$ | Z-7 | 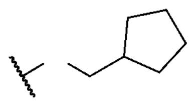 | 1*S*,4*R*(cis) |

(continued)

| Example No. | $X_1$ | $X_2$ | $X_3$ | $R_1$ | Z | G | Z configuration |
|---|---|---|---|---|---|---|---|
| **I-520** | F | $CF_3$ | H | (*R*)-$CF_3$ | Z-7 | 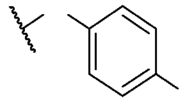 | 1*S*,4*R*(cis) |
| **I-521** | F | $CF_3$ | H | (*R*)-$CF_3$ | Z-7 | (*R*)-$OCH(CH_3)C{\equiv}CH$ | 1*S*,4*R*(cis) |
| **I-522** | F | $CF_3$ | H | (*R*)-$CF_3$ | Z-7 | (*S*)-$OCH(CH_3)C{\equiv}CH$ | 1*S*,4*R*(cis) |
| **I-523** | Cl | $CF_3$ | H | (*R*)-$CF_3$ | Z-7 | OH | 1*S*,4*R*(cis) |
| **I-524** | Cl | $CF_3$ | H | (*R*)-$CF_3$ | Z-7 | $OCH_3$ | 1*S*,4*R*(cis) |
| **I-525** | Cl | $CF_3$ | H | (*R*)-$CF_3$ | Z-7 | (*R*)-$OCH(CH_3)CH_2CH_3$ | 1*S*,4*R*(cis) |
| **I-526** | Cl | $CF_3$ | H | (*R*)-$CF_3$ | Z-7 | (*S*)-$OCH(CH_3)CH_2CH_3$ | 1*S*,4*R*(cis) |
| **I-527** | Cl | $CF_3$ | H | (*R*)-$CF_3$ | Z-7 | 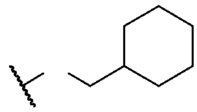 | 1*S*,4*R*(cis) |
| **I-528** | Cl | $CF_3$ | H | (*R*)-$CF_3$ | Z-7 | 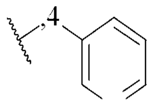 | 1*S*,4*R*(cis) |
| **I-529** | F | $OCF_3$ | H | (*R*)-$CF_3$ | Z-7 | $OCH_3$ | 1*S*,4*R*(cis) |
| **I-530** | F | $CF_3$ | H | (*R*)-$CFH_2$ | Z-7 | $OCH_3$ | 1*S*,4*R*(cis) |
| **I-531** | F | $OCF_3$ | H | (*R*)-$CFH_2$ | Z-7 | $OCH_3$ | 1*S*,4*R*(cis) |
| **I-532** | F | $CF_3$ | H | (*R*)-$CF_2H$ | Z-7 | $OCH_3$ | 1*S*,4*R*(cis) |
| **I-533** | F | $OCF_3$ | H | (*R*)-$CF_2H$ | Z-7 | $OCH_3$ | 1*S*,4*R*(cis) |
| **I-534** | F | $CF_3$ | H | (*S*)-$CH{=}CH_2$ | Z-7 | $OCH_3$ | 1*R*,4*S*(cis) |
| **I-535** | F | $CF_3$ | H | (*R*)-$CH_3$ | Z-7 | $OCH_3$ | 1*R*,4*S*(cis) |
| **I-536** | F | $CF_3$ | H | (*R*)-$CF_3$ | Z-7 | $OCH_3$ | 1*R*,4*S*(cis) |
| **I-537** | F | $OCF_3$ | H | (*S*)-$CH{=}CH_2$ | Z-7 | $OCH_3$ | 1*R*,4*S*(cis) |
| **I-538** | F | F | H | (*S*)-$CH{=}CH_2$ | Z-1 | $ON{=}C(CH_3)_2$ | cis |
| **I-539** | F | Cl | H | (*S*)-$CH{=}CH_2$ | Z-1 | $ON{=}C(CH_3)_2$ | cis |
| **I-540** | F | $CF_3$ | H | (*S*)-$CH{=}CH_2$ | Z-1 | $ON{=}C(CH_3)_2$ | cis |
| **I-541** | F | $OCF_3$ | H | (*S*)-$CH{=}CH_2$ | Z-1 | $ON{=}C(CH_3)_2$ | cis |
| **I-542** | Cl | Cl | H | (*S*)-$CH{=}CH_2$ | Z-1 | $ON{=}C(CH_3)_2$ | cis |
| **I-543** | F | F | H | (*R*)-$CH_3$ | Z-1 | $ON{=}C(CH_3)_2$ | cis |
| **I-544** | F | Cl | H | (*R*)-$CH_3$ | Z-1 | $ON{=}C(CH_3)_2$ | cis |
| **I-545** | F | $CF_3$ | H | (*R*)-$CH_3$ | Z-1 | $ON{=}C(CH_3)_2$ | cis |
| **I-546** | F | $OCF_3$ | H | (*R*)-$CH_3$ | Z-1 | $ON{=}C(CH_3)_2$ | cis |
| **I-547** | Cl | Cl | H | (*R*)-$CH_3$ | Z-1 | $ON{=}C(CH_3)_2$ | cis |
| **I-548** | F | F | H | (*R*)-$CF_3$ | Z-1 | $ON{=}C(CH_3)_2$ | cis |
| **I-549** | F | Cl | H | (*R*)-$CF_3$ | Z-1 | $ON{=}C(CH_3)_2$ | cis |
| **I-550** | F | $CF_3$ | H | (*R*)-$CF_3$ | Z-1 | $ON{=}C(CH_3)_2$ | cis |
| **I-551** | F | $OCF_3$ | H | (*R*)-$CF_3$ | Z-1 | $ON{=}C(CH_3)_2$ | cis |
| **I-552** | Cl | Cl | H | (*R*)-$CF_3$ | Z-1 | $ON{=}C(CH_3)_2$ | cis |
| **I-553** | F | $CF_3$ | H | (*S*)-$CH{=}CH_2$ | Z-5 | $ON{=}C(CH_3)_2$ | cis-(rac) |

(continued)

| Example No. | $X_1$ | $X_2$ | $X_3$ | $R_1$ | Z | G | Z configuration |
|---|---|---|---|---|---|---|---|
| **I-554** | F | $OCF_3$ | H | (*S*)-$CH=CH_2$ | Z-5 | $ON=C(CH_3)_2$ | cis-(rac) |
| **I-555** | F | $OCF_2H$ | H | (*S*)-$CH=CH_2$ | Z-5 | $ON=C(CH_3)_2$ | cis-(rac) |
| **I-556** | F | F | H | (*S*)-$CH=CH_2$ | Z-6 | $ON=C(CH_3)_2$ | 1*R*,3*S*(cis) |
| **I-557** | F | Cl | H | (*S*)-$CH=CH_2$ | Z-6 | $ON=C(CH_3)_2$ | 1*R*,3*S*(cis) |
| **I-558** | F | $CF_3$ | H | (*S*)-$CH=CH_2$ | Z-6 | $ON=C(CH_3)_2$ | 1*R*,3*S*(cis) |
| **I-559** | F | $OCF_3$ | H | (*S*)-$CH=CH_2$ | Z-6 | $ON=C(CH_3)_2$ | 1*R*,3*S*(cis) |
| **I-560** | F | $CF_3$ | H | (*S*)-$CH=CH_2$ | Z-7 | $ON=C(CH_3)_2$ | 1*S*,4*R*(cis) |
| **I-561** | F | $OCF_3$ | H | (*S*)-$CH=CH_2$ | Z-7 | $ON=C(CH_3)_2$ | 1*S*,4*R*(cis) |
| **I-562** | Cl | $CF_3$ | H | (*S*)-$CH=CH_2$ | Z-7 | $ON=C(CH_3)_2$ | 1*S*,4*R*(cis) |
| **I-563** | Cl | $OCF_3$ | H | (*S*)-$CH=CH_2$ | Z-7 | $ON=C(CH_3)_2$ | 1*S*,4*R*(cis) |
| **I-564** | F | F | F | (*S*)-$CH=CH_2$ | Z-7 | $ON=C(CH_3)_2$ | 1*S*,4*R*(cis) |
| **I-565** | F | $CF_3$ | H | (*R*)-$CH_3$ | Z-7 | $ON=C(CH_3)_2$ | 1*S*,4*R*(cis) |
| **I-566** | Cl | $CF_3$ | H | (*R*)-$CH_3$ | Z-7 | $ON=C(CH_3)_2$ | 1*S*,4*R*(cis) |
| **I-567** | F | $CF_3$ | H | (*R*)-$CF_3$ | Z-7 | $ON=C(CH_3)_2$ | 1*S*,4*R*(cis) |
| **I-568** | Cl | $CF_3$ | H | (*R*)-$CF_3$ | Z-7 | $ON=C(CH_3)_2$ | 1*S*,4*R*(cis) |
| **I-569** | F | F | H | (*R*)-$CH_3$ | Z-1 | $OCH_2CH_2SCH_3$ | cis |
| **I-570** | F | $OCF_3$ | H | (*S*)-$CH=CH_2$ | Z-5 | $OCH_3$ | cis-(rac) |
| **I-571** | F | $OCF_2H$ | H | (*S*)-$CH=CH_2$ | Z-5 | $OCH_3$ | cis-(rac) |
| **I-572** | Cl | $OCF_3$ | H | (*S*)-$CH=CH_2$ | Z-5 | $OCH_3$ | cis-(rac) |
| **I-573** | Cl | $OCF_2H$ | H | (*S*)-$CH=CH_2$ | Z-5 | $OCH_3$ | cis-(rac) |

The compounds according to the invention can be prepared by various processes listed below:

Scheme 1:

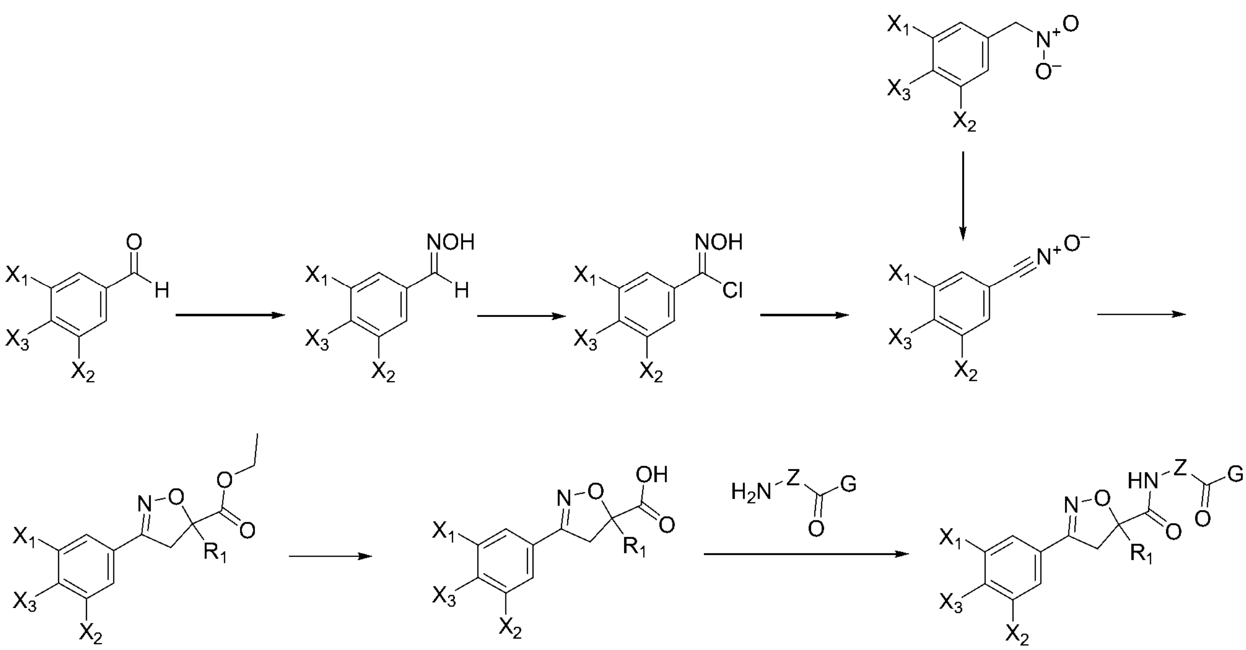

In Scheme 1 and the schemes which follow. Such 1,3-dipolar cycloadditions of nitrile oxides with suitable

dipolarophiles are described, for example, in Reviews: 1,3 dipolar Cycloaddition Chemistry, Padwa, ed. Wiley, New York, 1984; Kanemasa and Tsuge, Heterocycles 1990, 30, 719. For preparation of chloroximes, see Kim, Jae N., Ryu, Eung K. J. Org. Chem. 1992, 57, 6649).

Compounds according to the invention substituted in the 4 and 5 positions of the isoxazoline ring system can likewise be prepared by 1,3-dipolar cycloaddition by using suitable 1,2-disubstituted olefins as dipolarophiles. Usually, this reaction gives diastereomer mixtures which can be separated by column chromatography. Optically active isoxazolines can be obtained by chiral HPLC of suitable precursors or end products and also by enantioselective reactions such as, for example, enzymatic ester or amide cleavage or by using chiral auxiliaries at the dipolarophile, as described by Olssen (J. Org. Chem. 1988, 53, 2468).

For preparation of the compounds according to the invention, it is also possible to use suitably substituted 2-alkoxyacrylamides (Scheme 3). These are obtainable from the acrylic esters described in Scheme 2 after hydrolysis and amide formation.

Scheme 2:

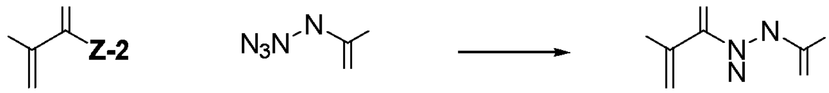

One option for activating the acrylic acid is carbodiimides, for example EDCI (Chen, F. M. F.; Benoiton, N. L. Synthesis 1979, 709). For preparation of acrylamides, see U.S. Pat. No. 2,521,902, JP60112746, J. of Polymer Science 1979, 17 (6), 1655. Suitably substituted acrylamides can be reacted in a 1,3-cycloaddition reaction with nitrile oxides to give the compounds according to the invention (Scheme 3).

Scheme 3:

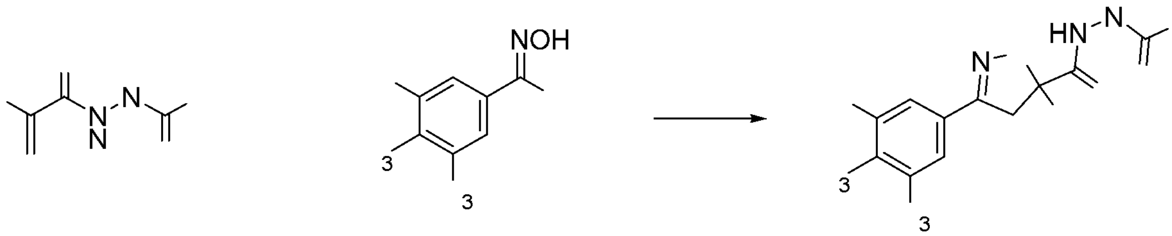

Transformations of the functional groups $R^3$ are possible either at the alkene stage or at the isoxazoline stage.

Collections of compounds of the formula (I) and/or salts thereof which can be synthesized by the above-mentioned reactions can also be prepared in a parallelized manner, in which case this may be accomplished in a manual, partly automated or fully automated manner. It is possible, for example, to automate the conduct of the reaction, the workup or the purification of the products and/or intermediates. Overall, this is understood to mean a procedure as described, for example, by D. Tiebes in Combinatorial Chemistry-Synthesis, Analysis, Screening (editor: Gunther Jung), Wiley, 1999, on pages 1 to 34.

The compounds of the formula (I) according to the invention (and/or salts thereof), referred to collectively as "compounds according to the invention" hereinafter, have excellent herbicidal efficacy against a broad spectrum of economically important monocotyledonous and dicotyledonous annual harmful plants. may effectively control a variety of weeds, may achieve good results at low doses, and may be used as herbicide. Therefore, the present invention further includes a use of the compounds of the general formula (I) in control of weeds.

The present invention therefore also provides a method for controlling unwanted plants or for regulating the growth of plants, preferably in plant crops, in which one or more compound(s) of the invention is/are applied to the plants (for example harmful plants such as monocotyledonous or dicotyledonous weeds or unwanted crop plants), the seed (for example grains, seeds or vegetative propagules such as tubers or shoot parts with buds) or the area on which the plants grow (for example the area under cultivation). The compounds of the invention can be deployed, for example, prior to sowing (if appropriate also by incorporation into the soil), prior to emergence or after emergence. Specific examples of some representatives of the monocotyledonous and dicotyledonous weed flora which can be controlled by the compounds of the invention are as follows, though the enumeration is not intended to impose a restriction to particular species.

Monocotyledonous harmful plants of the genera: *Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis,*

*Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.*

Dicotyledonous weeds of the genera: *Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.*

When the compound of the present invention is applied to soil before seedling, the growth of harmful plant seeds stops after treatment, and harmful plants stay in a growth period at the time of application, or die completely after a period of time, thereby eliminating competition of weeds harmful to crop plants in a lasting manner at an extremely early time point.

When the compound of the present invention is applied to green plant sites after seedling, the growth stops after treatment, and harmful plants stay in a growth period at the time of application, or die completely after a period of time, thereby eliminating competition of weeds harmful to crop plants in a lasting manner at an extremely early time point.

The compounds of the present invention can be selective in crops of useful plants and can also be used as non-selective herbicides.

Therefore, the technical solution of the present invention further includes a use of the compounds of the general formula (I) in control of weeds.

In addition, the compounds of the general formula (I) of the present invention are also applicable to drying and/or defoliation of plants.

As mentioned earlier, the present invention provides a pesticide herbicide, which is composed of an active ingredient and excipients, where the active ingredient includes at least one of the foregoing uracil compounds containing a carboxylate fragment.

Preferably, the content of the active ingredient in the pesticide herbicide is 0.1-99.9 weight%.

The present invention has no special limitations on specific types of the excipients in the herbicide, such as various surfactants and solvents commonly used in the field of herbicides.

The compounds according to the invention can be applied in the customary formulations in the form of wettable powders, emulsifiable concentrates, sprayable solutions, dusts or granules. The invention therefore also provides herbicidal compositions comprising compounds of the formula I. The compounds of the formula I can be formulated in various ways depending on the prevailing biological and/or chemico-physical parameters. Examples of suitable formulation options are: wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), oil dispersions (OD), oil- or water-based dispersions, oil-miscible solutions, dusts (DP), capsule suspensions (CS), seed-dressing compositions, granules for broadcasting and soil application, granules (GR) in the form of microgranules, spray granules, coating granules and adsorption granules, water-dispersible granules (WG), water-soluble granules (SG), ULV formulations, microcapsules and waxes. These individual formulation types are known in principle and are described, for example, in Winnacker-Küchler, "Chemische Technologie" [Chemical Technology], Volume 7, C. Hauser Verlag Munich, 4th. Edition 1986; Wade van Valkenburg, "Pesticide Formulations", Marcel Dekker, N. Y. , 1973; K. Martens, "Spray Drying" Handbook, 3rd Ed. 1979, G. Goodwin Ltd. London.

The necessary formulation auxiliaries, such as inert materials, surfactants, solvents and other additives, are likewise known and are described, for example, in Watkins, "Handbook of Insecticide Dust Diluents and Carriers", 2nd Ed. , Darland Books, Caldwell N. J. , H. v. Olphen, "Introduction to Clay Colloid Chemistry"; 2nd Ed. , J. Wiley & Sons, N. Y. ; C. Marsden, "Solvents Guide"; 2nd Ed. , Interscience, N. Y. 1963; McCutcheon's "Detergents and Emulsifiers Annual", MC Publ. Corp. , Ridgewood N. J. ; Sisley and Wood, "Encyclopedia of Surface Active Agents", Chem. Publ. Co. Inc. , N. Y. 1964; Schönfeldt, "Grenzflüchenaktive Äthylenoxidaddkte" [Surface-active ethylene oxide adducts], Wiss. Verlagagesell. Stuttgart 1976; Winnacker-Küchler, "Chemische Technologie" [Chemical Technology], Volume 7, C. Hauser Verlag Munich, 4th Edition 1986.

Wettable powders are preparations which are uniformly dispersible in water and which contain, in addition to the active compound and as well as a diluent or inert substance, surfactants of ionic and/or nonionic type (wetting agents, dispersants), for example polyethoxylated alkyl phenols, polyethoxylated fatty alcohols, polyethoxylated fatty amines, fatty alcohol polyglycol ethersulfates, alkanesulfonates, alkylbenzenesulfonates, sodium ligninsulfonate, sodium 2,2'-dinaphthylmethane-6,6'-disulfonate, sodium dibutyinaphthalenesulfona-te or else sodium oleoylmethyltaurinate. To prepare the wettable powders, the herbicidally active compounds are finely ground, for example in customary apparatus such as hammer mills, fan mills and air-jet mills, and are mixed simultaneously or subsequently with the formulation auxiliaries.

Emulsifiable concentrates are prepared by dissolving the active compound in an organic solvent, for example butanol, cyclohexanone, dimethylformamide, xylene or else relatively highboiling aromatic compounds or hydrocarbons or mixtures of the solvents, with the addition of one or more surfactants of ionic and/or nonionic type (emulsifiers).

Examples of emulsifiers which can be used are calcium alkylarylsulfonates, such as Ca dodecylbenzenesulfonate, or nonionic emulsifiers, such as fatty acid polyglycol esters, alkylaryl polyglycol ethers, fatty alcohol polyglycol ethers, propylene oxide-ethylene oxide condensation products, alkyl polyethers, sorbitan esters, for example sorbitan fatty acid esters or polyoxyethylene sorbitan esters, for example polyoxyethylene sorbitan fatty acid esters.

Dusts are obtained by grinding the active compound with finely divided solid substances, for example talc, natural clays, such as kaolin, bentonite and pyrophyllite, or diatomaceous earth. Suspension concentrates can be water- or oil-based. They can be prepared, for example, by wet milling using commercially customary bead mills, with or without the addition of surfactants as already mentioned above, for example, in the case of the other formulation types.

Emulsions, for example oil-in-water emulsions (EW), can be prepared for example by means of stirrers, colloid mills and/or static mixers using aqueous organic solvents and, if desired, surfactants as already mentioned above, for example, in the case of the other formulation types.

Granules can be prepared either by spraying the active compound onto adsorptive, granulated inert material or by applying active-compound concentrates to the surface of carriers such as sand, kaolinites or granulated inert material, by means of adhesive binders, for example polyvinyl alcohol, sodium polyacrylate or else mineral oils. Suitable active compounds can also be granulated in the manner which is customary for the preparation of fertilizer granules, if desired as a mixture with fertilizers. Water-dispersible granules are generally prepared by the customary processes, such as spray-drying, fluidized-bed granulation, disk granulation, mixing using high-speed mixers, and extrusion without solid inert material.

For the preparation of disk, fluidized-bed, extruder and spray granules, see for example processes in "Spray-Drying Handbook" 3rd ed. 1979, G. Goodwin Ltd. , London; J. E. Browning, "Agglomeration", Chemical and Engineering 1967, pages 147 ff. ; "Perry's Chemical Engineer's Handbook", 5th Ed. , McGraw-Hill, New York 1973, pp. 8-57. For further details on the formulation of crop protection products, see for example G. C. Klingman, "Weed Control as a Science", John Wiley and Sons Inc. , New York, 1961, pages 81-96 and J. D. Freyer, S. A. Evans, "Weed Control Handbook", 5th Ed. , Blackwell Scientific Publications, Oxford, 1968, pages 101-103.

The agrochemical formulations generally contain from 0.1 to 99% by weight, in particular from 0.1 to 95% by weight, of active compound of the formula (I). In wettable powders the concentration of active compound is, for example, from about 10 to 99% by weight, the remainder to 100% by weight consisting of customary formulation constituents. In emulsifiable concentrates the concentration of active compound can be from about 1 to 90%, preferably from 5 to 80%, by weight. Formulations in the form of dusts contain from 1 to 30% by weight of active compound, preferably most commonly from 5 to 20% by weight of active compound, while sprayable solutions contain from about 0. 05 to 80%, preferably from 2 to 50%, by weight of active compound. In the case of water-dispersible granules the content of active compound depends partly on whether the active compound is in liquid or solid form and on the granulation auxiliaries, fillers, etc. that are used. In water-dispersible granules the content of active compound, for example, is between 1 and 95% by weight, preferably between 10 and 80% by weight.

In addition, said formulations of active compound may comprise the tackifiers, wetting agents, dispersants, emulsifiers, penetrants, preservatives, antifreeze agents, solvents, fillers, carriers, colorants, antifoams, evaporation inhibitors and pH and viscosity regulators which are customary in each case.

Based on these formulations it is also possible to produce combinations with other pesticidally active substances, for example insecticides, acaricides, herbicides and fungicides, and also with safeners, fertilizers and/or growth regulators, for example in the form of a ready-mix or tank mix.

For application, the formulations in commercial form are, if appropriate, diluted in a customary manner, for example in the case of wettable powders, emulsifiable concentrates, dispersions and water-dispersible granules with water. Dust-type preparations, granules for soil application or granules for scattering and sprayable solutions are not normally diluted further with other inert substances prior to application.

The required application rate of the compounds of the formula (I) varies with the external conditions, including, inter alia, temperature, humidity and the type of herbicide used. It can vary within wide limits, for example between 0.001 and 1.0 kg/ha or more of active substance, but it is preferably between 0.005 and 750 g/ha.

A carrier is a natural or synthetic organic or inorganic substance with which the active compounds are mixed or combined for better applicability, in particular for application to plants or plant parts or seed. The carrier, which may be solid or liquid, is generally inert and should be suitable for use in agriculture.

Useful solid or liquid carriers include: for example ammonium salts and natural rock dusts, such as kaolins, clays, talc, chalk, quartz, attapulgite, montmorillonite or diatomaceous earth, and synthetic rock dusts, such as finely divided silica, alumina and natural or synthetic silicates, resins, waxes, solid fertilizers, water, alcohols, especially butanol, organic solvents, mineral and vegetable oils, and derivatives thereof. It is likewise possible to use mixtures of such carriers. Useful solid carriers for granules include: for example crushed and fractionated natural rocks such as calcite, marble, pumice, sepiolite, dolomite, and synthetic granules of inorganic and organic meals, and also granules of organic material such as sawdust, coconut shells, corn cobs and tobacco stalks.

Suitable liquefied gaseous extenders or carriers are liquids which are gaseous at standard temperature and

under atmospheric pressure, for example aerosol propellants such as halogenated hydrocarbons, or else butane, propane, nitrogen and carbon dioxide.

In the formulations, it is possible to use tackifiers such as carboxymethylcellulose, natural and synthetic polymers in the form of powders, granules or latices, such as gum arabic, polyvinyl alcohol and polyvinyl acetate, or else natural phospholipids such as cephalins and lecithins and synthetic phospholipids. Further additives may be mineral and vegetable oils.

When the extender used is water, it is also possible to use, for example, organic solvents as auxiliary solvents. Suitable liquid solvents are essentially: aromatics such as xylene, toluene or alkylnaphthalenes, chlorinated aromatics and chlorinated aliphatic hydrocarbons such as chlorobenzenes, chloroethylenes or dichloromethane, aliphatic hydrocarbons such as cyclohexane or paraffins, for example mineral oil fractions, mineral and vegetable oils, alcohols such as butanol or glycol and their ethers and esters, ketones such as acetone, methyl ethyl ketone, methyl isobutyl ketone or cyclohexanone, strongly polar solvents such as dimethylformamide and dimethyl sulfoxide, and also water.

The compositions according to the invention may additionally comprise further components, for example surfactants. Useful surfactants are emulsifiers and/or foam formers, dispersants or wetting agents having ionic or nonionic properties, or mixtures of these surfactants. Examples thereof are salts of polyacrylic acid, salts of lignosulfonic acid, salts of phenolsulfonic acid or naphthalenesulfonic acid, polycondensates of ethylene oxide with fatty alcohols or with fatty acids or with fatty amines, substituted phenols (preferably alkylphenols or arylphenols), salts of sulfosuccinic esters, taurine derivatives (preferably alkyl taurates), phosphoric esters of polyethoxylated alcohols or phenols, fatty acid esters of polyols, and derivatives of the compounds containing sulfates, sulfonates and phosphates, for example alkylaryl polyglycol ethers, alkylsulfonates, alkyl sulfates, arylsulfonates, protein hydrolyzates, lignosulfite waste liquors and methylcellulose. The presence of a surfactant is necessary if one of the active compounds and/or one of the inert carriers is insoluble in water and when application is effected in water. The proportion of surfactants is between 5 and 40 percent by weight of the inventive composition. It is possible to use dyes such as inorganic pigments, for example iron oxide, titanium oxide and Prussian Blue, and organic dyes such as alizarin dyes, azo dyes and metal phthalocyanine dyes, and trace nutrients such as salts of iron, manganese, boron, copper, cobalt, molybdenum and zinc.

If appropriate, it is also possible for other additional components to be present, for example protective colloids, binders, adhesives, thickeners, thixotropic substances, penetrants, stabilizers, sequestrants, complexing agents. In general, the active compounds can be combined with any solid or liquid additive commonly used for formulation purposes. In general, the compositions and formulations according to the invention contain between 0.05 and 99% by weight, 0.01 and 98% by weight, preferably between 0.1 and 95% by weight and more preferably between 0.5 and 90% active compound, most preferably between 10 and 70 percent by weight. The active compounds or compositions according to the invention can be used as such or, depending on their respective physical and/or chemical properties, in the form of their formulations or the use forms prepared therefrom, such as aerosols, capsule suspensions, cold-fogging concentrates, warm-fogging concentrates, encapsulated granules, fine granules, flowable concentrates for the treatment of seed, ready-to-use solutions, dustable powders, emulsifiable concentrates, oil-in-water emulsions, water-in-oil emulsions, macrogranules, microgranules, oil-dispersible powders, oil-miscible flowable concentrates, oil-miscible liquids, foams, pastes, pesticide coated seed, suspension concentrates, suspoemulsion concentrates, soluble concentrates, suspensions, wettable powders, soluble powders, dusts and granules, water-soluble granules or tablets, water-soluble powders for the treatment of seed, wettable powders, natural products and synthetic substances impregnated with active compound, and also microencapsulations in polymeric substances and in coating materials for seed, and also ULV cold-fogging and warm-fogging formulations.

The formulations mentioned can be produced in a manner known per se, for example by mixing the active compounds with at least one customary extender, solvent or diluent, emulsifier, dispersant and/or binder or fixative, wetting agent, water repellent, optionally siccatives and UV stabilizers and optionally dyes and pigments, antifoams, preservatives, secondary thickeners, tackifiers, gibberellins and other processing auxiliaries.

The compositions according to the invention include not only formulations which are already ready for use and can be deployed with a suitable apparatus onto the plant or the seed, but also commercial concentrates which have to be diluted with water prior to use.

The active compounds according to the invention may be present as such or in their (commercial standard) formulations, or else in the use forms prepared from these formulations as a mixture with other (known) active compounds, such as insecticides, attractants, sterilants, bactericides, acaricides, nematicides, fungicides, growth regulators, herbicides, fertilizers, safeners or semiochemicals.

The treatment according to the invention of the plants and plant parts with the active compounds or compositions is carried out directly or by action on their surroundings, habitat or storage space using customary treatment methods, for example by dipping, spraying, atomizing, irrigating, evaporating, dusting, fogging, broadcasting, foaming, painting, spreading-on, watering (drenching), drip irrigating and, in the case of propagation material, in particular in the case of seeds, furthermore as a powder for dry seed treatment, a solution for seed treatment, a water-soluble powder for slurry treatment, by incrusting, by coating with one or more coats, etc. It is furthermore possible to apply the active compounds by

the ultra-low volume method or to inject the active compound preparation or the active compound itself into the soil.

In order to enhance the control effect of the 3-phenylisoxazoline-5-carboxamide compounds of the present invention and increase the scope of use thereof, the 3-phenylisoxazoline-5-carboxamide compounds of the present invention can be used either alone or in combination with other commonly used herbicides, and there is no special limitation on the proportion of the said combination, and it can be selected in accordance with the proportion of the conventional use in the field, as long as the control effect can be enhanced and the scope of use can be increased, and safety can be improved. As long as the control effect can be enhanced after compounding, the range of use can be enlarged and the safety can be improved.

Suitable active compounds which can be combined with the active compounds according to the invention in mixed formulations or in a tank mix are, for example, known active compounds as described in for example World Herbicide New Product Technology Handbook, China Agricultural Science and Farming Techniques Press, 2010. 9 and in the literature cited therein. For example the following active compounds may be mentioned as herbicides which can be combined with the compounds of the formula I (note: the compounds are either named by the "common name" in accordance with the International Organization for Standardization (ISO) or by the chemical names, if appropriate together with a customary code number): acetochlor, butachlor, alachlor, propisochlor, metolachlor, s-metolachlor, pretilachlor, propachlor, ethachlor, napropamide, R-left handed napropamide, propanil, mefenacet, diphenamid, diflufenican, ethaprochlor, beflubutamid, bromobutide, dimethenamid, dimethenamid-P, etobenzanid, flufenacet, thenylchlor, metazachlor, isoxaben, flamprop-M-methyl, flamprop-M-propyl, allidochlor, pethoxamid, chloranocryl, cyprazine, mefluidide, monalide, delachlor, prynachlor, terbuchlor, xylachlor, dimethachlor, cisanilide, trimexachlor, clomeprop, propyzamide, pentanochlor, carbetamide, benzoylprop-ethyl, cyprazole, butenachlor, tebutam, benzipram, mogrton, dichlofluanid, naproanilide, diethatyl-ethyl, naptalam, flufenacet, benzadox, chlorthiamid, chlorophthalimide, isocarbamide, picolinafen, atrazine, simazine, prometryn, cyanatryn, simetryn, ametryn, propazine, dipropetryn, SSH-108, terbutryn, terbuthylazine, triaziflam, cyprazine, proglinazine, trietazine, prometon, simetone, aziprotryne, desmetryn, dimethametryn, procyazine, mesoprazine, sebuthylazine, secbumeton, terbumeton, methoprotryne, cyanatryn, ipazine, chlorazine, atraton, pendimethalin, eglinazine, cyanuric acid, indaziflam, chlorsulfuron, metsulfuron-methyl, bensulfuron methyl, chlorimuronethyl, tribenuron-methyl, thifensulfuron-methyl, pyrazosulfuron-ethyl, mesosulfuron, iodosulfuron-methyl sodium, foramsulfuron, cinosulfuron, triasulfuron, sulfometuron methyl, nicosulfuron, ethametsulfuron-methyl, amidosulfuron, ethoxysulfuron, cyclosulfamuron, rimsulfuron, azimsulfuron, flazasulfuron, monosulfuron, monosulfuron-ester, flucarbazonesodium, flupyrsulfuron-methyl, halosulfuron-methyl, oxasulfuron, imazosulfuron, primisulfuron, propoxycarbazone, prosulfuron, sulfosulfuron, trifloxysulfuron, triflusulfuron-methyl, tritosulfuron, sodium metsulfuron methyl, flucetosulfuron, HNPC-C, orthosulfamuron, propyrisulfuron, metazosulfuron, acifluorfen, fomesafen, lactofen, fluoroglycofen, oxyfluorfen, chlornitrofen, aclonifen, ethoxyfen-ethyl, bifenox, nitrofluorfen, chlomethoxyfen, fluorodifen, fluoronitrofen, furyloxyfen, nitrofen, TOPE, DMNP, PPG1013, AKH-7088, halosafen, chlortoluron, isoproturon, linuron, diuron, dymron, fluometuron, benzthiazuron, methabenzthiazuron, cumyluron, ethidimuron, isouron, tebuthiuron, buturon, chlorbromuron, methyldymron, phenobenzuron, SK-85, metobromuron, metoxuron, afesin, monuron, siduron, fenuron, fluothiuron, neburon, chloroxuron, noruron, isonoruron, 3-cyclooctyl-1, thiazfluron, tebuthiuron, difenoxuron, parafluron, methylamine tribunil, karbutilate, trimeturon, dimefuron, monisouron, anisuron, methiuron, chloreturon, tetrafluron, phenmedipham, phenmedipham-ethyl, desmedipham, asulam, terbucarb, barban, propham, chlorpropham, rowmate, swep, chlorbufam, carboxazole, chlorprocarb, fenasulam, BCPC, CPPC, carbasulam, butylate, benthiocarb, vernolate, molinate, triallate, dimepiperate, esprocarb, pyributicarb, cycloate, avadex, EPTC, ethiolate, orbencarb, pebulate, prosulfocarb, tiocarbazil, CDEC, dimexano, isopolinate, methiobencarb, 2,4-D butyl ester, MCPA-Na, 2,4-D isooctyl ester, MCPA isooctyl ester, 2,4-D sodium salt, 2,4-D dimethyla mine salt, MCPA-thioethyl, MCPA, 2,4-D propionic acid, high 2,4-D propionic acid salt, 2,4-D butyric acid, MCPA propionic acid, MCPA propionic acid salt, MCPA butyric acid, 2,4,5-D, 2,4,5-D propionic acid, 2,4,5-D butyric acid, MCPA amine salt, dicamba, erbon, chlorfenac, saison, TBA, chloramben, methoxy-TBA, diclofop-methyl, fluazifop-butyl, fluazifop-p-butyl, haloxyfopmethyl, haloxyfop-P, quizalofop-ethyl, quizalofop-p-ethyl, fenoxaprop-ethy, fenoxaprop-p-ethyl, propaquizafop, cyhalofop-butyl, metamifop, clodinafop-propargyl, fenthiaprop-ethyl, chloroazifop-propynyl, poppenate-methyl, trifopsime, isoxapyrifop, paraquat, diquat, oryzalin, ethalfluralin, isopropalin, nitralin, profluralin, prodinamine, benfluralin, fluchloraline, dinitramina, dipropalin, chlornidine, methalpropalin, dinoprop, glyphosate, anilofos, glufosinate ammonium, amiprophos-methyl, sulphosate, piperophos, bialaphos-sodium, bensulide, butamifos, phocarb, 2,4-DEP, H-9201, zytron, imazapyr, imazethapyr, imazaquin, imazamox, imazamox ammonium salt, imazapic, imazamethabenz-methyl, fluroxypyr, fluroxypyr isooctyl ester, clopyralid, picloram, trichlopyr, dithiopyr, haloxydine, 3,5,6-trichloro-2-pyridinol, thiazopyr, fluridone, aminopyralid, diflufenzopyr, triclopyr-butotyl, Cliodinate, sethoxydim, clethodim, cycloxydim, alloxydim, clefoxydim, butroxydim, tralkoxydim, tepraloxydim, buthidazole, metribuzin, hexazinone, metamitron, ethiozin, ametridione, amibuzin, bromoxynil, bromoxynil octanoate, ioxynil octanoate, ioxynil, dichlobenil, diphenatrile, pyraclonil, chloroxynil, iodobonil, flumetsulam, florasulam, penoxsulam, metosulam, cloransulam-methyl, diclosulam, pyroxsulam, benfuresate, bispyribacsodium, pyribenzoxim, pyriftalid, pyriminobac-methyl, pyrithiobac-sodium, benzobicylon, mesotrione, sulcotrione, tembotrione, tefuryltrione, bicyclopyrone, ketodpiradox, isoxaflutole, clomazone, fenoxasulfone, methiozolin, fluazolate, pyraflufen-ethyl, pyrazolynate, difenzoquat, pyrazoxyfen, benzofenap, nipyraclofen, pyrasulfotole, topramezone, pyroxasulfone, cafenstrole, flupoxam, aminotriazole, amicarbazone, azafenidin, carfentrazone-ethyl, sulfentrazone, bencarbazone, benzfendizone, butafenacil, bromacil, isocil, lenacil, terbacil, flupropacil, cinidonethyl, flumiclorac-pentyl, flumioxazin, propyzamide, MK-129, flumezin, pentachlorophenol, dinoseb, dinoterb, dinoterb acetate, dinosam, DNOC, chloronitrophene, medinoterb acetate, dinofenate, oxadiargyl, oxadiazon, pentoxazone, Flufenacet, fluthiacet-methyl, fentrazamide, flufenpyr-ethyl, pyrazon, brompyrazon, metflurazon, kusakira, dimidazon, oxapyrazon, norflurazon, pyridafol, quinclorac, quinmerac, bentazone, pyridate, oxaziclomefone, benazolin, clomazone, cinmethylin, ZJ0702, pyribambenz-propyl, indanofan, sodium chlorate, dalapon, trichloroacetic acid, monochloroacetic acid, hexachloroacetone, flupropanate, cyperquat, bromofenoxim, epronaz, methazole, flurtamone, benfuresate, ethofumesate, tioclorim, chlorthal, fluorochloridone, tavron, acrolein, bentranil, tridiphane, chlorfenpropmethyl, thidiarizonaimin, phenisopham, busoxinone, methoxyphenone, saflufenacil, clacyfos, chloropon, alorac, diethamquat, etnipromid, iprymidam, ipfencarbazone, thiencarbazone-methyl, pyrimisulfan, chlorflurazole, tripropindan, sulglycapin, prosulfalin, cambendichlor, aminocyclopyrachlor, rodethanil, benoxacor, fenclorim, flurazole, fenchlorazole-ethyl, cloquintocet-mexyl, oxabetrinil, MG/91, cyometrinil, DKA-24, mefenpyr-diethyl, furilazole, fluxofenim, isoxadifen-ethyl, dichlormid, halauxifen-methyl, DOW florpyrauxifen, UBH-509, D489, LS 82-556, KPP-300, NC-324, NC-330, KH-218, DPX-N8189, SC-0744, DOWCO535, DK-8910, V-53482, PP-600, MBH-001, KIH-9201, ET-751, KIH-6127 and KIH-2023.

Examples of the safener include benoxacor, BPCMS (CSB), cloquintocet, cloquintocet-mexyl, cumyluron, cyometrinil, cyprosulfamide, daimuron (dymron), dichlormid, dicyclonon (diclonon), dietholate, dimepiperate, disulphoton, fenchlorazole, fenchlorazole-ethyl, fenclorim, flurazole, fluxofenim, furilazole, hexim, isoxadifen, isoxadifen-ethyl, MCPA, mecoprop, mefenpyr, mefenpyr-diethyl, mephenate, metcamifen, methoxyphenone, 1,8-naphthalic anhydride (NA), octamethylene-diamine, oxabetrinil, 4-(dichloroacetyl)-1-oxa-4-azaspiro[4.5]decane (AD67, MON4660), 4-carboxy-3,4-dihydro-2H-oxo-benzopyran-4-acetic acid (CL304415), 2,2-dichloro-N-[2-oxo-2(propenylamino)ethyl]-N-2-propenylacetamide (DKA-24), 2-(dichloromethyl)-2-methyl-1,3-dioxolane (MG191), 2-propenyl 1-oxa-4-azaspiro[4,5]decane-4-carbodithioate (MG838), (3-dichloroacetyl-5-(2-furyl)-2,2-dimethyloxazolidine) (MON13900), (N-allyl-N-[(1,3-dioxolan-2-yl)methyl]dichloroacetamide (PPG-1292), 3-(dichloroacetyl)-2,2-dimethyl-1,3-oxazolidine (R28725), 3-(dichloroacetyl)-2,2,5-trimethyl-1,3-oxazolidine (R29148), and 1-dichloroacetylazepane (TI-35). These ingredients may be used alone or in combination of two or more species. When two or more species are used in combination, the proportions thereof may be arbitrarily determined.

As also described below, the treatment of transgenic seed with the active compounds according to the invention or compositions is of particular significance. This relates to the seed of plants containing at least one heterologous gene which enables the expression of a polypeptide or protein having insecticidal properties. The heterologous gene in transgenic seed can originate, for example, from microorganisms of the species *Bacillus, Rhizobium, Pseudomonas, Serratia, Trichoderma, Clavibacter, Glomus* or *Gliocladium.* This heterologous gene preferably originates from *Bacillus* sp., in which case the gene product is effective against the European corn borer and/or the Western corn rootworm. The heterologous gene more preferably originates from *Bacillus thuringiensis.*

In the context of the present invention, the inventive composition is applied to the seed alone or in a suitable formulation. Preferably, the seed is treated in a state in which it is sufficiently stable for no damage to occur in the course of treatment. In general, the seed can be treated at any time between harvest and sowing. It is customary to use seed which has been separated from the plant and freed from cobs, shells, stalks, coats, hairs or the flesh of the fruits. For example, it is possible to use seed which has been harvested, cleaned and dried down to a moisture content of less than 15% by weight. Alternatively, it is also possible to use seed which, after drying, for example, has been treated with water and then dried again.

In general, when treating the seed, it has to be ensured that the amount of the composition according to the invention and/or further additives applied to the seed is chosen such that the germination of the seed is not impaired and the plant which arises therefrom is not damaged. This has to be ensured particularly in the case of active compounds which can exhibit phytotoxic effects at certain application rates.

The compositions according to the invention can be applied directly, i.e. without containing any other components and without having been diluted. In general, it is preferable to apply the compositions to the seed in the form of a suitable formulation. Suitable formulations and methods for seed treatment are known to those skilled in the art and are described, for example, in the following documents: U.S. Pat. Nos. 4,272,417 A, 4,245,432 A, 4,808,430, 5,876,739, US 2003/0176428 A1, WO 2002/080675 A1, WO 2002/028186 A2.

The active compounds which can be used in accordance with the invention can be converted to the customary seed-dressing formulations, such as solutions, emulsions, suspensions, powders, foams, slurries or other coating compositions for seed, and also ULV formulations.

These formulations are produced in a known manner, by mixing the active compounds with customary additives, for example customary extenders and solvents or diluents, dyes, wetting agents, dispersants, emulsifiers, antifoams, preservatives, secondary thickeners, adhesives, gibberellins, and also water. Dyes which may be present in the seed-dressing formulations usable in accordance with the invention are all dyes which are customary for such purposes. It is

possible to use either pigments, which are sparingly soluble in water, or dyes, which are soluble in water. Examples include the dyes known by the names Rhodamine B, C.I. Pigment Red 112 and C.I. Solvent Red 1.

Useful wetting agents which may be present in the seed-dressing formulations usable in accordance with the invention are all substances which promote wetting and which are customary for the formulation of agrochemically active compounds. Alkyl naphthalenesulfonates, such as diisopropyl or diisobutyl naphthalenesulfonates, can be used with preference.

Suitable dispersants and/or emulsifiers which may be present in the seed-dressing formulations usable in accordance with the invention are all nonionic, anionic and cationic dispersants customary for the formulation of agrochemically active compounds. Preference is given to using nonionic or anionic dispersants or mixtures of nonionic or anionic dispersants. Suitable nonionic dispersants include especially ethylene oxide/propylene oxide block polymers, alkylphenol polyglycol ethers and tristryrylphenol polyglycol ether, and the phosphated or sulfated derivatives thereof. Suitable anionic dispersants are especially lignosulfonates, polyacrylic acid salts and arylsulfonate-formaldehyde condensates.

Antifoams which may be present in the seed-dressing formulations usable in accordance with the invention are all foam-inhibiting substances customary for the formulation of agrochemically active compounds. Silicone antifoams and magnesium stearate can be used with preference.

Preservatives which may be present in the seed-dressing formulations usable in accordance with the invention are all substances usable for such purposes in agrochemical compositions. Examples include dichlorophene and benzyl alcohol hemiformal.

Secondary thickeners which may be present in the seed-dressing formulations usable in accordance with the invention are all substances usable for such purposes in agrochemical compositions. Preferred examples include cellulose derivatives, acrylic acid derivatives, xanthan, modified clays and finely divided silica.

Useful stickers which may be present in the seed-dressing formulations usable in accordance with the invention are all customary binders usable in seed-dressing products. Preferred examples include polyvinylpyrrolidone, polyvinyl acetate, polyvinyl alcohol and tylose.

The seed-dressing formulations usable in accordance with the invention can be used, either directly or after previously having been diluted with water, for the treatment of a wide range of different seed, including the seed of transgenic plants. In this case, additional synergistic effects may also occur in interaction with the substances formed by expression.

For the treatment of seed with the seed-dressing formulations usable in accordance with the invention or with the preparations prepared therefrom by addition of water, useful equipment is all mixing units usable customarily for seed dressing. Specifically, the seed dressing procedure is to place the seed into a mixer, to add the particular desired amount of seed-dressing formulations, either as such or after prior dilution with water, and to mix them until the formulation is distributed homogeneously on the seed. If appropriate, this is followed by a drying operation.

The active compounds according to the invention, given good plant compatibility, favorable homeotherm toxicity and good environmental compatibility, are suitable for protection of plants and plant organs, for increasing harvest yields, and for improving the quality of the harvested crop. They can preferably be used as crop protection agents. They are active against normally sensitive and resistant species and also against all or specific stages of development.

Plants which can be treated in accordance with the invention include the following main crop plants: corn, soya bean, cotton, *Brassica* oil seeds such as *Brassica napus* (e.g. Canola), *Brassica rapa, B. juncea* (e.g. (field) mustard) and *Brassica carinata,* rice, wheat, sugar beet, sugar cane, oats, rye, barley, millet and sorghum, triticale, flax, grapes and various fruit and vegetables from various botanic taxa, for example *Rosaceae* sp. (for example pome fruits such as apples and pears, but also stone fruits such as apricots, cherries, almonds and peaches, and berry fruits such as strawberries), *Ribesioidae* sp., *Juglandaceae* sp., *Betulaceae* sp., *Anacardiaceae* sp., *Fagaceae* sp *., Moraceae* sp., *Oleaceae* sp., *Actinidaceae* sp., *Lauraceae* sp., *Musaceae* sp. (for example banana trees and plantations), *Rubiaceae* sp. (for example coffee), *Theaceae* sp., *Sterculiceae* sp., *Rutaceae* sp. (for example lemons, oranges and grapefruit); *Solanaceae* sp. (for example tomatoes, potatoes, peppers, eggplants), *Liliaceae* sp., *Compositae* sp. (for example lettuce, artichokes and chicory-including root chicory, endive or common chicory), *Umbelliferae* sp. (for example carrots, parsley, celery and celeriac), Cucurbitaceae sp. (for example cucumbers-including gherkins, pumpkins, watermelons, calabashes and melons), *Alliaceae* sp. (for example leeks and onions), *Cruciferae* sp. (for example white cabbage, red cabbage, broccoli, cauliflower, Brussels sprouts, pak choi, kohlrabi, radishes, horseradish, cress and chinese cabbage), *Leguminosae* sp. (for example peanuts, peas, and beans-for example common beans and broad beans), *Chenopodiaceae* sp. (for example Swiss chard, fodder beet, spinach, beetroot), Malvaceae (for example okra), *Asparagaceae* (for example asparagus); useful plants and ornamental plants in the garden and woods; and in each case genetically modified types of these plants.

As mentioned above, it is possible to treat all plants and their parts in accordance with the invention. In a preferred embodiment, wild plant species and plant cultivars, or those obtained by conventional biological breeding techniques, such as crossing or protoplast fusion, and parts thereof, are treated. In a further preferred embodiment, transgenic plants and plant cultivars obtained by genetic engineering methods, if appropriate in combination with conventional methods

(genetically modified organisms), and parts thereof are treated. The term "parts" or "parts of plants" or "plant parts" has been explained above. Particular preference is given in accordance with the invention to treating plants of the respective commercially customary plant cultivars or those that are in use. Plant cultivars are understood to mean plants having new properties ("traits") which have been grown by conventional breeding, by mutagenesis or by recombinant DNA techniques. They may be cultivars, varieties, biotypes or genotypes.

The treatment method according to the invention can be used for the treatment of genetically modified organisms (GMOs), e.g. plants or seeds. Genetically modified plants (or transgenic plants) are plants in which a heterologous gene has been stably integrated into the genome. The term "heterologous gene" means essentially a gene which is provided or assembled outside a plant and which, upon introduction into the nuclear genome, the chloroplast genome or the mitochondrial genome, imparts to the transformed plant novel or improved agronomical or other traits because it expresses a protein or polypeptide of interest or another gene which is present in the plant, or other genes which are present in the plant are down-regulated or switched off (for example by means of antisense technology, co-suppression technologies or RNAi technologies [RNA interference]). A heterologous gene that is located in the genome is also called a transgene. A transgene that is defined by its specific presence in the plant genome is called a transformation or transgenic event.

Depending on the plant species or plant cultivars, their location and growth conditions (soils, climate, vegetation period, diet), the inventive treatment may also result in superadditive ("synergistic") effects. For example, the following effects which exceed the effects actually to be expected are possible: reduced application rates and/or widened spectrum of activity and/or increased efficacy of the active ingredients and compositions which can be used in accordance with the invention, better plant growth, increased tolerance to high or low temperatures, increased tolerance to drought or to water or soil salinity, increased flowering performance, easier harvesting, accelerated maturation, higher harvest yields, bigger fruits, greater plant height, greener leaf color, earlier flowering, higher quality and/or a higher nutritional value of the harvested products, higher sugar concentration within the fruits, better storage stability and/or processability of the harvested products.

Plants and plant cultivars which are preferably treated in accordance with the invention include all plants which have genetic material which imparts particularly advantageous, useful traits to these plants (whether obtained by breeding and/or biotechnological means).

Examples of nematode-resistant plants are described, for example, in the following U.S. patent application Ser. Nos. 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396 and 12/497,221.

Plants that may be treated according to the invention are hybrid plants that already express the characteristics of heterosis, or hybrid effect, which results in generally higher yield, vigor, better health and resistance towards biotic and abiotic stress factors. Such plants are typically produced by crossing an inbred male-sterile parent line (the female crossbreeding parent) with another inbred male-fertile parent line (the male crossbreeding parent). Hybrid seed is typically harvested from the male-sterile plants and sold to growers. Male-sterile plants can sometimes (e.g. in corn) be produced by detasseling (i.e. the mechanical removal of the male reproductive organs or male flowers) but, more typically, male sterility is the result of genetic determinants in the plant genome. In that case, and especially when seed is the desired product to be harvested from the hybrid plants, it is typically beneficial to ensure that male fertility in hybrid plants, which contain the genetic determinants responsible for male sterility, is fully restored. This can be accomplished by ensuring that the male crossbreeding parents have appropriate fertility restorer genes which are capable of restoring the male fertility in hybrid plants that contain the genetic determinants responsible for male sterility. Genetic determinants for male sterility may be located in the cytoplasm. Examples of cytoplasmic male sterility (CMS) were for instance described for *Brassica* species. However, genetic determinants for male sterility can also be located in the nuclear genome. Male-sterile plants can also be obtained by plant biotechnology methods such as genetic engineering. A particularly useful means of obtaining male-sterile plants is described in WO 89/10396 in which, for example, a ribonuclease such as a barnase is selectively expressed in the tapetum cells in the stamens. Fertility can then be restored by expression in the tapetum cells of a ribonuclease inhibitor such as barstar.

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Herbicide-tolerant plants are for example glyphosate-tolerant plants, i.e. plants made tolerant to the herbicide glyphosate or salts thereof. Plants can be made tolerant to glyphosate by various methods. Thus, for example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium *Salmonella typhimurium* (Comai et al., 1983, Science, 221, 370-371), the CP4 gene of the bacterium *Agrobacterium* sp. (Barry et al., 1992, Curr. Topics Plant Physiol. 7, 139-145), the genes encoding a *petunia* EPSPS (Shah et al., 1986,

Science 233, 478-481), a tomato EPSPS (Gasser et al., 1988, J. Biol. Chem. 263, 4280-4289) or an *Eleusine* EPSPS (WO 01/66704). It can also be a mutated EPSPS. Glyphosate-tolerant plants can also be obtained by expressing a gene that encodes a glyphosate oxidoreductase enzyme. Glyphosate-tolerant plants can also be obtained by expressing a gene that encodes a glyphosate acetyltransferase enzyme. Glyphosate-tolerant plants can also be obtained by selecting plants containing naturally-occurring mutations of the above mentioned genes. Plants which express EPSPS genes which impart glyphosate tolerance have been described. Plants which express other genes which impart glyphosate tolerance, for example decarboxylase genes, have been described.

Other herbicide-resistant plants are for example plants made tolerant to herbicides inhibiting the enzyme glutamine synthase, such as bialaphos, phosphinothricin or glufosinate. Such plants can be obtained by expressing an enzyme detoxifying the herbicide or a mutant glutamine synthase enzyme that is resistant to inhibition. One example of such an effective detoxifying enzyme is an enzyme encoding a phosphinothricin acetyltransferase (such as the bar or pat protein from *Streptomyces* species). Plants expressing an exogenous phosphinothricin acetyltransferase have been described.

Further herbicide-tolerant plants are also plants that have been made tolerant to the herbicides inhibiting the enzyme hydroxyphenylpyruvate dioxygenase (HPPD). Hydroxyphenylpyruvate dioxygenases are enzymes that catalyze the reaction in which para-hydroxyphenylpyruvate (HPP) is converted to homogentisate. Plants tolerant to HPPD inhibitors can be transformed with a gene encoding a naturally-occurring resistant HPPD enzyme, or a gene encoding a mutated or chimeric HPPD enzyme, as described in WO 96/38567, WO 99/24585, WO 99/24586, WO 2009/144079, WO 2002/046387 or U.S. Pat. No. 6,768,044. Tolerance to HPPD inhibitors can also be obtained by transforming plants with genes encoding certain enzymes enabling the formation of homogentisate despite inhibition of the native HPPD enzyme by the HPPD inhibitor. Such plants are described in WO 99/34008 and WO 02/36787. Tolerance of plants to HPPD inhibitors can also be improved by transforming plants with a gene encoding a prephenate dehydrogenase enzyme in addition to a gene encoding an HPPD-tolerant enzyme, as described in WO 2004/024928. In addition, plants can be made more tolerant to HPPD inhibitors by inserting into the genome thereof a gene which encodes an enzyme which metabolizes or degrades HPPD inhibitors, for example CYP450 enzymes (see WO 2007/103567 and WO 2008/150473).

Other herbicide-resistant plants are plants which have been rendered tolerant to acetolactate synthase (ALS) inhibitors. Known ALS inhibitors include, for example, sulfonylurea, imidazolinone, triazolopyrimidines, pyrimidinyloxy(thio)benzoates, and/or sulfonylaminocarbonyltriazolinone herbicides. It is known that different mutations in the ALS enzyme (also known as acetohydroxy acid synthase, AHAS) confer tolerance to different herbicides and groups of herbicides, as described, for example, in Tranel and Wright (Weed Science 2002, 50, 700-712). The production of sulfonylurea-tolerant plants and imidazolinone-tolerant plants has been described. Further sulfonylurea- and imidazolinone-tolerant plants have also been described.

Further plants tolerant to imidazolinones and/or sulfonylureas can be obtained by induced mutagenesis, by selection in cell cultures in the presence of the herbicide or by mutation breeding (cf., for example, for soya beans U.S. Pat. No. 5,084,082, for rice WO 97/41218, for sugar beet U.S. Pat. No. 5,773,702 and WO 99/057965, for lettuce U.S. Pat. No. 5,198,599 or for sunflower WO 01/065922).

Plants or plant cultivars (obtained by plant biotechnology methods such as genetic engineering) which may also be treated according to the invention are tolerant to abiotic stress factors. Such plants can be obtained by genetic transformation, or by selection of plants containing a mutation imparting such stress resistance. Particularly useful stress-tolerant plants include the following:

a. plants which contain a transgene capable of reducing the expression and/or the activity of the poly(ADP-ribose) polymerase (PARP) gene in the plant cells or plants;
b. plants which contain a stress tolerance-enhancing transgene capable of reducing the expression and/or the activity of the PARG-encoding genes of the plants or plant cells;
c. plants which contain a stress tolerance-enhancing transgene coding for a plant-functional enzyme of the nicotinamide adenine dinucleotide salvage biosynthesis pathway, including nicotinamidase, nicotinate phosphoribosyltransferase, nicotinic acid mononucleotide adenyltransferase, nicotinamide adenine dinucleotide synthetase or nicotinamide phosphoribosyltransferase.

Plants or plant cultivars (obtained by plant biotechnology methods such as genetic engineering) which may also be treated according to the invention show altered quantity, quality and/or storage stability of the harvested product and/or altered properties of specific components of the harvested product such as, for example:

1) Transgenic plants which synthesize a modified starch which, in its physicochemical characteristics, in particular the amylose content or the amylose/amylopectin ratio, the degree of branching, the average chain length, the side chain distribution, the viscosity behavior, the gelling strength, the starch granule size and/or the starch granule morphology, is changed in comparison with the synthesized starch in wild-type plant cells or plants, so that this modified starch is

better suited to specific applications.
2) Transgenic plants which synthesize non-starch carbohydrate polymers or which synthesize non-starch carbohydrate polymers with altered properties in comparison to wild-type plants without genetic modification. Examples are plants which produce polyfructose, especially of the inulin and levan type, plants which produce alpha-1,4-glucans, plants which produce alpha-1,6-branched alpha-1,4-glucans, and plants producing alternan.
3) Transgenic plants which produce hyaluronan.
4) Transgenic plants or hybrid plants such as onions with particular properties, such as "high soluble solids content", "low pungency" (LP) and/or "long storage" (LS).

Plants or plant cultivars (obtained by plant biotechnology methods such as genetic engineering) which may also be treated according to the invention are plants, such as cotton plants, with altered fiber characteristics. Such plants can be obtained by genetic transformation, or by selection of plants containing a mutation imparting such altered fiber characteristics and include:

a) plants, such as cotton plants, containing an altered form of cellulose synthase genes;
b) plants, such as cotton plants, which contain an altered form of rsw2 or rsw3 homologous nucleic acids, such as cotton plants with an increased expression of sucrose phosphate synthase;
c) plants, such as cotton plants, with increased expression of sucrose synthase;
d) plants, such as cotton plants, wherein the timing of the plasmodesmatal gating at the base of the fiber cell is altered, for example through downregulation of fiber-selective β-1,3-glucanase;
e) plants, such as cotton plants, which have fibers with altered reactivity, for example through expression of the N-acetylglucosaminetransferase gene, including nodC, and chitin synthase genes.

Plants or plant cultivars (obtained by plant biotechnology methods such as genetic engineering) which may also be treated according to the invention are plants, such as oilseed rape or related *Brassica* plants, with altered oil profile characteristics. Such plants can be obtained by genetic transformation, or by selection of plants containing a mutation imparting such altered oil characteristics and include:

a) plants, such as oilseed rape plants, which produce oil having a high oleic acid content;
b) plants, such as oilseed rape plants, which produce oil having a low linolenic acid content;
c) plants, such as oilseed rape plants, producing oil having a low level of saturated fatty acids.

Plants or plant cultivars (which can be obtained by plant biotechnology methods such as genetic engineering) which may also be treated according to the invention are plants such as potatoes which are virus-resistant, for example to the potato virus Y (SY230 and SY233 events from Tecnoplant, Argentina), or which are resistant to diseases such as potato late blight (e.g. RB gene), or which exhibit reduced cold-induced sweetness (which bear the genes Nt-Inh, II-INV) or which exhibit the dwarf phenotype (A-20 oxidase gene).

Plants or plant cultivars (obtained by plant biotechnology methods such as genetic engineering) which may also be treated according to the invention are plants, such as oilseed rape or related *Brassica* plants, with altered seed shattering characteristics. Such plants can be obtained by genetic transformation, or by selection of plants containing a mutation imparting such altered characteristics, and include plants such as oilseed rape with retarded or reduced seed shattering.

Particularly useful transgenic plants which can be treated according to the invention are plants with transformation events or combinations of transformation events which are the subject of granted or pending petitions for nonregulated status in the USA at the Animal and Plant Health Inspection Service (APHIS) of the United States Department of Agriculture (USDA). Information relating to this is available at any time from APHIS (4700 River Road Riverdale, Md. 20737, USA), for example via the website http://www.aphis.usda.gov/brs/not_reg.html. At the filing date of this application, the petitions with the following information were either granted or pending at the APHIS:

- Petition: Identification number of the petition. The technical description of the transformation event can be found in the specific petition document available from APHIS on the website via the petition number. These descriptions are hereby disclosed by reference.
- Extension of a petition: Reference to an earlier petition for which an extension of scope or term is being requested.
- Institution: Name of the person submitting the petition.
- Regulated article: The plant species in question.
- Transgenic phenotype: The trait imparted to the plant by the transformation event.
- Transformation event or line: The name of the event(s) (sometimes also referred to as line(s)) for which nonregulated status is being requested.

- APHIS documents: Various documents which have been published by APHIS with regard to the petition or can be obtained from APHIS on request.

Particularly useful transgenic plants which can be treated in accordance with the invention are plants which comprise one or more genes which code for one or more toxins, for example the transgenic plants which are sold under the following trade names: YIELD GARD® (for example corn, cotton, soya beans), KnockOut® (for example corn), BiteGard® (for example corn), BT-Xtra® (for example corn), StarLink® (for example corn), Bollgard® (cotton), Nucotn® (cotton), Nucotn 33B® (cotton), NatureGard® (for example corn), Protecta® and NewLeaf® (potato). Examples of herbicide-tolerant plants include corn varieties, cotton varieties and soya bean varieties which are available under the following trade names: Roundup Ready® (tolerance to glyphosates, for example corn, cotton, soya beans), Liberty Link® (tolerance to phosphinothricin, for example oilseed rape), IMI® (tolerance to imidazolinone) and SCS® (tolerance to sulfonylurea), for example corn. Herbicide-resistant plants (plants bred in a conventional manner for herbicide tolerance) which may be mentioned include the varieties sold under the name Clearfield® (for example corn).

Particularly useful transgenic plants which may be treated according to the invention are plants containing transformation events, or a combination of transformation events, and that are listed for example in the databases for various national or regional regulatory agencies (see for example http://gmoinfo.jrc.it/gmp_browse.aspx and http://cera-gmc.org/index.php?evidcode=&hstIDXCode=&gType=&AbbrCode=&atCode=&stCode=&coID Code=&action=gm_crop_database&mode=Submit).

When using the active compounds according to the invention in transgenic crops, in addition to the effects against harmful plants which can be observed in other crops, there are frequently effects which are specific for the application in the respective transgenic crop, for example a modified or specifically broadened spectrum of weeds which can be controlled, modified application rates which can be used for the application, preferably good combinability with the herbicides to which the transgenic crops are resistant, and an effect on the growth and the yield of the transgenic crop plants. The invention therefore also provides for the use of the compounds according to the invention as herbicides for controlling harmful plants in transgenic crop plants.

In addition, the substances according to the invention have outstanding growth-regulating properties in crop plants. They engage in the plant metabolism in a regulating manner and can this be employed for the targeted control of plant constituents and for facilitating harvesting, for example by provoking desiccation and stunted growth. Furthermore, they are also suitable for generally regulating and inhibiting undesirable vegetative growth, without destroying the plants in the process. Inhibition of vegetative growth plays an important role in many monocotyledon and dicotyledon crops because lodging can be reduced hereby, or prevented completely.

If the name of a compound in the present invention is in conflict with the structural formula, the structural formula shall prevail unless the structural formula is obviously wrong.

The compound of formula (I) provided by the present invention has exhibited better herbicidal activity and safety compared to those in the prior art.

## DETAILED DESCRIPTION

The following embodiments are used to illustrate the invention and should not be regarded as limiting the invention in any way. The protection scope claimed by the present invention is recited in the claims. In this field, any simple replacement or improvement made to the present invention by the person skilled in the art shall fall within the protection scope of the present invention.

Several methods for preparing compounds of the present invention are explained in detail in the following schemes and examples. The raw materials can be purchased from the market or prepared using methods known in the literature or as detailed in the explanation. The person skilled in the art should understand that the compounds of the present invention can also be synthesized using other synthesis routes. Although specific raw materials and conditions in the synthesis route have been explained below, they can be easily replaced with other similar raw materials and conditions, and various isomers of the compounds prepared by variants or variations of the preparation method of the present invention shall fall within the protection scope of the present invention. In addition, the preparation method described below can be further modified according to the disclosure of the present invention, using conventional chemical methods well-known to one skilled in the art, for example, protecting appropriate groups during the reaction process, and so on.

The method examples provided below are intended to promote further understanding of the preparation method of the present invention, and the specific substances, types, and conditions used are determined as further explanations of the present invention, not as limitations to the reasonable protection scope of the present invention. The raw materials and reagents used in the synthesis of the compounds described below can either be purchased from the market or easily prepared by one skilled in the art.

The analytical instruments described in the examples are as follows:

I. High Performance Liquid Chromatography (hereinafter referred to as HPLC): using 1260 Infinity II equipment, Agilent Technologies

Column: Agilent Eclipse Plus C18 3.5 μm, 4.6 * 100 mm
Mobile phase: A: water+0.1% phosphoric acid; B: Acetonitrile, temperature: 30 °C
Gradient: 10% B to 95% B within 15 min; 95% B for 3 min
Flow rate: 1 mL/min

II. Ultra High Performance Liquid Chromatography-Tandem Mass Spectrometer (hereinafter referred to as LC-MS): using ACQUITY H-Class UPLC-SQ Detector 2 equipment, Waters

Column: ACQUITY UPLC® BEH C18 1.7 μm. 2.1 * 50 mm Column
Mobile phase: A: water+0.2% formic acid; B: Acetonitrile, temperature: 30 °C
Gradient: 10% B to 95% B within 5 min; 95% B for 1 min
Flow rate: 0.5 mL/min
MS method: ESI positive and negative, mass range (m/z): 100 to 800

III. Gas Chromatography-Tandem Mass Spectrometer (hereinafter referred to as GC-MS): using 7890B GC System-5977A MSD equipment, Agilent Technologies

Column: HP-5MS UI, Agilent Technologies, 0.25 μm, 30 m * 0.250 mm
Sample injector temperature: 250 °C
Chromatographic column flow rate: helium 1 mL/min
Method: remain the temperature of 40 °C for 2 min, raise the temperature to 280 °C at a rate of 20 °C/min, remain the temperature of 280 °C for 5 min, a total time of 19 min
MSD transmission line temperature: 280 °C
EI ion source temperature: 230 °C, MS quadrupole temperature: 150 °C, scanning range: 30.00 to 400.00

IV. Automatic Polarimeter: using Rudolph Research Analytical, Autopol® II Automatic Polarimeter

Wavelength: 589 nm
Cell: 50.00 mm
Temperature: 20 °C
They are reported as specific rotations including the concentration "c" of the measured compound (in g / 100 mL) and the solvent used.

In addition, the chemical shift value of proton nuclear magnetic resonance spectrum (hereinafter referred to as $^1$H-NMR) recorded below is measured at 400MHz (Bruker, AVANCE III HD 400M) in deuterated chloroform ($CDCl_3$) solvent using $Me_4Si$ (tetramethylsilane) as the standard substance. In the case of determination in deuterated dimethyl sulfoxide solvent, it is shown as "(DMSO-d6)" in the data of chemical shift value. It should be noted that the symbols in the chemical shift values of $^1$H-NMR represent the following meanings.
s: singlet, d: doublet, dd: double doublet, dt: double triplet, td: triple doublet, ddd: double double doublet, t: triplet, q: quartet, sep: septet, m: multiplet, brs: broad singlet. In addition, in the presence of two or more stereoisomers, each chemical shift value is marked with "and" for signals that can be analyzed.
The examples of representative compounds are as follows, and the synthesis methods of other compounds are similar, which will not be explained in detail here.
The following examples provide a detailed illustration of the present invention.

**Example 1**

Preparation of intermediate **II-1**

Step 1: Preparation of intermediate **II-1.1**

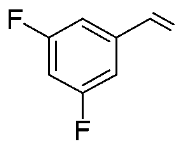
Z-2

At room temperature, hydroxylamine hydrochloride (58.7 g, 0.84 mol), ethanol (300 mL) and sodium acetate (69 g, 0.84 mol) were added to a 500 mL single-necked flask, and stirred well. 3,5-difluorobenzaldehyde (100 g, 0.7 mol) was added dropwise and the mixture was reacted for 3 h. After the reaction was completed, the solvent was removed under reduced pressure. Extraction was performed with 100 mL of dichloromethane twice. The organic phases were combined, washed once with saturated saline solution, dried with anhydrous sodium sulfate, and distilled under reduced pressure to remove solvent to give intermediate **II-1.1** (white solid, 108 g).

Step 2: Preparation of intermediate **II-1.2**

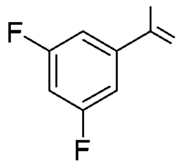
Z-2

At room temperature, the intermediate **II-1.1** (5 g, 31.8 mmol) and *N,N*-dimethylformamide (10 mL) were added to a 50 mL single-necked flask and stirred under an ice bath. *N*-chlorosuccinimide (5.15 g, 38.57 mmol) was added and the reaction was continued under the ice bath for 3 h. After the reaction was completed, the mixture was extracted with 30 mL of dichloromethane twice. The organic phases were combined, washed once with saturated saline solution, dried with anhydrous sodium sulfate, and distilled under reduced pressure to remove solvent to give intermediate **II-1.2** (white solid, 5 g).

Step 3: Preparation of intermediate **II-1.3**

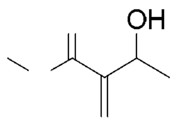

At room temperature, 40% acetaldehyde aqueous solution (110.125 g, 1 mol) and 1,4-dioxane (70 mL) were added to a 500 mL single-necked flask and stirred. Methyl acrylate (258 g, 3 mol) and triethylenediamine (112.17 g, 1 mol) were added and the mixture was reacted for 3 h. After the reaction was completed, the mixture was distilled under reduced pressure to remove solvent, and then extracted with 200 mL of dichloromethane twice. The organic phases were combined, washed once with saturated saline solution, dried with anhydrous sodium sulfate, and distilled under reduced pressure to remove solvent to give intermediate **II-1.3** (yellow liquid, 85 g).

Step 4: Preparation of intermediate II**-1.4**

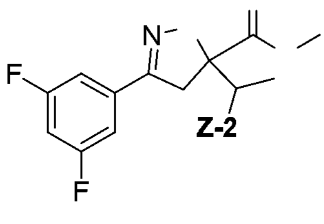
Z-2

At room temperature, the intermediate **II-1.2** (2 g, 10.44 mmol) and isopropanol (20 mL) were added to a 50 mL

single-necked flask, and stirred to dissolve. Intermediate **II-1.3** (1.36 g, 10.44 mmol) and sodium bicarbonate (4.4 g, 52.2 mmol) were added, and the mixture was heated to 50 °C and reacted for 2 h. After the reaction was completed, the reaction solution was filtered, and the filtrate was extracted with 30 mL of dichloromethane twice. The organic phases were combined, washed once with saturated saline solution, dried with anhydrous sodium sulfate, distilled under reduced pressure to remove solvent, and purified by column chromatography (eluent: petroleum ether: ethyl acetate=5:1) to give intermediate **II-1.4** (white solid, 2.6 g).

Step 5: Preparation of intermediate **II-1.5**

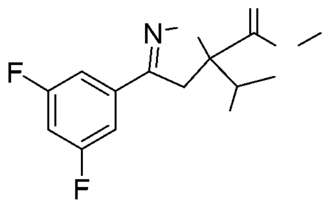

At room temperature, the intermediate **II-1.4** (2.6 g, 9.1 mmol) and dichloromethane (10 mL) were added to a 50 mL single-necked flask under an ice bath. Then, pyridine (1.46 mL, 18.2 mmol) was added, trifluoromethanesulfonic anhydride (2.30 mL, 13.67 mmol) was added dropwise, and the mixture was reacted at room temperature for 2 h. After the reaction was completed, the mixture was extracted with 30 mL of dichloromethane twice. The organic phases were combined, washed once with saturated saline solution, dried with anhydrous sodium sulfate, and distilled under reduced pressure to remove solvent to give crude intermediate **II-1.5** (yellow solid, 3.2 g). It was directly used for the next step without further treatment.

Step 6: Preparation of intermediate **II-1.6**

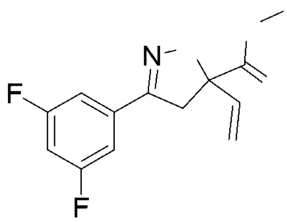

At room temperature, the intermediate **II-1.5** (3.2 g, 7.67 mmol) and N,N-dimethylacetamide (20 mL) were added to a 50 mL single-necked flask, and stirred to dissolve. 1,8-diazabicyclo[5.4.0]undecane-7-ene (1.375 mL, 9.204 mmol) was added dropwise and the mixture was stirred overnight at room temperature. After the reaction was completed, it was extracted with 30 mL of ethyl acetate twice. The organic phases were combined, washed once with saturated saline solution, dried with anhydrous sodium sulfate, distilled under reduced pressure to remove solvent, and purified by column chromatography (eluent: petroleum ether: ethyl acetate=5:1) to give intermediate II-**1.6** (white solid, 1.39 g). $^1$H-NMR (400 MHz, CDCl3) $\delta$ 7.18 (dd, *J*=8.0, 2.3 Hz, 2H), 6.87 (tt, *J*=8.7, 2.3 Hz, 1H), 6.13 (dd, *J*=17.2, 10.7 Hz, 1H), 5.55 (d, *J*=17.2 Hz, 1H), 5.38 (d, *J*=10.7 Hz, 1H), 3.93 (d, *J*=17.0 Hz, 1H), 3.83 (s, 3H), 3.34 (d, *J*=17.0 Hz, 1H).

Step 7: Preparation of intermediate **II-1**

Z-2

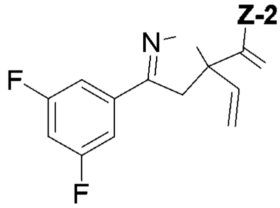

At room temperature, the intermediate **II-1.6** (6 g, 22.4 mmol) and tetrahydrofuran (30 mL) were added to a 100 mL single-necked flask, and stirred to dissolve. 20% sodium hydroxide aqueous solution (6.72 g, 33.6 mmol) was added and the mixture was refluxed and reacted for 5 h. After the reaction was completed, the reaction solution was cooled to room temperature, and the pH value was adjusted to 1-2 with dilute hydrochloric acid (10%). Solid was precipitated, filtered, and dried to give intermediate **II-1** (white solid, 5g). $^1$H-NMR (400 MHz, $CDCl_3$) $\delta$ 8.88 (s, 1H), 7.22-7.13 (m, 2H), 6.97-6.84 (m, 1H), 6.16 (dd, *J*=17.2, 10.7 Hz, 1H), 5.63 (d, *J*=17.2 Hz, 1H), 5.44 (d, *J*=10.7 Hz, 1H), 3.92 (d, *J*=17.1 Hz, 1H), 3.40 (d, *J*=17.1 Hz, 1H).

**Example 2**

Preparation of intermediate **II-19**

Step 1: Preparation of intermediate **II-19.1**

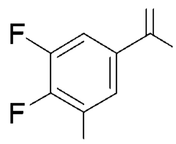

At room temperature, compound 3-chloro-4,5-difluorobenzoic acid (5 g, 25.97 mmol) and dichloromethane (50 mL) were added to a 25mL single-necked flask. Then, 2 drops of *N,N*-dimethylformamide (DMF) was added, followed by adding oxalyl chloride (3.3 mL, 38.95 mmol). Intense gas release was observed. The mixture was stirred at room temperature for 2 h, then distilled under reduced pressure to remove the solvent and excess oxalyl chloride. The obtained crude intermediate **II-19.1** was directly used for the next step without further purification.

Step 2: Preparation of Intermediate **II-19.2**

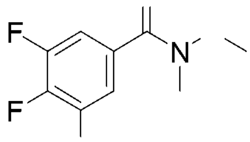

At room temperature, methoxymethylamine (2.38 g, 38.95 mmol), triethylamine (3.94 g, 38.95 mmol) and 10 mL of dichloromethane were added to a 25 mL single-necked flask, cooled to 0 °C, and stirred. All the crude intermediate **II-19.1** prepared in the previous step was dissolved in 20 mL of dichloromethane, and dropped into the reaction solution. Then, the mixture was heated to room temperature, and stirred for 2 h. After the reaction was completed, the mixture was added with water and stirred for 10 min, standing for layering. The organic phase was dried with anhydrous sodium sulfate and concentrated under reduced pressure. The residue was purified by reverse-phase column chromatography to give intermediate **II-19.2** (white solid, 5 g).

Step 3: Preparation of Intermediate **II-19.3**

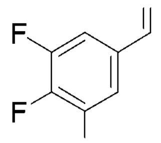

At room temperature, the intermediate **II-19.2** (5 g, 21.3 mmol) and tetrahydrofuran (20 mL) were added to a 100 mL reaction flask, and cooled to -20 °C. A toluene solution of diisobutyl aluminum hydride (1.5M) (21.3 mL, 31.95 mmol) was added dropwise, and the mixture was reacted for 2 h. After the reaction was completed, the mixture was added with a saturated aqueous solution of potassium sodium tartrate, extracted with ethyl acetate, and purified by column chromatography to give the intermediate **II-19.3** (yellow liquid, 1.26 g). $^1$H-NMR (400 MHz, DMSO) $\delta$ 9.94 (s, 1H), 8.06 (dt, *J*=6.2, 1.7 Hz, 1H), 8.03-7.95 (m, 1H).

Step 4: Preparation of Intermediate **II-19.4**

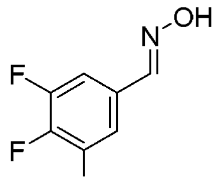

At room temperature, hydroxylamine hydrochloride (595 mg, 8.56 mmol), ethanol (10 mL) and sodium acetate (702 mg, 8.56 mmol) were added to a 50 mL single-necked flask, and stirred well.

The intermediate **II-19.3** (1.26 g, 7.14 mmol) was added dropwise, and the mixture was reacted for 3 h. After the reaction was completed, the solvent was removed under reduced pressure. The mixture was extracted with dichloromethane twice. The organic phases were combined, washed once with saturated saline solution, dried with anhydrous sodium sulfate, and distilled under reduced pressure to remove solvent to give intermediate **II-19.4** (white solid, 1.25 g).

Step 5: Preparation of Intermediate **II-19.5**

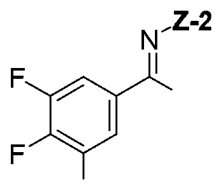

At room temperature, the intermediate **II-19.4** (1.25 g, 6.52 mmol) and *N,N*-dimethylformamide (20 mL) were added to a 50 mL single-necked flask and stirred under an ice bath. *N*-chlorosuccinimide (1.04 g, 7.83 mmol) was added and the reaction was continued under the ice bath for 3 h. After the reaction was completed, the reaction solution was extracted with 30 mL of dichloromethane twice. The organic phases were combined, washed once with saturated saline solution, dried with anhydrous sodium sulfate, and distilled under reduced pressure to remove solvent to give intermediate **II-19.5** (yellow oil, 1.5 g).

Step 6: Preparation of Intermediate **II-19.6**

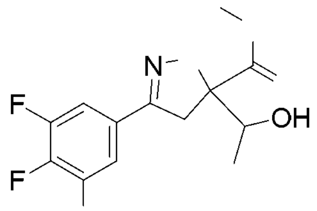

At room temperature, the intermediate **II-19.5** (700 mg, 3.1 mmol) and isopropanol (10 mL) were added to a 50 mL single-necked flask, and stirred to dissolve. Then, the compound 3-hydroxy-2-methylenebutanoate methyl ester (403 mg, 3.1 mmol) and sodium bicarbonate (1.3 g, 15.5 mmol) were added, and the mixture was heated to 50 °C and reacted for 2 h. After the reaction was completed, the reaction solution was filtered, and the filtrate was extracted with 30 mL of dichloromethane twice. The organic phases were combined, washed once with saturated saline solution, dried with anhydrous sodium sulfate, distilled under reduced pressure to remove solvent, and purified by column chromatography (eluent: petroleum ether: ethyl acetate=5:1) to give intermediate **II-19.6** (yellow solid, 880 mg).

Step 7: Preparation of Intermediate **II-19.7**

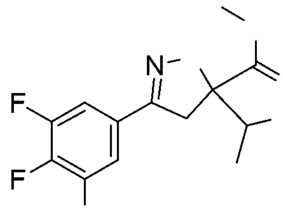

At room temperature, the intermediate **II-19.6** (880 mg, 2.75 mmol) and dichloromethane (20 mL) were added to a 50 mL single-necked flask under an ice bath. Then, pyridine (445 μL, 5.5 mmol) was added, trifluoromethanesulfonic anhydride (695 μL, 4.13 mmol) was added dropwise, and the mixture was reacted at room temperature for 2 h. After the reaction was completed, the reaction solution was extracted with 30 mL of dichloromethane twice. The organic phases were combined, washed once with saturated saline solution, dried with anhydrous sodium sulfate, and distilled under reduced pressure to remove solvent to give crude intermediate **II-19.7** (yellow oil, 1.27 g). It was directly used for the next step without further treatment.

Step 8: Preparation of Intermediate **II-19.8**

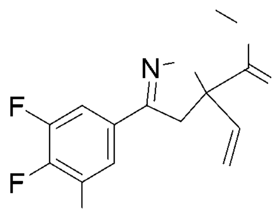

At room temperature, the intermediate **II-19.7** (1.20 g, 2.66 mmol) and N,N-dimethylformamide (15 mL) were added to a 50 mL single-necked flask, and stirred to dissolve. 1,8-diazabicyclo[5.4.0]undecane-7-ene was added dropwise (476 μL, 3.19 mmol) and the mixture was stirred overnight at room temperature. After the reaction was completed, it was extracted with 30 mL of ethyl acetate twice. The organic phases were combined, washed once with saturated saline solution, dried with anhydrous sodium sulfate, distilled under reduced pressure to remove solvent, and purified by column chromatography (eluent: petroleum ether: ethyl acetate=5:1) to give intermediate **II-19.8** (oily solid, 497 mg). $^1$H-NMR (400 MHz, DMSO) $\delta$ 7.89-7.68 (m, 2H), 6.15 (dd, *J*=17.3, 10.7 Hz, 1H), 5.41 (dd, *J*=29.0, 14.0 Hz, 2H), 3.96 (d, *J*=17.8 Hz, 1H), 3.75 (s, 3H), 3.69 (d, *J*=17.8 Hz, 1H).

Step 9: Preparation of Intermediate **II-19**

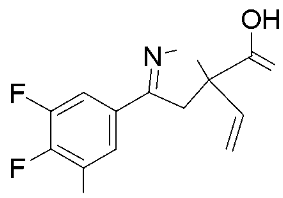

At room temperature, the intermediate **II-19.8** (497 mg, 1.65 mmol) and tetrahydrofuran (10 mL) were added to a 100 mL single-necked flask, and stirred to dissolve. 20% sodium hydroxide aqueous solution (330 mg, 1.65 mmol) was added, and the mixture was reacted at room temperature for 1 h. After the reaction was completed, the pH value of the reaction solution was adjusted to 1-2 with dilute hydrochloric acid (10%). Solid was precipitated, filtered, and dried to give intermediate **II-19** (white solid, 400 mg). $^1$H-NMR (400 MHz, DMSO) $\delta$ 13.57 (s, 1H), 7.93-7.67 (m, 2H), 6.15 (dd, *J*=17.3, 10.7 Hz, 1H), 5.44 (dd, *J*=17.3, 0.5 Hz, 1H), 5.34 (d, *J*=10.7 Hz, 1H), 3.90 (d, *J*=17.7 Hz, 1H), 3.62 (d, *J*=17.7 Hz, 1H).

**Example 3**

Preparation of intermediate **II-20**

Step 1: Preparation of intermediate **II-20.1**

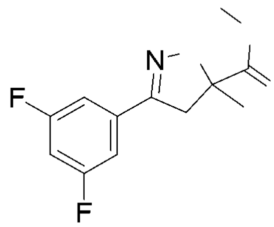

At room temperature, the intermediate **II-19.5** (3 g, 15.7 mmol) and isopropanol (20 mL) were added to a 50 mL reaction flask. Methyl methacrylate (1.84 mL, 17.2 mmol) and sodium bicarbonate (6.6 g, 78.5 mmol) were added at room temperature, and the mixture was heated to 50 °C and reacted for 3 h. After the reaction was completed, the mixture was purified by column chromatography to give intermediate **II-20.1** (white solid, 4 g). $^1$H-NMR (400 MHz, DMSO) $\delta$ 7.60-7.45 (m, 3H), 4.27 (d, *J*=15.6 Hz, 1H), 3.94 (d, *J*=15.6 Hz, 1H), 3.73 (s, 3H), 1.64 (s, 3H).

Step 2: Preparation of intermediate **II-20**

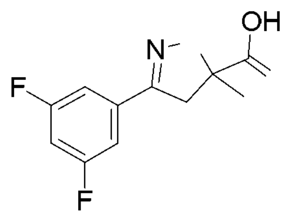

At room temperature, the intermediate **II-20.1** (4 g, 15.67 mmol) and tetrahydrofuran (20 mL) were added to a 100 mL single-necked flask, and stirred to dissolve. 20% sodium hydroxide aqueous solution (3.76 g, 18.8 mmol) was added, and the mixture was reacted at room temperature for 1 h. After the reaction was completed, the pH value of the reaction solution was adjusted to 1-2 with dilute hydrochloric acid (10%). Solid was precipitated, filtered, and dried to give intermediate **II-20** (white solid, 3.6 g). $^1$H-NMR (400 MHz, DMSO) $\delta$ 13.31 (s, 1H), 7.40 (d, *J*=7.3 Hz, 3H), 3.82 (d, *J*=17.6 Hz, 1H), 3.40 (d, *J*=17.5 Hz, 1H), 1.58 (s, 3H).

**Example 4**

Preparation of intermediate **II-28**

Step 1: Preparation of intermediate **II-28.1**

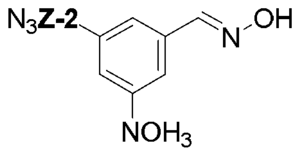

The intermediate **II-28.1** was prepared by reacting 3,5-dimethoxybenzaldehyde with hydroxylamine hydrochloride, which was similar to the preparation procedure of intermediate **II-1.1.**

Step 2: Preparation of intermediate **II-28.2**

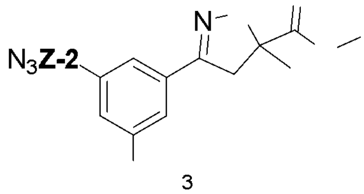

At room temperature, the intermediate **II-28.1** (1 g, 5.5 mmol) and dichloromethane (5 mL) were added to a 25 mL single-necked flask, followed by adding triethylamine (1.68 g, 13.2 mmol). The reaction mixture was stirred under ice bath condition for 10 min, followed by slowly adding sodium hypochlorite solution (10% available chlorine) (5.5 mL, 7.4 mmol). The mixture was stirred at 0 °C for 1 h, and then methyl methacrylate (553 g, 5.52 mmol) was added. The mixture was stirred at 0 °C for 1 h, and then distilled under reduced pressure to remove solvent. Water was added, and the mixture was extracted with ethyl acetate twice. The organic phases were combined, and distilled under reduced pressure to remove solvent to give intermediate **II-28.2** (white solid, 970 mg).

Step 3: Preparation of intermediate **II-28**

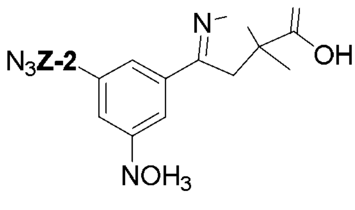

At room temperature, the intermediate **II-28.2** (970 mg, 3.47 mmol) and tetrahydrofuran (20 mL) were added to a 100 mL single-necked flask, and stirred to dissolve. 20% sodium hydroxide aqueous solution (800 mg, 4 mmol) was added, and the mixture was reacted at room temperature for 1 h. After the reaction was completed, the pH value of the reaction solution was adjusted to 1-2 with dilute hydrochloric acid (10%). Solid was precipitated, filtered, and dried to give intermediate **II-28** (white solid, 850 mg). $^1$H-NMR (400 MHz, DMSO) $\delta$ 13.11 (s, 1H), 6.80 (d, *J*=2.2 Hz, 2H), 6.59 (t, *J*=2.1 Hz, 1H), 3.77 (s, 6H), 3.38 (d, 2H), 1.55 (s, 3H).

**Example 5**

Preparation of intermediate **II-35**

Step 1: Preparation of intermediate **II-35.1**

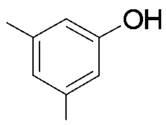

3

At 0 °C, 3-chloro-5-hydroxybenzonitrile (3 g, 19 mmol), difluorobromomethyltrimethylsilane (7.8 g, 38 mmol), and dichloromethane (30 mL) were added to a 250 mL single-necked flask, and stirred for 20 min. Then, 20% potassium hydroxide aqueous solution (27 mL, 114 mmol) was added dropwise and the mixture was reacted for 1.5 h. After the reaction was completed, the mixture was extracted with 30 mL of dichloromethane twice. The organic phases were combined, washed once with saturated saline solution, dried with anhydrous sodium sulfate, distilled under reduced pressure to remove solvent, and purified by column chromatography to give intermediate **II-35.1** (light yellow oil, 2.89 g).

Step 2: Preparation of intermediate **II-35.2**

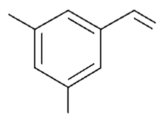

3

At room temperature, the intermediate **II-35.2** (2.89 g, 14.2 mmol), sodium hypophosphite (2.5 g, 28.4 mmol), pyridine (8 mL), acetic acid (2 mL), water (2 mL) and Raney Ni (water-moist, 0.2 g) were added to a 250 mL single-necked flask. The reaction mixture was refluxed under $N_2$ atmosphere for 10 h. After the reaction was completed, the mixture was cooled to room temperature, and diluted with 20 mL of ethyl acetate. The solid suspension in the mixture was removed by filtration, the solvent was removed under vacuum, and purification was performed through column chromatography (petroleum ether: ethyl acetate=3: 1) to give **II-35.2** (yellow oil, 2.4 g).

Step 3: Preparation of intermediate **II-35.3**

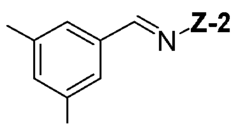

3

The intermediate **II-35.3** was prepared by reacting 3-chloro-5-(difluoromethoxy)benzaldehyde with hydroxylamine hydrochloride, which was similar to the preparation procedure of intermediate **II-1.1**.

Step 4: Preparation of intermediate **II-35.4**

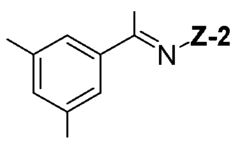

3

The intermediate **II-35.4** was prepared by reacting **II-35.3** with *N*-chlorosuccinimide, which was similar to the

preparation procedure of intermediate **II-1.2**.

Step 5: Preparation of intermediate **II-35.5**

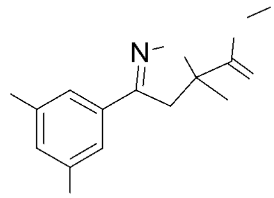

3

The intermediate **II-35.5** was prepared by reacting **II-35.4** with methyl methacrylate, which was similar to the preparation procedure of intermediate **II-20.1**.

Step 6: Preparation of intermediate **II-35**

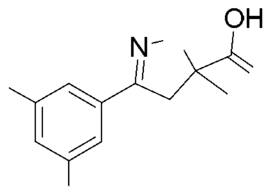

3

At room temperature, the intermediate **II-35.5** (1.52 g, 4.76 mmol), tetrahydrofuran (5 mL) and water (20 mL) were added to a 100 mL single-necked flask, and stirred well. The mixture was added with lithium hydroxide monohydrate (220 mg, 5.2 mmol) and reacted for 30 min. After the reaction was completed, 1M hydrochloric acid was added dropwise, and the pH was adjusted to 3-4. 20 mL of ethyl acetate was added for extraction. The organic phases were combined, washed once with saturated saline solution, dried with anhydrous sodium sulfate, and distilled under reduced pressure to remove solvent to give **II-35** (light yellow solid, 1.41 g). $^1$H-NMR (400 MHz, DMSO) $\delta$ 13.26 (s, 1H), 7.56-7.40 (m, 3H), 7.32 (ddt, *J*=9.5, 4.7, 2.8 Hz, 1H), 3.81 (d, *J*=17.4 Hz, 1H), 3.40 (d, *J*=17.4 Hz, 1H), 1.58 (s, 3H).

**Example 6**

Preparation of intermediate **II-39**

Step 1: Preparation of intermediate **II-39.1**

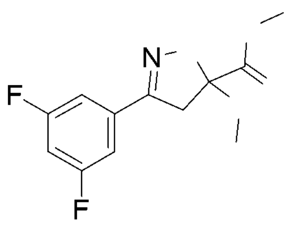

At room temperature, the intermediate **II-19.5** (2.44 g, 12.7 mmol) and isopropanol (10 mL) were added to a 50 mL reaction flask. Methyl methoxyacrylate (1.47 mg, 12.7 mmol) and sodium bicarbonate (5.3 g, 63.5 mmol) were added at room temperature, and the mixture was heated to 50 °C and reacted for 3 h. After the reaction was completed, the mixture was purified by column chromatography to give intermediate **II-39.1** (white solid, 3g). $^1$H-NMR (400 MHz, DMSO) $\delta$ 7.47-7.40 (m, 3H), 3.93 (d, *J*=18.8 Hz, 1H), 3.80 (s, 3H), 3.74 (d, *J*=18.8 Hz, 1H), 3.30 (s, 3H).

Step 2: Preparation of intermediate **II-39**

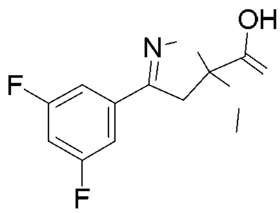

At room temperature, the intermediate **II-39.1** (3 g, 11.06 mmol) and tetrahydrofuran (10 mL) were added to a 50 mL single-necked flask, and stirred to dissolve. Lithium hydroxide monohydrate (464 mg, 11.06 mmol) was added, and the mixture was reacted at room temperature for 1 h. After the reaction was completed, the pH of the reaction solution was adjusted to 1-2 with dilute hydrochloric acid (10%). Solid was precipitated and filtered. The filter cake was dried to give intermediate **II-39** (white solid, 2 g). $^1$H-NMR (400 MHz, DMSO) $\delta$ 13.86 (s, 1H), 7.51-7.38 (m, 3H), 3.87 (d, *J*=18.7 Hz, 1H), 3.67 (d, *J*=18.7 Hz, 1H), 3.31 (s, 3H).

**Example 7**

Preparation of intermediate **II-69**

Step 1: Preparation of intermediate **II-69.1**

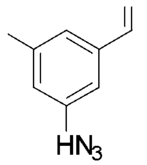

1-bromo-3-chloro-5-(trifluoromethyl)benzene (20 g, 77.09 mmol) and tetrahydrofuran (80 mL) were added to a 500 mL three-necked flask. Tetrahydrofuran solution of isopropyl magnesium chloride (2 mol/L) (115.6 mL, 231.27 mmol) was added under an ice bath, and the reaction solution was stirred at room temperature for 1 h and turned pink. N,N-dimethylformamide (16.9 g, 231.27 mmol) was added under an ice bath, and the mixture was stirred overnight at room temperature. After the reaction was completed, excess dilute hydrochloric acid (10%) was added dropwise. The mixture was filtered under reduced pressure to remove insoluble substances. The filtrate was distilled under reduced pressure to remove solvent, and extracted with 1000 mL of dichloromethane twice. The organic phases were combined, washed once with saturated saline solution, dried with anhydrous sodium sulfate, and distilled under reduced pressure to remove solvent to give intermediate **II-69.1** (yellow white solid, 12 g). $^1$H-NMR (400 MHz, $CDCl_3$) $\delta$ 9.94 (s, 1H), 7.98-7.91 (m, 2H), 7.78-7.73 (m, 1H).

Step 2: Preparation of intermediate **II-69.2**

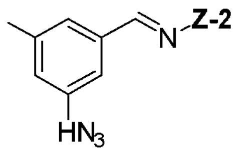

The intermediate **II-69.2** was prepared by reacting 3-chloro-5-(trifluoromethyl)benzaldehyde with hydroxylamine hydrochloride, which was similar to the preparation procedure of intermediate **II-1.1.** $^1$H-NMR (400 MHz, DMSO) $\delta$ 11.76 (s, 1H), 8.25 (s, 1H), 7.95-7.82 (m, 3H).

Step 3: Preparation of intermediate **II-69.3**

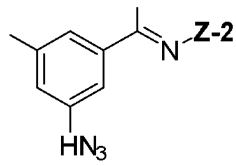

The intermediate **II-69.2** was reacted with *N*-chlorosuccinimide to give intermediate **II-69.3,** which was similar to the preparation procedure of intermediate **II-1.2.** $^1$H-NMR (400 MHz, DMSO) $\delta$ 12.94 (s, 1H), 8.07-7.92 (m, 3H).

Step 4: Preparation of intermediate **II-69**

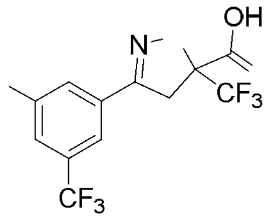

At room temperature, the intermediate **II-69.3** (5 g, 19 mmol) and isopropanol (20 mL) were added to a 50 mL single-necked flask, and stirred to dissolve. 2-(trifluoromethyl) acrylic acid (2.67 g, 19 mmol) and sodium bicarbonate (3.66 g, 97.5 mmol) were added, and the mixture was heated to 50 °C and reacted for 2 h. After the reaction was completed, the mixture was filtered to remove insoluble substances and extracted with 30 mL of dichloromethane twice. The organic phases were combined, washed once with saturated saline solution, dried with anhydrous sodium sulfate, distilled under reduced pressure to remove solvent, and purified by column chromatography to give intermediate **II-69** (white solid, 5.1 g). $^1$H-NMR (400 MHz, DMSO) $\delta$ 8.12 (s, 1H), 8.06 (s, 1H), 8.03 (s, 1H), 4.38-4.20 (m, 2H).

**Example 8**

Preparation of intermediate **II-77**

Step 1: Preparation of intermediate **II-77.1**

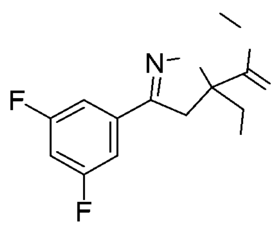

At room temperature, the intermediate **II-19.5** (1 g, 5.2 mmol) and isopropanol (20 mL) were added to a 50 mL reaction flask. Methyl 2-(chloromethyl)acrylate (0.7 g, 5.3 mmol) and sodium bicarbonate (2.2 g, 26.2 mmol) were added at room temperature, the mixture was heated to 50 °C, and reacted for 3 h. After the reaction was completed, the mixture was purified by column chromatography to give intermediate **II-77.1** (white solid, 1.4 g).

Step 2: Preparation of intermediate **II-77:**

Z-2

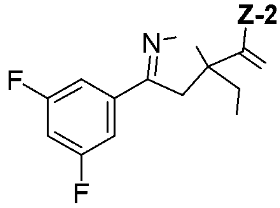

At room temperature, the intermediate **II-77.1** (1.4 g, 5.13 mmol) and tetrahydrofuran (20 mL) were added to a 100 mL single-necked flask, and stirred to dissolve. 20% aqueous solution of lithium hydroxide monohydrate (0.26 g, 6.15 mmol) was added, and the mixture was reacted at room temperature for 1 h. After the reaction was completed, the pH of the mixture was adjusted to 1-2 with dilute hydrochloric acid (10%). The organic phase was extracted with ethyl acetate, dried with anhydrous sodium sulfate, and distilled under reduced pressure to remove solvent to give intermediate **II-77** (white solid, 1.3 g). $^1$H-NMR (400 MHz, DMSO) $\delta$ 7.52-7.36 (m, 3H), 4.11-4.01 (m, 2H), 3.90 (d, *J*=18.0 Hz, 1H), 3.62 (d, *J*=18.0 Hz, 1H).

**Example 9**

Preparation of intermediate **II-110**

Step 1: Preparation of intermediate **II-110.1**

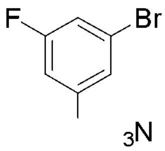

At room temperature, 3-bromo-5-fluorophenol (5 g, 26.18 mmol) was dissolved in isopropanol (15 mL) and 20% sodium hydroxide aqueous solution (7.8 g, 39.27 mmol) was added. After stirring for 30 min, monochlorodifluoromethane ($CHClF_2$) was introduced for gas displacement, and the reaction was allowed to react at 30 °C for 12 h. After the reaction was completed, the liquid was separated, the upper isopropanol phase was taken and distilled under reduced pressure to remove isopropanol. 20 mL of water was added, and the mixture was placed in the Dean Stark device and heated to 100 °C and refluxed. The lower liquid in the Dean Stark device was collected to give intermediate **II-110.1** (colorless oil, 4.1 g).

Step 2: Preparation of intermediate **II-110.2**

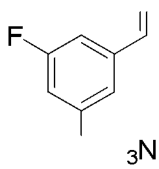

The intermediate **II-110.1** (1 g, 4.15 mmol) was dissolved in tetrahydrofuran (2 mL) under an ice bath. Tetrahydrofuran solution of isopropyl magnesium chloride (2M) (6.22 mL, 12.45 mmol) was added at 0 °C. After reacting at 0 °C for 1.5 h, N,N-dimethylformamide (0.9 g, 12.45 mmol) was added, and the mixture was reacted at room temperature for 1 h. After the reaction was completed, cold saturated ammonium chloride solution was added for quenching. The aqueous phase was extracted three times with ethyl acetate, and then purified by column chromatography to give intermediate **II-110.2** (yellow oil, 0.56 g).

Step 3: Preparation of intermediate **II-110.3**

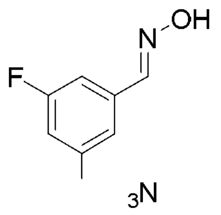

The intermediate **II-110.3** was prepared by reacting intermediate **II-110.2** with hydroxylamine hydrochloride, which was similar to the preparation procedure of intermediate **II-1.1.**

Step 4: Preparation of intermediate **II-110.4**

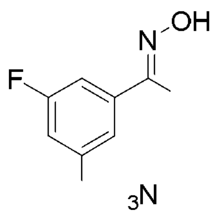

The intermediate **II-110.3** was reacted with N-chlorosuccinimide to prepare intermediate **II-110**.**4**, which was similar to the preparation procedure of intermediate **II-1.2.**

Step 5: Preparation of intermediate **II-110.5**

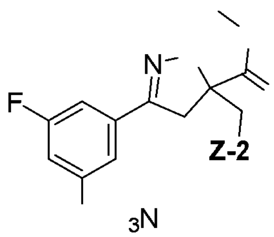

At room temperature, the intermediate **II-110.4** (6.0 g, 25.0 mmol) and isopropanol (20 mL) were added to a 50 mL reaction flask, methyl 2-(chloromethyl)acrylate (2.9 g, 25.0 mmol) and sodium bicarbonate (10.4 g, 124.1 mmol) were added, and the mixture was heated to 50 °C and reacted for 3 h. After the reaction was completed, the mixture was purified by column chromatography to give intermediate **II-110.5** (white solid, 1.4 g).

Step 6: Preparation of intermediate **II-110.6**

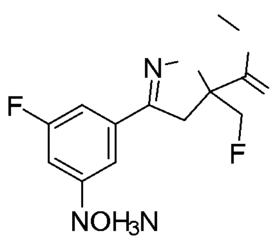

The intermediate **II-110.5** (785 mg, 2.3 mmol) was dissolved in dichloromethane (10 mL). Diethylaminosulfur trifluoride (DAST) (939 mg, 5.8 mmol) was added dropwise at 0 °C. Then, the mixture was placed in a microwave reactor and reacted at 60 °C for 10 h. After the reaction was completed, the reaction solution was added to saturated sodium

bicarbonate aqueous solution, extracted with dichloromethane twice, dried with anhydrous sodium sulfate, distilled under reduced pressure to remove solvent, and purified by column chromatography to give intermediate **II-110.6** (white solid, 700 mg).

Step 7: Preparation of intermediate **II-110**

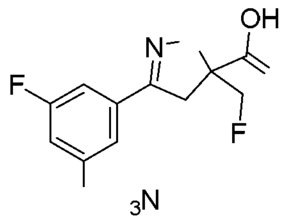

At room temperature, the intermediate **II-110.6** (700 mg, 2.06 mmol) and tetrahydrofuran (20 mL) were added to a 100 mL single-necked flask, and stirred to dissolve. 20% aqueous solution of lithium hydroxide monohydrate (95 mg, 2.27 mmol) was added, and the mixture was reacted at room temperature for 1 h. After the reaction was completed, the pH of the mixture was adjusted to 1-2 with dilute hydrochloric acid (10%), and the mixture was extracted with ethyl acetate, dried with anhydrous sodium sulfate and distilled under reduced pressure to remove solvent to give intermediate **II-110** (yellow solid, 650 mg). $^1$H-NMR (400 MHz, DMSO) $\delta$ 13.87 (s, 1H), 7.68-7.09 (m, 4H), 4.95-4.58 (m, 2H), 3.82 (dd, *J*=17.9, 1.9 Hz, 1H), 3.61 (dd, *J*=17.9, 1.9 Hz, 1H).

**Example 10**

Preparation of intermediate **II-115**

Step 1: Preparation of intermediate **II-115.1**

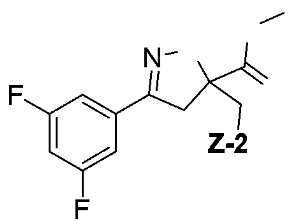

The intermediate **II-1.2** was reacted with methyl 2-(hydroxymethyl)acrylate to prepare intermediate **II-115.1,** which was similar to the preparation procedure of intermediate **II-110.5.**

Step 2: Preparation of intermediate **II-115.2**

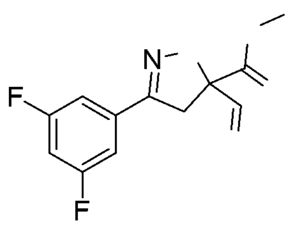

At room temperature, the intermediate **II-115.1** (1.5 g, 5.5 mmol), Dess-Martin periodinane (2.8 g, 6.6 mmol) and dichloromethane (15 mL) were added to a 100 mL single-necked flask, and the mixture was reacted for 30 min. After the reaction was completed, the reaction was quenched with 30 mL of saturated sodium bicarbonate aqueous solution, and

the solid suspension was removed by filtration. The filtrate was extracted with 30 mL of dichloromethane twice. The organic phases were combined, and purified by column chromatography to give intermediate **II-115.2** (white solid, 1.1 g).

Step 3: Preparation of intermediate **II-115.3**

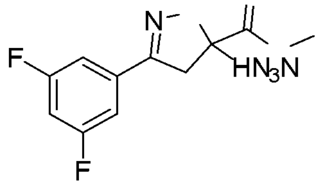

At room temperature, the intermediate **II-115.2** (1.1 g, 4.1 mmol), diethylaminosulfur trifluoride (DAST) (2 g, 12.3 mmol) and dichloromethane (15 mL) were added to a 100 mL single-necked flask, stirred well, and refluxed at 50 °C for 24 h. After the reaction was completed, the reaction was quenched with 30 mL of saturated sodium bicarbonate aqueous solution at room temperature, and the mixture was extracted with 30 mL of dichloromethane twice. The organic phases were combined, and purified by reverse-phase column chromatography to give intermediate **II-115.3** (yellow oil, 700 mg).

Step 4: Preparation of intermediate **II-115**

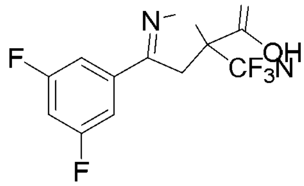

At room temperature, the intermediate **II-115.3** (700 mg, 2.4 mmol), tetrahydrofuran (5 mL) and water (20 mL) were added to a 100 mL single-necked flask, and stirred well. Lithium hydroxide monohydrate (111 mg, 2.6 mmol) was added and the mixture was reacted for 30 min. After the reaction was completed, 1M hydrochloric acid was added dropwise, and the pH was adjusted to 3-4. The mixture was extracted with 20 mL of ethyl acetate. The organic phases were combined, washed once with saturated saline solution, dried with anhydrous sodium sulfate, and distilled under reduced pressure to remove solvent to give intermediate **II-115** (yellow solid, 650 mg). $^1$H-NMR (400 MHz, DMSO) $\delta$ 14.43 (s, 1H), 7.46 (ddd, *J*=12.1, 6.7, 2.2 Hz, 3H), 6.54 (t, *J*=53.9 Hz, 1H), 4.01-3.81 (m, 2H).

## Example 11

Preparation of intermediate **II-136**

Step 1: Preparation of intermediate **II-136.1**

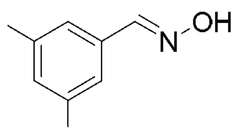

At room temperature, 3,5-dichlorobenzaldehyde (5 g, 28.6 mmol), hydroxylamine hydrochloride (3.17 g, 45.8 mmol), sodium acetate (4.69 g, 57.2 mmol) and ethanol (30 mL) were added to a 100 mL single-necked flask, stirred well, and reacted at room temperature for 1.5 h. After the reaction was completed, the solvent was removed under reduced pressure. The mixture was extracted with 30 mL of dichloromethane twice. The organic phases were combined, washed once with saturated saline solution, dried with anhydrous sodium sulfate, and distilled under reduced pressure to remove solvent to give intermediate **II-136.1** (white solid, 5.4 g).

Step 2: Preparation of intermediate **II-136.2**

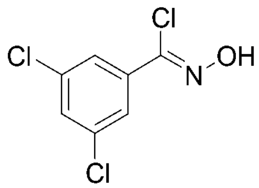

At room temperature, the intermediate **II-136.1** (5.4 g, 28.4 mmol) and *N,N*-dimethylformamide (10 mL) were added to a 50 mL single-necked flask and stirred under an ice bath. *N*-chlorosuccinimide (4.5 g, 34.1 mmol) was added and the reaction was continued under the ice bath for 3 h. After the reaction was completed, the mixture was extracted with 30 mL of dichloromethane twice. The organic phases were combined, washed once with saturated saline solution, dried with anhydrous sodium sulfate, and distilled under reduced pressure to remove solvent to give intermediate **II-136.2** (white solid, 6.1 g).

Step 3: Preparation of intermediate **II-136.3**

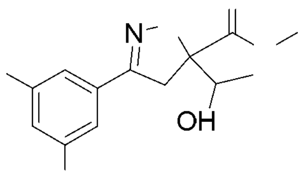

The intermediate **II-136.2** was reacted with intermediate **II-1.3** to prepare intermediate **II-136.3**, which was similar to the preparation procedure of intermediate **II-1.4.**

Step 4: Preparation of intermediate **II-136.4**

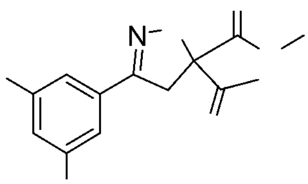

At room temperature, the intermediate **II-136.3** (1.5 g, 4.72 mmol) and dichloromethane (10 mL) were added to a 50 mL single-necked flask, and stirred well. The mixture was stirred for 20 min under a condition of -10 °C. Then, Dess-Martin periodinane (3 g, 7.08 mmol) was slowly added to the mixture. After addition, the mixture was transferred to a condition of room temperature and stirred for 2 h. After the reaction was completed, the reaction was quenched with 30 mL of saturated sodium bicarbonate aqueous solution, and the mixture was extracted with 30 mL of dichloromethane twice. The organic phases were combined, and purified by reverse-phase column chromatography to give intermediate **II-136.4** (white solid, 1.35 g).

Step 5: Preparation of intermediate **II-136.5**

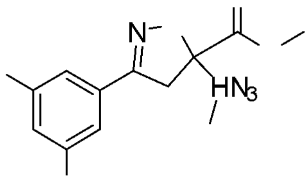

At room temperature, the intermediate **II-136.4** (1.0 g, 3.16 mmol) and dichloromethane (10 mL) were added to a 50 mL single-necked flask, and cooled to -10 °C. Diethylaminosulphur trifluoride (DAST) (1.27 g, 7.9 mmol) was slowly added dropwise, and the mixture was refluxed at 40 °C and reacted for 5 h after dropwise addition. After the reaction was completed, the reaction was quenched with 30 mL of saturated sodium bicarbonate aqueous solution, and the mixture was extracted with 30 mL of dichloromethane twice. The organic phases were combined, distilled under reduced pressure to remove solvent, and purified by column chromatography to give intermediate **II-136.5** (light yellow solid, 500 mg).

Step 6: Preparation of intermediate **II-136**

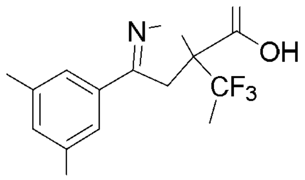

At room temperature, the intermediate (500 mg, 1.48 mmol), tetrahydrofuran (5 mL) and water (20 mL) were added to a 100 mL single-necked flask, and stirred well. Lithium hydroxide monohydrate (68 mg, 1.6 mmol) was added and the mixture was reacted for 30 min. After the reaction was completed, 1M hydrochloric acid was added dropwise, and the pH value was adjusted to 3-4. The mixture was extracted with 20 mL of ethyl acetate. The organic phases were combined, washed once with saturated saline solution, dried with anhydrous sodium sulfate, and distilled under reduced pressure to remove solvent to give **II-136** (white solid, 450 mg). $^1$H-NMR (400 MHz, DMSO) $\delta$ 7.77 (s, 3H), 3.98 (d, *J*=1.6 Hz, 2H), 1.94-1.77 (m, 3H).

**Example 12**

Preparation of intermediate **II-155**

Step 1: Preparation of intermediate **II-155.1**

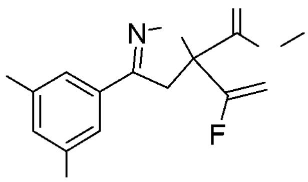

At room temperature, the intermediate **II-136.4** (1.0 g, 3.16 mmol) and dichloromethane (10 mL) were added to a 50 mL microwave reaction flask, cooled to -10 °C, and stirred. Diethylaminosulfur trifluoride (DSAT) (1.27 g, 7.9 mmol) was slowly added dropwise. After dropwise addition, the mixture was placed in a microwave reactor and reacted at 60 °C for 5 h. After the reaction was completed, the reaction was quenched with 30 mL of saturated sodium bicarbonate aqueous solution, and the mixture was extracted with 30 mL of dichloromethane twice. The organic phases were combined, distilled under reduced pressure to remove solvent, and purified by column chromatography to give the intermediate **II-136.5** (yellow oil, 540 mg).

Step 2: Preparation of intermediate **II-155**

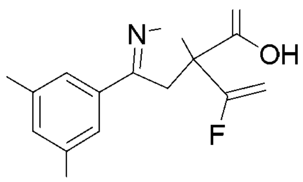

At room temperature, the intermediate **II-136.5** (540 mg, 1.7 mmol), tetrahydrofuran (5 mL) and water (20 mL) were added to a 100 mL single-necked flask, and stirred well. Lithium hydroxide monohydrate (78 mg, 1.9 mmol) was added and the mixture was reacted for 30 min. After the reaction was completed, hydrochloric acid (1M) was added dropwise, and the pH value was adjusted to 3-4. The mixture was extracted with 20 mL of ethyl acetate. The organic phases were combined, washed once with saturated saline solution, dried with anhydrous sodium sulfate, and distilled under reduced pressure to remove solvent to give intermediate **II-155** (white solid, 510 mg). $^1$H-NMR (400 MHz, CDCl3) $\delta$ 10.65 (s, 1H), 7.34 (dd, *J*=53.4, 2.0 Hz, 3H), 5.08-4.77 (m, 2H), 3.92 (d, *J*=17.4 Hz, 1H), 3.56 (d, *J*=17.4 Hz, 1H).

Referring to the above methods, other intermediates **II** can be obtained by using corresponding substituted benzaldehyde. The corresponding substituted benzaldehyde can be purchased in the market or easily prepared by ordinary those skilled in the art. Part of the prepared intermediates **II** and $^1$H-NMR thereof were shown in Table A.

Table A

| NO. | Structure | $^1$H NMR |
|---|---|---|
| **II-2** | 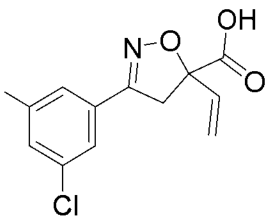 | $^1$H NMR (400 MHz, DMSO) $\delta$ 13.38 (s, 1H), 7.67 - 7.47 (m, 3H), 6.15 (dd, *J* = 17.2, 10.7 Hz, 1H), 5.39 (dd, *J* = 44.1, 14.0 Hz, 2H), 3.90 (d, *J* = 17.7 Hz, 1H), 3.63 (d, *J* = 17.7 Hz, 1H). |
| **II-3** | 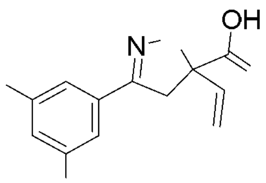 | $^1$H NMR (400 MHz, DMSO) $\delta$ 7.72 (d, *J* = 1.0 Hz, 3H), 6.16 (ddd, *J* = 17.3, 10.8, 1.0 Hz, 1H), 5.46 (dd, *J* = 17.4, 0.9 Hz, 1H), 5.37 - 5.21 (m, 1H), 4.11 - 3.77 (m, 1H), 3.65 (d, *J* = 18.0 Hz, 1H). |
| **II-4** | 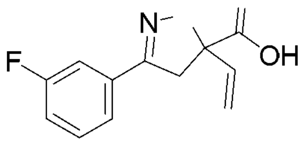 | $^1$H NMR (400 MHz, DMSO) $\delta$ 13.55 (s, 1H), 7.58 - 7.50 (m, 3H), 7.38 - 7.27 (m, 1H), 6.17 (dd, *J* = 17.2, 10.7 Hz, 1H), 5.46 (d, *J* = 17.2 Hz, 1H), 5.34 (d, *J*= 10.7 Hz, 1H), 3.90 (d, *J* = 17.6 Hz, 1H), 3.61 (d, *J* = 17.6 Hz, 1H). |
| **II-5** | 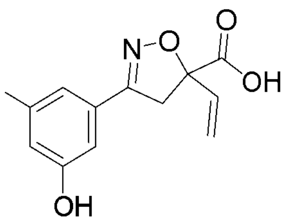 | $^1$H NMR (400 MHz, DMSO) $\delta$ 13.59 (s, 1H), 8.05 - 7.97 (m, 2H), 7.94 - 7.87 (m, 1H), 6.16 (dd, *J* = 17.2, 10.7 Hz, 1H), 5.44 (dd, *J* = 10.7, 0.9 Hz, 1H), 5.33 (dd, *J* = 10.7 Hz, 1H), 3.92 (d, *J*= 17.8 Hz, 1H), 3.65 (d, *J* = 17.8 Hz, 1H). |
| **II-6** | 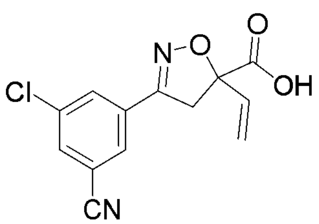 | $^1$H NMR (400 MHz, DMSO) $\delta$ 13.57 (s, 1H), 8.19 - 8.01 (m, 3H), 6.15 (dd, *J* = 17.3, 10.7 Hz, 1H), 5.45 (d, *J* = 17.3 Hz, 1H), 5.34 (d, *J*= 10.7 Hz, 1H), 3.94 (d, *J* = 17.8 Hz, 1H), 3.67 (d, *J*= 17.8 Hz, 1H). |

(continued)

| NO. | Structure | $^1$H NMR |
|---|---|---|
| **II-7** | 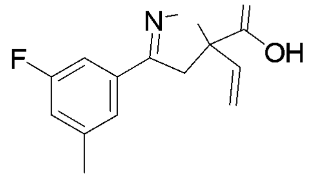 | $^1$H NMR (400 MHz, $CDCl_3$) $\delta$ 7.22 (s, 1H), 7.18 (d, *J* = 9.1 Hz, 1H), 6.97 (d, *J* = 9.2 Hz, 1H), 6.16 (dd, *J* = 17.2, 10.7 Hz, 1H), 5.63 (d, *J*= 17.2 Hz, 1H), 5.43 (d, *J* = 10.7 Hz, 1H), 3.94 (d, *J* = 17.1 Hz, 1H), 3.42 (d, *J* = 17.1 Hz, 1H), 2.38 (s, 3H). |
| **II-8** | 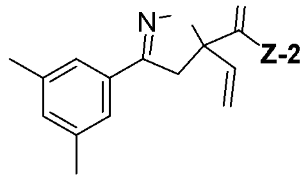 | $^1$H NMR (400 MHz, $CDCl_3$) $\delta$ 7.26 (s, 2H), 7.08 (s, 1H), 6.16 (dd, *J* = 17.2, 10.7 Hz, 1H), 5.62 (d, *J* = 17.2 Hz, 1H), 5.41 (d, *J* = 10.7 Hz, 1H), 3.96 (d, *J* = 17.1 Hz, 1H), 3.44 (d, *J* = 17.1 Hz, 1H), 2.33 (s, 6H). |
| **II-9** | 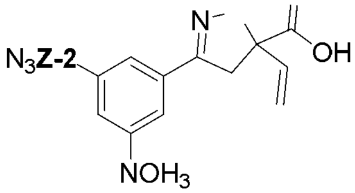 | $^1$H NMR (400 MHz, DMSO) $\delta$ 13.45 (s, 1H), 6.82 (d, J = 2.0 Hz, 2H), 6.60 (s, 1H), 6.15 (dd, J = 17.3, 10.7 Hz, 1H), 5.38 (dd, J = 48.3, 14.0 Hz, 2H), 3.85 (d, J = 17.5 Hz, 1H), 3.78 (s, 6H), 3.58 (d, J = 17.6 Hz, 1H). |
| **II-10** | 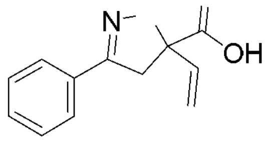 | $^1$H NMR (400 MHz, DMSO) $\delta$ 13.42 (s, 1H), 7.91 - 7.57 (m, 2H), 7.47 (dd, J = 5.1, 1.4 Hz, 3H), 6.17 (dd, J = 17.3, 10.7 Hz, 1H), 5.54 - 5.24 (m, 2H), 3.93 - 3.50 (m, 2H). |
| **II-11** | 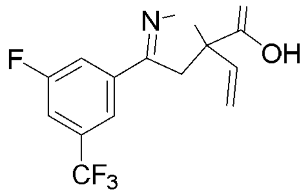 | $^1$H NMR (400 MHz, DMSO) $\delta$ 13.55 (s, 1H), 7.85 (dd, *J* = 11.4, 7.0 Hz, 3H), 6.17 (dd, *J* = 17.3, 10.7 Hz, 1H), 5.46 (d, *J* = 17.0 Hz, 1H), 5.35 (d, *J* = 10.7 Hz, 1H), 3.97 (d, *J* = 17.7 Hz, 1H), 3.70 (d, *J* = 17.7 Hz, 1H). |
| **II-12** | 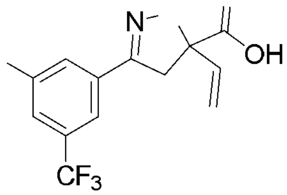 | $^1$H NMR (400 MHz, DMSO) $\delta$ 13.06 (s, 1H), 8.05 (t, J = 1.7 Hz, 1H), 8.02 - 7.95 (m, 2H), 6.17 (dd, J = 17.3, 10.7 Hz, 1H), 5.47 (dd, J = 17.2, 0.9 Hz, 1H), 5.35 (dd, J = 10.7, 0.8 Hz, 1H), 3.98 (d, J = 17.7 Hz, 1H), 3.73 (d, J = 17.9 Hz, 1H). |
| **II-13** | 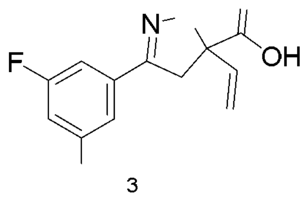 | $^1$H NMR (400 MHz, DMSO) $\delta$ 7.65 - 7.50 (m, 3H), 6.22 - 6.08 (m, 1H), 5.40 (dd, J = 17.3, 1.0 Hz, 1H), 5.27 (dd, J = 10.7, 0.9 Hz, 1H), 3.88 (d, J = 17.6 Hz, 1H), 3.58 (d, J = 17.7 Hz, 1H). |
| **II-14** | 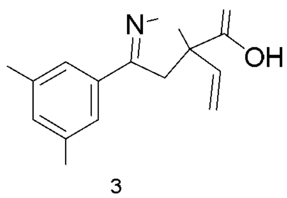 | $^1$H NMR (400 MHz, DMSO) $\delta$ 13.58 (s, 1H), 7.84 - 7.77 (m, 1H), 7.71 (s, 1H), 7.66 (s, 1H), 6.16 (dd, *J* = 17.3, 10.7 Hz, 1H), 5.45 (d, *J* = 17.3 Hz, 1H), 5.34 (d, *J* = 10.7 Hz, 1H), 3.93 (d, *J* = 17.7 Hz, 1H), 3.67 (d, *J* = 17.7 Hz, 1H). |

(continued)

| NO. | Structure | $^1$H NMR |
|---|---|---|
| **II-15** | 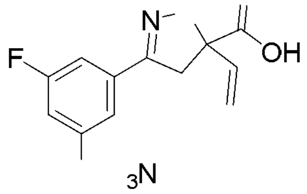 | $^1$H NMR (400 MHz, DMSO) $\delta$ 13.62 (s, 1H), 7.61 - 7.18 (m, 4H), 6.22 - 6.07 (m, 1H), 5.44 (dd, $J$ = 17.3, 0.9 Hz, 1H), 5.33 (dd, $J$ = 10.7, 0.8 Hz, 1H), 3.90 (d, $J$ = 17.7 Hz, 1H), 3.62 (d, $J$= 17.7 Hz, 1H). |
| **II-16** | 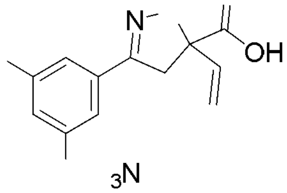 | |
| **II-17** | 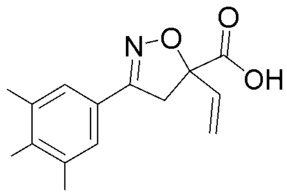 | $^1$H NMR (400 MHz, DMSO) $\delta$ 13.54 (s, 1H), 7.67 (dd, $J$ = 8.5, 6.9 Hz, 2H), 6.15 (dd, $J$ = 17.3, 10.7 Hz, 1H), 5.44 (d, $J$ = 17.3 Hz, 1H), 5.34 (d, $J$ = 10.7 Hz, 1H), 3.89 (d, $J$ = 17.7 Hz, 1H), 3.60 (d, $J$ = 17.7 Hz, 1H). |
| **II-18** | 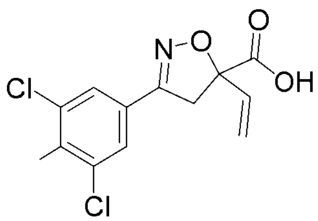 | $^1$H NMR (400 MHz, DMSO) $\delta$ 13.53 (s, 1H), 7.90 (d, $J$ = 6.4 Hz, 2H), 6.15 (dd, $J$ = 17.2, 10.7 Hz, 1H), 5.44 (d, $J$ = 17.3 Hz, 1H), 5.34 (d, $J$ = 10.7 Hz, 1H), 3.91 (d, $J$ = 17.7 Hz, 1H), 3.64 (d, $J$ = 17.7 Hz, 1H). |
| **II-21** | 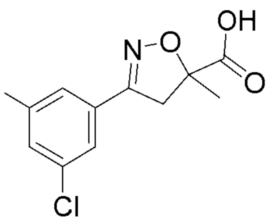 | $^1$H NMR (400 MHz, DMSO) $\delta$ 13.34 (s, 1H), 7.62 - 7.55 (m, 2H), 7.55 - 7.48 (m, 1H), 3.82 (d, $J$ = 17.6 Hz, 1H), 3.41 (d, $J$ = 17.6 Hz, 1H), 1.57 (s, 3H). |
| **II-22** | 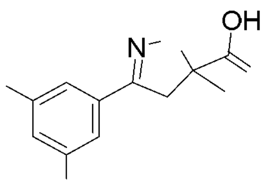 | $^1$H NMR (400 MHz, DMSO) $\delta$ 13.29 (s, 1H), 7.71 (dd, $J$ = 14.7, 2.0 Hz, 3H), 3.82 (d, $J$ = 17.6 Hz, 1H), 3.41 (d, $J$ = 17.6 Hz, 1H), 1.56 (s, 3H). |
| **II-23** | 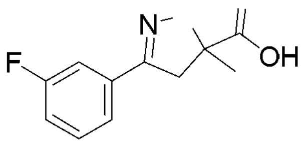 | $^1$H NMR (400 MHz, DMSO) $\delta$ 13.26 (s, 1H), 7.56 - 7.40 (m, 3H), 7.32 (ddt, $J$ = 9.5, 4.7, 2.8 Hz, 1H), 3.81 (d, $J$ = 17.4 Hz, 1H), 3.40 (d, $J$ = 17.4 Hz, 1H), 1.58 (s, 3H). |
| **II-24** | 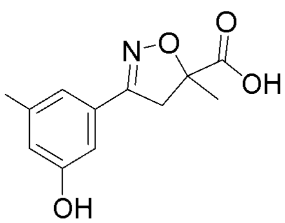 | $^1$H NMR (400 MHz, DMSO) $\delta$ 13.37 (s, 1H), 8.06 - 7.94 (m, 2H), 7.94 - 7.80 (m, 1H), 3.86 (d, $J$ = 17.7 Hz, 1H), 3.43 (d, $J$ = 17.6 Hz, 1H), 1.58 (s, 3H). |

(continued)

| NO. | Structure | $^1$H NMR |
|---|---|---|
| **II-25** | 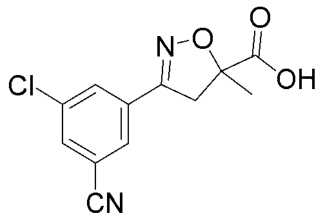 | $^1$H NMR (400 MHz, DMSO) $\delta$ 13.33 (s, 1H), 8.16 (s, 1H), 8.10 (d, *J* = 3.9 Hz, 1H), 8.05 (d, *J* = 1.5 Hz, 1H), 3.86 (d, *J* = 17.6 Hz, 1H), 3.43 (d, *J* = 17.6 Hz, 1H), 1.57 (s, 3H). |
| **II-26** | 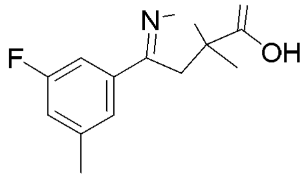 | $^1$H NMR (400 MHz, $CDCl_3$) $\delta$ 7.17 (s, 1H), 7.12 (d, *J* = 9.0 Hz, 1H), 6.92 (d, *J* = 9.2 Hz, 1H), 3.81 (d, *J* = 17.2 Hz, 2H), 3.19 (d, *J* = 17.1 Hz, 1H), 2.34 (s, 3H), 1.71 (s, 3H). |
| **II-27** | 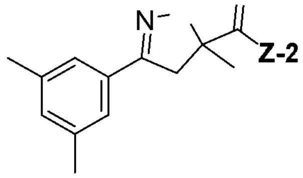 | $^1$H NMR (400 MHz, $CDCl_3$) $\delta$ 7.25 (s, 2H), 7.07 (s, 1H), 3.86 (d, *J* = 17.1 Hz, 1H), 3.28 (d, *J* = 17.1 Hz, 1H), 2.33 (s, 6H), 1.75 (s, 3H). |
| **II-29** | 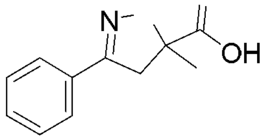 | $^1$H NMR (400 MHz, DMSO) $\delta$ 13.23 (s, 1H), 7.68 (s, 2H), 7.47 (s, 3H), 3.80 (d, *J* = 17.3 Hz, 1H), 3.39 (d, *J* = 17.3 Hz, 1H), 1.58 (s, 3H). |
| **II-30** | 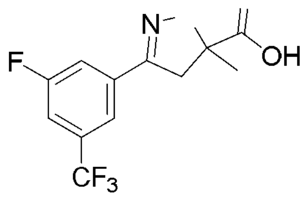 | 1H NMR (400 MHz, DMSO) $\delta$ 13.01 (s, 1H), 7.92 - 7.71 (m, 3H), 3.90 (d, J = 17.6 Hz, 1H), 3.48 (d, J = 17.6 Hz, 1H), 1.60 (s, 3H). |
| **II-31** | 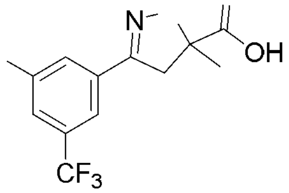 | $^1$H NMR (400 MHz, DMSO) $\delta$ 13.26 (s, 1H), 7.56 - 7.40 (m, 3H), 7.32 (ddt, *J* = 9.5, 4.7, 2.8 Hz, 1H), 3.81 (d, *J* = 17.4 Hz, 1H), 3.40 (d, *J* = 17.4 Hz, 1H), 1.58 (s, 3H). |
| **II-32** | 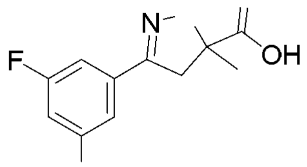 3 | $^1$H NMR (400 MHz, DMSO) $\delta$ 13.47 (s, 1H), 7.64 - 7.46 (m, 3H), 3.83 (d, *J* = 17.5 Hz, 1H), 3.39 (d, *J* = 17.6 Hz, 1H), 1.56 (s, 3H). |
| **II-33** | 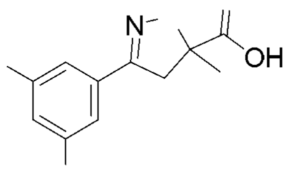 3 | $^1$H NMR (400 MHz, DMSO) $\delta$ 13.32 (s, 1H), 7.77 (t, *J* = 1.4 Hz, 1H), 7.70 (s, 1H), 7.64 (s, 1H), 3.85 (d, *J* = 17.6 Hz, 1H), 3.44 (d, J = 17.6 Hz, 1H), 1.57 (s, 3H). |

(continued)

| NO. | Structure | $^1$H NMR |
|---|---|---|
| **II-34** | 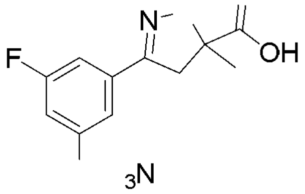 | $^1$H NMR (400 MHz, DMSO) $\delta$ 13.37 (s, 1H), 7.61 - 7.19 (m, 4H), 3.82 (d, $J$ = 17.6 Hz, 1H), 3.40 (d, $J$ = 17.5 Hz, 1H), 1.57 (s, 3H). |
| **II-36** | 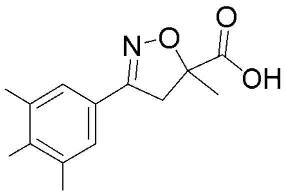 | $^1$H NMR (400 MHz, DMSO) $\delta$ 13.16 (s, 1H), 7.89 - 7.46 (m, 2H), 3.80 (d, $J$ = 17.5 Hz, 1H), 3.36 (d, $J$ = 17.6 Hz, 1H), 1.57 (s, 3H). |
| **II-37** | 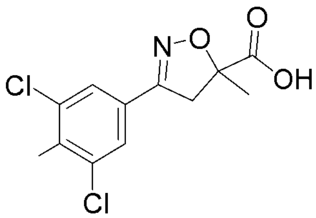 | $^1$H NMR (400 MHz, DMSO) $\delta$ 13.27 (s, 1H), 7.87 (d, $J$ = 6.4 Hz, 2H), 3.83 (d, $J$ = 17.5 Hz, 1H), 3.41 (d, $J$ = 17.6 Hz, 1H), 1.57 (s, 3H). |
| **II-38** | 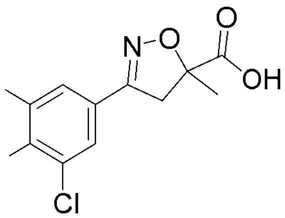 | $^1$H NMR (400 MHz, DMSO) $\delta$ 13.27 (s, 1H), 7.83 - 7.68 (m, 2H), 3.82 (d, $J$ = 17.6 Hz, 1H), 3.39 (d, $J$ = 17.6 Hz, 1H), 1.56 (s, 3H). |
| **II-40** | 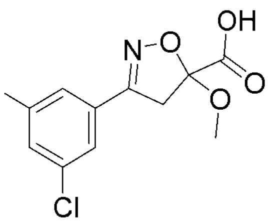 | |
| **II-41** | 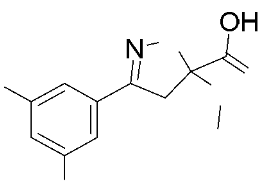 | |
| **II-42** | 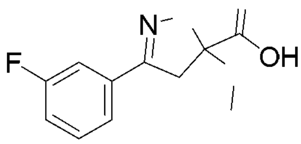 | |

(continued)

| NO. | Structure | 1H NMR |
|---|---|---|
| II-43 | 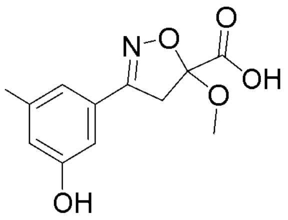 | |
| II-44 | 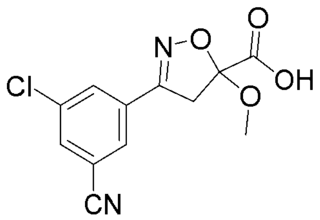 | |
| II-45 | 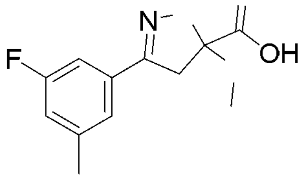 | |
| II-46 | 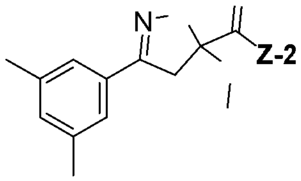 | |
| II-47 | 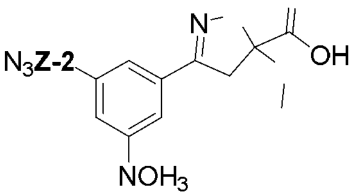 | |
| II-48 | 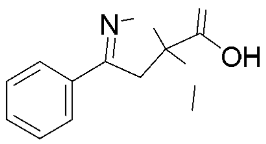 | |
| II-49 | 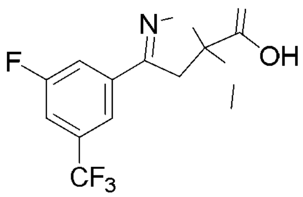 | |
| II-50 | 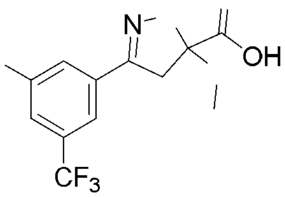 | |

(continued)

| NO. | Structure | 1H NMR |
|---|---|---|
| **II-51** | 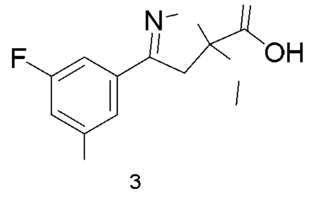 | |
| **II-52** | 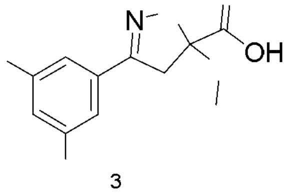 | |
| **II-53** | 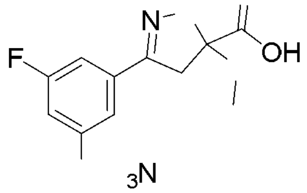 | |
| **II-54** | 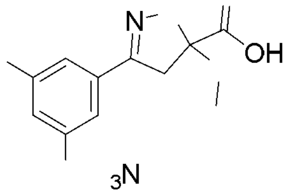 | |
| **II-55** | 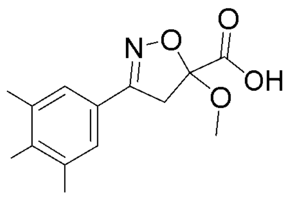 | |
| **II-56** | 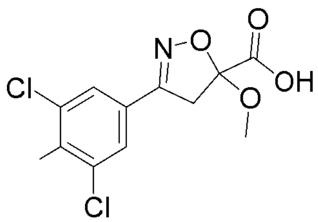 | |
| **II-57** | 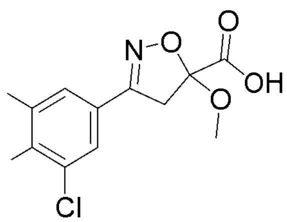 | |

(continued)

| NO. | Structure | $^1$H NMR |
|---|---|---|
| **II-58** | 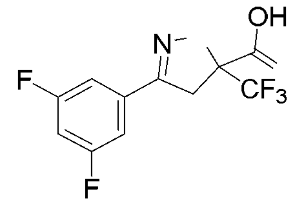 | $^1$H NMR (400 MHz, DMSO) $\delta$ 13.79 (s, 1H), 7.69 - 7.35 (m, 3H), 4.18 (s, 2H). |
| **II-59** | 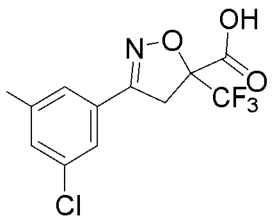 | $^1$H NMR (400 MHz, DMSO) $\delta$ 7.75 - 7.51 (m, 3H), 4.20 (d, *J* = 1.7 Hz, 2H). |
| **II-60** | 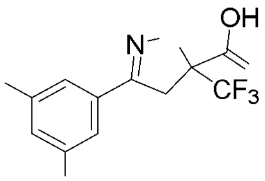 | $^1$H NMR (400 MHz, DMSO) $\delta$ 7.80 (q, *J* = 1.4 Hz, 3H), 4.21 (d, *J* = *3.3* Hz, 2H). |
| **II-61** | 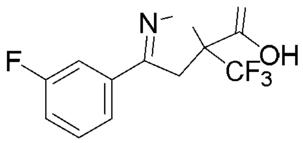 | $^1$H NMR (400 MHz, DMSO) $\delta$ 14.06 (s, 1H), 7.67 - 7.50 (m, 3H), 7.39 (td, *J* = 8.5, 2.7 Hz, 1H), 4.18 (d, *J* = *3.2* Hz, 2H). |
| **II-62** | 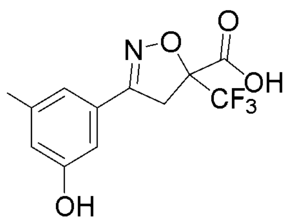 | $^1$H NMR (400 MHz, DMSO) $\delta$ 8.03 (s, 2H), 8.03 - 7.93 (m, 1H), 4.23 (s, 2H). |
| **II-63** | 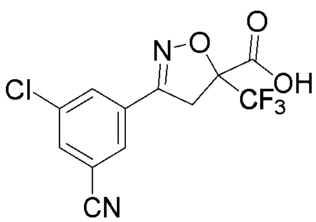 | $^1$H NMR (400 MHz, DMSO) $\delta$ 13.65 (s, 1H), 8.28 - 8.09 (m, 3H), 4.23 (d, *J* = 2.5 Hz, 2H). |
| **II-64** | 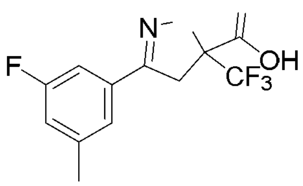 | $^1$H NMR (400 MHz, DMSO) $\delta$ 7.45 (s, 1H), 7.37 (d, *J* = 9.5 Hz, 1H), 7.23 (d, *J* = 9.6 Hz, 1H), 4.14 (s, 2H), 2.37 (s, 3H). |
| **II-65** | 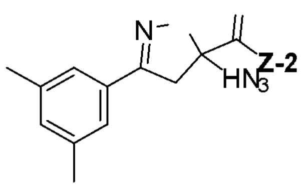 | $^1$H NMR (400 MHz, DMSO) $\delta$ 7.37 (s, 2H), 7.16 (s, 1H), 4.10 (d, *J* = *7.7* Hz, 2H), 2.31 (s, 6H). |

(continued)

| NO. | Structure | $^1$H NMR |
|---|---|---|
| **II-66** | 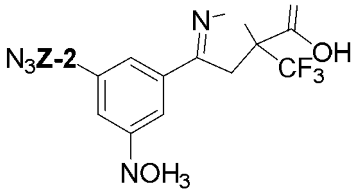 | $^1$H NMR (400 MHz, DMSO) $\delta$ 6.88 (d, J = 2.2 Hz, 2H), 6.66 (dd, J = 10.4, 8.3 Hz, 1H), 4.13 (s, 2H), 3.78 (s, 6H). |
| **II-67** | 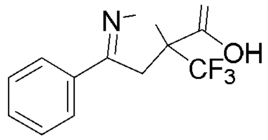 | $^1$H NMR (400 MHz, DMSO) $\delta$ 13.72 (s, 1H), 7.75 (d, *J* = 7.5 Hz, 2H), 7.50 (dt, *J* = 14.2, 7.0 Hz, 3H), 4.22 - 4.08 (m, 2H). |
| **II-68** | 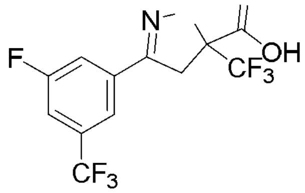 | $^1$H NMR (400 MHz, DMSO) $\delta$ 7.99 - 7.82 (m, 3H), 4.37 - 4.23 (m, 2H). |
| **II-70** | 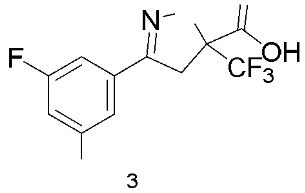 | $^1$H NMR (400 MHz, DMSO) $\delta$ 14.83 (s, 1H), 7.78 - 7.55 (m, 3H), 4.21 (d, *J* = 1.9 Hz, 2H). |
| **II-71** | 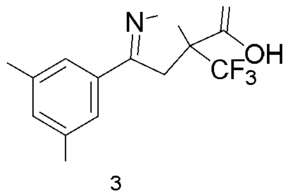 | $^1$H NMR (400 MHz, DMSO) $\delta$ 14.87 (s, 1H), 7.87 (s, 1H), 7.79 (s, 1H), 7.73 (s, 1H), 4.32 - 4.15 (m, 2H). |
| **II-72** | 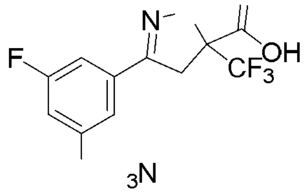 | $^1$H NMR (400 MHz, DMSO) $\delta$ 7.61 - 7.19 (m, 4H), 4.18 (s, 2H). |
| **II-73** | 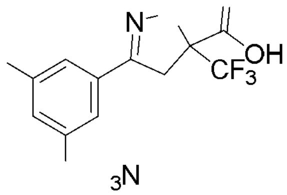 | |
| **II-74** | 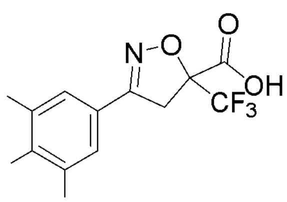 | $^1$H NMR (400 MHz, DMSO) $\delta$ 7.73 (dd, *J* = 13.2, 6.4 Hz, 2H), 4.15 (s, 2H). |

(continued)

| NO. | Structure | 1H NMR |
|---|---|---|
| **II-75** | 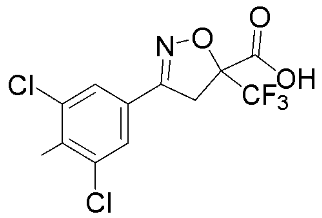 | $^1$H NMR (400 MHz, DMSO) δ 7.94 (d, J = 6.4 Hz, 2H), 3.97 (d, J = 8.6 Hz, 2H). |
| **II-76** | 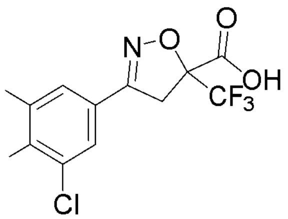 | $^1$H NMR (400 MHz, $CDCl_3$) δ 7.51 - 7.45 (m, 2H), 3.98 (d, *J*= 17.8 Hz, 1H), 3.78 (d, *J* = 17.8 Hz, 1H) |
| **II-78** | 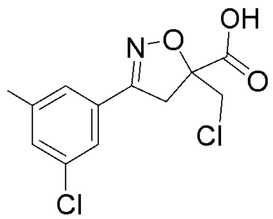 | $^1$H NMR (400 MHz, DMSO-) δ 13.90 (s, 1H), 7.69 - 7.51 (m, 3H), 4.10 - 4.02 (m, 2H), 3.91 (d, *J* = 18.0 Hz, 1H), 3.63 (d, *J* = 18.0 Hz, 1H). |
| **II-79** | 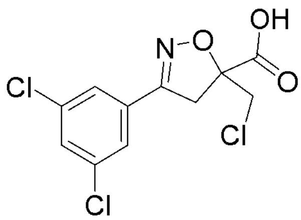 | $^1$H NMR (400 MHz, DMSO) δ 13.85 (s, 1H), 7.75 (dd, *J* = 9.4, 1.9 Hz, 3H), 4.05 (d, *J* = 2.4 Hz, 2H), 3.92 (d, *J* = 18.0 Hz, 1H), 3.64 (d, *J* = 18.0 Hz, 1H). |
| **II-80** | 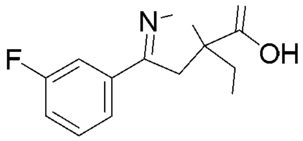 | |
| **II-81** | 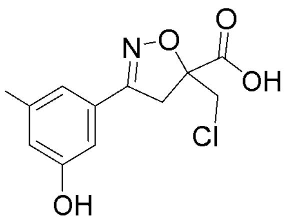 | |
| **II-82** | 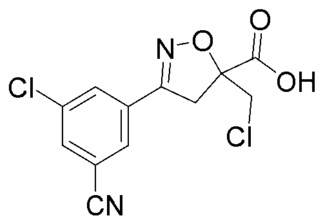 | |

(continued)

| NO. | Structure | 1H NMR |
|---|---|---|
| **II-83** | 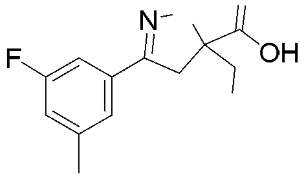 | |
| **II-84** | 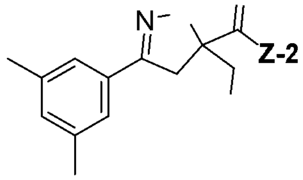 | |
| **II-85** | 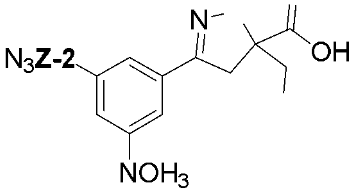 | |
| **II-86** | 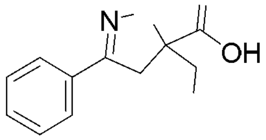 | |
| **II-87** | 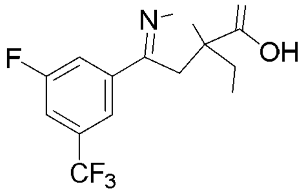 | |
| **II-88** | 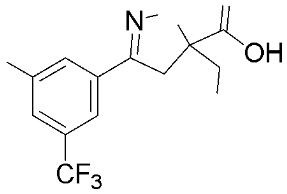 | |
| **II-89** | 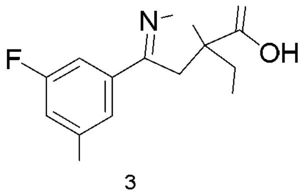 | |
| **II-90** | 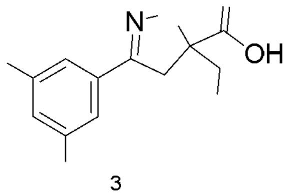 | |

(continued)

| NO. | Structure | $^1$H NMR |
|---|---|---|
| **II-91** | 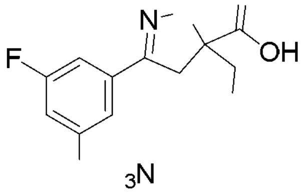 | |
| **II-92** | 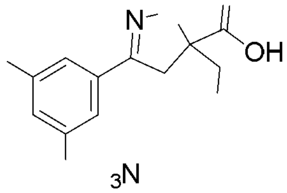 | |
| **II-93** | 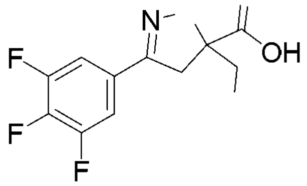 | |
| **II-94** | 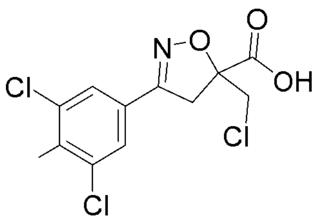 | |
| **II-95** | 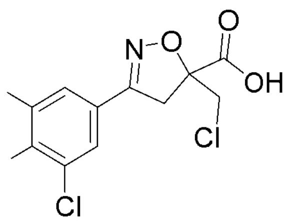 | |
| **II-96** | 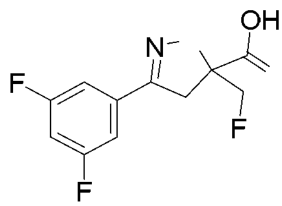 | $^1$H NMR (400 MHz, DMSO) $\delta$ 13.84 (s, 1H), 7.83 - 7.26 (m, 3H), 4.76 (ddd, *J* = 46.7, 26.9, 10.2 Hz, 2H), 3.81 (dd, *J* = 17.9, 1.6 Hz, 1H), 3.60 (d, *J* = 17.9 Hz, 1H). |
| **II-97** | 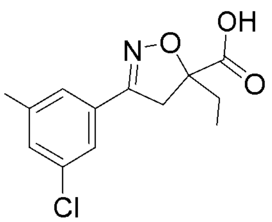 | $^1$H NMR (400 MHz, DMSO) $\delta$ 13.87 (s, 1H), 7.68 - 7.47 (m, 3H), 4.91 - 4.60 (m, 2H), 3.82 (dd, *J* = 17.9, 1.9 Hz, 1H), 3.61 (dd, *J* = 17.9, 1.9 Hz, 1H). |

(continued)

| NO. | Structure | $^1$H NMR |
|---|---|---|
| **II-98** | 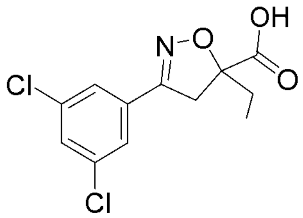 | |
| **II-99** | 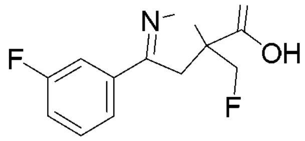 | |
| **II-100** | 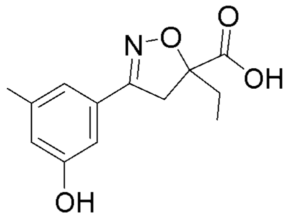 | |
| **II-101** | 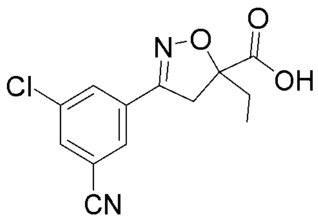 | $^1$H NMR (400 MHz, DMSO) $\delta$ 13.63 (s, 1H), 8.20 - 8.16 (m, 1H), 8.13 (t, $J$ = 1.3 Hz, 1H), 8.07 (t, $J$ = 1.7 Hz, 1H), 4.76 (ddd, $J$ = 46.7, 28.7, 10.2 Hz, 2H), 3.85 (dd, $J$ = 18.0, 1.6 Hz, 1H), 3.64 (d, $J$ = 18.0 Hz, 1H). |
| **II-102** | 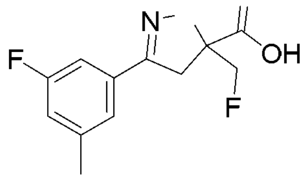 | |
| **II-103** | 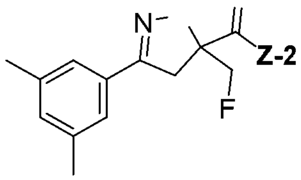 | |
| **II-104** | 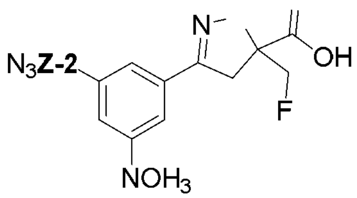 | |
| **II-105** | 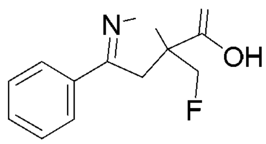 | |

(continued)

| NO. | Structure | 1H NMR |
|---|---|---|
| **II-106** | 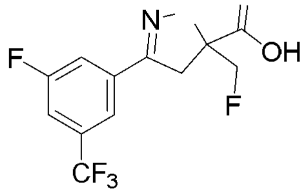 | $^1$H NMR (400 MHz, DMSO) $\delta$ 7.88 - 7.80 (m, 2H), 7.76 (dt, J = 8.6, 2.0 Hz, 1H), 4.77 (ddd, J = 46.7, 29.8, 10.2 Hz, 2H), 3.89 (dd, J = 17.9, 1.9 Hz, 1H), 3.67 (d, J = 17.9 Hz, 1H). |
| **II-107** | 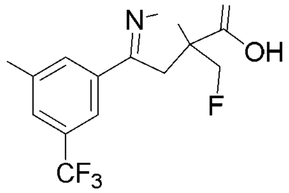 | |
| **II-108** | 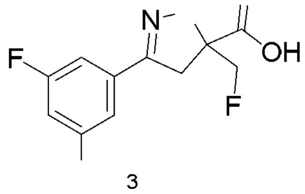 | $^1$H NMR (400 MHz, DMSO) $\delta$ 13.94 (s, 1H), 7.74 - 7.48 (m, 3H), 4.93 - 4.60 (m, 2H), 3.85 (dd, *J* = 17.9, 1.9 Hz, 1H), 3.63 (dd, *J* = 17.9, 1.9 Hz, 1H). |
| **II-109** | 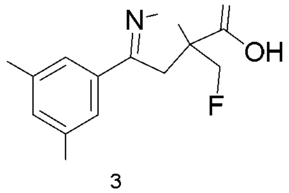 | $^1$H NMR (400 MHz, DMSO) $\delta$ 13.70 (s, 1H), 7.80 (t, *J* = 1.5 Hz, 1H), 7.73 (s, 1H), 7.66 (s, 1H), 4.76 (ddd, *J* = 46.7, 29.6, 10.2 Hz, 2H), 3.85 (dd, *J* = 17.9, 1.5 Hz, 1H), 3.64 (d, *J* = 17.9 Hz, 1H). |
| **II-111** | 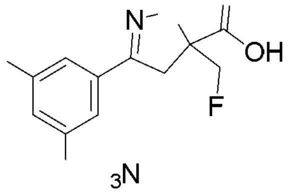 | |
| **II-112** | 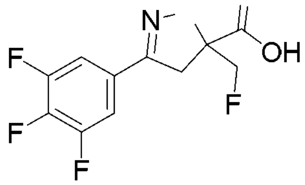 | $^1$H NMR (400 MHz, DMSO) $\delta$ 13.61 (s, 1H), 7.89 - 7.54 (m, 2H), 4.76 (ddd, *J* = 46.7, 25.9, 10.2 Hz, 2H), 3.80 (dd, *J* = 17.9, 1.7 Hz, 1H), 3.59 (d, *J* = 17.9 Hz, 1H). |
| **II-113** | 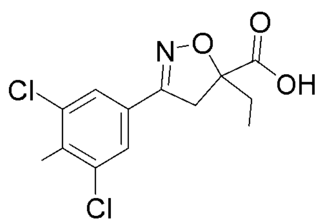 | |

(continued)

| NO. | Structure | ¹H NMR |
|---|---|---|
| **II-114** | 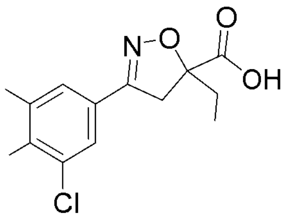 | |
| **II-116** | 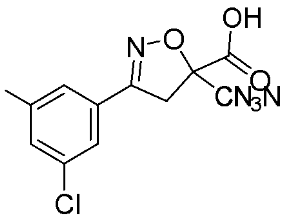 | ¹H NMR (400 MHz, DMSO) $\delta$ 14.42 (s, 1H), 7.71 - 7.50 (m, 3H), 6.53 (t, J = 54.0 Hz, 1H), 4.03 - 3.81 (m, 2H). |
| **II-117** | 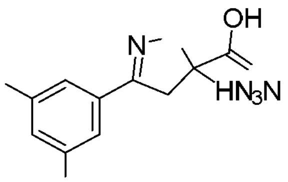 | |
| **II-118** | 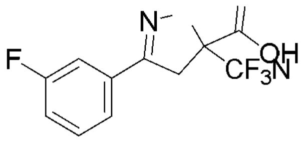 | |
| **II-119** | 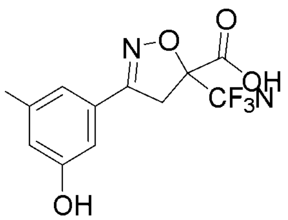 | |
| **II-120** | 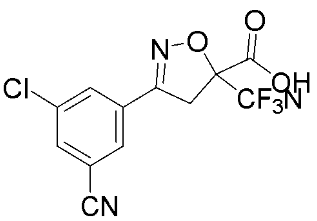 | |
| **II-121** | 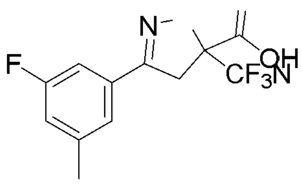 | |
| **II-122** | 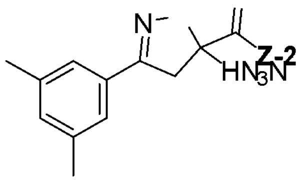 | |

(continued)

| NO. | Structure | $^1$H NMR |
|---|---|---|
| **II-123** | 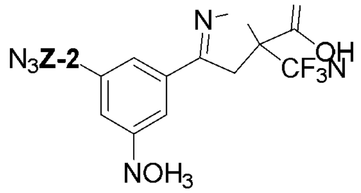 | |
| **II-124** | 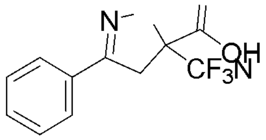 | |
| **II-125** | 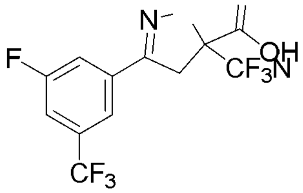 | $^1$H NMR (400 MHz, DMSO) $\delta$ 14.42 (s, 1H), 8.06 - 7.74 (m, 3H), 6.55 (t, *J* = 53.9 Hz, 1H), 4.19 - 3.86 (m, 2H). |
| **II-126** | 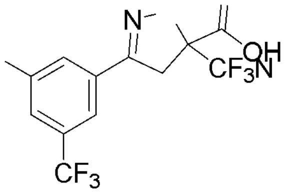 | |
| **II-127** | 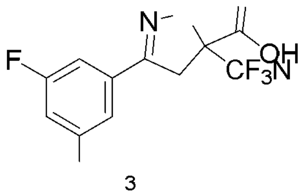 | |
| **II-128** | 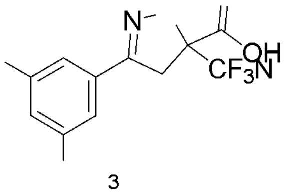 | |
| **II-129** | 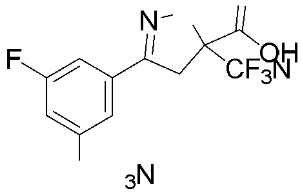 | |
| **II-130** | 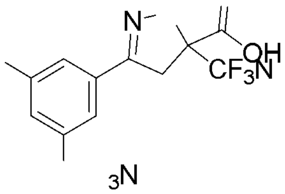 | |

(continued)

| NO. | Structure | 1H NMR |
|---|---|---|
| **II-131** | 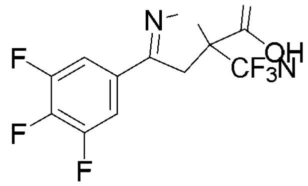 | |
| **II-132** | 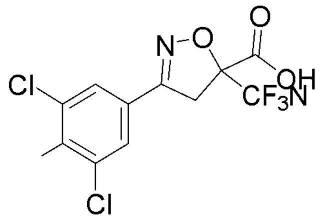 | |
| **II-133** | 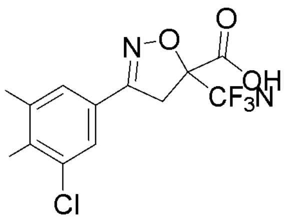 | |
| **II-134** | 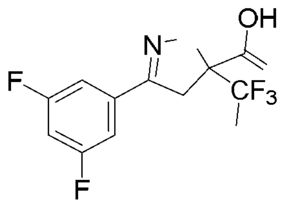 | $^1$H NMR (400 MHz, DMSO) $\delta$ 14.18 (s, 1H), 7.52 - 7.38 (m, 3H), 3.94 (s, 2H), 1.80 (t, $J$ = 19.5 Hz, 3H). |
| **II-135** | 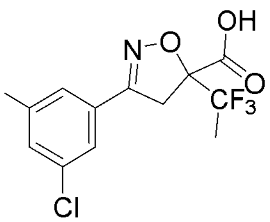 | |
| **II-137** | 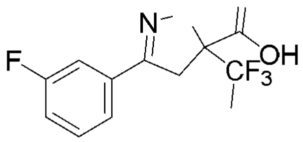 | |
| **II-138** | 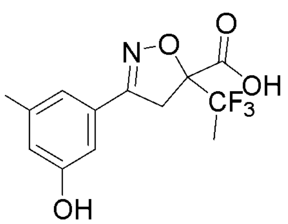 | |

(continued)

| NO. | Structure | $^1$H NMR |
|---|---|---|
| **II-139** | 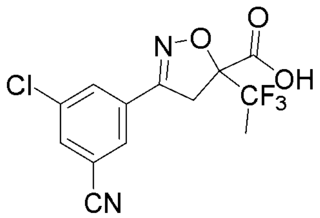 | |
| **II-140** | 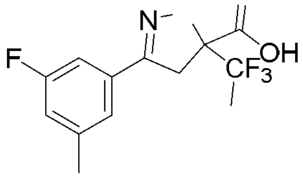 | |
| **II-141** | 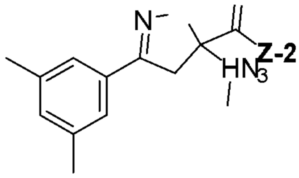 | |
| **II-142** | 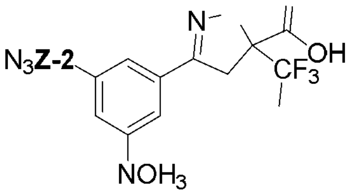 | |
| **II-143** | 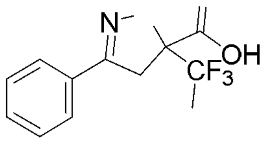 | |
| **II-144** | 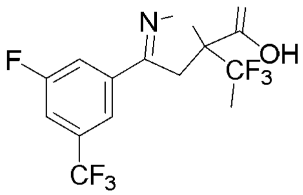 | |
| **II-145** | 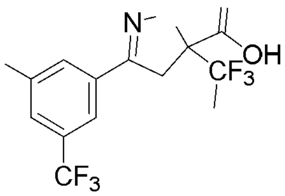 | |
| **II-146** | 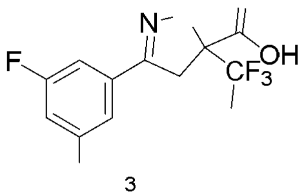 | |

(continued)

| NO. | Structure | $^1$H NMR |
|---|---|---|
| **II-147** | 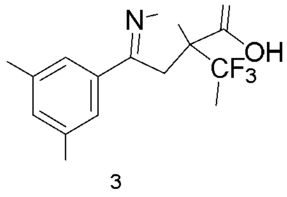 | |
| **II-148** | 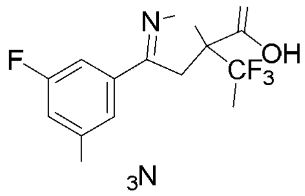 | |
| **II-149** | 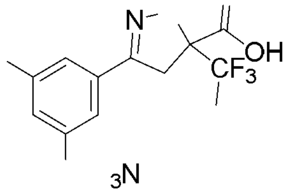 | |
| **II-150** | 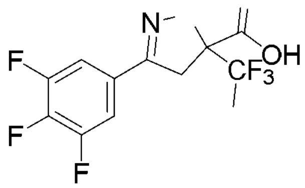 | |
| **II-151** | 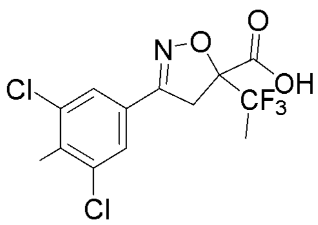 | |
| **II-152** | 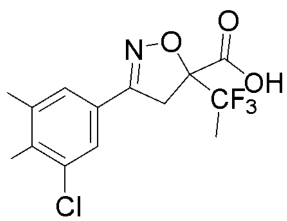 | |
| **II-153** | 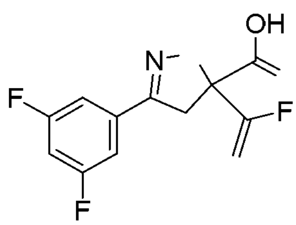 | $^1$H NMR (400 MHz, DMSO) $\delta$ 14.18 (s, 1H), 7.66 - 7.34 (m, 3H), 5.10 (dd, $J$ = 16.0, 4.2 Hz, 1H), 5.01 (dd, $J$ = 17.1, 4.2 Hz, 1H), 3.97 (q, $J$ = 18.1 Hz, 2H). |

(continued)

| NO. | Structure | $^1$H NMR |
|---|---|---|
| **II-154** | 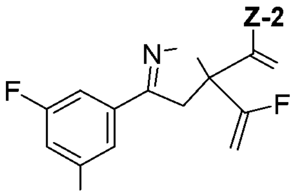 | |
| **II-156** | 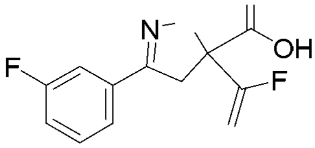 | |
| **II-157** | 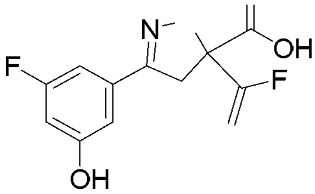 | |
| **II-158** | 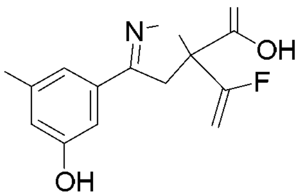 | |
| **II-159** | 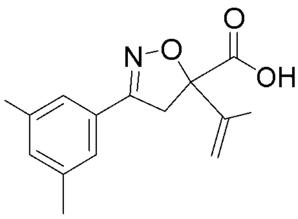 | |
| **II-160** | 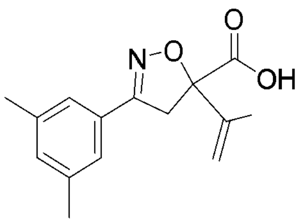 | |
| **II-161** | 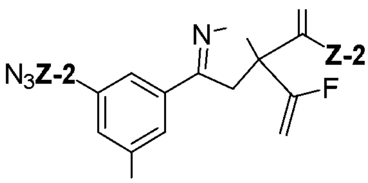 3 | |
| **II-162** | 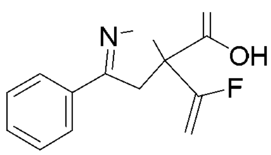 | |

(continued)

| NO. | Structure | $^{1}$H NMR |
|---|---|---|
| **II-163** | 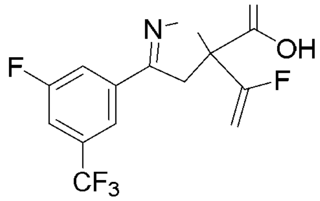 | |
| **II-164** | 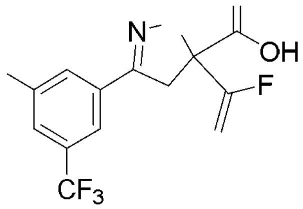 | |
| **II-165** | 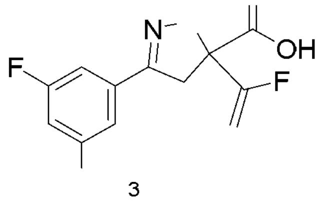 | |
| **II-166** | 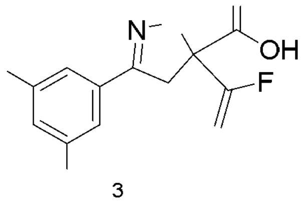 | |
| **II-167** | 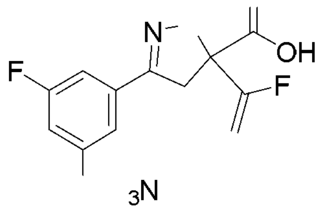 | |
| **II-168** | 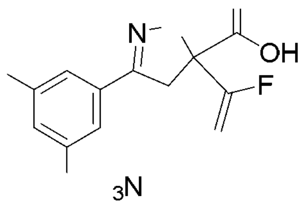 | |
| **II-169** | 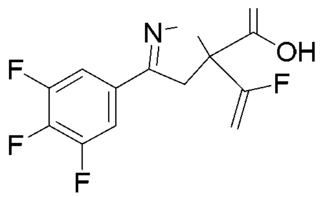 | |

(continued)

| NO. | Structure | ¹H NMR |
|---|---|---|
| **II-170** | 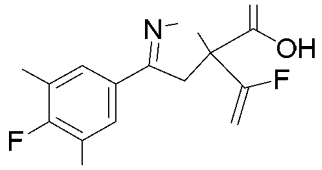 | |
| **II-171** | 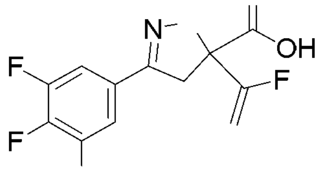 | |

The above prepared intermediates **II** can be separated by chiral column to obtain enantiomers which were S configuration and R configuration, respectively.

Analytical separation methods:

Method A:

Instrument: Agilent 1260 Infinity II
Column: Chiralpak AD-H, 250 x 4.6 mm I.D., 5 μm
Mobile phase: A represents n-hexane (0.2% formic acid), B represents isopropanol, isocratic: B%=25%
Flow rate: 1.0 mL/min, column temperature: 30 °C
Wavelength: 265 nm
Running time: 20 min

Method B:

Instrument: Agilent 1260 Infinity II
Column: Chiralpak AD-H, 250 x 4.6 mm I.D., 5 μm
Mobile phase: A represents n-hexane (0.2% formic acid), B represents isopropanol, isocratic: B%=20%
Flow rate: 1.0 mL/min, column temperature: 30 °C
Wavelength: 265 nm
Running time: 20 min

Method C:

Instrument: Agilent 1260 Infinity II
Column: Chiralpak AD-H, 250 x 4.6 mm I.D., 5 μm
Mobile phase: A represents n-hexane (0.2% formic acid), B represents isopropanol, isocratic: B%=15%
Flow rate: 1.0 mL/min, column temperature: 30 °C
Wavelength: 265 nm
Running time: 20 min

Method D:

Instrument: Agilent 1260 Infinity
Column: Chiralpak AD-H, 250 x 4.6 mm I.D., 5 μm
Mobile phase: A represents n-hexane (0.05% trifluoroacetic acid), B represents isopropanol, isocratic: B%=12%
Flow rate: 1.0 mL/min, column temperature: 30 °C
Wavelength: 265 nm
Running time: 20 min

Method E:

Instrument: Agilent 1260 Infinity
Column: Chiralpak AD-H, 250 x 4.6 mm I.D., 5 μm
Mobile phase: A represents n-hexane, B represents isopropanol, isocratic: B%=25%
Flow rate: 1.0 mL/min, column temperature: 30 °C
Wavelength: 265 nm
Running time: 20 min

Method F:

Instrument: Agilent 1260 Infinity
Column: Chiralpak AD-H, 250 x 4.6 mm I.D., 5 μm
Mobile phase: A represents n-hexane, B represents isopropanol, isocratic: B%=30%
Flow rate: 1.0 mL/min, column temperature: 30 °C
Wavelength: 265 nm
Running time: 20 min

Method G:

Instrument: Agilent 1260 Infinity
Column: Chiralpak AD-H, 250 x 4.6 mm I.D., 5 μm
Mobile phase: A represents n-hexane, B represents isopropanol, isocratic: B%=10%
Flow rate: 1.0 mL/min, column temperature: 30 °C
Wavelength: 265 nm
Running time: 20 min

Method H:

Instrument: Agilent 1260 Infinity
Column: Chiralpak AD-H, 250 x 4.6 mm I.D., 5 μm
Mobile phase: A represents n-hexane, B represents isopropanol, isocratic: B%=25%
Flow rate: 1.0 mL/min, column temperature: 30 °C
Wavelength: 265 nm
Running time: 20 min

Preparative separation methods:

Method 1:

Instrument: Gilson GX-281

Column: Chiralpak AD-H, 250 x 30 mm I.D., 5 μm
Mobile phase: A represents n-hexane (0.2% formic acid), B represents isopropanol, isocratic: B%=25%
Flow rate: 31 mL/min, column temperature: 30 °C
Wavelength: 265 nm
Running time: 20 min

Method 2:

Instrument: Gilson GX-281
Column: Chiralpak AD-H, 250 x 30 mm I.D., 5 μm
Mobile phase: A represents n-hexane (0.2% formic acid), B represents isopropanol, isocratic: B%=20%
Flow rate: 33 mL/min, column temperature: 30 °C
Wavelength: 265 nm
Running time: 20 min

Method 3:

Instrument: Gilson GX-281
Column: Chiralpak AD-H, 250 x 30 mm I.D., 5 μm
Mobile phase: A represents n-hexane (0.2% formic acid), B represents isopropanol, isocratic: B%=15%
Flow rate: 34 mL/min, column temperature: 30 °C
Wavelength: 265 nm
Running time: 20 min

Method 4:

Instrument: Gilson GX-281
Column: Chiralpak AD-H, 250 x 30 mm I.D., 5 μm
Mobile phase: A represents n-hexane (0.05% trifluoroacetic acid), B represents isopropanol, gradient: B%=12%
Flow rate: 34 mL/min, column temperature: 30 °C
Wavelength: 265 nm
Running time: 20 min

Intermediates **II-1** can be separated to give enantiomer **II-1-A** and enantiomer **II-1-B** (Method 1):

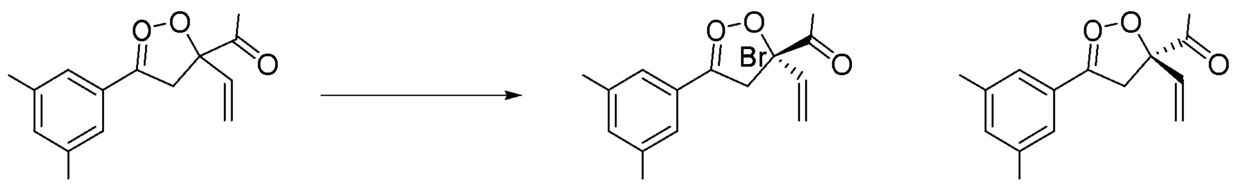

5.0 g of intermediate **II-1** (purity 95%) was weighed. The sample was dissolved in n-hexane: isopropanol=75:25 to prepare a solution of 40 mg/mL, with an injection volume of 5 mL. After separation, the intermediate **II-1-A** (2.36 g, purity 98%) and intermediate **II-1-B** (2.13 g, purity 99%) were obtained.

**II-1-A**: Retention time: 8.82 min (Method 1), 100ee% (Method A), $[\alpha]_D^{20}$= -57.85° (c=0.130, in MeOH).

**II-1-B:** Retention time: 6.19 min (Method 1), 100ee% (Method A), $[\alpha]_D^{20}$=61.32° (c=0.181, in MeOH).

The above methods can be used to give a single chiral isomer of other intermediate **II,** as shown in Table B.

Table B

| NO. | Structure | Preparative separation method Retention time | Analytical separation method ee% | [α] |
|---|---|---|---|---|
| **II-A** | 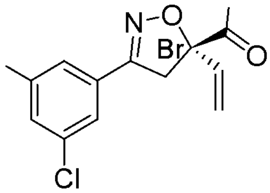 | Method 1<br>12.485 min | Method A<br>100 | $[\alpha]_D^{20}$= -60.51°<br>(c=0.241, in MeOH). |
| **II-11-A** | 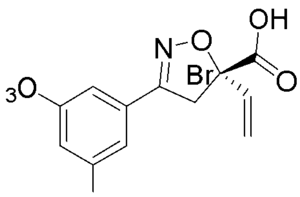 | Method 1<br>8.325 min | Method A<br>100 | $[\alpha]_D^{20}$= -50.34°<br>(c=0.427, in MeOH). |
| **II-12-A** | 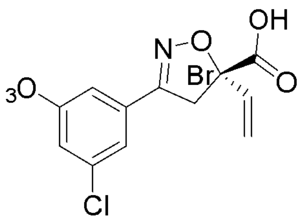 | Method 1<br>10.191 min | Method A<br>100 | $[\alpha]_D^{20}$= -47.22°<br>(c=0.131, in MeOH). |
| **II-13-A** | 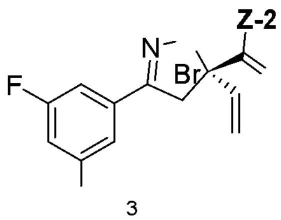 | Method 1<br>7.657 min | Method A<br>100 | $[\alpha]_D^{20}$= -49.32°<br>(c=0.109 in MeOH) |
| **II-14-A** | 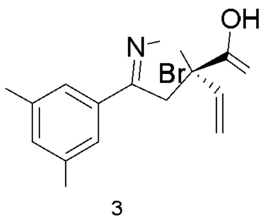 | Method 1<br>8.284 min | Method A<br>100 | $[\alpha]_D^{20}$= -48.12°<br>(c=0.199 in MeOH) |
| **II-20-A** | 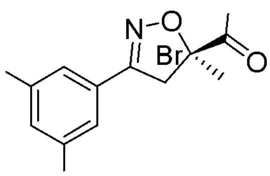 | Method 1<br>8.37 min | Method A<br>100 | $[\alpha]_D^{20}$= -130.61°<br>(c=0.211 in MeOH) |
| **II-21-A** | 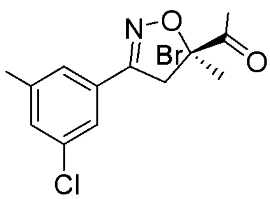 | Method 2<br>8.763 min | Method B<br>100 | $[\alpha]_D^{20}$= -132.71°<br>(c=0.192 in MeOH) |

(continued)

| NO. | Structure | Preparative separation method Retention time | Analytical separation method ee% | [α] |
|---|---|---|---|---|
| **II-30-A** | 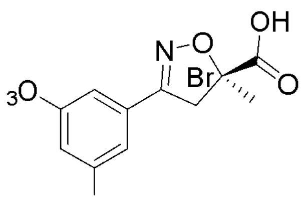 | Method 2<br>7.16 min | Method B<br>100 | $[\alpha]_D^{20}$= -115.68°<br>(c=0.156 in MeOH) |
| **II-31-A** | 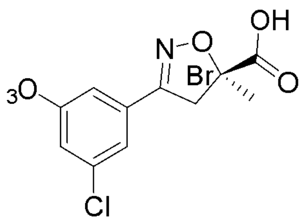 | Method 1<br>7.522 min | Method B<br>100 | $[\alpha]_D^{20}$= -111.29°<br>(c=0.126 in MeOH) |
| **II-32-A** | 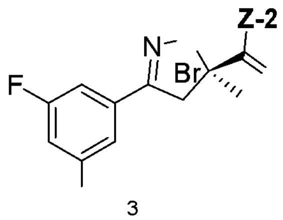 | Method 2<br>6.941 min | Method A<br>99.14 | $[\alpha]_D^{20}$= -102.12°<br>(c=0.104 in MeOH) |
| **II-58-A** | 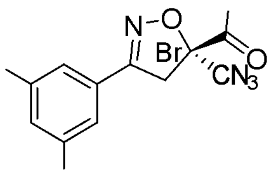 | Method 1<br>6.978 min | Method A<br>100 | $[\alpha]_D^{20}$= -85.20°<br>(c=0.232 in MeOH) |
| **II-59-A** | 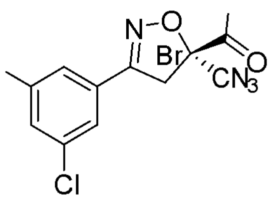 | Method 2<br>8.084 min | Method B<br>100 | $[\alpha]_D^{20}$= -102.91°<br>(c=0.110 in MeOH) |
| **II-68-A** | 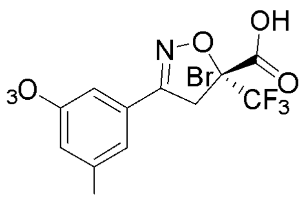 | Method 2<br>6.344 min | Method B<br>100 | $[\alpha]_D^{20}$= -77.11°<br>(c=0.147 in MeOH) |
| **II-69-A** | 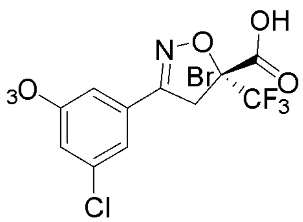 | Method 1<br>6.216 min | Method B<br>100 | $[\alpha]_D^{20}$= -66.33°<br>(c=0.119 in MeOH) |

(continued)

| NO. | Structure | Preparative separation method Retention time | Analytical separation method ee% | [α] |
|---|---|---|---|---|
| **II-70-A** | 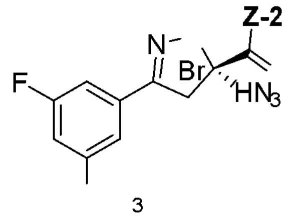 | Method 3<br>6.379 min | Method C<br>100 | $[\alpha]_D^{20}$= -79.35°<br>(c=0.123 in MeOH) |
| **II-106-A** | 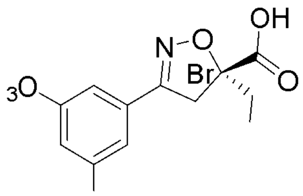 | Method 3<br>8.876 min | Method C<br>100 | $[\alpha]_D^{20}$= -92.62°<br>(c=0.149 in MeOH) |
| **II-108-A** | 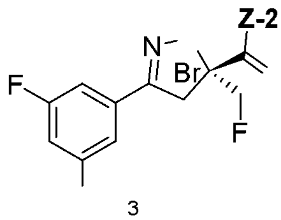 | Method 4<br>9.618 min | Method C<br>98.74 | $[\alpha]_D^{20}$= -86.73°<br>(c=0.208 in MeOH) |
| **II-115-A** | 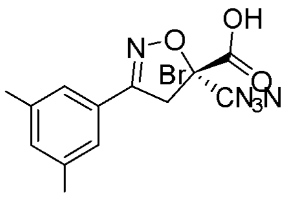 | Method 3<br>8.577 min | Method C<br>100 | $[\alpha]_D^{20}$= -169.09°<br>(c=0.110 in MeOH) |
| **II-116-A** | 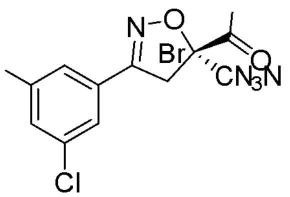 | Method 2<br>8.702 min | Method B<br>100 | $[\alpha]_D^{20}$= -152.08°<br>(c=0.137 in MeOH) |
| **II-125-A** | 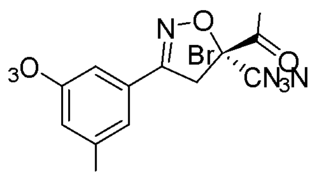 | Method 3<br>6.608 min | Method C<br>100 | $[\alpha]_D^{20}$= -138.56°<br>(c=0.077 in MeOH) |
| **II-127-A** | 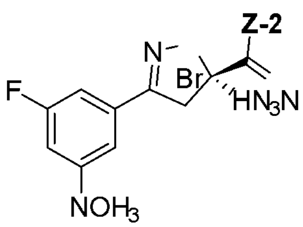 | Method 4<br>8.240 min | Method A<br>100 | $[\alpha]_D^{20}$= -120.68°<br>(c=0.124 in MeOH) |

**Examples I-10**

Preparation of compound **I-10**

Step 1: Preparation of intermediate **I-10.1**

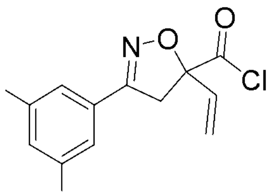

At room temperature, the intermediate **II-1** (1.0 g, 3.95 mmol), oxalyl chloride (750 mg, 5.95 mmol), dichloromethane (15 mL) and N,N-dimethylformamide (3 drops) were added to a 100 mL single-necked flask. The reaction mixture was stirred in $N_2$ atmosphere for 30 min. After the reaction was completed, the mixture was distilled under reduced pressure to remove solvent and excess oxalyl chloride to give intermediate **I-10.1** (yellow oil, 1 g).

Step 2: Preparation of intermediate **I-10.2**

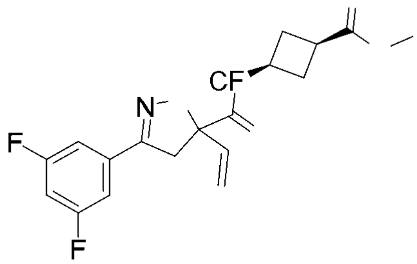

At room temperature, commercially available cis-methyl 3-aminocyclobutanecarboxylate hydrochloride (250 mg, 1.51 mmol), triethylamine (292 mg, 2.9 mmol), and dichloromethane (2 mL) were added to a 25 mL single-necked flask. The intermediate **I-10.1** (300 mg, 1.1 mmol) dissolved in 2 mL of dichloromethane was added at 0 °C. The mixture was heated to room temperature while stirring for 6 h. After the reaction was completed, the mixture was added with water and stirred, standing for layering. The lower organic phase was separated, dried with anhydrous sodium sulfate, concentrated under reduced pressure and purified by reverse-phase column chromatography to give intermediate **I-10.2** (white solid, 200 mg).

Step 3: Preparation of intermediate **I-10.3**

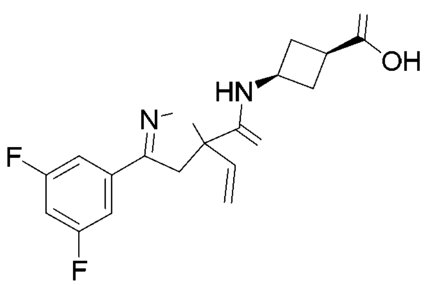

At room temperature, the intermediate **I-10.2** (178 mg, 0.488 mmol), tetrahydrofuran (2 mL) and water (0.5 mL) were added to a 25 mL single-necked flask, and stirred to dissolve. Lithium hydroxide monohydrate (23 mg, 0.55 mmol) was added, and the mixture was reacted at room temperature for 1 h. After the reaction was completed, the pH value of the mixture was adjusted to 1-2 with dilute hydrochloric acid (10%). The solid was precipitated and filtered. The filter cake was dried to give intermediate **I-10.3** (white solid, 120 mg).

Step 4: Preparation of intermediate **I-10.4**

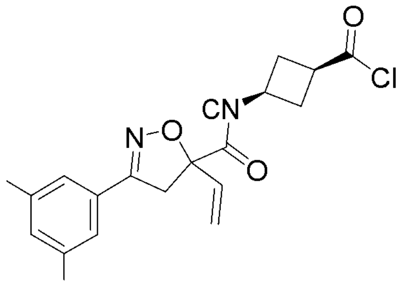

At room temperature, the intermediate **I-10.3** (500 mg, 1.48 mmol) and dichloromethane (5 mL) were added to a 25 mL single-necked flask. Then, 2 drops of N,Ndimethylformamide was added, followed by adding oxalyl chloride (200 μL, 2.32 mmol). Intense gas release was observed. The mixture was stirred at room temperature for 2 h, and then distilled under reduced pressure to remove the solvent and excess oxalyl chloride. The obtained crude intermediate **I-10.4** was used for the next step without further purification.

Step 5: Preparation of intermediate **I-10.5**

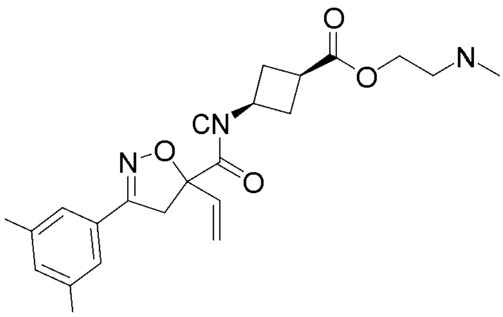

At room temperature, 2-(methylthio)ethanol (273 mg, 2.96 mmol), triethylamine (505 mg, 5mmol), and dichloromethane (2 mL) were added to a 25 mL single-necked flask. A solution of intermediate **I-10.4** from the previous step in dichloromethane (2 mL) was added at 0 °C, and the mixture was heated to room temperature while stirring for 2 h. After the reaction was completed, water was added and the mixture was stirred, standing for layering. The lower organic phase was separated, dried with anhydrous sodium sulfate and concentrated under reduced pressure. The residue was purified by reverse-phase column chromatography to give intermediate **I-10.5** (white solid, 355 mg).

Step 6: Preparation of intermediate **I-10.6**

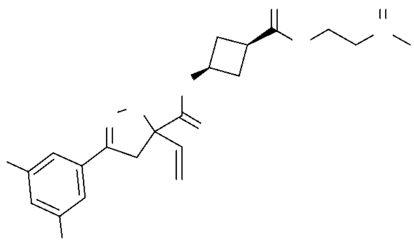

At room temperature, the intermediate **I-10.5** (145 mg, 0.342 mmol) and dichloromethane (5 mL) were added to a 25 mL single-necked flask, and stirred evenly under an ice bath. Metachloroperoxybenzoic acid (69 mg, 0.342 mmol, 85%) was added, and the mixture was reacted for 5 h. After the reaction was completed, the mixture was purified by column

chromatography (dichloromethane: acetic acid=99: 1) to give intermediate **I-10.6** (white solid, 102 mg).

Step 7: Preparation of compound **I-10**

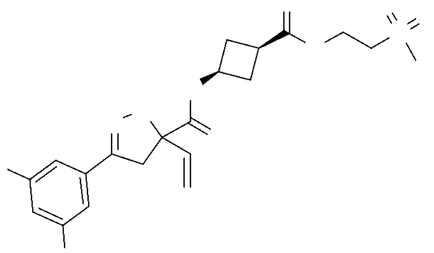

At room temperature, the intermediate **I-10.6** (130 mg, 0.306 mmol) and dichloromethane (5 mL) were added to a 25 mL single-necked flask, and stirred evenly at room temperature. Metachloroperoxybenzoic acid (155 mg, 0.766 mmol, 85%) was added, and the mixture was heated to 40 °C and reacted for 2 h. After the reaction was completed, the mixture was purified by column chromatography (dichloromethane: acetic acid=99:1) to give compound **I-10** (white solid, 70 mg). $^1$H-NMR (400 MHz, DMSO) $\delta$ 8.53 (d, *J*=7.7 Hz, 1H), 7.47-7.37 (m, 3H), 6.12 (dd, *J*=17.3, 10.7 Hz, 1H), 5.33 (dd, *J*=29.3, 14.0 Hz, 2H), 4.37 (t, *J*=5.8 Hz, 2H), 4.23-4.12 (m, 1H), 3.87 (d, *J*=17.8 Hz, 1H), 3.50 (dd, *J=15.5,* 11.7 Hz, 3H), 3.01 (s, 3H), 2.93-2.79 (m, 1H), 2.44-2.20 (m, 4H).

**Example I-20**

Preparation of compound **I-20**

Step 1: Preparation of intermediate **I-20.1**

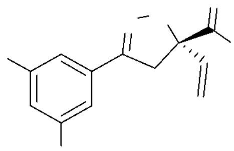

At room temperature, the intermediate **II-1-A** (2.0 g, 7.9 mmol), oxalyl chloride (1.5 g, 11.9 mmol), dichloromethane (15 mL) and *N,N*-dimethylformamide (3 drops) were added to a 100 mL single-necked flask. The reaction mixture was stirred in $N_2$ atmosphere for 30 min. After the reaction was completed, the solvent and excess oxalyl chloride were removed under vacuum to give intermediate **I-20.1** (yellow oil, 2.1 g).

Step 2: Preparation of intermediate **I-20.2**

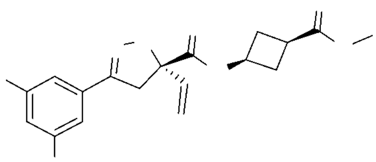

At 0 °C, cis-methyl 3-aminocyclobutanecarboxylate hydrochloride (1.9 g, 11.7 mmol), dichloromethane (15 mL) and triethylamine (3.9 g, 39 mmol) were added to a 100 mL single-necked flask. After stirring thoroughly for 20 min, a solution of the intermediate **I-20.1** (2.1 g, 7.8 mmol) in dichloromethane (5 mL) was added dropwise into the mixture. After dropwise addition was completed, the mixture was stirred and reacted at room temperature for 2 h. After the reaction was completed, the mixture was extracted with 30 mL of dichloromethane twice. The organic phases were combined, washed once with saturated saline solution, dried with anhydrous sodium sulfate, distilled under reduced pressure to remove

solvent, and purified by column chromatography (petroleum ether/ethyl acetate) to give intermediate **I-20.2** (light yellow solid, 2.7 g).

Step 3: Preparation of intermediate **I-20.3**

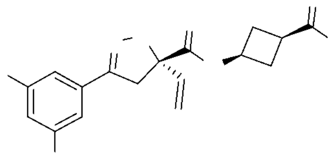

At room temperature, the intermediate **I-20.2** (2.7 g, 7.4 mmol), tetrahydrofuran (10 mL) and water (20 mL) were added to a 100 mL single-necked flask, and stirred well. Lithium hydroxide monohydrate (342 mg, 8.2 mmol) was added and the mixture was reacted for 30 min. After the reaction was completed, 1M hydrochloric acid was added dropwise, and the pH value was adjusted to 3-4. The mixture was extracted with 30 mL of ethyl acetate. The organic phases were combined, washed once with saturated saline solution, dried with anhydrous sodium sulfate, and distilled under reduced pressure to remove solvent to give intermediate **I-20.3** (white solid, 2.55 g).

Step 4: Preparation of intermediate **I-20.4**

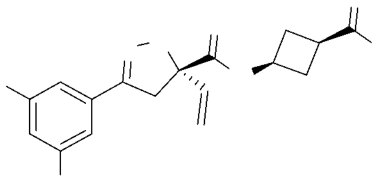

At room temperature, the intermediate **I-20.3** (200 mg, 0.57 mmol), oxalyl chloride (109 mg, 0.86 mmol), dichloromethane (4 mL) and N,N-dimethylformamide (2 drops) were added to a 25 mL single-necked flask. The reaction mixture was stirred in $N_2$ atmosphere for 30 min. After the reaction was completed, the solvent and excess oxalyl chloride were removed under vacuum to give intermediate **I-20.4** (yellow solid, 203 mg).

Step 5: Preparation of compound **I-20**

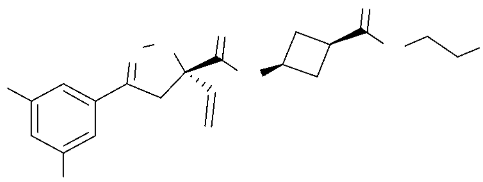

At room temperature, 3-hydroxypropionitrile (158 mg, 2.2 mmol) and dichloromethane (2 mL) were added to a 25 mL single-necked flask. After stirring thoroughly, a solution of the intermediate **I-20.4** (203 mg, 0.55 mmol) in dichloromethane (3 mL) was added dropwise, and the mixture was stirred and reacted for 2 h. After the reaction was completed, the mixture was distilled under reduced pressure to remove solvent. The residue was purified by reverse-phase column chromatography (acetonitrile/water) to give compound **I-20** (white solid, 177 mg). $^1$H-NMR (400 MHz, $CDCl_3$) $\delta$ 7.21-7.13 (m, 2H), 7.03 (d, *J*=8.3 Hz, 1H), 6.89 (tt, *J*=8.7, 2.3 Hz, 1H), 6.15 (dd, *J*=17.2, 10.7 Hz, 1H), 5.52 (d, *J*=17.2 Hz, 1H), 5.34 (d, *J*=10.7 Hz, 1H), 4.44-4.33 (m, 1H), 4.30 (t, *J*=6.3 Hz, 2H), 3.90 (d, *J=17.2* Hz, 1H), 3.31 (d, *J=17.3* Hz, 1H), 2.90 0 (tt, *J*=9.5, 7.9 Hz, 1H), 2.79-2.58 (m, 4H), 2.31-2.09 (m, 2H).

**Example I-253**

Preparation of compound **I-253**

Step 1: Preparation of intermediate **I-253.1**

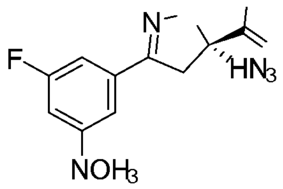

At room temperature, the intermediate **II-70-A** (350 mg, 0.97 mmol) was dissolved in dichloromethane (5 mL), and N,N-dimethylformamide (0.1 mL) was added, followed by adding oxalyl chloride (184.5 mg, 1.45 mmol) dropwise. The mixture was stirred at room temperature for 1 h to give the dichloromethane solution of intermediate **I-253.1,** which was used directly for the next step without additional treatment.

Step 2: Preparation of intermediate **I-253.2**

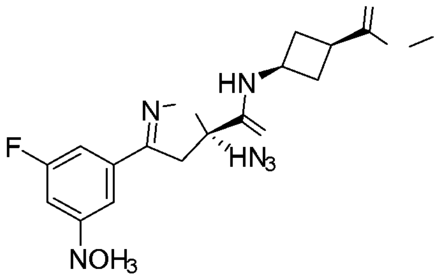

At room temperature, cis-methyl 3-aminocyclobutanecarboxylate hydrochloride (176.6mg, 1.07 mmol) was suspended in dichloromethane (5mL), and triethylamine (500.3mg, 4.94 mmol) was added to react for 30 min. The dichloromethane solution of intermediate **I-253.1** from the previous step was added dropwise under an ice bath, slowly returning the mixture to room temperature while stirring for 40 min. After the reaction was completed, the mixture was added with water and stirred, standing for layering. The lower organic phase was separated, distilled under reduced pressure to remove solvent, and purified by column chromatography to give intermediate **I-253.2** (white solid, 350 mg).

Step 3: Preparation of intermediate **I-253.3**

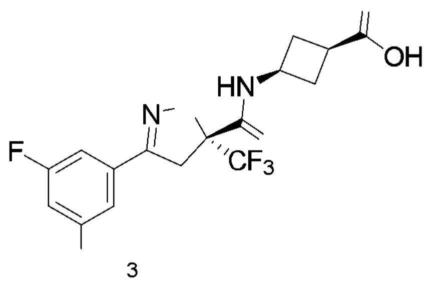

At room temperature, the intermediate **I-253.2** (350 mg, 0.74 mmol) was dissolved in tetrahydrofuran (10 mL) and an aqueous solution (10ml) of lithium hydroxide monohydrate (37.3 mg, 0.89 mmol) was added dropwise under an ice bath, slowly returning the mixture to room temperature while stirring for 40 min. After the reaction was completed, the pH value was adjusted to 1-2 with dilute hydrochloric acid (10%), and then saturated sodium chloride aqueous solution was added. The mixture was extracted with ethyl acetate. The organic phase was dried with anhydrous sodium sulfate, and

distilled under reduced pressure to remove solvent to give intermediate **I-253.3** (white solid, 340 mg).

Step 4: Preparation of intermediate **I-253.4**

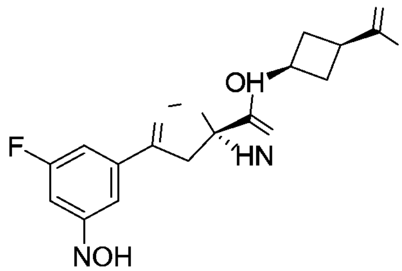

At room temperature, the intermediate **I-253.3** (150 mg, 0.33 mmol) was dissolved in dichloromethane (5 mL). After adding N,N-dimethylformamide (0.1 mL), oxalyl chloride (62.31 mg, 0.49 mmol) was added dropwise. The mixture was stirred at room temperature for 1 h to give a dichloromethane solution of intermediate **I-253.4.**

Step 5: Preparation of compound **I-253**

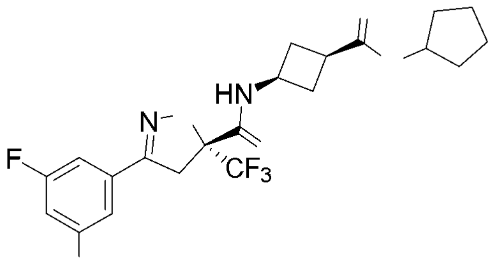

3

At room temperature, the dichloromethane solution of intermediate **I-253.4** from the previous step was added dropwise to cyclopentanol (1.5 mL) to react at room temperature for 2 h. After the reaction was completed, the mixture was distilled under reduced pressure to remove solvent. The residue was purified by column chromatography to give compound **I-253** (white solid, 117 mg). $^1$H-NMR (400 MHz, $CDCl_3$) $\delta$ 7.41-7.31 (m, 2H), 7.22 (d, *J*=8.3 Hz, 1H), 7.09 (dd, *J*=8.5, 2.5 Hz, 1H), 5.23-5.11 (m, 1H), 4.47-4.34 (m, 1H), 3.98 (d, *J=18.1* Hz, 1H), 3.76 (d, *J=18.1* Hz, 1H), 2.91-2.75 (m, 1H), 2.74-2.54 (m, 2H), 2.27-2.15 (m, 2H), 1.93-1.78 (m, 3H), 1.76-1.55 (m, 6H).

**Example I-364**

Preparation of compound **I-364**

Step 1: Preparation of intermediate **I-364.1**

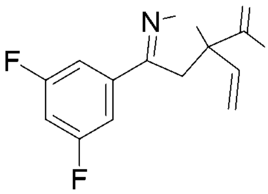

At room temperature, the intermediate **II-1** (150 mg, 0.59 mmol), dichloromethane (5 mL) and N,N-dimethylformamide (0.2 mL) were added to a single-necked flask under dried nitrogen gas, and stirred to dissolve. Oxalyl chloride

(113 mg, 0.88 mmol) was added, and a large number of bubbles were generated during the dropwise addition process. The mixture was stirred at room temperature for 20 min to give a dichloromethane solution of intermediate **1-364.1,** which was used directly for the next step without additional treatment.

Step 2: Preparation of compound **1-364**

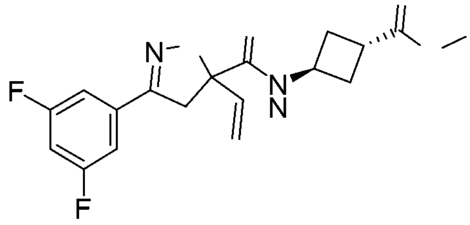

At room temperature, trans-methyl 3-aminocyclobutanecarboxylate hydrochloride (107 mg, 0.65 mmol), triethylamine (239 mg, 2.37 mmol), and dichloromethane (5 mL) were added sequentially to a single-necked flask, and stirred thoroughly for 20 min. Under an ice bath, a dichloromethane solution of the intermediate **1-364.1** from the previous step was added dropwise, and the mixture was stirred at room temperature for 30 min. After the reaction was completed, the mixture was distilled under reduced pressure to remove solvent, and purified by reversed-phase HPLC to give compound **1-364** (white solid, 206 mg). $^1$H-NMR (400 MHz, DMSO) $\delta$ 8.57 (d, *J*=7.9 Hz, 1H), 7.45-7.37 (m, 3H), 6.19-6.05 (m, 1H), 5.42-5.25 (m, 2H), 4.40 (dd, *J*=16.1, 8.1 Hz, 1H), 3.87 (d, *J*=17.8 Hz, 1H), 3.62 (s, 3H), 3.53 (d, *J*=17.8 Hz, 1H), 3.05-2.93 (m, 1H), 2.41-2.25 (m, 4H).

Example **1-385**

Preparation of compound **1-385**

Step 1: Preparation of intermediate **1-385.1**

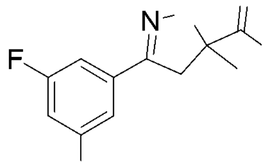

3

Under $N_2$ protection, the intermediate **11-34** (150 mg, 0.52 mmol), dichloromethane (5 mL) and N,N-dimethylformamide (0.2 mL) were added to a dried single-necked flask, and stirred to dissolve at room temperature. Oxalyl chloride (113 mg, 0.88 mmol) was added, and a large number of bubbles were generated during the dropwise addition process. The mixture was stirred at room temperature for 20 min to give a dichloromethane solution of intermediate **I-385.1,** which was left untreated and ready for use.

Step 2: Preparation of compound **I-385**

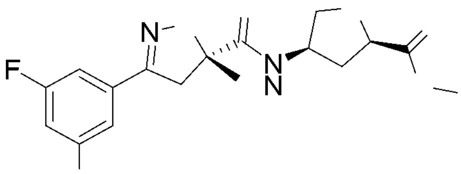

3

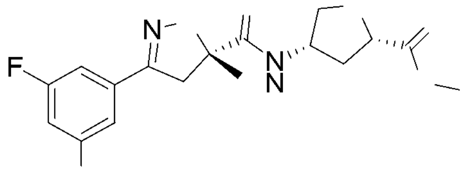

3

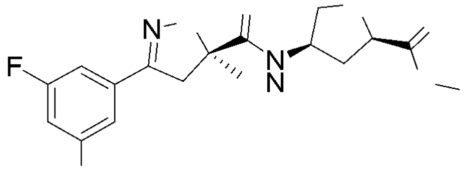 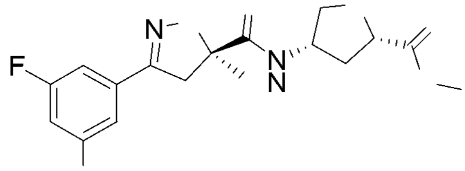

3 3

At room temperature, cis-4-aminotetrahydrofuran-2-methylformate hydrochloride (130 mg, 0.72 mmol), triethylamine (290 mg, 2.88 mmol), and dichloromethane (5 mL) were sequentially added to a single-necked flask. The reaction mixture was stirred thoroughly for 20 min under a condition of room temperature and. After the reaction solution was clear, the dichloromethane solution of intermediate **I-385.1** was added dropwise and stirred for 30 min at room temperature. Reaction was completed with TLC monitoring and was stopped. The mixture was distilled under reduced pressure to remove solvent, and purified by reversed-phase HPLC to give compound **I-385** (white powdery solid, 180 mg, a mixture of four optical isomers).

$^1$H-NMR (400 MHz, Chloroform-d) $\delta$ 7.54-7.43 (m, 1H), 7.24-7.17 (m, 2H), 6.97-6.91 (m, 1H), 6.55 (t, J=72.7 Hz, 1H), 4.64-4.50 (m, 2H), 4.07-3.99 (m, 1H), 3.99-3.87 (m, 1H), 3.85-3.71 (m, 4H), 3.18 (dd, J=17.3, 3.2 Hz, 1H), 2.61-2.48 (m, 1H), 2.15-1.98 (m, 2H), 1.70 (d, J=9.43, 3.2 Hz, 1H) Hz, 3H).

Compound **I-385** was separated by chiral SFC (Column: Chiralpak IC; Column Size: 250 * 60 mm, 10 um; Injection: 1 mL; Mobile Phase: $CO_2$: isopropanol=75:25; Flow rate: 80 mL/min; Wavelength: UV 220 nm; Temperature: 40 °C; Sample solution: 4 mg/mL in metal/dichloromethane) to give four optical isomers.

Optical isomer 1

$^1$H-NMR (400 MHz, Chloroform-d) $\delta$ 7.47 (d, *J*=7.9 Hz, 1H), 7.25-7.17 (m, 2H), 6.94 (dt, *J*=9.0, 2.0 Hz, 1H), 6.55 (t, *J*=72.7 Hz, 1H), 4.61-4.52 (m, 2H), 4.01 (dd, *J*=9.5, 5.2 Hz, 1H), 3.90 (dd, *J*=9.5, 2.2 Hz, 1H), 3.84 (s, 3H), 3.75 (d, *J*=17.3 Hz, 1H), 3.19 (d, *J*=17.3 Hz, 1H), 2.63-2.50 (m, 1H), 2.11 (dt, *J*=13.8, 2.6 Hz, 1H), 1.69 (s, 3H).

Optical isomer 2

$^1$H-NMR (400 MHz, Chloroform-d) $\delta$ 7.51 (d, *J*=8.1 Hz, 1H), 7.24-7.16 (m, 2H), 6.94 (dt, *J*=9.0, 2.1 Hz, 1H), 6.55 (t, *J*=72.7 Hz, 1H), 4.64-4.51 (m, 2H), 4.05 (dd, *J*=9.4, 5.2 Hz, 1H), 3.96 (dd, *J*=9.4, 2.1 Hz, 1H), 3.78 (s, 3H), 3.76 (d, *J*=17.3 Hz, 1H), 3.18 (d, *J*=17.3 Hz, 1H), 2.59-2.48 (m, 1H), 2.01 (dt, *J*=13.8, 2.6 Hz, 1H), 1.72 (s, 3H).

Optical isomer 3

$^1$H-NMR (400 MHz, Chloroform-d) $\delta$ 7.47 (d, *J*=7.9 Hz, 1H), 7.25-7.17 (m, 2H), 6.94 (dt, *J*=9.0, 2.0 Hz, 1H), 6.55 (t, *J*=72.7 Hz, 1H), 4.61-4.52 (m, 2H), 4.01 (dd, *J*=9.5, 5.2 Hz, 1H), 3.90 (dd, *J*=9.5, 2.2 Hz, 1H), 3.84 (s, 3H), 3.75 (d, *J*=17.3 Hz, 1H), 3.19 (d, *J*=17.3 Hz, 1H), 2.63-2.50 (m, 1H), 2.11 (dt, *J*=13.8, 2.6 Hz, 1H), 1.69 (s, 3H).

Optical isomer 4

$^1$H-NMR (400 MHz, Chloroform-d) $\delta$ 7.51 (d, *J*=8.1 Hz, 1H), 7.24-7.16 (m, 2H), 6.94 (dt, *J*=9.0, 2.1 Hz, 1H), 6.55 (t, *J*=72.7 Hz, 1H), 4.64-4.51 (m, 2H), 4.05 (dd, *J*=9.4, 5.2 Hz, 1H), 3.96 (dd, *J*=9.4, 2.1 Hz, 1H), 3.78 (s, 3H), 3.76 (d, *J*=17.3 Hz, 1H), 3.18 (d, *J*=17.3 Hz, 1H), 2.59-2.48 (m, 1H), 2.01 (dt, *J*=13.8, 2.6 Hz, 1H), 1.72 (s, 3H).

**Example 1-404**

Preparation of compound **I-404**

Step 1: Preparation of intermediate **i-404.1**

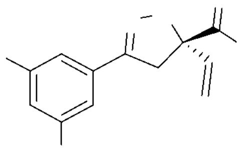

At room temperature, the intermediate **II-1-A** (200 mg, 0.79 mmol), oxalyl chloride (150 mg, 1.19 mmol), dichloromethane (5 mL) and *N,N*-dimethylformamide (1 drop) were added to a 100 mL single-necked flask. The reaction mixture was stirred in $N_2$ atmosphere for 30 min. After the reaction was completed, the solvent and excess oxalyl chloride were removed under reduced pressure to give intermediate **I-404.1** (yellow oil, 210 mg).

Step 2: Preparation of compound **I-404**

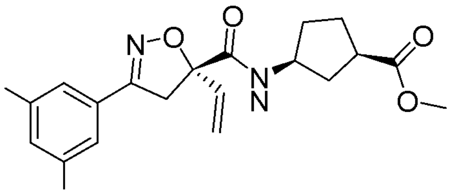

At 0 °C, (1*R*,3*S*)-methyl 3-aminocyclopentanecarboxylate hydrochloride (200 mg, 1.11 mmol), dichloromethane (5 mL) and triethylamine (337 mg, 3.33 mmol) were added to a 50 mL single-necked flask. After stirring thoroughly for 20 min, a solution of the intermediate **I-404.1** (210 mg, 0.78 mmol) in dichloromethane (3 mL) was added dropwise. After the dropwise addition, the mixture was stirred at room temperature and reacted for 2 h. After the reaction was completed, the mixture was extracted with 30 mL of dichloromethane twice. The organic phases were combined, washed once with saturated saline solution, dried with anhydrous sodium sulfate, distilled under reduced pressure to remove solvent, and purified by column chromatography (petroleum ether/ethyl acetate) to give intermediate **I-404** (white solid, 270 mg). $^1$H-NMR (400 MHz, DMSO) $\delta$ 8.15 (d, *J*=7.7 Hz, 1H), 7.49-7.35 (m, 3H), 6.14 (dd, *J*=17.3, 10.6 Hz, 1H), 5.37 (d, *J*=17.3 Hz, 1H), 5.30 (d, *J=10.6* Hz, 1H), 4.11 (dt, *J*=14.4, 7.2 Hz, 1H), 3.88 (d, *J*=17.7 Hz, 1H), 3.59 (s, 3H), 3.53 (d, *J*=17.8 Hz, 1H), 2.86-2.74 (m, 1H), 2.12-2.0 0 (m, 1H), 1.88-1.78 (m, 3H), 1.73 (dt, *J*=12.7, 8.7 Hz, 1H), 1.65-1.53 (m, 1H).

**Example 1-416**

Preparation of compound **I-416**

Step 1: Preparation of compound **I-416**

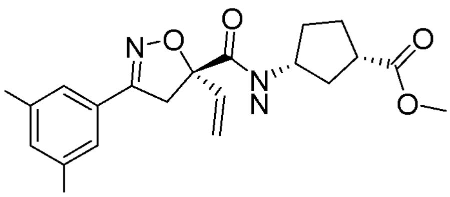

At 0 °C, (1*R*,3*S*)-methyl 3-aminocyclopentanecarboxylate hydrochloride (200 mg, 1.11 mmol), dichloromethane (5 mL) and triethylamine (337 mg, 3.33 mmol) were added to a 50 mL single-necked flask. After stirring thoroughly for 20 min, a solution of the intermediate **I-404.1** (210 mg, 0.78 mmol) in dichloromethane (3 mL) was added dropwise. After the dropwise addition, the mixture was stirred at room temperature and reacted for 2 h. After the reaction was completed, the mixture was extracted with 30 mL of dichloromethane twice. The organic phases were combined, washed once with saturated saline solution, dried with anhydrous sodium sulfate, distilled under reduced pressure to remove solvent, and purified by column chromatography (petroleum ether/ethyl acetate) to give intermediate **I-416** (white solid, 260 mg). $^1$H-NMR (400 MHz, DMSO) $\delta$ 8.16 (d, *J*=7.8 Hz, 1H), 7.47-7.36 (m, 3H), 6.14 (dd, *J=17.3,* 10.6 Hz, 1H), 5.38 (dd, *J*=17.3, 0.6 Hz, 1H), 5.34-5.26 (m, 1H), 4.17-4.04 (m, 1H), 3.88 (d, *J*=17.7 Hz, 1H), 3.61 (s, 3H), 3.53 (d, *J*=17.8 Hz, 1H), 2.81 (p, *J*=8.3 Hz, 1H), 2.10 (dt, *J*=12.7, 7.3 Hz, 1H), 1.87-1.76 (m, 3H), 1.75-1.67 (m, 1H), 1.65-1.52 (m, 1H).

**Example I-452**

Preparation of compound **I-452**

Step 1: Preparation of intermediate **I-452.1**

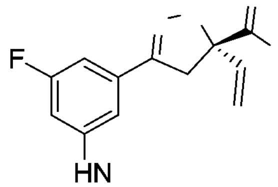

At room temperature, the intermediate **II-11-A** (2.8 g, 9.2 mmol), oxalyl chloride (1.76 g, 13.8 mmol), dichloromethane (15 mL) and *N,N*-dimethylformamide (3 drops) were added to a 100 mL single-necked flask. The reaction mixture was stirred in $N_2$ atmosphere for 30 min. After the reaction was completed, the mixture was distilled under reduced pressure to remove solvent and excess oxalyl chloride to give intermediate **I-452.1** (yellow oil, 2.9 g).

Step 2: Preparation of intermediate **I-452.2**

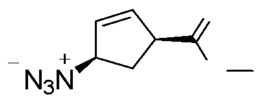

The intermediate **I-452.2** can be prepared from commercially available (1*R*,4*S*)2-azabicyclo [2.2.1] hept-5-en-3-one (Vince lactam, CAS No.: 79200-56-9), using the method described in Marco D. Migliore et al.: J Med. Chem. (2007, 50, 6485-6492). $^1$H-NMR (400 MHz, DMSO) $\delta$ 8.29 (s, 3H), 6.12-6.04 (m, 1H), 5.92-5.84 (m, 1H), 4.24-4.12 (m, 1H), 3.74-3.67 (m, 1H), 3.66 (s, 3H), 2.56 (dt, *J=13.9,* 8.6 Hz, 1H), 1.94 (dt, *J=13.6,* 6.7 Hz, 1H).

Step 3: Preparation of intermediate **I-452.3**

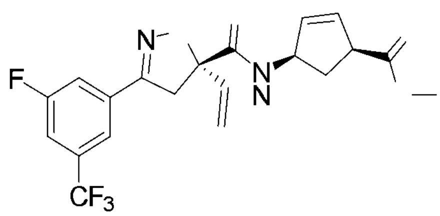

At 0 °C, intermediate **I-452.2** (2.4 g, 13.5 mmol), dichloromethane (15 mL) and triethylamine (4.5 g, 45 mmol) were added to a 100 mL single-necked flask. After stirring thoroughly for 30 min, a solution of the intermediate **I-452.1** (2.9 g, 9.0 mmol) in dichloromethane (10 mL) was added dropwise into the mixture. After the dropwise addition, the mixture was stirred and reacted for 2 h at room temperature. After the reaction was completed, the mixture was extracted with 30 mL of dichloromethane twice. The organic phases were combined, washed once with saturated saline solution, dried with anhydrous sodium sulfate, distilled under reduced pressure to remove solvent, and purified by column chromatography (petroleum ether/ethyl acetate) to give intermediate **I-452.3** (light yellow solid, 3.6 g).

Step 4: Preparation of intermediate **I-452.4**

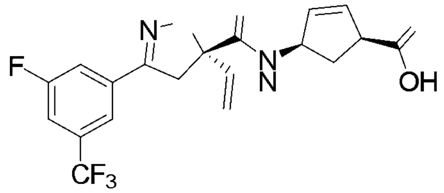

At room temperature, the intermediate **I-452.3** (3.6 g, 8.5 mmol), tetrahydrofuran (10 mL) and water (20 mL) were added to a 100 mL single-necked flask, and stirred well. Lithium hydroxide monohydrate (390 mg, 9.3 mmol) was added to react for 30 min. After the reaction was completed, 1M hydrochloric acid was added dropwise, and the pH value was adjusted to 3-4. The mixture was extracted with 30 mL of ethyl acetate. The organic phases were combined, washed once with saturated saline solution, dried with anhydrous sodium sulfate, and distilled under reduced pressure to remove solvent to give intermediate **I-452.4** (white solid, 3.45 g).

Step 5: Preparation of intermediate **I-452.5**

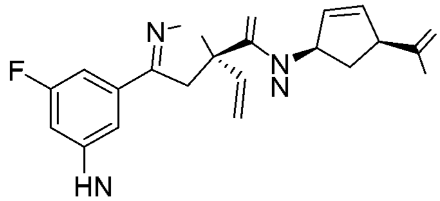

At room temperature, the intermediate **I-452.4** (200 mg, 0.49 mmol), oxalyl chloride (93 mg, 0.73 mmol), dichloromethane (4 mL) and *N,N*-dimethylformamide (2 drops) were added to a 25 mL single-necked flask. The reaction mixture was stirred in $N_2$ atmosphere for 30 min. After the reaction was completed, the mixture was distilled under reduced pressure to remove solvent and excess oxalyl chloride to give intermediate **I-452.5** (yellow solid, 202 mg).

Step 6: Preparation of compound **I-452**

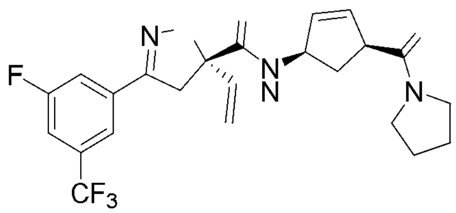

At room temperature, tetrahydropyrrole (134 mg, 1.9 mmol), triethylamine (242 mg, 2.4 mmol), and dichloromethane (2 mL) were added to a 25 mL single-necked flask. After stirring thoroughly, a solution of intermediate **I-452.5** (202 mg, 0.47 mmol) in dichloromethane (3 mL) was added dropwise, and the mixture was stirred and reacted for 2 h. After the reaction was completed, the mixture was distilled under reduced pressure to remove solvent. The residue was purified by reverse-phase column chromatography (acetonitrile/water) to give **I-452** (white solid, 177 mg). $^1$H-NMR (400 MHz, $CDCl_3$) $\delta$ 7.99 (d, *J*=8.9 Hz, 1H), 7.67 (s, 1H), 7.57 (dt, *J*=9.0, 1.9 Hz, 1H), 7.35 (dt, *J*=8.3, 2.0 Hz, 1H), 6.16 (dd, *J*=17.3, 10.7 Hz, 1H), 5.95-5.84 (m, 2H), 5.53 (d, *J*=17.2 Hz, 1H), 5.30 (d, *J*=10.7 Hz, 1H), 5.02 (tt, *J*=8.5, 2.4 Hz, 1H), 3.96 (d, *J*=17.1 Hz), 1H), 3.69-3.59 (m, 1H), 3.58-3.42 (m, 4H), 3.33 (d, *J*=17.1 Hz, 1H), 2.39-2.25 (m, 1H), 2.02-1.82 (m, 5H).

**Example 1-466**

Preparation of compound **1-466**

Step 1: Preparation of intermediate **I-466.1**

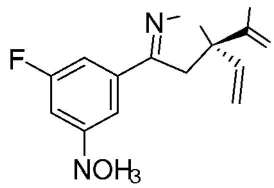

At room temperature, the intermediate **11-13-A** (4 g, 12.5 mmol) was dissolved in dichloromethane (25 mL). *N,N*-dimethylformamide (1 mL) was added, followed by adding oxalyl chloride (2.4 g, 18.8 mmol) dropwise. The mixture was stirred at room temperature for 1 h to give a dichloromethane solution of the intermediate **I-466.1.**

Step 2: Preparation of intermediate **I-466.2**

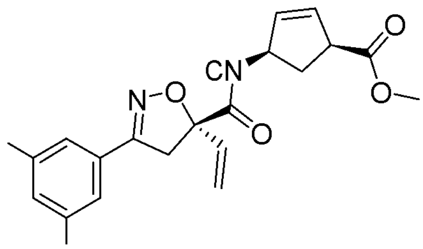

At room temperature, (1*S*,4*R*)-methyl 4-aminocyclopent-2-enecarboxylate hydrochloride (2.5 g, 13.8 mmol) was suspended in dichloromethane (25 mL). Triethylamine (6.5 g, 63.4 mmol) was added and stirred for 30 min. A dichloromethane solution of the intermediate **I-466.1** from the previous step was added dropwise under an ice bath, slowly returning the mixture to room temperature while stirring for 40 min. After the reaction was completed, the mixture was added with water and stirred, standing for layering. The lower organic phase was separated, distilled under reduced pressure to remove solvent, and purified by column chromatography to give the intermediate **I-466.2** (yellow oil, 4.5 g).

Step 3: Preparation of intermediate **I-466.3**

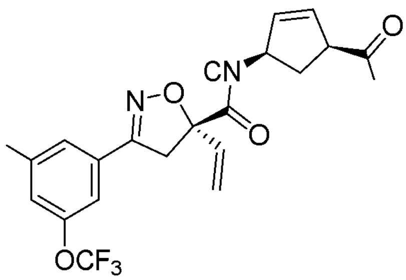

At room temperature, the intermediate **I-466.2** (4.5 g, 9.5 mmol) was dissolved in tetrahydrofuran (20 mL). The aqueous solution (20 mL) of lithium hydroxide monohydrate (476.4 mg, 11.4 mmol) was added dropwise under an ice bath (20 mL), slowly returning the mixture to room temperature while stirring for 50 min. After the reaction was completed, the pH value was adjusted to 1-2 with dilute hydrochloric acid (10%), and saturated aqueous solution of sodium chloride was added. The mixture was extracted with ethyl acetate, and purified by column chromatography to give the intermediate **I-466.3** (colorless oil, 3.6 g). $^1$H-NMR (400 MHz, DMSO) $\delta$ 12.45 (s, 1H), 8.08 (d, *J*=7.9 Hz, 1H), 7.66-7.51 (m, 3H), 6.15 (dd, *J*=17.3, 10.7 Hz, 1H), 5.96-5.88 (m, 1H), 5.80-5.76 (m, 1H), 5.39 (d, *J=17.3* Hz, 1H), 5.30 (d, *J=10.6* Hz, 1H), 4.86-4.74 (m, 1H), 3.92 (d, *J=17.8* Hz, 1H), 3.58 (d, *J*=17.8 Hz, 1H) 3.49-3.40 (m, 1H), 2.44-2.32 (m, 1H), 1.90-1.77 (m, 1H).

Step 4: Preparation of intermediate **I-466.4**

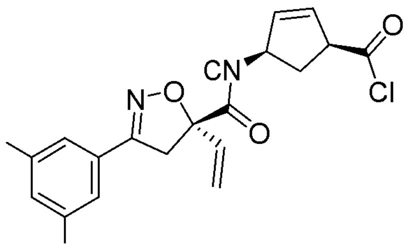

At room temperature, the intermediate **I-466.3** (220 mg, 0.51 mmol) was dissolved in dichloromethane (5 mL). *N,N*-dimethylformamide (0.1 mL) was added, followed by adding oxalyl chloride (97.8 mg, 0.77 mmol) dropwise. The mixture was stirred at room temperature for 1 h to give a dichloromethane solution of the intermediate **I-466.4.**

Step 5: Preparation of compound **I-466**

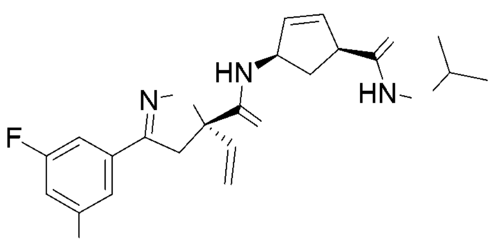

3

At room temperature, 2-(aminooxy) propane hydrochloride (114.6 mg, 1.03 mmol) was suspended in dichloromethane (5 mL). Triethylamine (260.0 g, 2.57 mmol) was added and stirred for 30 min. A dichloromethane solution of the intermediate **I-466.4** was added dropwise under an ice bath, slowly returning the mixture to room temperature while stirring for 40 min. After the reaction was completed, the mixture was washed with water, distilled under reduced pressure to remove the solvent, and purified by column chromatography to give compound **I-466** (white solid, 178 mg). $^1$H-NMR (400 MHz, DMSO) $\delta$ 10.97 (s, 1H), 8.04 (d, *J*=8.4 Hz, 1H), 7.67-7.59 (m, 1H), 7.56 (d, *J*=7.2 Hz, 2H), 6.18-6.09 (m, 1H), 5.82 (s, 2H), 5.43-5.28 (m, 2H), 4.88-4.78 (m, 1H), 4.05-3.94 (m, 1H), 3.89 (dd, *J*=17.8, 4.0 Hz, 1H), 3.59 (dd, *J*=17.8, 4.0 Hz, 1H), 3.24 (dd, *J*=8.2, 3.7 Hz, 1H), 2.33-2.20 (m, 1H), 1.73-1.63 (m, 1H), 1.12 (d, *J*=6.2 Hz, 6H).

**Example I-525**

Preparation of compound **I-525**

Step 1: Preparation of intermediate **I-525.1**

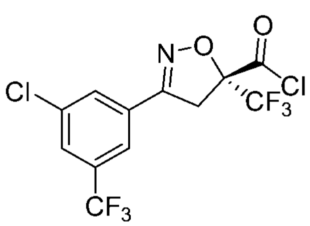

The intermediate **II-69-A** (200 mg, 0.55 mmol), dichloromethane (5 mL) and *N,N*-dimethylformamide (0.2 mL) were added to a dried single-necked flask under $N_2$ protection, and stirred to dissolve at room temperature. Oxalyl chloride (105 mg, 0.83 mmol) was added, and a large number of bubbles were generated during the dropwise addition process. The

mixture was stirred at room temperature for 20 min to give a dichloromethane solution of the intermediate I-**525.1.**

Step 2: Preparation of intermediate **I-525.2**

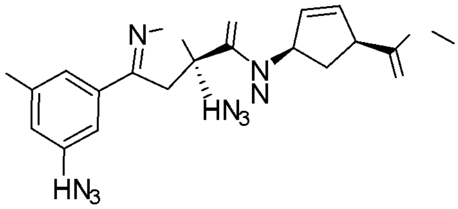

At room temperature, (1S,4R)-methyl 4-aminocyclopent-2-enecarboxylate hydrochloride (146 mg, 0.83 mmol), triethylamine (194 mg, 1.93 mmol), and dichloromethane (5 mL) were added to a 25 mL single-necked flask. The reaction mixture was thoroughly stirred to dissociate for 20 min under a condition of room temperature. Under an ice bath, a dichloromethane solution of intermediate **I-525.1** was added dropwise, and the mixture was stirred for 30 min at room temperature. After the reaction was completed, the solvent was removed under reduced pressure, and compound **I-525.2** (oily solid, 200 mg) was obtained through purification by reverse-phase column chromatography. $^1$H-NMR (400 MHz, DMSO) $\delta$ 8.63 (d, *J*=7.7 Hz, 1H), 8.12 (s, 1H), 8.08 (s, 1H), 8.04 (s, 1H), 5.93 (dt, *J=5.6,* 2.1 Hz, 1H), 5.79 (dt, *J*=5.6, 2.3 Hz, 1H), 4.88-4.80 (m, 1H), 4.34-4.11 (m, 2H), 3.62 (s, 3H), 3.60-3.53 (m, 1H), 2.48-2.40 (m, 1H), 1.98-1.87 (m, 1H).

Step 3: Preparation of intermediate **I-525.3**

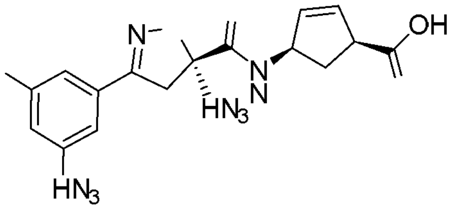

Intermediate **I-525.2** (200 mg, 0.41 mmol), tetrahydrofuran (5 mL) and water (2 mL) were thoroughly stirred at room temperature for 5 min. Then, lithium hydroxide monohydrate (26 mg, 0.62 mmol) was added and the mixture was stirred at room temperature for 3 h. The reaction was completed with LC-MS monitoring. 1M hydrochloric acid was added dropwise, and the pH value was adjusted to 3-4. The mixture was extracted with ethyl acetate. The organic phases were combined, distilled under reduced pressure to remove solvent, and purified by column chromatography to give intermediate **I-525.3** (190 mg). $^1$H-NMR (400 MHz, $CDCl_3$) $\delta$ 7.89-7.78 (m, 2H), 7.75-7.69 (m, 1H), 7.40 (d, J=8.9 Hz, 1H), 6.09-6.03 (m, 1H), 6.00-5.93 (m, 1H), 5.08 (t, *J*=8.7 Hz, 1H), 4.03 (d, *J*=18.1 Hz, 1H), 3.79 (d, *J*=18.1 Hz, 1H), 3.62-3.54 (m, 1H), 2.53-2.40 (m, 1H), 2.02-1.92 (m, 1H).

Step 4: Preparation of intermediate **I-525.4**

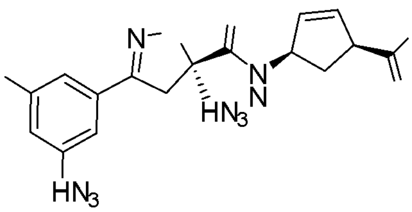

Under $N_2$ protection, the intermediate **I-525.3** (180 mg, 0.38 mmol), dichloromethane (5 mL) and N,N-dimethylformamide (0.2 mL) were added to a dried single-necked flask, and stirred to dissolve at room temperature. Oxalyl chloride (72 mg, 0.57 mmol) was added and a large number of bubbles were generated during the dropwise addition process. The

mixture was stirred at room temperature for 20 min to give a dichloromethane solution the intermediate **I-525.4.**

Step 5: Preparation of compound **I-525**

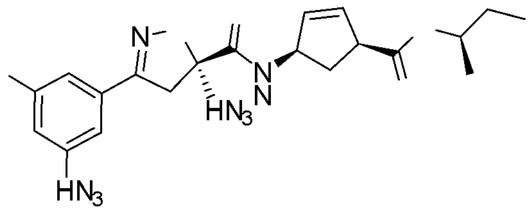

At room temperature, (*R*)-butane-2-ol (42 mg, 0.57 mmol) and dichloromethane (5 mL) were added sequentially to a single-necked flask, and stirred thoroughly for 5 min. The intermediate **I-525.4** was added dropwise and stirred at room temperature for 30 min. After the reaction was completed, the mixture was distilled under reduced pressure to remove solvent, and purified by reversed-phase HPLC to give compound **I-525** (oily solid, 107 mg). $^1$H-NMR (400 MHz, $CDCl_3$) $\delta$ 7.85 (d, *J=1.7* Hz, 1H), 7.80 (s, 1H), 7.72 (d, *J*=1.9 Hz, 1H), 7.40 (d, *J*=8.8 Hz, 1H), 6.04-5.99 (m, 1H), 5.97-5.89 (m, 1H), 5.07 (t, *J*=8.7 Hz, 1H), 4.93-4.80 (m, 1H), 4.05 (d, *J*=18.0 Hz, 1H), 3.79 (d, *J*=18.0 Hz, 1H), 3.54-3.47 (m, 1H), 2.43 (dt, *J*=14.1, 8.4 Hz, 1H), 1.99-1.91 (m, 1H), 1.70-1.50 (m, 2H), 1.23 (d, *J*=6.3 Hz, 3H), 0.88 (t, *J*=7.4 Hz, 3H).

**Example 1-535**

Preparation of compound **I-535**

Step 1: Preparation of intermediate **I-535.1**

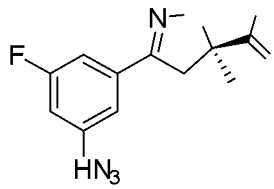

Under $N_2$ protection, the intermediate **II-30-A** (200 mg, 0.69 mmol), dichloromethane (5 mL) and N,N-dimethylformamide (0.2 mL) were added to a dried single-necked flask, and stirred to dissolved at room temperature. Oxalyl chloride (130.4 mg, 1.04 mmol) was added, and a large number of bubbles were generated during the dropwise addition process. The mixture was stirred at room temperature for 20 min to give a dichloromethane solution of intermediate **I-535.1** for use.

Step 2: Preparation of compound **I-535**

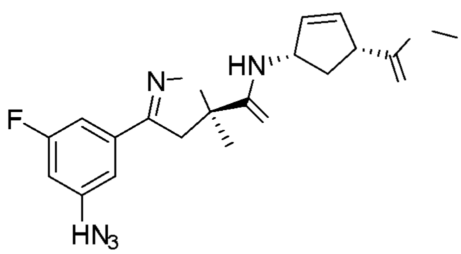

At room temperature, (1*S*,4*R*)-methyl 4-aminocyclopent-2-enecarboxylate hydrochloride (183 mg, 1.03 mmol), triethylamine (244 mg, 2.41 mmol) and dichloromethane (5 mL) were added to a 25 mL single-necked flask. The reaction

mixture was stirred thoroughly for 20 min under a condition of room temperature, and cooled to 0 °C. The intermediate **I-535.1** was added dropwise, returning the mixture to room temperature while stirring for 30 min. After the reaction was completed, the mixture was distilled under reduced pressure to remove solvent, and purified by reversed-phase HPLC to give compound **I-535** (oily solid, 200 mg). $^1$H-NMR (400 MHz, $CDCl_3$) δ 7.67 (s, 1H), 7.60-7.54 (m, 1H), 7.41-7.36 (m, 1H), 7.21 (d, *J*=8.9 Hz, 1H), 5.97-5.92 (m, 1H), 5.84-5.77 (m, 1H), 5.06-4.97 (m, 1H), 3.84 (d, *J*=17.3 Hz, 1H), 3.76 (s, 3H), 3.58-3.50 (m, 1H), 3.22 (d, *J*=17.3 Hz, 1H), 2.59-2.48 (m, 1H), 2.00 1.92 (m, 1H), 1.75 (s, 3H).

The preparation was carried out analogously to the preparation examples given above, the analytical data were shown in Table C and Table D.

Table C

| NO. | $^1$H NMR |
|---|---|
| **I-1** | $^1$H NMR (400 MHz, DMSO-$d_6$) δ 12.09 (s, 1H), 8.50 (d, *J* = 7.7 Hz, 1H), 7.40 (dd, *J* = 14.2, 4.8 Hz, 3H), 6.13 (dd, *J* = 17.3, 10.7 Hz, 1H), 5.34 (dd, *J* = 31.0, 14.0 Hz, 2H), 4.22 - 4.06 (m, 1H), 3.87 (d, *J* = 17.8 Hz, 1H), 3.52 (d, *J* = 17.8 Hz, 1H), 2.80 - 2.64 (m, 1H), 2.42 - 2.29 (m, 2H), 2.27 - 2.20 (m, 2H). |
| **I-3** | $^1$H NMR (400 MHz, DMSO) δ 8.54 (d, *J* = 7.8 Hz, 1H), 7.40 (dd, *J* = 14.3, 4.8 Hz, 3H), 6.13 (dd, *J* = 17.3, 10.7 Hz, 1H), 5.33 (dd, *J* = 30.8, 14.0 Hz, 2H), 4.23 - 4.11 (m, 1H), 3.87 (d, *J* = 17.7 Hz, 1H), 3.59 (s, 3H), 3.52 (d, *J* = 17.8 Hz, 1H), 2.87 - 2.75 (m, 1H), 2.44 - 2.31 (m, 2H), 2.31 - 2.21 (m, 2H). |
| **I-4** | $^1$H NMR (400 MHz, DMSO) δ 8.54 (d, *J* = 7.8 Hz, 1H), 7.40 (dd, *J* = 14.3, 4.8 Hz, 3H), 6.13 (dd, *J* = 17.3, 10.7 Hz, 1H), 5.33 (dd, *J* = 30.8, 14.0 Hz, 2H), 4.26 - 4.09 (m, 1H), 3.87 (d, *J* = 17.7 Hz, 1H), 3.59 (s, 3H), 3.52 (d, *J* = 17.8 Hz, 1H), 2.87 - 2.76 (m, 1H), 2.43 - 2.32 (m, 2H), 2.32 - 2.19 (m, 2H). |
| **I-5** | $^1$H NMR (400 MHz, $CDCl_3$) δ 7.23 - 7.15 (m, 2H), 7.10 (d, *J* = 8.1 Hz, 1H), 6.89 (ddd, *J* = 8.7, 5.4, 2.1 Hz, 1H), 6.16 (dd, *J* = 17.2, 10.7 Hz, 1H), 5.52 (d, *J* = 17.2 Hz, 1H), 5.33 (d, *J* = 10.7 Hz, 1H), 4.46 - 4.29 (m, 1H), 4.14 (q, *J* = 7.1 Hz, 2H), 3.91 (d, *J* = 17.3 Hz, 1H), 3.32 (d, *J* = 17.3 Hz, 1H), 2.91 - 2.75 (m, 1H), 2.62 (ttd, *J* = 15.5, 7.7, 4.8 Hz, 2H), 2.20 (qd, *J* = *9.7,* 5.9 Hz, 2H), 1.26 (t, *J*= 7.1 Hz, 3H). |
| **I-6** | $^1$H NMR (400 MHz, $CDCl_3$) δ 7.21 - 7.11 (m, 2H), 6.98 (d, J = 8.0 Hz, 1H), 6.89 (tt, J = 8.7, 2.3 Hz, 1H), 6.15 (dd, J = 17.3, 10.7 Hz, 1H), 5.53 (d, J = 17.2 Hz, 1H), 5.34 (d, J = 10.7 Hz, 1H), 4.40 - 4.26 (m, 1H), 4.09 (t, J = 6.7 Hz, 2H), 3.89 (d, J = 17.2 Hz, 1H), 3.30 (d, J = 17.2 Hz, 1H), 2.89 - 2.75 (m, 1H), 2.69 - 2.53 (m, 2H), 2.24 - 2.10 (m, 2H), 1.65 - 1.59 (m, 2H), 1.37 (dq, J = 14.7, 7.4 Hz, 2H), 0.93 (t, J = 7.4 Hz, 3H). |
| **I-7** | $^1$H NMR (400 MHz, $CDCl_3$) δ 7.23 - 7.15 (m, 2H), 7.12 (d, *J* = 8.2 Hz, 1H), 7.00 - 6.81 (m, 1H), 6.16 (dd, *J* = 17.2, 10.7 Hz, 1H), 5.52 (d, *J* = 17.2 Hz, 1H), 5.33 (d, *J* = 10.7 Hz, 1H), 4.43 - 4.28 (m, 1H), 4.14 - 4.00 (m, 2H), 3.97 - 3.84 (m, 1H), 3.32 (d, *J* = 17.3 Hz, 1H), 2.90 - 2.75 (m, 1H), 2.62 (ttd, *J* = 15.4, 7.7, 4.8 Hz, 2H), 2.20 (ddd, *J* = 20.1, 9.7, 5.4 Hz, 2H), 1.70 - 1.55 (m, 2H), 1.46 - 1.17 (m, 8H), 0.88 (t, *J* = 6.7 Hz, 3H). |
| **I-8** | $^1$H NMR (400 MHz, DMSO-$d_6$) δ 8.55 (d, *J* = 7.7 Hz, 1H), 7.48 - 7.36 (m, 3H), 6.12 (dd, *J* = 17.3, 10.7 Hz, 1H), 5.33 (dd, *J* = 29.7, 13.9 Hz, 2H), 4.24 - 4.09 (m, 3H), 3.87 (d, *J* = 17.8 Hz, 1H), 3.52 (d, *J* = 17.8 Hz, 1H), 2.91 - 2.75 (m, 1H), 2.69 (t, *J* = 6.6 Hz, 2H), 2.45 - 2.19 (m, 4H), 2.09 (s, 3H). |
| **I-9** | $^1$H NMR (400 MHz, DMSO-$d_6$) δ 8.55 (d, *J* = 7.7 Hz, 1H), 7.54 - 7.31 (m, 3H), 6.12 (dd, *J* = 17.3, 10.7 Hz, 1H), 5.41 - 5.25 (m, 2H), 4.45 - 4.37 (m, 1H), 4.37 - 4.27 (m, 1H), 4.24 - 4.11 (m, 1H), 3.87 (d, *J* = 17.7 Hz, 1H), 3.52 (d, *J* = 17.8 Hz, 1H), 3.11 (ddd, *J* = 13.7, 8.7, 5.0 Hz, 1H), 2.95 (dt, *J* = 13.7, 4.8 Hz, 1H), 2.90 - 2.75 (m, 1H), 2.59 (s, 3H), 2.44 - 2.20 (m, 4H). |
| **I-10** | $^1$H NMR (400 MHz, DMSO-$d_6$) δ 8.53 (d, *J* = 7.7 Hz, 1H), 7.47 - 7.37 (m, 3H), 6.12 (dd, *J* = 17.3, 10.7 Hz, 1H), 5.33 (dd, *J* = 29.3, 14.0 Hz, 2H), 4.37 (t, *J* = 5.8 Hz, 2H), 4.23 - 4.12 (m, 1H), 3.87 (d, *J* = 17.8 Hz, 1H), 3.50 (dd, *J* = 15.5, 11.7 Hz, 3H), 3.01 (s, 3H), 2.93 - 2.79 (m, 1H), 2.44 - 2.20 (m, 4H). |
| **I-11** | $^1$H NMR (400 MHz, $CDCl_3$) δ 7.17 (tq, *J* = 7.1, 5.0, 3.6 Hz, 2H), 7.00 (d, *J* = 8.4 Hz, 1H), 6.89 (tt, *J* = 8.7, 2.4 Hz, 1H), 6.15 (dd, *J* = 17.3, 10.7 Hz, 1H), 5.53 (d, *J* = 17.2 Hz, 1H), 5.43 (qd, *J* = 6.7, 2.2 Hz, 1H), 5.34 (d, *J* = 10.7 Hz, 1H), 4.36 (h, *J* = 8.4 Hz, 1H), 3.89 (d, *J* = 17.2 Hz, 1H), 3.31 (d, *J* = 17.2 Hz, 1H), 2.98 - 2.77 (m, 1H), 2.75 - 2.56 (m, 2H), 2.46 (d, *J*= 2.2 Hz, 1H), 2.19 (q, *J*= 9.8 Hz, 2H), 1.63 (s, 1H), 1.51 (d, *J* = 6.6 Hz, 3H). |
| **I-12** | $^1$H NMR (400 MHz, $CDCl_3$) δ 7.21 - 7.12 (m, 2H), 6.99 (d, *J* = 8.4 Hz, 1H), 6.89 (tt, *J* = 8.7, 2.3 Hz, 1H), 6.15 (dd, *J* = 17.2, 10.7 Hz, 1H), 5.53 (d, *J* = 17.3 Hz, 1H), 5.43 (qd, *J* = 6.7, 2.1 Hz, 1H), 5.34 (d, *J* = 10.7 Hz, 1H), 4.43 - 4.28 (m, 1H), 3.89 (d, *J* = 17.3 Hz, 1H), 3.31 (d, *J* = 17.2 Hz, 1H), 2.85 *(tt, J* = 9.5, 7.9 Hz, 1H), 2.73 - 2.54 (m, 2H), 2.46 (d, *J* = 2.1 Hz, 1H), 2.19 (dq, *J* = 11.5, 9.3 Hz, 2H), 1.62 (s, 1H), 1.51 (d, *J* = 6.8 Hz, 3H). |

(continued)

| NO. | $^1H$ NMR |
|---|---|
| **I-13** | $^1H$ NMR (400 MHz, $CDCl_3$) $\delta$ 7.19 (t, *J* = 6.4 Hz, 2H), 7.13 (d, *J* = 8.2 Hz, 1H), 6.90 (ddd, *J* = 8.7, 5.4, 2.1 Hz, 1H), 6.15 (dd, *J* = 17.2, 10.7 Hz, 1H), 5.52 (d, *J* = 17.2 Hz, 1H), 5.33 (d, *J* = 10.7 Hz, 1H), 4.44 - 4.27 (m, 1H), 4.13 (dq, *J* = 9.4, 3.2 Hz, 1H), 3.91 (d, *J* = 17.3 Hz, 1H), 3.33 (d, *J* = 17.3 Hz, 1H), 2.86 - 2.71 (m, 1H), 2.60 (ttd, *J* = 15.5, 7.7, 4.9 Hz, 2H), 2.20 (ddd, *J* = 20.2, 9.6, 5.6 Hz, 2H), 0.81 - 0.58 (m, 4H). |
| **I-14** | $^1H$ NMR (400 MHz, $CDCl_3$) $\delta$ 7.17 (t, *J* = 6.3 Hz, 2H), 7.12 (d, *J* = 8.1 Hz, 1H), 6.89 (t, *J* = 8.7 Hz, 1H), 6.16 (dd, *J* = 17.2, 10.7 Hz, 1H), 5.52 (d, *J* = 17.2 Hz, 1H), 5.33 (d, *J* = 10.7 Hz, 1H), 4.48 - 4.26 (m, 1H), 4.06 (d, *J* = 6.8 Hz, 2H), 3.92 (d, *J* = 17.3 Hz, 1H), 3.33 (d, *J* = 17.3 Hz, 1H), 2.92 - 2.77 (m, 1H), 2.71 - 2.49 (m, 3H), 2.21 (qd, *J* = 9.6, 6.6 Hz, 2H), 2.12 - 1.99 (m, 2H), 2.00 - 1.82 (m, 2H), 1.82 - 1.67 (m, 2H). |
| **I-15** | $^1H$ NMR (400 MHz, $CDCl_3$) $\delta$ 7.23 - 7.14 (m, 2H), 7.10 (d, *J* = 7.7 Hz, 1H), 6.89 (t, *J* = 8.7 Hz, 1H), 6.15 (dd, *J* = 17.2, 10.7 Hz, 1H), 5.52 (d, *J* = 17.2 Hz, 1H), 5.34 (d, *J* = 10.7 Hz, 1H), 4.49 (q, *J* = 8.4 Hz, 2H), 4.39 (dt, *J* = 16.8, 8.5 Hz, 1H), 3.91 (d, *J* = 17.3 Hz, 1H), 3.33 (d, *J* = 17.3 Hz, 1H), 3.06 - 2.87 (m, 1H), 2.80 - 2.55 (m, 2H), 2.25 (qd, *J*= 9.9, 4.7 Hz, 2H). |
| **I-16** | $^1H$ NMR (400 MHz, $CDCl_3$) $\delta$ 7.21 - 7.13 (m, 2H), 7.08 (d, *J* = 8.1 Hz, 1H), 6.89 (tt, *J* = 8.7, 2.2 Hz, 1H), 6.22 - 5.90 (m, 2H), 5.52 (d, *J* = 17.2 Hz, 1H), 5.34 (d, *J* = 10.7 Hz, 1H), 4.61 (t, *J* = 13.7 Hz, 2H), 4.46 - 4.31 (m, 1H), 3.90 (d, *J* = 17.3 Hz, 1H), 3.32 (d, *J* = 17.3 Hz, 1H), 3.02 - 2.89 (m, 1H), 2.68 (ttd, *J* = 15.6, 7.8, 4.8 Hz, 2H), 2.31 - 2.17 (m, 2H). |
| **I-17** | $^1H$ NMR (400 MHz, $CDCl_3$) $\delta$ 7.18 (d, J = 5.9 Hz, 2H), 7.10 (d, J = 7.9 Hz, 1H), 6.89 (t, J = 8.6 Hz, 1H), 6.15 (dd, J = 17.2, 10.7 Hz, 1H), 5.52 (d, J = 17.2 Hz, 1H), 5.33 (d, J = 10.7 Hz, 1H), 4.45 - 4.30 (m, 1H), 4.18 (t, J = 6.4 Hz, 2H), 3.91 (d, J = 17.3 Hz, 1H), 3.44 (t, J = 6.1 Hz, 2H), 3.38 - 3.24 (m, 4H), 2.92 - 2.73 (m, 1H), 2.72 - 2.53 (m, 2H), 2.20 (td, J = 15.3, 9.7 Hz, 2H), 1.98 - 1.79 (m, 2H). |
| **I-18** | $^1H$ NMR (400 MHz, $CDCl_3$) $\delta$ 7.18 (d, *J* = 5.8 Hz, 2H), 7.11 (d, *J* = 7.8 Hz, 1H), 6.89 (t, *J* = 8.4 Hz, 1H), 6.15 (dd, *J* = 17.2, 10.7 Hz, 1H), 5.52 (d, *J* = 17.2 Hz, 1H), 5.33 (d, *J* = *10.7* Hz, 1H), 5.09 (dd, *J* = 10.2, 6.0 Hz, 1H), 4.43 - 4.26 (m, 1H), 3.91 (d, *J* = 17.3 Hz, 1H), 3.50 - 3.26 (m, 6H), 2.91 - 2.78 (m, 1H), 2.72 - 2.53 (m, 2H), 2.28 - 2.11 (m, 2H), 1.22 (d, *J* = 6.3 Hz, 3H). |
| **I-19** | $^1H$ NMR (400 MHz, $CDCl_3$) $\delta$ 7.24 - 7.11 (m, 2H), 7.02 (d, *J* = 8.2 Hz, 1H), 6.89 (tt, *J* = 8.7, 2.3 Hz, 1H), 6.15 (dd, *J* = 17.2, 10.7 Hz, 1H), 5.52 (d, *J* = 17.2 Hz, 1H), 5.33 (d, *J* = 10.7 Hz, 1H), 5.09 (pd, *J* = 6.4, 4.1 Hz, 1H), 4.42 - 4.27 (m, 1H), 3.89 (d, *J* = 17.2 Hz, 1H), 3.49 - 3.26 (m, 6H), 2.91 - 2.77 (m, 1H), 2.62 (ttd, *J* = 15.3, 7.7, 4.8 Hz, 2H), 2.17 (q, *J* = 9.5 Hz, 2H), 1.23 (d, *J* = 6.5 Hz, 3H). |
| **I-20** | $^1H$ NMR (400 MHz, $CDCl_3$) $\delta$ 7.21 - 7.13 (m, 2H), 7.03 (d, *J* = 8.3 Hz, 1H), 6.89 (tt, *J* = 8.7, 2.3 Hz, 1H), 6.15 (dd, *J* = 17.2, 10.7 Hz, 1H), 5.52 (d, *J* = 17.2 Hz, 1H), 5.34 (d, *J* = 10.7 Hz, 1H), 4.44 - 4.33 (m, 1H), 4.30 (t, *J* = 6.3 Hz, 2H), 3.90 (d, *J* = 17.2 Hz, 1H), 3.31 (d, *J* = 17.3 Hz, 1H), 2.90 (tt, *J* = 9.5, 7.9 Hz, 1H), 2.79 - 2.58 (m, 4H), 2.31 - 2.09 (m, 2H). |
| **I-21** | $^1H$ NMR (400 MHz, $CDCl_3$) $\delta$ 7.44 (d, *J* = 8.5 Hz, 1H), 7.16 (dt, *J* = 6.2, 2.1 Hz, 2H), 6.88 (tt, *J* = 8.7, 2.3 Hz, 1H), 6.15 (dd, *J* = 17.2, 10.7 Hz, 1H), 5.73 (s, 1H), 5.53 (t, *J* = 15.4 Hz, 2H), 5.33 (d, *J* = 10.7 Hz, 1H), 4.36 (hept, *J* = 8.1, 7.6 Hz, 1H), 3.89 (d, *J* = 17.2 Hz, 1H), 3.31 (d, *J* = 17.3 Hz, 1H), 2.77 - 2.65 (m, 1H), 2.65 - 2.49 (m, 2H), 2.42 - 2.25 (m, 2H). |
| **I-22** | $^1H$ NMR (400 MHz, $CDCl_3$) $\delta$ 9.01 (s, 1H), 7.50 (s, 1H), 7.16 (h, *J* = 4.9 Hz, 2H), 6.89 (tt, *J* = 8.7, 2.3 Hz, 1H), 6.14 (dd, *J* = 17.3, 10.7 Hz, 1H), 5.50 (d, *J*= 17.2 Hz, 1H), 5.33 (d, *J* = 10.6 Hz, 1H), 4.36 (d, *J* = 8.8 Hz, 1H), 3.90 (d, *J* = 17.3 Hz, 1H), 3.32 (d, *J* = 17.3 Hz, 1H), 2.62 (d, *J*= 38.5 Hz, 3H), 2.27 (d, *J* = 16.7 Hz, 2H). |
| **I-23** | $^1H$ NMR (400 MHz, $CDCl_3$) $\delta$ 8.30 (s, 1H), 7.24 - 7.07 (m, 3H), 6.93 - 6.83 (m, 1H), 6.14 (dd, *J*= 17.3, 10.7 Hz, 1H), 5.53 (d, *J* = 17.2 Hz, 1H), 5.33 (d, *J* = 10.7 Hz, 1H), 4.35 (s, 1H), 3.88 (d, *J* = 17.2 Hz, 1H), 3.83 - 3.64 (m, 3H), 3.30 (d, *J* = 17.2 Hz, 1H), 2.75 - 2.47 (m, 3H), 2.38 - 2.21 (m, 2H). |
| **I-24** | $^1H$ NMR (400 MHz, $CDCl_3$) $\delta$ 7.16 (ddd, *J*= 14.3, 7.6, 4.7 Hz, 3H), 6.88 (tt, *J* = 8.8, 2.4 Hz, 1H), 6.15 (dd, *J* = 17.2, 10.7 Hz, 1H), 5.53 (d, *J* = 17.2 Hz, 2H), 5.33 (d, *J* = 10.7 Hz, 1H), 4.43 - 4.23 (m, 1H), 3.88 (d, *J* = 17.2 Hz, 1H), 3.30 (d, *J* = 17.2 Hz, 1H), 2.82 (d, *J* = 4.8 Hz, 3H), 2.69 - 2.43 (m, 3H), 2.25 (ddp, *J* = 11.6, 8.9, 4.0, 3.6 Hz, 2H). |
| **I-25** | $^1H$ NMR (400 MHz, $CDCl_3$) $\delta$ 7.25 - 7.11 (m, 3H), 6.90 (tt, *J* = 8.6, 2.4 Hz, 1H), 6.15 (dd, *J* = 17.2, 10.7 Hz, 1H), 5.53 *(d, J*= 17.2 Hz, 1H), 5.35 (d, *J* = 10.6 Hz, 1H), 4.26 (h, *J* = 8.4 Hz, 1H), 3.92 (d, *J* = 17.3 Hz, 1H), 3.33 (d, *J* = 14.0 Hz, 4H), 2.86 (q, *J*= 8.8 Hz, 1H), 2.73 - 2.53 (m, 2H), 2.45 (q, *J* = 9.6 Hz, 2H). |

(continued)

| NO. | $^1$H NMR |
|---|---|
| **I-26** | $^1$H NMR (400 MHz, $CDCl_3$) δ 7.23 - 7.01 (m, 3H), 6.88 (tt, *J* = 8.7, 2.3 Hz, 1H), 6.14 (dd, *J* = 17.2, 10.7 Hz, 1H), 5.52 *(d, J* = 17.2 Hz, 1H), 5.33 (d, *J* = 10.7 Hz, 1H), 4.43 - 4.24 (m, 1H), 3.88 (d, *J* = 17.2 Hz, 1H), 3.45 (t, *J* = 6.8 Hz, 2H), 3.40 - 3.22 (m, 3H), 2.89 (tt, *J = 9.3,* 7.9 Hz, 1H), 2.69 - 2.47 (m, 2H), 2.34 - 2.17 (m, 2H), 1.97 - 1.82 (m, 4H). |
| **I-27** | $^1$H NMR (400 MHz, DMSO-$d_6$) δ 12.09 (s, 1H), 8.50 (d, *J*= 7.7 Hz, 1H), 7.57 (dd, *J* = 23.7, 9.9 Hz, 3H), 6.13 (dd, *J*= 17.2, 10.7 Hz, 1H), 5.34 (dd, *J* = 31.8, 14.0 Hz, 2H), 4.21 - 4.08 (m, 1H), 3.88 (d, *J* = 17.8 Hz, 1H), 3.54 (d, *J* = 17.8 Hz, 1H), 2.77 - 2.64 (m, 1H), 2.41 - 2.29 (m, 2H), 2.27 - 2.21 (m, 2H). |
| **I-29** | $^1$H NMR (400 MHz, DMSO-$d_6$) δ 8.53 (d, *J* = 7.7 Hz, 1H), 7.64 - 7.48 (m, 3H), 6.12 (dd, *J* = 17.3, 10.6 Hz, 1H), 5.33 (dd, *J* = 30.9, 14.0 Hz, 2H), 4.16 (dd, *J* = 16.4, 8.5 Hz, 1H), 3.87 (d, *J* = 17.7 Hz, 1H), 3.60 (d, *J* = 12.8 Hz, 3H), 3.53 (d, *J* = 17.8 Hz, 1H), 2.88 - 2.73 (m, 1H), 2.44 - 2.18 (m, 4H). |
| **I-31** | $^1$H NMR (400 MHz, $CDCl_3$) δ 7.41 (t, *J* = 1.6 Hz, 1H), 7.31 - 7.27 (m, 1H), 7.22 - 7.12 (m, 1H), 7.03 (d, *J* = 8.4 Hz, 1H), 6.15 (dd, *J* = 17.3, 10.7 Hz, 1H), 5.52 (d, *J* = 17.2 Hz, 1H), 5.33 (d, *J* = 10.7 Hz, 1H), 4.43 - 4.28 (m, 1H), 4.07 (t, *J* = 6.8 Hz, 2H), 3.90 *(d, J* = 17.2 Hz, 1H), 3.31 (d, *J* = 17.2 Hz, 1H), 2.89 - 2.76 (m, 1H), 2.72 - 2.54 (m, 2H), 2.27 - 2.10 (m, 2H), 1.69 - 1.56 (m, 2H), 1.40 - 1.23 (m, 4H), 0.99 - 0.85 (m, 3H). |
| **I-32** | $^1$H NMR (400 MHz, $CDCl_3$) δ 7.41 (d, *J* = 1.6 Hz, 1H), 7.27 (s, 1H), 7.19 - 7.13 (m, 1H), 7.02 (d, *J* = 8.3 Hz, 1H), 6.14 (dd, *J* = 17.2, 10.7 Hz, 1H), 5.84 - 5.73 (m, 1H), 5.65 - 5.55 (m, 1H), 5.52 (d, *J* = 17.2 Hz, 1H), 5.33 (d, *J* = 10.7 Hz, 1H), 4.55 - 4.48 (m, 2H), 4.40 - 4.28 (m, 1H), 3.90 (d, *J* = 17.2 Hz, 1H), 3.30 (d, *J* = 17.3 Hz, 1H), 2.89 - 2.75 (m, 1H), 2.72 - 2.55 (m, 2H), 2.25 - 2.12 (m, 2H), 1.73 (dq, *J* = 6.6, 1.3 Hz, 3H). |
| **I-33** | $^1$H NMR (400 MHz, $CDCl_3$) δ 7.41 (d, *J* = 1.6 Hz, 1H), 7.32 - 7.26 (m, 1H), 7.23 - 7.12 (m, 1H), 7.06 (d, *J* = 8.4 Hz, 1H), 6.15 (dd, *J* = 17.3, 10.7 Hz, 1H), 5.52 (d, *J* = 17.2 Hz, 1H), 5.33 (d, *J* = 10.7 Hz, 1H), 5.15 - 5.01 (m, 1H), 4.46 - 4.27 (m, 1H), 3.90 (d, *J* = 17.3 Hz, 1H), 3.52 - 3.24 (m, 6H), 2.90 - 2.77 (m, 1H), 2.71 - 2.52 (m, 2H), 2.28 - 2.09 (m, 2H), 1.23 (d, *J* = 6.5 Hz, 3H). |
| **I-34** | $^1$H NMR (400 MHz, $CDCl_3$) δ 7.41 (t, *J* = 1.6 Hz, 1H), 7.30 - 7.27 (m, 1H), 7.19 - 7.13 (m, 1H), 7.00 (d, *J* = 8.3 Hz, 1H), 6.14 (dd, *J* = 17.2, 10.7 Hz, 1H), 5.52 (d, *J* = 17.2 Hz, 1H), 5.37 - 5.30 (m, 1H), 5.14 - 5.04 (m, 1H), 4.42 - 4.28 (m, 1H), 3.90 (d, *J* = 17.2 Hz, 1H), 3.49 - 3.38 (m, 2H), 3.36 (s, 3H), 3.30 (d, *J* = 17.2 Hz, 1H), 2.91 - 2.77 (m, 1H), 2.71 - 2.55 (m, 2H), 2.25 - 2.08 (m, 2H), 1.22 (d, *J* = 6.5 Hz, 3H). |
| **I-35** | $^1$H NMR (400 MHz, $CDCl_3$) δ 7.41 (s, 1H), 7.33 - 7.23 (m, 1H), 7.20 - 7.13 (m, 1H), 7.08 (d, *J* = 8.4 Hz, 1H), 6.15 (dd, *J* = 17.2, 10.7 Hz, 1H), 5.52 (d, *J* = 17.2 Hz, 1H), 5.33 (d, *J* = 10.7 Hz, 1H), 4.42 - 4.28 (m, 1H), 3.98 - 3.84 (m, 3H), 3.31 (d, *J* = 17.3 Hz, 1H), 2.92 - 2.78 (m, 1H), 2.72 - 2.54 (m, 2H), 2.30 - 2.12 (m, 2H), 1.21 - 1.02 (m, 1H), 0.66 - 0.50 (m, 2H), 0.28 (t, *J*= 5.2 Hz, 2H). |
| **I-36** | $^1$H NMR (400 MHz, DMSO) δ 10.67 (s, 1H), 8.50 (d, *J*= 7.6 Hz, 1H), 7.63 - 7.50 (m, 3H), 6.20 - 6.06 (m, 1H), 5.43 - 5.34 (m, 1H), 5.34 - 5.27 (m, 1H), 4.20 - 4.06 (m, 1H), 3.99 - 3.91 (m, 1H), 3.88 (d, *J* = 17.6 Hz, 1H), 3.54 (d, *J* = 17.7 Hz, 1H), 2.58 - 2.46 (m, 1H), 2.33 - 2.12 (m, 4H), 1.11 (d, *J* = 6.1 Hz, 6H). |
| **I-37** | $^1$H NMR (400 MHz, DMSO-$d_6$) δ 12.08 (s, 1H), 8.48 (d, *J* = 7.7 Hz, 1H), 7.74 (d, *J* = 1.9 Hz, 1H), 7.72 (d, *J* = 1.9 Hz, 2H), 6.12 (dd, *J* = 17.3, 10.6 Hz, 1H), 5.37 (d, *J* = 17.2 Hz, 1H), 5.29 (d, *J* = 10.7 Hz, 1H), 4.20 - 4.07 (m, 1H), 3.88 (d, *J* = 17.8 Hz, 1H), 3.55 (d, *J* = 17.7 Hz, 1H), 2.76 - 2.65 (m, 1H), 2.37 - 2.28 (m, 2H), 2.28 - 2.16 (m, 2H). |
| **I-39** | $^1$H NMR (400 MHz, DMSO-$d_6$) δ 8.53 (d, *J* = 7.8 Hz, 1H), 7.76 (t, *J* = 1.9 Hz, 1H), 7.72 (d, *J* = 1.9 Hz, 2H), 6.12 (dd, *J* = 17.2, 10.7 Hz, 1H), 5.38 (dd, *J* = 17.2, 1.0 Hz, 1H), 5.30 (dd, *J* = 10.6, 1.0 Hz, 1H), 4.25 - 4.09 (m, 1H), 3.88 (d, *J* = 17.8 Hz, 1H), 3.60 (s, 3H), 3.55 *(d, J*= 17.8 Hz, 1H), 2.87 - 2.76 (m, 1H), 2.43 - 2.32 (m, 2H), 2.30 - 2.20 (m, 2H). |
| **I-41** | $^1$H NMR (400 MHz, DMSO-$d_6$) δ 12.14 (s, 1H), 8.57 (d, *J* = 7.7 Hz, 1H), 8.09 - 7.90 (m, 3H), 6.18 (dd, *J* = 17.3, 10.7 Hz, 1H), 5.49 - 5.32 (m, 2H), 4.20 (m, 1H), 3.97 (d, *J* = 17.8 Hz, 1H), 3.60 (d, *J* = 17.8 Hz, 1H), 2.83 - 2.70 (m, 1H), 2.46 - 2.19 (m, 4H). |
| **I-43** | $^1$H NMR (400 MHz, Chloroform-d) δ 7.71 (t, *J* = 1.6 Hz, 1H), 7.63 (m, 1H), 7.43 (m, 1H), 6.97 (d, *J* = 8.3 Hz, 1H), 6.15 (m, 1H), 5.53 (dd, *J* = 17.3, 1.6 Hz, 1H), 5.36 (dd, *J* = 10.7, 1.6 Hz, 1H), 4.44 - 4.29 (m, 1H), 3.93 (dd, *J* = 17.2, 1.6 Hz, 1H), 3.70 (d, *J* = 1.6 Hz, 3H), 3.32 (dd, *J* = 17.3, 1.6 Hz, 1H), 2.85 (m, 1H), 2.72 - 2.55 (m, 2H), 2.26 - 2.14 (m, 2H). |

(continued)

| NO. | $^1H$ NMR |
|---|---|
| **I-47** | $^1H$ NMR (400 MHz, DMSO-$d_6$) δ 8.53 (d, *J*= 7.8 Hz, 1H), 8.18 - 8.15 (m, 1H), 8.11 (t, *J* = 1.3 Hz, 1H), 8.08 - 8.03 (m, 1H), 6.12 (*dd, J* = 17.3, 10.7 Hz, 1H), 5.42 - 5.26 (m, 2H), 4.22 - 4.10 (m, 1H), 3.92 (d, *J*= 17.8 Hz, 1H), 3.59 (s, 3H), 3.54 (d, *J* = 17.8 Hz, 1H), 2.91 - 2.74 (m, 1H), 2.43 - 2.31 (m, 2H), 2.31 - 2.21 (m, 2H). |
| **I-49** | $^1H$ NMR (400 MHz, Chloroform-d) δ 7.68 (s, 1H), 7.59 (dt, *J* = 8.9, 2.0 Hz, 1H), 7.49 - 7.35 (m, 2H), 6.17 (dd, *J* = 17.2, 10.7 Hz, 1H), 5.53 (d, *J* = 17.2 Hz, 1H), 5.35 (d, *J* = 10.7 Hz, 1H), 4.46 - 4.33 (m, 1H), 4.02 (d, *J* = 4.0 Hz, 1H), 3.41 (d, *J* = 17.4 Hz, 1H), 2.96 - 2.82 (m, 1H), 2.74 - 2.56 (m, 2H), 2.36 - 2.22 (m, 2H). |
| **I-51** | $^1H$ NMR (400 MHz, DMSO-$d_6$) δ 8.54 (d, *J* = 7.8 Hz, 1H), 7.94 - 7.75 (m, 3H), 6.13 (dd, *J*= 17.2, 10.7 Hz, 1H), 5.43 - 5.26 (m, 2H), 4.25 - 4.08 (m, 1H), 3.94 (d, *J* = 17.8 Hz, 1H), 3.61 (d, *J* = 13.8 Hz, 4H), 2.82 (tt, *J* = 10.0, 8.0 Hz, 1H), 2.43 - 2.20 (m, 4H). |
| **I-55** | $^1H$ NMR (400 MHz, Chloroform-d) δ 7.80 (d, *J*= 19.0 Hz, 2H), 7.66 (s, 1H), 7.10 (d, *J = 8.3* Hz, 1H), 6.16 (dd, *J* = 17.2, 10.7 Hz, 1H), 5.53 (d, *J* = 17.2 Hz, 1H), 5.34 (d, *J*= 10.7 Hz, 1H), 4.37 (h, *J*= 8.3 Hz, 1H), 3.98 (d, *J*= 17.3 Hz, 1H), 3.69 (s, 3H), 3.36 (d, *J = 17.3* Hz, 1H), 2.92 - 2.78 (m, 1H), 2.71 - 2.53 (m, 2H), 2.31 - 2.14 (m, 2H). |
| **I-57** | $^1H$ NMR (400 MHz, DMSO-$d_6$) δ 12.15 (s, 1H), 8.56 (d, *J* = 7.7 Hz, 1H), 7.70 - 7.55 (m, 2H), 6.18 (dd, *J*= 17.2, 10.7 Hz, 1H), 5.50 - 5.30 (m, 2H), 4.20 (h, *J* = 8.3 Hz, 1H), 3.95 (d, *J* = 17.7 Hz, 1H), 3.60 (d, *J* = 17.8 Hz, 1H), 2.86 - 2.70 (m, 1H), 2.33 (m, 4H). |
| **I-59** | $^1H$ NMR (400 MHz, Chloroform-d) δ 7.31 (m, 2H), 7.08 - 6.94 (m, 2H), 6.15 (dd, *J* = 17.3, 10.7 Hz, 1H), 5.53 (d, *J* = 17.2 Hz, 1H), 5.34 (d, *J* = 10.7 Hz, 1H), 4.45 - 4.27 (m, 1H), 3.91 (d, *J* = 17.2 Hz, 1H), 3.69 (s, 3H), 3.31 (d, *J* = 17.2 Hz, 1H), 2.84 (m, 1H), 2.71 - 2.55 (m, 2H), 2.24 - 2.13 (m, 2H) |
| **I-61** | 1H NMR (400 MHz, $CDCl_3$) δ 7.33 (dd, J = 8.1, 1.9 Hz, 2H), 7.06 (dd, J = 20.9, 8.4 Hz, 2H), 6.15 (dd, J = 17.2, 10.7 Hz, 1H), 5.52 (d, J = 17.2 Hz, 1H), 5.34 (d, J = 10.7 Hz, 1H), 4.66 (dd, J = 4.6, 2.3 Hz, 2H), 4.46 - 4.29 (m, 1H), 3.93 (d, J = 17.3 Hz, 1H), 3.33 (d, J = 17.3 Hz, 1H), 2.96 - 2.79 (m, 1H), 2.64 (ttd, J = 15.6, 7.7, 4.9 Hz, 2H), 2.23 (ddd, J = 20.4, 9.8, 5.3 Hz, 2H), 1.86 (t, J = 2.3 Hz, 3H). |
| **I-62** | 1H NMR (400 MHz, $CDCl_3$) δ 7.31 (dd, J = 8.0, 2.0 Hz, 2H), 7.01 (dd, J = 19.2, 8.4 Hz, 2H), 6.15 (dd, J = 17.2, 10.7 Hz, 1H), 5.53 (d, J = 17.2 Hz, 1H), 5.34 (d, J = 10.7 Hz, 1H), 5.08 - 4.88 (m, 1H), 4.42 - 4.24 (m, 1H), 3.91 (d, J = 17.3 Hz, 1H), 3.31 (d, J = 17.3 Hz, 1H), 2.85 - 2.73 (m, 1H), 2.69 - 2.53 (m, 2H), 2.40 - 2.28 (m, 2H), 2.22 - 1.96 (m, 4H), 1.86 - 1.74 (m, 1H), 1.70 - 1.54 (m, 1H). |
| **I-63** | 1H NMR (400 MHz, $CDCl_3$) δ 7.32 (d, J = 9.0 Hz, 2H), 7.04 (t, J = 7.5 Hz, 2H), 6.15 (dd, J = 17.2, 10.7 Hz, 1H), 5.53 (d, J = 17.2 Hz, 1H), 5.34 (d, J = 10.7 Hz, 1H), 4.49 - 4.24 (m, 1H), 3.91 (t, J = 11.7 Hz, 3H), 3.33 (d, J = 17.3 Hz, 1H), 2.90 - 2.77 (m, 1H), 2.72 - 2.53 (m, 2H), 2.26 - 2.11 (m, 2H), 1.76 - 1.69 (m, 4H), 1.36 - 0.80 (m, 7H). |
| **I-64** | 1H NMR (400 MHz, $CDCl_3$) δ 7.37 - 7.29 (m, 2H), 7.06 (dd, J = 18.8, 8.2 Hz, 2H), 6.15 (dd, J = 17.2, 10.7 Hz, 1H), 5.87 (tt, J = 53.1, 3.6 Hz, 1H), 5.52 (d, J = 17.2 Hz, 1H), 5.34 (d, J = 10.7 Hz, 1H), 4.50 (t, J = 13.0 Hz, 2H), 4.45 - 4.29 (m, 1H), 3.93 (d, J = 17.3 Hz, 1H), 3.34 (d, J = 17.3 Hz, 1H), 3.00 - 2.84 (m, 1H), 2.78 - 2.53 (m, 2H), 2.36 - 2.11 (m, 2H). |
| **I-65** | 1H NMR (400 MHz, $CDCl_3$) δ 7.24 (dd, J = 8.0, 1.6 Hz, 2H), 6.97 (t, J = 7.8 Hz, 2H), 6.08 (dd, J = 17.2, 10.7 Hz, 1H), 5.45 (d, J = 17.2 Hz, 1H), 5.25 (t, J = 8.4 Hz, 1H), 4.29 (dp, J = 16.7, 8.4 Hz, 1H), 4.21 - 4.09 (m, 2H), 3.84 (d, J = 17.3 Hz, 1H), 3.62 - 3.51 (m, 2H), 3.50 - 3.39 (m, 2H), 3.25 (d, J = 17.3 Hz, 1H), 2.89 - 2.75 (m, 1H), 2.56 (ttd, J = 15.6, 7.7, 4.8 Hz, 2H), 2.13 (ddd, J = 20.1, 9.6, 4.9 Hz, 2H), 1.14 (t, J = 7.0 Hz, 3H). |
| **I-66** | 1H NMR (400 MHz, $CDCl_3$) δ 7.32 (d, J = 10.6 Hz, 2H), 7.06 (dd, J = 21.8, 8.3 Hz, 2H), 6.15 (dd, J = 17.2, 10.7 Hz, 1H), 5.53 (d, J = 17.2 Hz, 1H), 5.33 (d, J = 10.7 Hz, 1H), 4.45 - 4.31 (m, 1H), 3.92 (d, J = 17.2 Hz, 1H), 3.66 (s, 3H), 3.32 (d, J = 17.2 Hz, 1H), 3.27 - 3.14 (m, 4H), 2.57 (ttd, J = 15.4, 7.7, 4.8 Hz, 2H), 2.23 (qd, J = 9.9, 3.7 Hz, 2H). |
| **I-69** | $^1H$ NMR (400 MHz, DMSO-$d_6$) δ 8.53 (d, *J* = 7.8 Hz, 1H), 7.88 - 7.56 (m, 3H), 6.12 (dd, *J* = 17.2, 10.6 Hz, 1H), 5.37 (d, *J* = 17.3 Hz, 1H), 5.29 (d, *J* = 10.7 Hz, 1H), 4.26 - 4.09 (m, 1H), 3.90 (d, *J* = 17.8 Hz, 1H), 3.59 (s, 3H), 3.55 (d, *J* = 17.8 Hz, 1H), 2.87 - 2.76 (m, 1H), 2.36 (dt, *J*= 13.0, 6.5 Hz, 2H), 2.25 (ddd, *J* = 19.9, 10.1, 4.1 Hz, 2H). |

(continued)

| NO. | $^1$H NMR |
|---|---|
| **I-71** | $^1$H NMR (400 MHz, DMSO-$d_6$) $\delta$ 12.15 (s, 1H), 8.56 (d, *J* = 7.7 Hz, 1H), 7.47 (m, 1H), 7.40 (t, *J* = 73.2 Hz, 1H), 7.33 (m, 1H), 6.18 (m, 1H), 5.49 - 5.30 (m, 2H), 4.20 (m, 1H), 3.93 (d, *J* = 17.7 Hz, 1H), 3.58 (d, *J* = 17.7 Hz, 1H), 2.83 - 2.69 (m, 1H), 2.46 - 2.21 (m, 4H). |
| **I-73** | $^1$H NMR (400 MHz, Chloroform-d) $\delta$ 7.25 - 7.19 (m, 2H), 7.05 - 6.91 (m, 2H), 6.54 (t, *J* = 72.6 Hz, 1H), 6.15 (dd, *J* = 17.2, 10.7 Hz, 1H), 5.53 (d, *J* = 17.2 Hz, 1H), 5.34 (d, *J* = 10.7 Hz, 1H), 4.42 - 4.28 (m, 1H), 3.90 (d, *J* = 17.3 Hz, 1H), 3.69 (s, 3H), 3.31 (d, *J* = 17.2 Hz, 1H), 2.84 (m, 1H), 2.70 - 2.56 (m, 2H), 2.24 - 2.12 (m, 2H). |
| **I-75** | $^1$H NMR (400 MHz, DMSO-$d_6$) $\delta$ 12.11 (s, 1H), 8.49 (d, *J* = 7.7 Hz, 1H), 7.61 (t, *J* = 1.6 Hz, 1H), 7.45 (dt, *J* = 8.2, 2.0 Hz, 2H), 7.38 (t, *J* = 7.2 Hz, 1H), 6.13 (dd, *J* = 17.3, 10.6 Hz, 1H), 5.43 - 5.23 (m, 2H), 4.21 - 4.08 (m, 1H), 3.89 (d, *J* = 17.7 Hz, 1H), 3.54 (d, *J*= 17.7 Hz, 1H), 2.76 - 2.66 (m, 1H), 2.41 - 2.17 (m, 4H). |
| **I-77** | $^1$H NMR (400 MHz, DMSO-$d_6$) $\delta$ 8.54 (d, *J*= 7.8 Hz, 1H), 7.62 (d, *J*= 1.8 Hz, 1H), 7.46 (d, *J* = 1.6 Hz, 2H), 7.39 (t, *J* = 7.2 Hz, 1H), 6.21 - 6.06 (m, 1H), 5.44 - 5.23 (m, 2H), 4.23 - 4.10 (m, 1H), 3.88 (d, *J* = 17.8 Hz, 1H), 3.62 - 3.57 (m, 3H), 3.54 (d, *J* = 17.8 Hz, 1H), 2.88 - 2.76 (m, 1H), 2.43 - 2.20 (m, 4H). |
| **I-79** | $^1$H NMR (400 MHz, Chloroform-d) $\delta$ 9.67 (s, 1H), 7.75 (d, *J* = 8.7 Hz, 1H), 7.36 (td, *J* = 10.7, 9.4, 5.6 Hz, 3H), 7.21 - 7.06 (m, 1H), 6.19 (ddd, *J* = 17.3, 10.8, 1.2 Hz, 1H), 5.53 (d, *J* = 17.2 Hz, 1H), 5.33 (d, *J* = 10.7 Hz, 1H), 4.50 - 4.35 (m, 1H), 4.04 - 3.89 (m, 1H), 3.45 - 3.31 (m, 1H), 2.96 - 2.77 (m, 1H), 2.73 - 2.51 (m, 2H), 2.46 - 2.23 (m, 2H). |
| **I-81** | $^1$H NMR (400 MHz, Chloroform-d) $\delta$ 7.44 - 7.32 (m, 3H), 7.18 - 7.09 (m, 1H), 7.04 (d, *J* = 8.4 Hz, 1H), 6.16 (dd, *J* = 17.2, 10.7 Hz, 1H), 5.53 (d, *J* = 17.3 Hz, 1H), 5.32 (d, *J* = 10.7 Hz, 1H), 4.42 - 4.26 (m, 1H), 3.92 (d, *J* = 17.2 Hz, 1H), 3.69 (s, 3H), 3.34 (d, *J* = 17.2 Hz, 1H), 2.83 (tt, *J* = 9.5, 7.9 Hz, 1H), 2.71 - 2.53 (m, 2H), 2.27 - 2.10 (m, 2H). |
| **I-85** | $^1$H NMR (400 MHz, $CDCl_3$-*d*) $\delta$ 7.23 - 7.14 (m, 2H), 7.02 (d, *J* = 8.0 Hz, 1H), 6.95 (d, *J* = *9.2* Hz, 1H), 6.15 (dd, *J* = 17.2, 10.7 Hz, 1H), 5.52 (d, *J* = 17.3 Hz, 1H), 5.32 (d, *J* = 10.8 Hz, 1H), 4.42 - 4.26 (m, 1H), 3.90 (d, *J* = 17.3 Hz, 1H), 3.69 (s, 3H), 3.32 *(d, J* = 17.2 Hz, 1H), 2.88 - 2.76 (m, 1H), 2.70 - 2.53 (m, 2H), 2.37 (s, 3H), 2.26 - 2.11 (m, 2H). |
| **I-89** | $^1$H NMR (400 MHz, DMSO-$d_6$) $\delta$ 8.54 (d, *J* = 7.7 Hz, 1H), 7.66 (dd, *J* = 8.3, 7.0 Hz, 2H), 6.12 (dd, *J* = 17.3, 10.6 Hz, 1H), 5.44 - 5.24 (m, 2H), 4.25 - 4.11 (m, 1H), 3.88 (d, *J* = 17.8 Hz, 1H), 3.59 (s, 3H), 3.50 (d, *J* = 17.8 Hz, 1H), 2.90 - 2.74 (m, 1H), 2.43 - 2.19 (m, 4H). |
| **I-93** | $^1$H NMR (400 MHz, DMSO-$d_6$) $\delta$ 8.54 (d, *J* = 7.8 Hz, 1H), 7.84 - 7.71 (m, 2H), 6.12 (dd, *J* = 17.3, 10.7 Hz, 1H), 5.33 (ddd, *J* = 14.0, 11.3, 0.7 Hz, 2H), 4.22 - 4.10 (m, 1H), 3.88 (d, *J* = 17.8 Hz, 1H), 3.59 (s, 3H), 3.52 (d, *J* = 17.8 Hz, 1H), 2.89 - 2.76 (m, 1H), 2.44 - 2.18 (m, 4H). |
| **I-97** | $^1$H NMR (400 MHz, DMSO-$d_6$) $\delta$ 8.53 (d, *J* = 7.8 Hz, 1H), 7.89 (d, *J* = 6.4 Hz, 2H), 6.11 (dd, *J* = 17.2, 10.6 Hz, 1H), 5.33 (dd, *J* = 30.0, 14.0 Hz, 2H), 4.22 - 4.09 (m, 1H), 3.89 (d, *J* = 17.8 Hz, 1H), 3.59 (s, 3H), 3.54 (d, *J* = 17.8 Hz, 1H), 2.89 - 2.75 (m, 1H), 2.44 - 2.32 (m, 2H), 2.25 (ddd, *J* = 19.7, 10.0, 4.9 Hz, 2H). |
| **I-101** | $^1$H NMR (400 MHz, DMSO-$d_6$) $\delta$ 8.53 (d, *J* = 7.8 Hz, 1H), 7.75 - 7.62 (m, 2H), 7.47 (d, *J* = *6.5* Hz, 3H), 6.22 - 6.06 (m, 1H), 5.38 (d, *J*= 17.3 Hz, 1H), 5.28 (d, *J* = 10.6 Hz, 1H), 4.23 - 4.11 (m, 1H), 3.85 (d, *J* = 17.5 Hz, 1H), 3.59 (s, 3H), 3.50 (d, *J*= 17.5 Hz, 1H), 2.87 - 2.76 (m, 1H), 2.43 - 2.17 (m, 4H). |
| **I-105** | $^1$H NMR (400 MHz, $CDCl_3$-*d*) $\delta$ 7.26 - 7.21 (m, 2H), 7.07 (s, 2H), 6.17 (dd, *J* = 17.2, 10.7 Hz, 1H), 5.52 (d, *J* = 17.3 Hz, 1H), 5.30 (d, *J* = 3.5 Hz, 1H), 4.44 - 4.24 (m, 1H), 3.91 (d, *J* = 17.2 Hz, 1H), 3.69 (s, 3H), 3.34 (d, *J* = 17.2 Hz, 1H), 2.89 - 2.75 (m, 1H), 2.69 - 2.53 (m, 2H), 2.33 (s, 6H), 2.24 - 2.10 (m, 2H). |
| **I-109** | $^1$H NMR (400 MHz, DMSO-$d_6$) $\delta$ 8.51 (d, *J* = 7.8 Hz, 1H), 6.82 (d, *J* = 2.1 Hz, 2H), 6.60 (t, *J* = 2.0 Hz, 1H), 6.18 - 6.06 (m, 1H), 5.36 (d, *J* = 17.3 Hz, 1H), 5.27 (d, *J* = 10.6 Hz, 1H), 4.24 - 4.12 (m, 1H), 3.82 (d, *J* = 17.6 Hz, 1H), 3.77 (s, 6H), 3.59 (s, 3H), 3.49 (d, *J* = 17.6 Hz, 1H), 2.87 - 2.74 (m, 1H), 2.40 - 2.21 (m, 4H). |
| **I-111** | $^1$H NMR (400 MHz, DMSO-$d_6$) $\delta$ 12.08 (s, 1H), 8.40 (d, *J* = 7.8 Hz, 1H), 7.39 (dd, *J* = 8.1, 5.1 Hz, 3H), 4.22 - 4.08 (m, 1H), 3.75 (d, *J* = 17.7 Hz, 1H), 3.33 (d, *J* = 17.7 Hz, 1H), 2.77 - 2.64 (m, 1H), 2.41 - 2.14 (m, 4H), 1.53 (s, 3H). |
| **I-113** | $^1$H NMR (400 MHz, DMSO-$d_6$) $\delta$ 8.44 (d, *J* = 7.8 Hz, 1H), 7.51 - 7.30 (m, 3H), 4.22 - 4.10 (m, 1H), 3.74 (d, *J* = 17.7 Hz, 1H), 3.59 (s, 3H), 3.33 (d, *J* = 17.7 Hz, 1H), 2.88 - 2.76 (m, 1H), 2.42 - 2.18 (m, 4H), 1.53 (s, 3H). |

(continued)

| NO. | $^1H$ NMR |
|---|---|
| **I-114** | $^1H$ NMR (400 MHz, DMSO-$d_6$) $\delta$ 8.44 (d, J = 7.8 Hz, 1H), 7.51 - 7.30 (m, 3H), 4.22 - 4.10 (m, 1H), 3.74 (d, J = 17.7 Hz, 1H), 3.59 (s, 3H), 3.33 (d, J = 17.7 Hz, 1H), 2.88 - 2.76 (m, 1H), 2.42 - 2.18 (m, 4H), 1.53 (s, 3H). |
| **I-115** | $^1H$ NMR (400 MHz, DMSO-$d_6$) $\delta$ 8.42 (d, *J*= 7.8 Hz, 1H), 7.45 - 7.34 (m, 3H), 4.23 - 4.07 (m, 3H), 3.74 (d, *J* = 17.7 Hz, 1H), 3.37 - 3.27 (d, *J* = 17.7 Hz, 1H), 2.87 - 2.76 (m, 1H), 2.69 (t, *J* = 6.6 Hz, 2H), 2.44 - 2.14 (m, 4H), 2.09 (s, 3H), 1.53 (s, 3H). |
| **I-116** | $^1H$ NMR (400 MHz, DMSO-$d_6$) $\delta$ 8.45 (d, *J* = 7.9 Hz, 1H), 7.46 - 7.33 (m, 3H), 4.45 - 4.36 (m, 1H), 4.36 - 4.26 (m, 1H), 4.24 - 4.10 (m, 1H), 3.74 (d, *J* = 17.7 Hz, 1H), 3.33 (d, *J* = 17.7 Hz, 1H), 3.11 (ddd, *J* = 13.7, 8.7, 5.0 Hz, 1H), 2.94 (dt, *J* = 13.7, 4.8 Hz, 1H), 2.90 - 2.78 (m, 1H), 2.59 (s, 3H), 2.44 - 2.18 (m, 4H), 1.53 (s, 3H). |
| **I-117** | $^1H$ NMR (400 MHz, DMSO-$d_6$) $\delta$ 8.43 (d, *J* = 7.7 Hz, 1H), 7.40 (d, *J* = 5.7 Hz, 3H), 4.37 (t, *J* = 5.7 Hz, 2H), 4.25 - 4.08 (m, 1H), 3.74 (d, *J* = 17.7 Hz, 1H), 3.49 (t, *J* = 5.5 Hz, 2H), 3.31 (s, 1H), 3.01 (s, 3H), 2.93 - 2.78 (m, 1H), 2.46 - 2.18 (m, 4H), 1.53 (s, 3H). |
| **I-118** | $^1H$ NMR (400 MHz, DMSO-$d_6$) $\delta$ 8.43 (d, *J* = 7.8 Hz, 1H), 7.45 - 7.34 (m, 3H), 4.21 - 4.09 (m, 3H), 3.74 (d, *J* = 17.7 Hz, 1H), 3.33 (d, *J* = 17.7 Hz, 1H), 2.87 - 2.75 (m, 1H), 2.72 (t, *J* = 6.7 Hz, 2H), 2.58 - 2.52 (m, 2H), 2.43 - 2.17 (m, 4H), 1.53 (s, 3H), 1.17 (t, *J* = 7.4 Hz, 3H). |
| **I-119** | $^1H$ NMR (400 MHz, DMSO-$d_6$) $\delta$ 8.45 (d, *J* = 7.8 Hz, 1H), 7.45 - 7.33 (m, 3H), 4.45 - 4.36 (m, 1H), 4.36 - 4.25 (m, 1H), 4.23 - 4.10 (m, 1H), 3.74 (d, *J* = 17.7 Hz, 1H), 3.33 (d, *J* = 17.7 Hz, 1H), 3.06 (ddd, *J* = 13.9, 8.9, 5.1 Hz, 1H), 2.96 - 2.88 (m, 1H), 2.87 - 2.77 (m, 2H), 2.68 (dq, *J* = 14.8, 7.4 Hz, 1H), 2.45 - 2.19 (m, 4H), 1.53 (s, 3H), 1.18 (t, *J* = 7.5 Hz, 3H). |
| **I-120** | $^1H$ NMR (400 MHz, DMSO-$d_6$) $\delta$ 8.43 (d, *J* = 7.7 Hz, 1H), 7.47 - 7.33 (m, 3H), 4.36 (t, *J* = 5.7 Hz, 2H), 4.24 - 4.10 (m, 1H), 3.74 (d, *J* = 17.7 Hz, 1H), 3.47 (t, *J* = 5.7 Hz, 2H), 3.33 (d, *J* = 17.7 Hz, 1H), 3.11 (q, *J* = 7.4 Hz, 2H), 2.91 - 2.77 (m, 1H), 2.46 - 2.15 (m, 4H), 1.53 (s, 3H), 1.22 (t, *J*= 7.4 Hz, 3H). |
| **I-121** | $^1H$ NMR (400 MHz, $CDCl_3$) $\delta$ 7.16 (dt, *J* = 6.4, 2.1 Hz, 2H), 7.04 (d, *J* = 8.5 Hz, 1H), 6.89 (tt, *J* = 8.7, 2.3 Hz, 1H), 4.71 - 4.62 (m, 1H), 4.62 - 4.51 (m, 1H), 4.40 - 4.26 (m, 3H), 3.75 (d, *J* = 17.3 Hz, 1H), 3.18 (d, *J* = 17.3 Hz, 1H), 2.90 (tt, *J* = 9.5, 7.9 Hz, 1H), 2.74 - 2.56 (m, 2H), 2.20 (ddt, *J* = 16.3, 11.1, 9.3 Hz, 2H), 1.71 (s, 3H). |
| **I-122** | 1H NMR (400 MHz, $CDCl_3$) $\delta$ 7.22 - 7.13 (m, 2H), 7.08 (d, J = 8.1 Hz, 1H), 6.89 (tt, J = 8.6, 2.2 Hz, 1H), 4.77 (d, J = 10.8 Hz, 2H), 4.46 - 4.33 (m, 1H), 3.76 (d, J = 17.3 Hz, 1H), 3.19 (d, J = 17.3 Hz, 1H), 3.09 - 2.90 (m, 1H), 2.71 (ttd, J = 15.6, 7.8, 4.8 Hz, 2H), 2.26 (tt, J = 22.4, 11.2 Hz, 2H), 1.71 (s, 3H). |
| **I-123** | $^1H$ NMR (400 MHz, $CDCl_3$) $\delta$ 7.21 - 7.11 (m, 2H), 7.05 (d, *J* = 8.2 Hz, 1H), 6.88 (tt, *J = 8.7, 2.3* Hz, 1H), 4.41 - 4.28 (m, 1H), 4.27 - 4.19 (m, 2H), 3.75 (d, *J* = 17.3 Hz, 1H), 3.59 (dd, *J* = 11.5, 6.9 Hz, 2H), 3.39 (s, 3H), 3.18 (t, *J* = 11.1 Hz, 1H), 2.94 - 2.82 (m, 1H), 2.63 (ttd, *J* = 15.6, 7.7, 4.9 Hz, 2H), 2.19 (dt, *J*= 20.4, 9.4 Hz, 2H), 1.71 (s, 3H). |
| **I-124** | 1H NMR (400 MHz, $CDCl_3$) $\delta$ 7.18 - 7.10 (m, 2H), 7.05 (d, J = 8.1 Hz, 1H), 6.86 (tt, J = 8.7, 2.2 Hz, 1H), 5.88 - 5.74 (m, 1H), 5.32 (p, J = 6.4 Hz, 1H), 5.21 (dd, J = 17.3, 1.0 Hz, 1H), 5.16 - 5.07 (m, 1H), 4.38 - 4.22 (m, 1H), 3.73 (d, J = 17.3 Hz, 1H), 3.16 (d, J = 17.3 Hz, 1H), 2.87 - 2.73 (m, 1H), 2.70 - 2.50 (m, 2H), 2.24 - 2.07 (m, 2H), 1.68 (s, 3H), 1.29 (dd, J = 6.5, 2.0 Hz, 3H). |
| **I-125** | 1H NMR (400 MHz, $CDCl_3$) $\delta$ 7.16 (tt, J = 12.9, 6.5 Hz, 3H), 6.88 (tt, J = 8.7, 2.2 Hz, 1H), 4.40 - 4.29 (m, 1H), 4.08 (ddd, J = 17.6, 11.6, 4.9 Hz, 2H), 3.75 (d, J = 17.4 Hz, 1H), 3.61 - 3.52 (m, 1H), 3.38 (s, 3H), 3.18 (d, J = 17.4 Hz, 1H), 2.95 - 2.83 (m, 1H), 2.64 (ttd, J = 15.6, 7.8, 4.9 Hz, 2H), 2.20 (ddd, J = 25.9, 14.9, 6.7 Hz, 2H), 1.71 (s, 3H), 1.17 (d, J = 6.4 Hz, 3H). |
| **I-126** | $^1H$ NMR (400 MHz, $CDCl_3$) $\delta$ 7.20 - 7.11 (m, 2H), 7.03 (d, *J* = 8.0 Hz, 1H), 6.88 (tt, *J* = 8.7, 2.2 Hz, 1H), 4.41 - 4.28 (m, 1H), 4.08 (ddd, *J* = 17.6, 11.6, 5.0 Hz, 2H), 3.75 (d, *J* = 17.3 Hz, 1H), 3.59 - 3.51 (m, 1H), 3.37 (s, 3H), 3.17 (d, *J* = 17.3 Hz, 1H), 2.94 - 2.82 (m, 1H), 2.64 (ttd, *J* = 15.6, 7.7, 4.8 Hz, 2H), 2.18 (dt, *J* = 20.4, 9.5 Hz, 2H), 1.71 (s, 3H), 1.16 (d, *J* = 6.4 Hz, 3H). |
| **I-127** | $^1H$ NMR (400 MHz, $CDCl_3$) $\delta$ 7.22 - 7.12 (m, 2H), 7.04 (d, *J*= 8.5 Hz, 1H), 6.88 (tt, *J* = 8.7, 2.4 Hz, 1H), 5.00 (hept, *J* = 6.2 Hz, 1H), 4.44 - 4.22 (m, 1H), 3.75 (d, *J* = 17.4 Hz, 1H), 3.18 (d, *J* = 17.3 Hz, 1H), 2.78 (tt, *J* = 9.4, 7.9 Hz, 1H), 2.61 (ddp, *J* = 21.6, 11.1, 3.9, 3.1 Hz, 2H), 2.26 - 2.09 (m, 2H), 1.69 (d, *J* = 20.0 Hz, 4H), 1.23 (dd, *J* = 6.3, 1.7 Hz, 6H). |

(continued)

| NO. | $^1$H NMR |
|---|---|
| **I-128** | $^1$H NMR (400 MHz, $CDCl_3$) $\delta$ 7.21 - 7.11 (m, 2H), 7.03 (d, *J* = 8.4 Hz, 1H), 6.88 (tt, *J* = 8.7, 2.4 Hz, 1H), 4.84 (h, *J* = 6.3 Hz, 1H), 4.48 - 4.25 (m, 1H), 3.75 (d, *J = 17.3* Hz, 1H), 3.18 (d, *J* = 17.3 Hz, 1H), 2.80 (tt, *J* = 9.4, 7.9 Hz, 1H), 2.69 - 2.50 (m, 2H), 2.31 - 2.05 (m, 2H), 1.79 - 1.45 (m, 6H), 1.20 (d, *J* = 6.3 Hz, 3H), 0.88 (t, *J = 7.4* Hz, 3H). |
| **I-129** | $^1$H NMR (400 MHz, $CDCl_3$) $\delta$ 7.21 - 7.11 (m, 2H), 7.03 (d, *J* = 8.4 Hz, 1H), 6.88 (tt, *J* = 8.7, 2.3 Hz, 1H), 4.84 (h, *J* = 6.3 Hz, 1H), 4.43 - 4.21 (m, 1H), 3.75 (d, *J* = 17.3 Hz, 1H), 3.18 (d, *J* = 17.4 Hz, 1H), 2.80 (tt, *J* = 9.5, 7.9 Hz, 1H), 2.72 - 2.46 (m, 2H), 2.15 (tt, *J* = 11.6, 9.2 Hz, 2H), 1.66 (s, 1H), 1.63 - 1.49 (m, 2H), 1.20 (d, *J* = 6.3 Hz, 3H), 0.89 (t, *J* = 7.5 Hz, 3H). |
| **I-130** | $^1$H NMR (400 MHz, $CDCl_3$) $\delta$ 7.16 (dt, *J*= 6.3, 2.1 Hz, 2H), 7.02 (d, *J* = 8.5 Hz, 1H), 6.94 - 6.84 (m, 1H), 4.43 - 4.26 (m, 1H), 4.13 (tt, *J* = 6.6, 3.3 Hz, 1H), 3.75 (d, *J* = 17.4 Hz, 1H), 3.17 (d, *J* = 17.3 Hz, 1H), 2.78 (tt, *J*= 9.4, 7.9 Hz, 1H), 2.67 - 2.51 (m, 2H), 2.16 (ddt, *J*= 16.3, 11.3, 9.4 Hz, 2H), 1.71 (s, 3H), 1.63 (s, 1H), 0.75 - 0.63 (m, 4H). |
| **I-131** | 1H NMR (400 MHz, $CDCl_3$) $\delta$ 7.19 (dd, J = 19.6, 6.9 Hz, 3H), 6.88 (t, J = 8.4 Hz, 1H), 5.63 (s, 1H), 4.32 (dt, J = 15.6, 7.8 Hz, 1H), 3.74 (d, J = 17.3 Hz, 1H), 3.39 - 3.25 (m, 2H), 3.17 (d, J = 17.3 Hz, 1H), 2.74 - 2.42 (m, 3H), 2.36 - 2.14 (m, 2H), 1.70 (s, 3H), 1.13 (t, J = 7.2 Hz, 3H). |
| **I-132** | $^1$H NMR (400 MHz, DMSO-$d_6$) $\delta$ 12.14 (s, 1H), 8.45 (m, 1H), 7.79 - 7.47 (m, 3H), 4.19 (h, *J* = 8.3 Hz, 1H), 3.80 (d, *J* = 17.7 Hz, 1H), 3.37 (d, *J* = 17.7 Hz, 2H), 2.72 (m, 1H), 2.50 - 2.15 (m, 4H), 1.58 (s, 3H). |
| **I-134** | $^1$H NMR (400 MHz, Chloroform-d) $\delta$ 7.40 (d, *J* = 1.5 Hz, 1H), 7.27 (m, 1H), 7.16 (m, 1H), 7.02 (d, *J* = 8.4 Hz, 1H), 4.34 (h, *J* = 8.3 Hz, 1H), 3.75 (dd, *J* = 17.3, 1.1 Hz, 1H), 3.69 (d, *J* = 1.1 Hz, 3H), 3.17 (dd, *J* = 17.3, 1.1 Hz, 1H), 2.89 - 2.78 (m, 1H), 2.62 (m, 2H), 2.18 (m, 2H), 1.71 (s, 3H). |
| **I-135** | $^1$H NMR (400 MHz, Chloroform-d) δ 7.40 (d, J = 1.5 Hz, 1H), 7.27 (m, 1H), 7.16 (m, 1H), 7.02 (d, J = 8.4 Hz, 1H), 4.34 (h, J = 8.3 Hz, 1H), 3.75 (dd, J = 17.3, 1.1 Hz, 1H), 3.69 (d, J = 1.1 Hz, 3H), 3.17 (dd, J = 17.3, 1.1 Hz, 1H), 2.89 - 2.78 (m, 1H), 2.62 (m, 2H), 2.18 (m, 2H), 1.71 (s, 3H). |
| **I-136** | $^1$H NMR (400 MHz, $CDCl_3$) $\delta$ 7.40 (t, *J* = 1.7 Hz, 1H), 7.30 - 7.26 (m, 1H), 7.18 - 7.13 (m, 1H), 7.05 (d, *J* = 8.5 Hz, 1H), 5.06 - 4.93 (m, 1H), 4.40 -4.27 (m, 1H), 3.75 (d, *J* = 17.3 Hz, 1H), 3.17 (d, *J* = 17.3 Hz, 1H), 2.83 - 2.70 (m, 1H), 2.69 - 2.51 (m, 2H), 2.26 - 2.05 (m, 2H), 1.71 (s, 3H), 1.23 (dd, *J* = 6.3, 1.7 Hz, 6H). |
| **I-137** | $^1$H NMR (400 MHz, DMSO-$d_6$) $\delta$ 12.08 (s, 1H), 8.38 (d, *J*= 7.8 Hz, 1H), 7.73 (d, *J* = 2.0 Hz, 1H), 7.69 (d, *J* = 1.9 Hz, 2H), 4.21 - 4.07 (m, 1H), 3.75 (d, *J* = 17.7 Hz, 1H), 3.37 (d, *J* = 17.7 Hz, 1H), 2.76 - 2.65 (m, 1H), 2.40 - 2.14 (m, 4H), 1.53 (s, 3H). |
| **I-139** | $^1$H NMR (400 MHz, Chloroform-d) $\delta$ 7.51 (d, *J* = 1.8 Hz, 2H), 7.41 (t, *J* = 1.8 Hz, 1H), 7.03 (d, *J* = 8.4 Hz, 1H), 4.42 - 4.27 (m, 1H), 3.75 (d, *J* = 17.3 Hz, 1H), 3.69 (d, *J* = 1.3 Hz, 3H), 3.17 (d, *J = 17.3* Hz, 1H), 2.93 - 2.75 (m, 1H), 2.71 - 2.53 (m, 2H), 2.28 - 2.10 (m, 2H), 1.71 (s, 3H). |
| **I-141** | $^1$H NMR (400 MHz, DMSO-$d_6$) $\delta$ 8.40 (d, *J* = 7.8 Hz, 1H), 8.01 - 7.93 (m, 2H), 7.87 (m, 1H), 4.21 - 4.07 (m, 1H), 3.80 (d, *J* = 17.7 Hz, 1H), 3.36 (d, *J* = 17.7 Hz, 1H), 2.76 - 2.62 (m, 1H), 2.44 - 2.13 (m, 4H), 1.55 (s, 3H). |
| **I-143** | $^1$H NMR (400 MHz, Chloroform-d) $\delta$ 7.69 (p, *J*= 1.4 Hz, 1H), 7.61 (m, 1H), 7.45 - 7.39 (m, 1H), 7.00 (d, *J*= 8.3 Hz, 1H), 4.42 - 4.26 (m, 1H), 3.84 - 3.76 (m, 1H), 3.69 (s, 3H), 3.18 (m, 1H), 2.92 - 2.78 (m, 1H), 2.63 (m, 2H), 2.27 - 2.10 (m, 2H), 1.72 (s, 3H). |
| **I-147** | $^1$H NMR (400 MHz, DMSO-$d_6$) $\delta$ 8.44 (d, *J* = 7.8 Hz, 1H), 8.19 - 8.00 (m, 3H), 4.23 - 4.09 (m, 1H), 3.79 (d, *J* = 17.7 Hz, 1H), 3.59 (s, 3H), 3.39 (d, *J* = 17.7 Hz, 1H), 2.88 - 2.76 (m, 1H), 2.44 - 2.17 (m, 4H), 1.53 (s, 3H). |
| **I-149** | $^1$H NMR (400 MHz, DMSO-$d_6$) $\delta$ 12.05 (s, 1H), 8.37 (d, *J* = 7.8 Hz, 1H), 7.86 - 7.67 (m, 3H), 4.16 (ddt, *J* = 16.9, 9.2, 7.7 Hz, 1H), 3.82 (d, *J* = 17.7 Hz, 1H), 3.39 (d, *J* = 17.7 Hz, 1H), 2.71 (tt, *J* = 9.9, 8.0 Hz, 1H), 2.44 - 2.15 (m, 4H), 1.55 (s, 3H). |
| **I-151** | $^1$H NMR (400 MHz, DMSO-$d_6$) $\delta$ 8.43 (d, *J*= 7.8 Hz, 1H), 7.83 (d, *J* = 7.9 Hz, 3H), 4.25 - 4.09 (m, 1H), 3.81 (d, *J* = 17.7 Hz, 1H), 3.59 (s, 3H), 3.40 (d, *J* = 17.7 Hz, 1H), 2.82 (tt, *J* = 10.0, 8.0 Hz, 1H), 2.45 - 2.16 (m, 4H), 1.54 (s, 3H). |

(continued)

| NO. | $^1$H NMR |
|---|---|
| **I-153** | $^1$H NMR (400 MHz, DMSO-$d_6$) $\delta$ 12.07 (s, 1H), 8.38 (d, *J* = 7.8 Hz, 1H), 8.11 - 7.87 (m, 3H), 4.23 - 4.08 (m, 1H), 3.83 (d, *J* = 17.6 Hz, 1H), 3.42 (d, *J* = 17.7 Hz, 1H), 2.71 (tt, *J* = 10.0, 8.0 Hz, 1H), 2.44 - 2.15 (m, 4H), 1.55 (s, 3H). |
| **I-155** | $^1$H NMR (400 MHz, DMSO-$d_6$) $\delta$ 8.42 (d, *J* = 7.9 Hz, 1H), 8.06 - 7.90 (m, 3H), 4.23 - 4.10 (m, 1H), 3.82 (d, *J* = 17.7 Hz, 1H), 3.59 (s, 3H), 3.42 (d, *J* = 17.7 Hz, 1H), 2.82 (tt, *J* = 10.0, 8.0 Hz, 1H), 2.43 - 2.18 (m, 4H), 1.54 (s, 3H). |
| **I-157** | $^1$H NMR (400 MHz, DMSO-$d_6$) $\delta$ 12.08 (s, 1H), 8.40 (d, *J* = 7.8 Hz, 1H), 7.62 - 7.50 (m, 3H), 4.14 (m, 1H), 3.76 (d, *J* = 17.6 Hz, 1H), 3.36 (d, *J* = 17.6 Hz, 1H), 2.70 (m, 1H), 2.41 - 2.14 (m, 4H), 1.53 (s, 3H). |
| **I-159** | $^1$H NMR (400 MHz, Chloroform-d) $\delta$ 7.33 - 7.28 (m, 2H), 7.07 - 6.98 (m, 2H), 4.34 (h, *J* = 8.4 Hz, 1H), 3.76 (dd, *J* = 17.3, 1.0 Hz, 1H), 3.69 (d, *J* = 1.0 Hz, 3H), 3.18 (dd, *J* = 17.3, 1.0 Hz, 1H), 2.90 - 2.78 (m, 1H), 2.72 - 2.54 (m, 2H), 2.18 (m, 2H), 1.71 (s, 3H). |
| **I-161** | $^1$H NMR (400 MHz, $CDCl_3$) $\delta$ 7.33 - 7.28 (m, 2H), 7.06 - 6.98 (m, 2H), 4.69 (d, *J*= 2.4 Hz, 2H), 4.42 - 4.30 (m, 1H), 3.77 (d, *J* = 17.4 Hz, 1H), 3.19 (d, *J*= 17.4 Hz, 1H), 2.96 - 2.82 (m, 1H), 2.73 - 2.56 (m, 2H), 2.49 (t, *J* = 2.4 Hz, 1H), 2.31 - 2.13 (m, 2H), 1.72 (s, 3H). |
| **I-164** | $^1$H NMR (400 MHz, DMSO-$d_6$) $\delta$ 8.41 (d, *J* = 7.8 Hz, 1H), 7.83 - 7.53 (m, 3H), 4.26 - 4.05 (m, 1H), 3.77 (d, *J* = 17.7 Hz, 1H), 3.59 (s, 3H), 3.39 (s, 1H), 2.88 - 2.78 (m, 1H), 2.45 - 2.14 (m, 4H), 1.53 (s, 3H). |
| **I-166** | $^1$H NMR (400 MHz, DMSO-$d_6$) $\delta$ 12.09 (s, 1H), 8.41 (d, *J* = 7.8 Hz, 1H), 7.42 - 7.39 (m, 1H), 7.38 (t, *J* = 72.3 Hz, 1H), 7.33 (m, 1H), 7.27 (m, 1H), 4.21 - 4.06 (m, 1H), 3.76 (d, *J* = 17.7 Hz, 1H), 3.31 (s, 1H), 2.76 - 2.63 (m, 1H), 2.40 - 2.12 (m, 4H), 1.53 (s, 3H). |
| **I-168** | $^1$H NMR (400 MHz, Chloroform-d) $\delta$ 7.24 - 7.18 (m, 2H), 7.03 (d, *J* = 8.4 Hz, 1H), 6.94 (m, 1H), 6.54 (t, *J* = 73.2 Hz, 1H), 4.41 - 4.27 (m, 1H), 3.76 (d, *J* = 17.3 Hz, 1H), 3.69 (s, 3H), 3.18 (d, *J* = 17.3 Hz, 1H), 2.84 (m, 1H), 2.71 - 2.54 (m, 2H), 2.18 (m, 2H), 1.71 (s, 3H). |
| **I-170** | $^1$H NMR (400 MHz, DMSO-$d_6$) $\delta$ 12.09 (s, 1H), 8.40 (d, *J* = 7.8 Hz, 1H), 7.59 (t, *J* = 1.7 Hz, 1H), 7.46 - 7.44 (m, 2H), 7.38 (t, *J* = 7.2 Hz, 1H), 4.13 (ddt, *J* = 16.9, 9.2, 7.7 Hz, 1H), 3.76 (d, *J* = 17.6 Hz, 1H), 3.33 (s, *J* = 17.6 Hz, 1H), 2.70 (tt, *J* = 9.9, 8.0 Hz, 1H), 2.42 - 2.11 (m, 4H), 1.53 (s, 3H). |
| **I-172** | $^1$H NMR (400 MHz, DMSO-$d_6$) $\delta$ 8.42 (d, *J* = 7.9 Hz, 1H), 7.59 (t, *J* = 1.6 Hz, 1H), 7.44 (t, *J* = 1.6 Hz, 2H), 7.38 (t, *J* = 7.2 Hz, 1H), 4.26 - 4.05 (m, 1H), 3.75 (d, *J* = 17.6 Hz, 1H), 3.59 (s, 3H), 3.37 (d, *J* = 17.6 Hz, 1H), 2.81 (tt, *J* = 10.0, 8.0 Hz, 1H), 2.43 - 2.17 (m, 4H), 1.53 (s, 3H). |
| **I-174** | $^1$H NMR (400 MHz, DMSO-$d_6$) $\delta$ 11.69 (s, 1H), 8.39 (d, *J* = 7.8 Hz, 1H), 7.57 - 7.44 (m, 3H), 7.37 - 7.27 (m, 1H), 4.20 - 4.06 (m, 1H), 3.74 (d, *J* = 17.5 Hz, 1H), 3.35 (d, *J* = 17.5 Hz, 1H), 2.70 (tt, *J*= 10.0, 8.0 Hz, 1H), 2.39 - 2.15 (m, 4H), 1.53 (s, 3H). |
| **I-176** | $^1$H NMR (400 MHz, DMSO-$d_6$) $\delta$ 8.43 (d, *J* = 7.9 Hz, 1H), 7.58 - 7.43 (m, 3H), 7.40 - 7.25 (m, 1H), 4.17 (h, *J* = 8.4 Hz, 1H), 3.73 (d, *J* = 17.5 Hz, 1H), 3.59 (s, 3H), 3.32 (s, 1H), 2.89 - 2.71 (m, 1H), 2.45 - 2.17 (m, 4H), 1.53 (s, 3H). |
| **I-180** | $^1$H NMR (400 MHz, $CDCl_3$-*d*) $\delta$ 7.21 - 7.13 (m, 2H), 7.06 (d, *J* = 8.0 Hz, 1H), 6.95 (d, *J* = 9.3 Hz, 1H), 4.40 - 4.27 (m, 1H), 3.76 (d, *J* = 17.3 Hz, 1H), 3.69 (s, 3H), 3.19 (d, *J* = 17.3 Hz, 1H), 2.89 - 2.75 (m, 1H), 2.70 - 2.51 (m, 2H), 2.37 (s, 3H), 2.29 - 2.10 (m, 2H), 1.70 (s, 3H). |
| **I-184** | $^1$H NMR (400 MHz, DMSO-$d_6$) $\delta$ 8.43 (d, *J* = 7.8 Hz, 1H), 7.64 (dd, *J* = 8.3, 7.1 Hz, 2H), 4.24 - 4.09 (m, 1H), 3.75 (d, *J* = 17.7 Hz, 1H), 3.59 (s, 3H), 3.28 (d, *J* = 17.7 Hz, 1H), 2.90 - 2.74 (m, 1H), 2.44 - 2.16 (m, 4H), 1.53 (s, 3H). |
| **I-188** | $^1$H NMR (400 MHz, DMSO-$d_6$) $\delta$ 8.43 (d, *J* = 7.8 Hz, 1H), 7.83 - 7.68 (m, 2H), 4.22 - 4.08 (m, 1H), 3.76 (d, *J* = 17.7 Hz, 1H), 3.59 (s, 3H), 3.30 (s, 1H), 2.86 - 2.76 (m, 1H), 2.43 - 2.31 (m, 2H), 2.28 - 2.14 (m, 2H), 1.52 (s, 3H). |
| **I-192** | $^1$H NMR (400 MHz, DMSO-$d_6$) $\delta$ 8.41 (d, *J* = 7.8 Hz, 1H), 7.87 (d, *J* = 6.4 Hz, 2H), 4.24 - 4.06 (m, 1H), 3.76 (d, *J* = 17.7 Hz, 1H), 3.59 (s, 3H), 3.36 (s, 1H), 2.89 - 2.74 (m, 1H), 2.41 - 2.19 (m, 4H), 1.52 (s, 3H). |
| **I-196** | $^1$H NMR (400 MHz, DMSO-$d_6$) $\delta$ 8.43 (d, *J* = 7.8 Hz, 1H), 7.72 - 7.61 (m, 2H), 7.46 (d, *J* = 3.8 Hz, 3H), 4.21 - 4.09 (m, 1H), 3.72 (d, *J* = 17.4 Hz, 1H), 3.58 (s, 3H), 3.30 (s, 1H), 2.87 - 2.74 (m, 1H), 2.43 - 2.15 (m, 4H), 1.52 (s, 3H). |

(continued)

| NO. | $^1H$ NMR |
|---|---|
| **I-200** | $^1H$ NMR (400 MHz, $CDCl_3$-*d*) $\delta$ 7.26 (s, 2H), 7.08 (d, *J* = 11.3 Hz, 2H), 4.39 - 4.24 (m, 1H), 3.78 (d, *J* = 17.3 Hz, 1H), 3.69 (s, 3H), 3.20 (d, *J* = 17.2 Hz, 1H), 2.88 - 2.75 (m, 1H), 2.68 - 2.52 (m, 2H), 2.33 (s, 6H), 2.24 - 2.09 (m, 2H), 1.69 (s, 3H). |
| **I-204** | $^1H$ NMR (400 MHz, DMSO-$d_6$) $\delta$ 8.40 (d, *J* = 7.8 Hz, 1H), 6.80 (d, *J* = 2.1 Hz, 2H), 6.59 (t, *J* = 2.1 Hz, 1H), 4.22 - 4.08 (m, 1H), 3.77 (s, 6H), 3.70 (d, *J* = 17.5 Hz, 1H), 3.59 (s, 3H), 3.28 (s, 1H), 2.86 - 2.73 (m, 1H), 2.42 - 2.16 (m, 4H), 1.51 (s, 3H). |
| **I-206** | $^1H$ NMR (400 MHz, DMSO-$d_6$) $\delta$ 12.11 (s, 1H), 8.98 (d, *J* = 7.5 Hz, 1H), 7.64 - 7.35 (m, 3H), 4.27 - 4.00 (m, 3H), 2.85 - 2.64 (m, 1H), 2.45 - 2.22 (m, 4H). |
| **I-208** | $^1H$ NMR (400 MHz, DMSO-$d_6$) $\delta$ 9.02 (d, *J* = 7.4 Hz, 1H), 7.50 (d, *J* = 7.5 Hz, 3H), 4.27 - 4.02 (m, 3H), 3.60 (s, 3H), 2.93 - 2.77 (m, 1H), 2.46 - 2.24 (m, 4H). |
| **I-210** | $^1H$ NMR (400 MHz, $CDCl_3$) $\delta$ 7.22 - 7.16 (m, 2H), 7.11 - 7.04 (m, 1H), 6.98 - 6.91 (m, 1H), 4.48 - 4.32 (m, 1H), 4.06 (t, *J* = 6.8 Hz, 2H), 3.94 (d, *J* = 18.1 Hz, 1H), 3.73 (d, *J*= 18.1 Hz, 1H), 2.93 - 2.80 (m, 1H), 2.75 - 2.57 (m, 2H), 2.29 - 2.16 (m, 2H), 1.72 - 1.60 (m, 2H), 0.94 (t, *J* = 7.4 Hz, 3H). |
| **I-211** | $^1H$ NMR (400 MHz, $CDCl_3$) $\delta$ 7.23 - 7.15 (m, 2H), 7.08 (d, *J* = 8.3 Hz, 1H), 6.98 - 6.91 (m, 1H), 4.47 - 4.32 (m, 1H), 3.94 (d, *J* = 18.1 Hz, 1H), 3.88 (d, *J* = 6.7 Hz, 2H), 3.73 (d, *J* = 18.1 Hz, 1H), 2.95 - 2.81 (m, 1H), 2.78 - 2.58 (m, 2H), 2.30 - 2.16 (m, 2H), 2.00 - 1.87 (m, 1H), 0.93 (d, *J* = 6.7 Hz, 6H). |
| **I-212** | $^1H$ NMR (400 MHz, $CDCl_3$) $\delta$ 7.24 - 7.15 (m, 2H), 7.12 - 7.06 (m, 1H), 6.99 - 6.90 (m, 1H), 4.84 -4.71 (m, 1H), 4.46 - 4.33 (m, 1H), 3.94 (d, *J* = 18.1 Hz, 1H), 3.73 (d, *J* = 18.1 Hz, 1H), 2.90 - 2.76 (m, 1H), 2.76 - 2.56 (m, 2H), 2.26 - 2.12 (m, 2H), 1.90 - 1.80 (m, 2H), 1.79 - 1.67 (m, 2H), 1.47 - 1.30 (m, 5H). |
| **I-213** | $^1H$ NMR (400 MHz, $CDCl_3$) $\delta$ 7.22 - 7.15 (m, 2H), 7.09 (d, *J* = 8.2 Hz, 1H), 6.98 - 6.91 (m, 1H), 4.47 - 4.33 (m, 1H), 3.98 (d, *J* = 7.2 Hz, 2H), 3.94 (d, *J* = 18.1 Hz, 1H), 3.73 (d, *J* = 18.1 Hz, 1H), 2.94 - 2.80 (m, 1H), 2.74 - 2.57 (m, 2H), 2.28 - 2.14 (m, 3H), 1.82 - 1.67 (m, 2H), 1.66 - 1.49 (m, 3H), 1.29 - 1.17 (m, 3H). |
| **I-214** | $^1H$ NMR (400 MHz, $CDCl_3$) $\delta$ 7.24 - 7.15 (m, 2H), 7.13 - 7.05 (m, 1H), 7.00 - 6.90 (m, 1H), 4.49 - 4.35 (m, 3H), 3.95 (d, *J* = 18.1 Hz, 1H), 3.74 (d, *J* = 18.1 Hz, 1H), 3.53 (t, *J* = 6.0 Hz, 2H), 2.99 - 2.85 (m, 1H), 2.82 - 2.58 (m, 2H), 2.35 - 2.18 (m, 2H). |
| **I-215** | $^1H$ NMR (400 MHz,$CDCl_3$) $\delta$ 7.24 - 7.15 (m, 2H), 7.10 - 7.03 (m, 1H), 6.98 - 6.91 (m, 1H), 6.11 - 5.77 (m, 1H), 4.49 - 4.36 (m, 1H), 4.36 - 4.24 (m, 2H), 3.94 (d, *J* = 18.0 Hz, 1H), 3.74 (d, *J* = 18.1 Hz, 1H), 3.01 - 2.89 (m, 1H), 2.80 - 2.62 (m, 2H), 2.31 - 2.19 (m, 2H). |
| **I-216** | $^1H$ NMR (400 MHz, $CDCl_3$) $\delta$ 7.23 - 7.15 (m, 2H), 7.10 - 7.03 (m, 1H), 6.98 - 6.91 (m, 1H), 5.86 - 5.70 (m, 1H), 5.14 - 5.06 (m, 2H), 4.46 - 4.32 (m, 1H), 4.16 (t, *J* = 6.7 Hz, 2H), 3.94 (d, *J* = 18.1 Hz, 1H), 3.73 (d, *J* = 18.1 Hz, 1H), 2.93 - 2.80 (m, 1H), 2.76 - 2.57 (m, 2H), 2.45 - 2.34 (m, 2H), 2.28 - 2.13 (m, 2H). |
| **I-217** | $^1H$ NMR (400 MHz, $CDCl_3$) $\delta$ 7.23 - 7.15 (m, 2H), 7.11 - 7.05 (m, 1H), 6.98 - 6.90 (m, 1H), 4.47 - 4.33 (m, 1H), 4.16 - 4.02 (m, 2H), 3.94 (d, *J* = 18.0 Hz, 1H), 3.73 (d, *J* = 18.1 Hz, 1H), 3.61 - 3.50 (m, 1H), 3.38 (s, 3H), 2.98 - 2.86 (m, 1H), 2.77 - 2.59 (m, 2H), 2.29 - 2.17 (m, 2H), 1.17 (d, *J* = 6.4 Hz, 3H). |
| **I-218** | 1H NMR (400 MHz, $CDCl_3$) $\delta$ 7.23 - 7.17 (m, 2H), 7.15 - 7.10 (m, 1H), 6.99 - 6.90 (m, 1H), 4.47 - 4.34 (m, 1H), 4.18 - 4.02 (m, 2H), 3.95 (d, J = 18.1 Hz, 1H), 3.74 (d, J = 18.0 Hz, 1H), 3.62 - 3.50 (m, 1H), 3.38 (s, 3H), 2.99 - 2.86 (m, 1H), 2.76 - 2.59 (m, 2H), 2.30 - 2.21 (m, 2H), 1.17 (d, J = 6.4 Hz, 3H). |
| **I-219** | $^1H$ NMR (400 MHz, $CDCl_3$) $\delta$ 7.23 - 7.15 (m, 2H), 7.12 - 7.03 (m, 1H), 6.98 - 6.90 (m, 1H), 4.46 - 4.34 (m, 1H), 4.16 - 4.02 (m, 2H), 3.94 (d, *J* = 18.1 Hz, 1H), 3.73 (d, *J* = 18.1 Hz, 1H), 3.61 - 3.51 (m, 1H), 3.38 (s, 3H), 2.98 - 2.86 (m, 1H), 2.76 - 2.61 (m, 2H), 2.29 - 2.17 (m, 2H), 1.17 (d, *J* = 6.4 Hz, 3H). |
| **I-220** | $^1H$ NMR (400 MHz, $CDCl_3$) $\delta$ 7.24 - 7.15 (m, 2H), 7.13 - 7.04 (m, 1H), 6.99 - 6.90 (m, 1H), 5.38 - 5.23 (m, 1H), 4.48 -4.31 (m, 1H), 3.94 *(d, J*= 18.1 Hz, 1H), 3.74 (d, *J* = 18.1 Hz, 1H), 2.96 - 2.84 (m, 1H), 2.85 - 2.58 (m, 2H), 2.49 - 2.42 (m, 1H), 2.30 - 2.15 (m, 2H), 1.89 - 1.71 (m, 2H), 1.08 - 0.94 (m, 3H). |
| **I-221** | $^1H$ NMR (400 MHz, DMSO) $\delta$ 10.84 (s, 1H), 9.00 (d, *J* = 7.4 Hz, 1H), 7.67 - 7.32 (m, 3H), 4.26 - 3.95 (m, 3H), 3.81 - 3.68 (m, 2H), 2.57 - 2.43 (m, 1H), 2.37 - 2.17 (m, 4H), 1.12 (t, *J* = 7.0 Hz, 3H). |
| **I-222** | $^1H$ NMR (400 MHz, $CDCl_3$) $\delta$ 7.23 - 7.18 (m, 2H), 7.17 - 7.13 (m, 1H), 6.97 - 6.90 (m, 1H), 4.46 - 4.32 (m, 1H), 3.93 (d, *J* = 18.1 Hz, 1H), 3.73 (d, *J* = 18.1 Hz, 1H), 3.62 - 3.48 (m, 2H), 3.36 - 3.26 (m, 2H), 3.07 - 2.92 (m, 1H), 2.73 - 2.50 (m, 2H), 2.37 - 2.21 (m, 2H), 1.68 - 1.60 (m, 2H), 1.59 - 1.45 (m, 4H). |

(continued)

| NO. | $^1H$ NMR |
|---|---|
| **I-223** | $^1H$ NMR (400 MHz, DMSO-$d_6$) δ 12.12 (s, 1H), 8.98 (d, *J* = 7.6 Hz, 1H), 7.73 - 7.57 (m, 3H), 4.31 - 4.02 (m, 3H), 2.82 - 2.68 (m, 1H), 2.32 (m, 4H). |
| **I-225** | $^1H$ NMR (400 MHz, Chloroform-d) δ 7.43 (d, *J* = 1.6 Hz, 1H), 7.34 - 7.28 (m, 1H), 7.22 (m, 1H), 7.10 (d, *J* = 8.3 Hz, 1H), 4.40 (h, *J* = 8.3 Hz, 1H), 3.95 *(d, J*= 18.1 Hz, 1H), 3.74 (d, *J* = 18.1 Hz, 1H), 3.71 (s, 3H), 2.93 - 2.82 (m, 1H), 2.67 (m, 2H), 2.23 (m, 2H). |
| **I-227** | $^1H$ NMR (400 MHz, $CDCl_3$) δ 7.43 (d, *J* = 1.7 Hz, 1H), 7.34 - 7.29 (m, 1H), 7.24 - 7.15 (m, 2H), 4.47 - 4.35 (m, 1H), 4.06 (t, *J* = 6.7 Hz, 2H), 3.97 (d, *J* = 18.1 Hz, 1H), 3.75 (d, *J* = 18.1 Hz, 1H), 2.92 - 2.81 (m, 1H), 2.75 - 2.57 (m, 2H), 2.30 - 2.16 (m, 2H), 1.72 - 1.61 (m, 2H), 0.94 (t, *J* = 7.4 Hz, 3H). |
| **I-228** | $^1H$ NMR (400 MHz, $CDCl_3$) δ 7.43 (s, 1H), 7.36 - 7.12 (m, 3H), 4.50 - 4.33 (m, 1H), 4.09 (t, *J* = 6.9 Hz, 2H), 3.97 (d, *J* = 18.1 Hz, 1H), 3.75 (d, *J* = 18.1 Hz, 1H), 2.94 - 2.79 (m, 1H), 2.78 - 2.53 (m, 2H), 2.33 - 2.13 (m, 2H), 1.63 (t, *J* = 7.0 Hz, 2H), 1.32 (d, *J* = 12.3 Hz, 6H), 0.90 (d, *J* = 6.1 Hz, 3H). |
| **I-229** | $^1H$ NMR (400 MHz, DMSO-$d_6$) δ 12.10 (s, 1H), 8.96 (d, *J* = 7.6 Hz, 1H), 7.99 - 7.67 (m, 3H), 4.24 - 3.99 (m, 3H), 2.78 - 2.66 (m, 1H), 2.43 - 2.19 (m, 4H). |
| **I-231** | $^1H$ NMR (400 MHz, DMSO-$d_6$) δ 8.99 (d, *J* = 7.6 Hz, 1H), 7.84 - 7.74 (m, 3H), 4.28 - 3.99 (m, 3H), 3.60 (s, 3H), 2.92 - 2.77 (m, 1H), 2.47 - 2.25 (m, 4H). |
| **I-233** | $^1H$ NMR (400 MHz, DMSO-$d_6$) δ 8.99 (d, *J* = 7.5 Hz, 1H), 8.11 - 8.02 (m, 2H), 7.96 (m 1H), 4.24 - 4.05 (m, 3H), 2.73 (m, 1H), 2.45 - 2.20 (m, 4H). |
| **I-235** | $^1H$ NMR (400 MHz, Chloroform-d) δ 7.74 (p, *J* = 1.4 Hz, 1H), 7.66 (m, 1H), 7.49 (m, 1H), 7.10 (d, *J* = 8.3 Hz, 1H), 4.49 - 4.35 (m, 1H), 4.00 (dd, *J* = 18.1, 2.0 Hz, 1H), 3.76 (dd, *J* = 18.1, 2.0 Hz, 1H), 3.72 (s, 3H), 2.95 - 2.83 (m, 1H), 2.78 - 2.58 (m, 2H), 2.24 (m, 2H). |
| **I-239** | $^1H$ NMR (400 MHz, DMSO-$d_6$) δ 9.02 (d, *J* = 7.6 Hz, 1H), 8.25 - 8.21 (m, 1H), 8.19 (t, *J* = 1.4 Hz, 1H), 8.14 - 8.09 (m, 1H), 4.29 - 4.04 (m, 3H), 3.59 (s, 3H), 2.92 - 2.77 (m, 1H), 2.45 - 2.24 (m, 4H). |
| **I-241** | $^1H$ NMR (400 MHz, DMSO-$d_6$) δ 12.12 (s, 1H), 8.99 (d, *J* = 7.6 Hz, 1H), 8.02 - 7.83 (m, 3H), 4.29 - 4.10 (m, 3H), 2.74 (tt, *J* = 10.0, 8.0 Hz, 1H), 2.46 - 2.22 (m, 4H). |
| **I-243** | $^1H$ NMR (400 MHz, DMSO-$d_6$) δ 9.01 (d, *J* = 7.6 Hz, 1H), 7.99 - 7.85 (m, 3H), 4.29 - 4.08 (m, 3H), 3.59 (s, 3H), 2.85 (tt, *J* = 9.9, 8.1 Hz, 1H), 2.47 - 2.25 (m, 4H). |
| **I-245** | $^1H$ NMR (400 MHz, Chloroform-d) δ 7.83 (d, *J* = 1.7 Hz, 1H), 7.78 (s, 1H), 7.72 (d, *J* = 1.8 Hz, 1H), 7.28 (s, 1H), 4.44 (h, *J* = 8.3 Hz, 1H), 4.03 (d, *J* = 18.1 Hz, 1H), 3.79 (d, *J* = 18.1 Hz, 1H), 3.01 - 2.86 (m, 1H), 2.81 - 2.60 (m, 2H), 2.38 - 2.24 (m, 2H). |
| **I-247** | $^1H$ NMR (400 MHz, DMSO-$d_6$) δ 8.99 (d, *J* = 7.6 Hz, 1H), 8.18 - 7.97 (m, 3H), 4.32 - 4.09 (m, 3H), 3.59 (s, 3H), 2.85 (tt, *J* = 10.0, 8.1 Hz, 1H), 2.46 - 2.25 (m, 4H). |
| **I-249** | $^1H$ NMR (400 MHz, DMSO-$d_6$) δ 12.11 (s, 1H), 8.98 (d, *J* = 7.6 Hz, 1H), 7.72 - 7.58 (m, 3H), 4.26 - 4.05 (m, 3H), 2.80 - 2.63 (m, 1H), 2.32 (m, 4H). |
| **I-251** | $^1H$ NMR (400 MHz, Chloroform-d) δ 7.34 (m, 2H), 7.17 - 7.05 (m, 2H), 4.39 (p, *J* = 8.3 Hz, 1H), 3.96 (d, *J* = 18.1 Hz, 1H), 3.74 (d, *J* = 18.1 Hz, 1H), 3.71 (s, 3H), 2.93 - 2.82 (m, 1H), 2.67 (m, 2H), 2.29 - 2.18 (m, 2H). |
| **I-253** | $^1H$ NMR (400 MHz, $CDCl_3$) δ 7.41 - 7.31 (m, 2H), 7.22 (d, *J* = 8.3 Hz, 1H), 7.09 (dd, *J* = 8.5, 2.5 Hz, 1H), 5.23 - 5.11 (m, 1H), 4.47 - 4.34 (m, 1H), 3.98 (d, *J* = 18.1 Hz, 1H), 3.76 (d, *J* = 18.1 Hz, 1H), 2.91 - 2.75 (m, 1H), 2.74 - 2.54 (m, 2H), 2.27 - 2.15 (m, 2H), 1.93 - 1.78 (m, 3H), 1.76 - 1.55 (m, 6H). |
| **I-254** | $^1H$ NMR (400 MHz, $CDCl_3$) δ 7.40 - 7.28 (m, 2H), 7.20 - 7.03 (m, 2H), 4.57 (t, *J* = 12.8 Hz, 2H), 4.53 - 4.34 (m, 1H), 3.97 (d, *J* = 18.1 Hz, 1H), 3.76 (d, *J* = 18.1 Hz, 1H), 3.05 - 2.90 (m, 1H), 2.80 - 2.65 (m, 2H), 2.39 - 2.21 (m, 2H). |
| **I-255** | $^1H$ NMR (400 MHz, $CDCl_3$) δ 7.38 - 7.30 (m, 2H), 7.15 - 7.05 (m, 2H), 4.70 (d, *J* = 2.5 Hz, 2H), 4.50 - 4.35 (m, 1H), 3.97 (d, *J* = 18.1 Hz, 1H), 3.75 (d, *J* = 18.0 Hz, 1H), 2.92 (tt, *J* = 9.3, 8.0 Hz, 1H), 2.78 - 2.61 (m, 2H), 2.49 (t, *J* = 2.5 Hz, 1H), 2.34 - 2.18 (m, 2H). |
| **I-258** | $^1H$ NMR (400 MHz, DMSO-$d_6$) δ 8.98 (d, *J* = 7.6 Hz, 1H), 7.86 (s, 1H), 7.75 (d, *J* = 24.2 Hz, 2H), 4.30 - 4.05 (m, 3H), 3.59 (s, 3H), 2.84 (s, 1H), 2.44 - 2.23 (m, 4H). |

(continued)

| NO. | $^1H$ NMR |
|---|---|
| **I-260** | $^1H$ NMR (400 MHz, DMSO-$d_6$) $\delta$ 12.12 (s, 1H), 8.99 (d, *J* = 7.6 Hz, 1H), 7.50 (m, 1H), 7.42 (m, 1H), 7.39 (t, *J* = 73.2 Hz, 1H), 7.34 (m, 1H), 4.28 - 4.02 (m, 3H), 2.74 (m, 1H), 2.45 - 2.20 (m, 4H). |
| **I-262** | $^1H$ NMR (400 MHz, Chloroform-d) $\delta$ 7.26 - 7.21 (m, 2H), 7.11 (d, *J* = 8.2 Hz, 1H), 7.00 (m, 1H), 6.56 (t, *J* = 72.4 Hz, 1H), 4.40 (h, *J* = 8.3 Hz, 1H), 3.96 (d, *J* = 18.1 Hz, 1H), 3.74 (d, *J* = 18.1 Hz, 1H), 3.70 (s, 3H), 2.87 (m, 1H), 2.67 (m, 2H), 2.29 - 2.18 (m, 2H). |
| **I-264** | $^1H$ NMR (400 MHz, DMSO-$d_6$) $\delta$ 12.14 (s, 1H), 9.00 (d, *J* = 7.6 Hz, 1H), 7.70 (t, *J* = 1.6 Hz, 1H), 7.55 (t, *J* = 1.8 Hz, 1H), 7.51 (t, *J* = 2.0 Hz, 1H), 7.40 (t, *J* = 7.2 Hz, 1H), 4.27 - 4.00 (m, 3H), 2.82 - 2.69 (m, 1H), 2.46 - 2.24 (m, 4H). |
| **I-266** | $^1H$ NMR (400 MHz, DMSO-$d_6$) $\delta$ 8.98 (d, *J* = 7.6 Hz, 1H), 7.68 (t, *J* = 1.6 Hz, 1H), 7.54 - 7.50 (m, 2H), 7.39 (t, *J* = 7.2 Hz, 1H), 4.25 - 4.03 (m, 3H), 3.59 (s, 3H), 2.84 (tt, *J* = 10.0, 8.1 Hz, 1H), 2.46 - 2.25 (m, 4H). |
| **I-268** | $^1H$ NMR (400 MHz, DMSO-$d_6$) $\delta$ 12.12 (s, 1H), 8.98 (d, *J* = 7.6 Hz, 1H), 7.65 - 7.49 (m, 3H), 7.36 (td, *J* = 8.5, 2.5 Hz, 1H), 4.26 - 4.01 (m, 3H), 2.74 (p, *J* = 9.0 Hz, 1H), 2.47 - 2.21 (m, 4H). |
| **I-270** | $^1H$ NMR (400 MHz, DMSO-$d_6$) $\delta$ 9.01 (d, *J* = 7.7 Hz, 1H), 7.67 - 7.51 (m, 3H), 7.40 (td, *J* = 8.7, 2.7 Hz, 1H), 4.29 - 4.00 (m, 3H), 3.60 (s, 3H), 2.93 - 2.79 (m, 1H), 2.47 - 2.25 (m, 4H). |
| **I-274** | $^1H$ NMR (400 MHz, DMSO-$d_6$) $\delta$ 8.99 (d, *J* = 7.6 Hz, 1H), 7.44 (s, 1H), 7.37 (d, *J* = 9.5 Hz, 1H), 7.23 (d, *J* = 9.7 Hz, 1H), 4.28 - 4.14 (m, 1H), 4.06 (d, *J* = 10.7 Hz, 2H), 3.59 (s, 3H), 2.83 (dd, *J* = 17.2, 8.9 Hz, 1H), 2.46 - 2.22 (m, 7H). |
| **I-278** | $^1H$ NMR (400 MHz, DMSO-$d_6$) $\delta$ 9.03 (d, *J* = 7.6 Hz, 1H), 7.81 - 7.66 (m, 2H), 4.28 - 4.00 (m, 3H), 3.59 (s, 3H), 2.91 - 2.78 (m, 1H), 2.46 - 2.23 (m, 4H). |
| **I-282** | $^1H$ NMR (400 MHz, DMSO-$d_6$) $\delta$ 9.01 (d, *J* = 7.7 Hz, 1H), 8.02 - 7.66 (m, 2H), 4.26 - 4.00 (m, 3H), 3.59 (s, 3H), 2.91 - 2.77 (m, 1H), 2.44 - 2.23 (m, 4H). |
| **I-286** | $^1H$ NMR (400 MHz, DMSO-$d_6$) $\delta$ 8.98 (d, *J* = 7.5 Hz, 1H), 7.96 (d, *J* = 6.3 Hz, 2H), 4.28 - 3.97 (m, 3H), 3.59 (s, 3H), 2.90 - 2.75 (m, 1H), 2.45 - 2.21 (m, 4H). |
| **I-290** | $^1H$ NMR (400 MHz, DMSO-$d_6$) $\delta$ 9.00 (d, *J* = 7.6 Hz, 1H), 7.76 (d, *J* = 7.0 Hz, 2H), 7.60 - 7.40 (m, 3H), 4.28 - 4.16 (m, 1H), 4.14 - 3.98 (m, 2H), 3.60 (s, 3H), 2.92 - 2.75 (m, 1H), 2.46 - 2.25 (m, 4H). |
| **I-294** | $^1H$ NMR (400 MHz, DMSO-$d_6$) $\delta$ 8.98 (d, *J* = 7.6 Hz, 1H), 7.37 (s, 2H), 7.16 (s, 1H), 4.20 (dd, *J* = 16.4, 8.3 Hz, 1H), 4.01 (d, *J* = 7.1 Hz, 2H), 3.59 (s, 3H), 2.90 - 2.77 (m, 1H), 2.43 - 2.31 (m, 4H), 2.30 (s, 6H). |
| **I-298** | $^1H$ NMR (400 MHz, $CDCl_3$-*d*) $\delta$ 7.11 (d, *J* = 8.1 Hz, 1H), 6.78 (s, 2H), 6.55 (s, 1H), 4.39 (d, *J* = 8.3 Hz, 1H), 3.94 (d, *J* = 18.0 Hz, 1H), 3.81 (s, 6H), 3.75 (d, *J* = 18.0 Hz, 1H), 3.69 (d, *J* = 1.0 Hz, 3H), 2.92 - 2.79 (m, 1H), 2.73 - 2.55 (m, 2H), 2.28 - 2.15 (m, 2H). |
| **I-303** | 1H NMR (400 MHz, $CDCl_3$) $\delta$ 7.23 - 7.13 (m, 2H), 7.10 (d, J = 8.3 Hz, 1H), 6.92 (tt, J = 8.7, 2.3 Hz, 1H), 6.13 (dd, J = 54.9, 53.5 Hz, 1H), 4.45 - 4.29 (m, 1H), 3.70 (s, 4H), 3.60 (d, J = 17.7 Hz, 1H), 2.86 (tt, J = 9.4, 8.0 Hz, 1H), 2.64 (dddd, J = 24.4, 12.3, 7.7, 4.0 Hz, 2H), 2.33 - 2.15 (m, 2H). |
| **I-307** | 1H NMR (400 MHz, $CDCl_3$) $\delta$ 7.40 (d, J = 1.7 Hz, 1H), 7.28 (dt, J = 8.8, 1.9 Hz, 1H), 7.18 (dt, J = 8.6, 2.0 Hz, 2H), 6.32 - 5.92 (m, 1H), 4.46 - 4.24 (m, 1H), 3.73 (d, J = 17.7 Hz, 2H), 3.69 (s, 3H), 3.60 (d, J = 17.7 Hz, 1H), 2.92 - 2.76 (m, 1H), 2.73 - 2.53 (m, 2H), 2.22 (dq, J = 11.4, 8.9 Hz, 2H). |
| **I-310** | $^1H$ NMR (400 MHz, DMSO-$d_6$) $\delta$ 8.64 (d, *J* = 7.8 Hz, 1H), 7.48 - 7.36 (m, 3H), 4.69 (ddd, *J* = 57.5, 46.9, 10.1 Hz, 2H), 4.27 - 4.12 (m, 1H), 3.69 (dd, *J* = 18.1, 1.6 Hz, 1H), 3.59 (s, 3H), 3.52 (d, *J* = 18.1 Hz, 1H), 2.89 - 2.76 (m, 1H), 2.45 - 2.21 (m, 4H). |
| **I-312** | $^1H$ NMR (400 MHz, DMSO-$d_6$) $\delta$ 12.09 (s, 1H), 8.61 (d, *J* = 7.7 Hz, 1H), 7.62 - 7.50 (m, 3H), 4.68 (m, 2H), 4.16 (h, *J* = 8.4 Hz, 1H), 3.70 (d, *J*= 18.1 Hz, 1H), 3.53 (d, *J* = 18.1 Hz, 1H), 2.79 - 2.65 (m, 1H), 2.42 - 2.18 (m, 4H). |
| **I-314** | $^1H$ NMR (400 MHz, Chloroform-d) $\delta$ 7.41 (s, 1H), 7.29 (m, 1H), 7.20 - 7.10 (m, 2H), 4.72 (m, 2H),, 4.35 (h, *J* = 8.3 Hz, 1H), 3.69 (d, *J* = 1.0 Hz, 3H), 3.64 (d, *J* = 17.5 Hz, 1H), 3.44 (d, *J* = 17.5 Hz, 1H), 2.85 (m, 1H), 2.63 (m, 2H), 2.32 - 2.10 (m, 2H). |

(continued)

| NO. | $^1$H NMR |
|---|---|
| **I-316** | $^1$H NMR (400 MHz, DMSO-$d_6$) $\delta$ 11.70 (s, 1H), 8.61 (d, $J$ = 7.7 Hz, 1H), 7.72 (dd, $J$ = 10.3, 1.9 Hz, 3H), 4.69 (ddd, $J$ = 64.0, 46.9, 10.1 Hz, 2H), 4.18 (dp, $J$ = 16.6, 8.0 Hz, 1H), 3.72 (dd, $J$ = 17.9, 2.0 Hz, 1H), 3.55 (d, $J$ = 18.1 Hz, 1H), 2.80 - 2.66 (m, 1H), 2.47 - 2.20 (m, 4H). |
| **I-318** | $^1$H NMR (400 MHz, DMSO-$d_6$) $\delta$ 8.64 (d, $J$ = 7.8 Hz, 1H), 7.76 (t, $J$ = 1.9 Hz, 1H), 7.71 (d, $J$ = 1.9 Hz, 2H), 4.68 (ddd, $J$ = 62.9, 46.9, 10.1 Hz, 2H), 4.19 (dp, $J$ = 16.6, 8.3, 7.9 Hz, 1H), 3.71 (dd, $J$ = 18.0, 1.8 Hz, 1H), 3.59 (s, 3H), 3.54 (d, $J$ = 18.1 Hz, 1H), 2.89 - 2.75 (m, 1H), 2.42 - 2.22 (m, 4H). |
| **I-322** | 1H NMR (400 MHz, Chloroform-*d*) $\delta$ 7.36 - 7.29 (m, 2H), 7.29 - 7.20 (m, 1H), 7.05 (d, J = 8.6 Hz, 1H), 4.87 - 4.59 (m, 2H), 4.44 - 4.27 (m, 1H), 3.69 (s, 3H), 3.66 (d, J = 17.5 Hz, 1H), 3.47 (d, J = 17.5 Hz, 1H), 2.91 - 2.80 (m, 1H), 2.72 - 2.55 (m, 2H), 2.31 - 2.17 (m, 2H). |
| **I-326** | 1H NMR (400 MHz, Chloroform-d) $\delta$ 7.25 - 7.20 (m, 2H), 7.14 (d, J = 8.3 Hz, 1H), 7.00 - 6.94 (m, 1H), 6.55 (t, J = 72.6 Hz, 1H), 4.88 - 4.57 (m, 2H) , 4.41 - 4.29 (m, 1H), 3.70 (s, 3H), 3.65 (d, J = 17.5 Hz, 1H), 3.45 (d, J = 17.5 Hz, 1H), 2.90 - 2.78 (m, 1H), 2.72 - 2.56 (m, 2H), 2.27 - 2.14 (m, 2H). |
| **I-328** | $^1$H NMR (400 MHz, DMSO) $\delta$ 8.64 (d, $J$ = 7.8 Hz, 1H), 7.78 (t, $J$ = 1.6 Hz, 1H), 7.73 (s, 1H), 7.66 (d, $J$ = 0.9 Hz, 1H), 4.69 (ddd, $J$ = 57.5, 46.9, 10.1 Hz, 2H), 4.26 - 4.12 (m, 1H), 3.73 (dd, $J$ = 18.1, 1.6 Hz, 1H), 3.59 (s, 3H), 3.56 (d, $J$ = 18.1 Hz, 1H), 2.88 - 2.76 (m, 1H), 2.44 - 2.22 (m, 4H). |
| **I-329** | $^1$H NMR (400 MHz, DMSO) $\delta$ 8.66 (d, $J$ = 7.8 Hz, 1H), 8.21 - 8.16 (m, 1H), 8.11 (t, $J$ = 1.3 Hz, 1H), 8.06 (t, $J$ = 1.7 Hz, 1H), 4.69 (ddd, $J$ = 57.5, 46.9, 10.1 Hz, 2H), 4.25 - 4.14 (m, 1H), 3.74 (dd, $J$ = 18.2, 1.5 Hz, 1H), 3.59 (s, 3H), 3.55 (d, $J$ = 18.2 Hz, 1H), 2.90 - 2.76 (m, 1H), 2.43 - 2.20 (m, 4H). |
| **I-330** | $^1$H NMR (400 MHz, DMSO-$d_6$) $\delta$ 8.66 (d, $J$ = 7.8 Hz, 1H), 7.88 - 7.77 (m, 3H), 4.79 (dd, $J$ = 46.5, 10.1 Hz, 1H), 4.64 (dd, $J$ = 47.3, 10.1 Hz, 1H), 4.29 - 4.15 (m, 1H), 3.78 (dd, $J$ = 18.1, 1.8 Hz, 1H), 3.62 (dd, $J$ = 18.1, 1.8 Hz, 1H), 3.59 (s, 3H), 2.96 - 2.74 (m, 1H), 2.46 - 2.21 (m, 4H). |
| **I-331** | $^1$H NMR (400 MHz, DMSO-$d_6$) $\delta$ 8.65 (d, $J$ = 7.8 Hz, 1H), 8.07 - 7.99 (m, 2H), 7.96 (d, $J$ = 2.1 Hz, 1H), 4.77 (dd, $J$ = 46.4, 10.1 Hz, 1H), 4.61 (dd, $J$ = 47.4, 10.1 Hz, 1H), 4.28 - 4.10 (m, 1H), 3.78 (dd, $J$ = 18.1, 1.8 Hz, 1H), 3.62 (dd, $J$ = 18.1, 1.8 Hz, 1H), 3.59 (s, 3H), 2.92 - 2.72 (m, 1H), 2.44 - 2.19 (m, 4H). |
| **I-332** | $^1$H NMR (400 MHz, DMSO) $\delta$ 8.66 (d, $J$ = 7.4 Hz, 1H), 7.73 - 7.57 (m, 2H), 4.86 - 4.51 (m, 2H), 4.27 - 4.09 (m, 1H), 3.69 (d, $J$ = 18.1 Hz, 1H), 3.59 (s, 3H), 3.50 (d, $J$ = 18.1 Hz, 1H), 2.91 - 2.73 (m, 1H), 2.43 - 2.20 (m, 4H). |
| **I-333** | $^1$H NMR (400 MHz, DMSO-$d_6$) $\delta$ 12.15 (s, 1H), 8.65 (m, 1H), 7.47 (m, 3H), 4.22 (p, $J$ = 8.3 Hz, 1H), 4.17 - 4.00 (m, 2H), 3.84 (d, $J$ = 18.2 Hz, 1H), 3.61 (d, $J$ = 18.2 Hz, 1H), 2.77 (p, $J$ = 9.0 Hz, 1H), 2.48 - 2.21 (m, 4H). |
| **I-335** | 1H NMR (400 MHz, Chloroform-d) $\delta$ 7.21 - 7.08 (m, 3H), 6.91 (m, 1H), 4.35 (h, $J$ = 8.4 Hz, 1H), 4.03 - 3.89 (m, 2H), 3.70 (d, $J$ = 1.0 Hz, 3H), 3.65 (d, $J$ = 17.6 Hz,, 1H), 3.53 (d, $J$ = 17.6 Hz, 1H), 2.85 (p, $J$ = 8.7 Hz, 1H), 2.64 (m, 2H), 2.21 (p, $J$ = 9.7 Hz, 2H). |
| **I-337** | $^1$H NMR (400 MHz, DMSO-$d_6$) $\delta$ 12.10 (s, 1H), 8.59 (d, $J$ = 7.6 Hz, 1H), 7.66 - 7.56 (m, 3H), 4.21 - 4.10 (m, 1H), 4.10 - 3.93 (m, 2H), 3.79 (d, $J$ = 18.1 Hz, 1H), 3.57 (d, $J$ = 18.2 Hz, 1H), 2.79 - 2.66 (m, 1H), 2.43 - 2.17 (m, 4H). |
| **I-339** | $^1$H NMR (400 MHz, Chloroform-d) $\delta$ 7.41 (q, $J$ = 1.5 Hz, 1H), 7.28 (m, 1H), 7.21 - 7.08 (m, 2H), 4.35 (h, $J$ = 8.3 Hz, 1H), 4.05 - 3.89 (m, 2H), 3.69 (s, 3H), 3.65 (dd, $J$ = 17.6, 1.2 Hz, 1H), 3.53 (dd, $J$ = 17.6, 1.2 Hz, 1H), 2.85 (m, 1H), 2.72 - 2.55 (m, 2H), 2.28 - 2.14 (m, 2H). |
| **I-341** | $^1$H NMR (400 MHz, DMSO-$d_6$) $\delta$ 11.70 (s, 1H), 8.58 (d, $J$ = 7.7 Hz, 1H), 7.73 (dd, $J$ = 11.8, 1.9 Hz, 3H), 4.22 - 4.10 (m, 1H), 4.07 (d, $J$ = 11.7 Hz, 1H), 3.97 (d, $J$ = 11.8 Hz, 1H), 3.78 - 3.74 (m, 1H), 3.58 (d, $J$ = 18.2 Hz, 1H), 2.81 - 2.66 (m, 1H), 2.46 - 2.18 (m, 4H). |
| **I-343** | $^1$H NMR (400 MHz, DMSO-$d_6$) $\delta$ 8.61 (d, $J$ = 7.7 Hz, 1H), 7.76 (t, $J$ = 1.9 Hz, 1H), 7.72 (d, $J$ = 1.9 Hz, 2H), 4.26 - 4.10 (m, 1H), 4.06 (d, $J$ = 11.7 Hz, 1H), 3.97 (d, $J$ = 11.8 Hz, 1H), 3.80 (d, $J$ = 18.2 Hz, 1H), 3.66 - 3.51 (m, 4H), 2.90 - 2.76 (m, 1H), 2.45 - 2.21 (m, 4H). |
| **I-347** | $^1$H NMR (400 MHz, DMSO-$d_6$) $\delta$ 8.80 (d, $J$ = 7.7 Hz, 1H), 7.44 (dd, $J$ = 13.8, 4.7 Hz, 3H), 4.26 - 4.11 (m, 1H), 3.93 - 3.81 (m, 2H), 3.59 (s, 3H), 2.92 - 2.76 (m, 1H), 2.46 - 2.21 (m, 4H), 1.74 (t, $J$ = 19.4 Hz, 3H). |

(continued)

| NO. | $^1H$ NMR |
|---|---|
| **I-349** | 1H NMR (400 MHz, Chloroform-d) δ 7.43 (t, J = 1.7 Hz, 1H), 7.33 - 7.28 (m, 1H), 7.21 - 7.16 (m, 1H), 7.12 (d, J = 8.2 Hz, 1H), 4.44 - 4.31 (m, 1H), 3.80 (d, J = 17.8 Hz, 1H), 3.70 (s, 3H), 3.65 (d, J = 17.8 Hz, 1H), 2.92 - 2.81 (m, 1H), 2.74 - 2.56 (m, 2H), 2.26 - 2.16 (m, 2H), 1.76 (t, J = 18.7 Hz, 3H). |
| **I-350** | $^1H$ NMR (400 MHz, DMSO-$d_6$) δ 12.11 (s, 1H), 8.77 (d, *J* = 7.6 Hz, 1H), 7.81 - 7.70 (m, 3H), 4.26 - 4.11 (m, 1H), 3.99 - 3.82 (m, 2H), 2.74 (tt, *J* = 9.9, 8.0 Hz, 1H), 2.45 - 2.21 (m, 4H), 1.77 (t, *J* = 19.4 Hz, 3H). |
| **I-352** | $^1H$ NMR (400 MHz, DMSO-$d_6$) δ 8.77 (d, *J* = 7.7 Hz, 1H), 7.83 - 7.68 (m, 3H), 4.25 - 4.09 (m, 1H), 3.99 - 3.80 (m, 2H), 3.59 (s, 3H), 2.83 (tt, *J* = 10.0, 8.1 Hz, 1H), 2.46 - 2.23 (m, 4H), 1.74 (t, *J* = 19.4 Hz, 3H). |
| **I-356** | $^1H$ NMR (400 MHz, DMSO-$d_6$) δ 8.77 (d, *J* = 7.7 Hz, 1H), 7.54 - 7.36 (m, 3H), 5.10 - 4.88 (m, 2H), 4.25 - 4.12 (m, 1H), 3.90 (s, 2H), 3.59 (s, 3H), 2.90 - 2.77 (m, 1H), 2.46 - 2.22 (m, 4H). |
| **I-357** | 1H NMR (400 MHz, Chloroform-d) δ 7.42 (d, J = 1.6 Hz, 1H), 7.32 - 7.27 (m, 1H), 7.21 - 7.16 (m, 1H), 7.07 (d, J = 8.2 Hz, 1H), 5.01 - 4.77 (m, 2H), 4.44 - 4.32 (m, 1H), 3.92 (d, J = 17.5 Hz, 1H), 3.70 (s, 3H), 3.64 (d, J = 17.5 Hz, 1H), 2.92 - 2.80 (m, 1H), 2.74 - 2.58 (m, 2H), 2.28 - 2.15 (m, 2H). |
| **I-358** | $^1H$ NMR (400 MHz, DMSO-$d_6$) δ 12.13 (s, 1H), 8.75 (d, *J* = 7.6 Hz, 1H), 7.84 - 7.66 (m, 3H), 5.12 - 5.05 (m, 1H), 4.99 (dd, *J* = 36.7, 4.2 Hz, 1H), 4.29 - 4.12 (m, 1H), 3.93 (s, 2H), 2.82 - 2.66 (m, 1H), 2.46 - 2.21 (m, 4H). |
| **I-360** | $^1H$ NMR (400 MHz, DMSO-$d_6$) δ 8.74 (d, *J* = 7.7 Hz, 1H), 7.84 - 7.69 (m, 3H), 5.08 - 5.04 (m, 1H), 4.97 (dd, *J* = 36.3, 4.2 Hz, 1H), 4.25 - 4.12 (m, 1H), 3.91 (s, 2H), 3.59 (s, 3H), 2.83 (tt, *J* = 9.9, 8.0 Hz, 1H), 2.46 - 2.23 (m, 4H). |
| **I-362** | $^1H$ NMR (400 MHz, DMSO) δ 8.67 (d, *J* = 7.9 Hz, 1H), 7.55 - 7.33 (m, 3H), 4.27 - 4.15 (m, 1H), 3.80 (d, *J* = 18.8 Hz, 1H), 3.62 (d, *J* = 16.3 Hz, 4H), 3.24 (s, 3H), 2.92 - 2.78 (m, 1H), 2.46 - 2.35 (m, 2H), 2.35 - 2.19 (m, 2H). |
| **I-363** | $^1H$ NMR (400 MHz, DMSO-$d_6$) δ 8.65 (d, *J* = 7.9 Hz, 1H), 7.83 - 7.70 (m, 3H), 4.29 - 4.16 (m, 1H), 3.82 (d, *J* = 18.8 Hz, 1H), 3.65 (d, *J* = 18.8 Hz, 1H), 3.60 (s, 3H), 3.24 (s, 3H), 2.92 - 2.76 (m, 1H), 2.46 - 2.35 (m, 2H), 2.35 - 2.23 (m, 2H). |
| **I-364** | 1H NMR (400 MHz, DMSO) δ 8.57 (d, J = 7.9 Hz, 1H), 7.45 - 7.37 (m, 3H), 6.19 - 6.05 (m, 1H), 5.42 - 5.25 (m, 2H), 4.40 (dd, J = 16.1, 8.1 Hz, 1H), 3.87 (d, J = 17.8 Hz, 1H), 3.62 (s, 3H), 3.53 (d, J = 17.8 Hz, 1H), 3.05 - 2.93 (m, 1H), 2.41 - 2.25 (m, 4H). |
| **I-365** | $^1H$ NMR (400 MHz, DMSO-$d_6$) δ 8.93 (s, 1H), 7.40 (dd, *J* = 14.6, 4.6 Hz, 3H), 6.12 (dd, *J* = 17.2, 10.6 Hz, 1H), 5.34 (dd, *J* = 28.3, 14.0 Hz, 2H), 3.92 (s, 1H), 3.60 (s, 3H), 3.52 (d, *J* = 17.8 Hz, 1H), 2.29 - 2.19 (m, 6H). |
| **I-366** | $^1H$ NMR (400 MHz, DMSO-$d_6$) δ 8.15 (s, 1H), 7.51 - 7.28 (m, 3H), 6.15 (dd, *J* = 17.3, 10.6 Hz, 1H), 5.34 (dd, *J* = 31.4, 14.0 Hz, 2H), 3.89 (d, *J* = 17.8 Hz, 1H), 3.60 (s, 3H), 3.53 (d, *J* = 17.8 Hz, 1H), 1.99 - 1.85 (m, 4H), 1.85 - 1.73 (m, 4H), 1.62 (d, *J* = 11.4 Hz, 2H). |
| **I-367** | $^1H$ NMR (400 MHz, DMSO-$d_6$) δ 7.41 (dd, *J* = 14.2, 4.8 Hz, 3H), 7.16 (s, 1H), 6.13 (dd, *J* = 17.3, 10.7 Hz, 1H), 5.33 (dd, *J* = 31.4, 14.0 Hz, 2H), 3.85 (d, *J* = 17.8 Hz, 1H), 3.56 (s, 3H), 3.52 (d, *J* = 17.8 Hz, 1H), 1.93 - 1.81 (m, 6H), 1.81 - 1.70 (m, 6H). |
| **I-368** | $^1H$ NMR (400 MHz, $CDCl_3$-*d*) δ 7.67 (s, 1H), 7.63 - 7.44 (m, 2H), 7.40 (d, *J* = 8.0 Hz, 1H), 6.16 (dd, *J* = 17.3, 10.7 Hz, 1H), 5.54 (dd, *J* = 17.3, 6.5 Hz, 1H), 5.35 (dd, *J* = 10.8, 5.8 Hz, 1H), 4.61 - 4.53 (m, 2H), 4.09 - 3.89 (m, 3H), 3.81 (d, *J* = 7.5 Hz, 3H), 3.36 (dd, *J* = 17.3, 3.9 Hz, 1H), 2.66 - 2.39 (m, 1H), 2.15 - 1.98 (m, 1H). |
| **I-369** | $^1H$ NMR (400 MHz, Chloroform-d) δ 7.54 - 7.42 (m, 1H), 7.35 - 7.28 (m, 2H), 7.03 (d, *J* = 8.7 Hz, 1H), 6.20 - 6.07 (m, 1H), 5.60 - 5.49 (m, 1H), 5.40 - 5.32 (m, 1H), 4.66 - 4.53 (m, 2H), 4.09 - 3.97 (m, 1H), 3.97 - 3.86 (m, 2H), 3.85 - 3.75 (m, 3H), 3.37 - 3.26 (m, 1H), 2.63 - 2.45 (m, 1H), 2.13 - 1.97 (m, 1H). |
| **I-370** | $^1H$ NMR (400 MHz, Chloroform-d) δ 7.41 (dd, *J* = 14.3, 8.3 Hz, 1H), 7.18 - 7.10 (m, 2H), 6.93 - 6.83 (m, 1H), 6.69 - 6.26 (m, 1H), 6.13 - 6.01 (m, 1H), 5.51 - 5.41 (m, 1H), 5.32 - 5.20 (m, 1H), 4.59 - 4.45 (m, 2H), 4.01 - 3.78 (m, 3H), 3.74 (dd, 3H), 3.32 - 3.17 (m, 1H), 2.54 - 2.40 (m, 1H), 2.09 - 1.90 (m, 1H) |
| **I-371** | $^1H$ NMR (400 MHz, Chloroform-d) δ 7.88 - 7.74 (m, 2H), 7.66 (s, 1H), 7.49 (dd, *J* = 14.7, 8.2 Hz, 1H), 6.15 (dd, *J* = 17.2, 10.7 Hz, 1H), 5.54 (dd, *J* = 17.3, 6.6 Hz, 1H), 5.35 (dd, *J* = 10.7, 5.7 Hz, 1H), 4.70 - 4.49 (m, 2H), 4.12 - 3.89 (m, 3H), 3.80 (s, 3H), 3.43 - 3.27 (m, 1H), 2.67 - 2.42 (m, 1H), 2.17 - 1.97 (m, 1H). |

(continued)

| NO. | $^1$H NMR |
|---|---|
| **I-372** | $^1$H NMR (400 MHz, DMSO-$d_6$) $\delta$ 8.20 (t, *J* = 6.3 Hz, 1H), 7.84 - 7.63 (m, 3H), 6.13 (ddd, *J* = 17.2, 10.6, 4.0 Hz, 1H), 5.35 (dd, *J* = 29.5, 14.0 Hz, 2H), 4.54 - 4.43 (m, 1H), 4.39 - 4.27 (m, 1H), 3.95 - 3.84 (m, 2H), 3.73 - 3.57 (m, 5H), 2.49 - 2.38 (m, 1H), 2.11 - 1.95 (m, 1H). |
| **I-373** | $^1$H NMR (400 MHz, Chloroform-d) $\delta$ 7.54 - 7.42 (m, 2H), 7.33 (s, 1H), 7.21 (d, *J* = 2.4 Hz, 1H), 6.56 (t, *J* = 72.7 Hz, 1H), 6.14 (dd, *J* = 17.2, 10.7 Hz, 1H), 5.53 (dd, *J* = 17.3, 6.3 Hz, 1H), 5.41 - 5.32 (m, 1H), 4.69 - 4.45 (m, 2H), 4.13 - 3.98 (m, 1H), 3.98 - 3.85 (m, 2H), 3.80 (d, *J* = 6.3 Hz, 3H), 3.32 (dd, *J* = 17.3, 3.5 Hz, 1H), 2.72 - 2.39 (m, 1H), 2.25 - 1.97 (m, 1H). |
| **I-374** | $^1$H NMR (400 MHz, $CDCl_3$-*d*) $\delta$ 7.47 (t, *J* = 7.7 Hz, 1H), 7.36 - 7.20 (m, 2H), 6.13 (dd, *J* = 17.2, 10.7 Hz, 1H), 5.53 (dd, *J* = 17.2, 6.2 Hz, 1H), 5.34 (dd, *J* = 10.6, 5.7 Hz, 1H), 4.70 - 4.45 (m, 2H), 4.03 (dd, *J* = 13.2, 7.8 Hz, 1H), 3.97 - 3.73 (m, 5H), 3.27 (dd, *J* = 17.2, 3.4 Hz, 1H), 2.66 - 2.45 (m, 1H), 2.18 - 1.93 (m, 1H). |
| **I-375** | $^1$H NMR (400 MHz, DMSO-$d_6$) $\delta$ 8.22 (t, *J* = 7.7 Hz, 1H), 7.79 (dd, *J* = 20.5, 7.4 Hz, 2H), 6.19 - 6.04 (m, 1H), 5.35 (dd, *J* = 25.1, 13.9 Hz, 2H), 4.53 - 4.43 (m, 1H), 4.40 - 4.26 (m, 1H), 3.97 - 3.81 (m, 2H), 3.75 - 3.49 (m, 5H), 2.49 - 2.39 (m, 1H), 2.08 - 2.02 (m, 1H). |
| **I-376** | $^1$H NMR (400 MHz, DMSO-$d_6$) $\delta$ 8.20 (t, *J* = 7.6 Hz, 1H), 7.91 (d, *J* = 6.4 Hz, 2H), 6.12 (ddd, *J* = 17.2, 10.6, 3.6 Hz, 1H), 5.35 (dd, *J* = 27.3, 14.0 Hz, 2H), 4.52 - 4.44 (m, 1H), 4.38 - 4.26 (m, 1H), 3.94 - 3.84 (m, 2H), 3.70 (d, *J* = 8.8 Hz, 1H), 3.66 (d, *J* = 11.8 Hz, 3H), 3.58 (d, *J* = 17.8 Hz, 1H), 2.49 - 2.39 (m, 1H), 2.10 - 2.02 (m, 1H). |
| **I-377** | $^1$H NMR (400 MHz, $CDCl_3$-*d*) $\delta$ 7.65 (s, 1H), 7.59 - 7.35 (m, 3H), 4.62 - 4.51 (m, 2H), 4.07 - 3.98 (m, 1H), 3.98 - 3.86 (m, 1H), 3.86 - 3.75 (m, 4H), 3.21 (dd, *J* = 17.3, 3.4 Hz, 1H), 2.62 - 2.46 (m, 1H), 2.14 - 1.95 (m, 1H), 1.71 (d, *J* = 9.5 Hz, 3H). |
| **I-378** | $^1$H NMR (400 MHz, DMSO-$d_6$) $\delta$ 12.58 (s, 1H), 8.10 (dd, *J* = 22.5, 7.3 Hz, 1H), 7.62 - 7.50 (m, 3H), 4.40 - 4.27 (m, 2H), 3.95 - 3.63 (m, 3H), 3.39 (dd, *J* = 17.7, 2.2 Hz, 1H), 2.49 - 2.37 (m, 1H), 2.08 - 1.93 (m, 1H), 1.54 (s, 3H). |
| **I-380** | $^1$H NMR (400 MHz, Chloroform-d) $\delta$ 7.49 (dd, *J* = 18.3, 8.2 Hz, 1H), 7.33 - 7.28 (m, 2H), 7.06 - 7.01 (m, 1H), 4.63 - 4.53 (m, 2H), 4.08 - 3.99 (m, 1H), 3.98 - 3.88 (m, 1H), 3.85 - 3.72 (m, 4H), 3.18 (dd, *J*= 17.3, 3.3 Hz, 1H), 2.63 - 2.44 (m, 1H), 2.16 - 1.98 (m, 1H), 1.71 (d, *J* = 9.5 Hz, 3H). |
| **I-382** | $^1$H NMR (400 MHz, Chloroform-d) $\delta$ 7.47 (t, *J* = 8.8 Hz, 1H), 7.36 - 7.28 (m, 2H), 7.03 (d, *J* = 8.6 Hz, 1H), 4.64 - 4.52 (m, 2H), 4.46 - 4.29 (m, 2H), 4.10 - 3.88 (m, 2H), 3.77 (dd, *J* = 17.3, 10.5 Hz, 1H), 3.18 (dd, *J* = 17.3, 4.0 Hz, 1H), 2.86 - 2.73 (m, 2H), 2.64 - 2.50 (m, 1H), 2.22 - 2.01 (m, 4H), 1.71 (d, *J* = 7.4 Hz, 3H). |
| **I-383** | 1H NMR (400 MHz, Chloroform-d) $\delta$ 7.59 (dd, J = 20.7, 7.8 Hz, 1H), 7.33 - 7.28 (m, 2H), 7.03 (d, J = 8.8 Hz, 1H), 4.73 - 4.50 (m, 4H), 4.07 - 3.88 (m, 2H), 3.84 - 3.72 (m, 1H), 3.21 - 3.04 (m, 3H), 2.75 - 2.64 (m, 3H), 2.61 - 2.50 (m, 1H), 2.25 - 2.09 (m, 1H), 1.74 - 1.67 (m, 3H). |
| **I-384** | $^1$H NMR (400 MHz, Chloroform-d) $\delta$ 7.45 (d, *J* = 7.5 Hz, 1H), 7.35 - 7.28 (m, 2H), 7.04 (d, *J* = 8.7 Hz, 1H), 4.70 (t, *J* = 5.7 Hz, 2H), 4.64 - 4.49 (m, 2H), 4.06 - 3.98 (m, 1H), 3.96 - 3.87 (m, 1H), 3.79 (d, *J* = 17.3 Hz, 1H), 3.59 - 3.39 (m, 2H), 3.19 (d, *J* = 17.3 Hz, 1H), 3.07 (s, 3H), 2.63 - 2.51 (m, 1H), 2.33 - 2.23 (m, 1H), 1.71 (s, 3H). |
| **I-385** | $^1$H NMR (400 MHz, Chloroform-d) $\delta$ 7.54 - 7.43 (m, 1H), 7.24 - 7.17 (m, 2H), 6.97 - 6.91 (m, 1H), 6.55 (t, *J* = 72.7 Hz, 1H), 4.64 - 4.50 (m, 2H), 4.07 - 3.99 (m, 1H), 3.99 - 3.87 (m, 1H), 3.85 - 3.71 (m, 4H), 3.18 (dd, *J* = 17.3, 3.2 Hz, 1H), 2.61 - 2.48 (m, 1H), 2.15 - 1.98 (m, 2H), 1.70 (d, *J* = 9.4 Hz, 3H). |
| **I-386** | $^1$H NMR (400 MHz, $CDCl_3$-*d*) $\delta$ 7.82 (d, *J* = 1.3 Hz, 1H), 7.75 (d, *J* = 11.2 Hz, 1H), 7.66 (s, 1H), 7.59 - 7.44 (m, 1H), 4.65 - 4.50 (m, 2H), 4.08 - 3.98 (m, 1H), 3.98 - 3.87 (m, 1H), 3.87 - 3.75 (m, 4H), 3.21 (dd, *J* = 17.3, 3.3 Hz, 1H), 2.64 - 2.45 (m, 1H), 2.16 - 1.95 (m, 1H), 1.72 (d, *J* = 9.5 Hz, 3H). |
| **I-387** | $^1$H NMR (400 MHz, $CDCl_3$-*d*) $\delta$ 7.55 (s, 1H), 7.54 - 7.44 (m, 1H), 7.41 (s, 1H), 7.29 (s, 1H), 4.65 - 4.48 (m, 2H), 4.08 - 3.98 (m, 1H), 3.98 - 3.86 (m, 1H), 3.86 - 3.72 (m, 4H), 3.18 (dd, *J* = 17.3, 3.3 Hz, 1H), 2.66 - 2.42 (m, 1H), 2.20 - 1.93 (m, 1H), 1.71 (d, *J* = 9.6 Hz, 3H). |
| **I-388** | $^1$H NMR (400 MHz, DMSO-$d_6$) $\delta$ 12.71 (s, 1H), 8.12 (dd, *J* = 23.3, 7.3 Hz, 1H), 7.59 (d, *J* = 1.6 Hz, 1H), 7.57 (t, *J* = 72, 1H), 7.45 (d, *J* = 2.1 Hz, 2H), 4.41 - 4.24 (m, 2H), 3.95 - 3.83 (m, 1H), 3.81 - 3.62 (m, 2H), 3.41 (d, *J* = 2.3 Hz, 1H), 2.45 (dt, *J* = 12.9, 8.7 Hz, 1H), 2.09 - 1.89 (m, 1H), 1.54 (s, 3H). |

(continued)

| NO. | $^1$H NMR |
|---|---|
| **I-390** | $^1$H NMR (400 MHz, DMSO-$d_6$) δ 8.09 (d, *J* = 7.1 Hz, 1H), 7.59 (t, *J*= 1.6 Hz, 1H), 7.57 (dt, *J* = 72, 1.2 Hz, 1H), 7.45 (dd, *J* = 3.1, 1.7 Hz, 2H), 4.52 -4.44 (m, 1H), 4.38 - 4.24 (m, 1H), 3.95 - 3.84 (m, 1H), 3.80 - 3.63(m, 2H), 3.67 (d, *J* = 20.0 Hz, 3H), 3.39 (d, *J* = 17.7 Hz, 1H), 2.49 - 2.40 (m, 1H), 2.13 - 1.94 (m, 1H), 1.54 (d, *J* = 3.1 Hz, 3H). |
| **I-392** | $^1$H NMR (400 MHz, $CDCl_3$-*d)* δ 7.77 (t, *J* = 9.1 Hz, 1H), 7.68 (s, 1H), 7.60 (d, *J* = 7.7 Hz, 1H), 7.45 (d, *J* = 8.0 Hz, 1H), 4.72 - 4.63 (m, 1H), 4.62 - 4.54 (m, 1H), 4.13 - 3.93 (m, 3H), 3.85 - 3.74 (m, 4H), 2.64 - 2.47 (m, 1H), 2.10 (t, *J* = 12.2 Hz, 1H). |
| **I-393** | $^1$H NMR (400 MHz, Chloroform-d) δ 7.77 (t, *J* = 8.9 Hz, 1H), 7.37 - 7.31 (m, 2H), 7.13 - 7.06 (m, 1H), 4.72 - 4.63 (m, 1H), 4.63 - 4.55 (m, 1H), 4.09 - 3.90 (m, 3H), 3.81 (d, *J* = 14.1 Hz, 3H), 3.78 (s, 1H), 2.63 - 2.46 (m, 1H), 2.16 - 2.05 (m, 1H). |
| **I-394** | $^1$H NMR (400 MHz, Chloroform-*d*) δ 7.76 (t, *J* = 8.8 Hz, 1H), 7.26 - 7.21 (m, 2H), 7.03 - 6.97 (m, 1H), 6.56 (t, *J* = 72.4 Hz, 1H), 4.72 - 4.63 (m, 1H), 4.63 - 4.55 (m, 1H), 4.11 - 3.89 (m, 3H), 3.81 (d, *J* = 13.3 Hz, 3H), 3.78 - 3.70 (m, 1H), 2.63 - 2.47 (m, 1H), 2.15 - 2.05 (m, 1H). |
| **I-395** | $^1$H NMR (400 MHz, DMSO-$d_6$) δ 12.73 (s, 1H), 8.71 (dd, *J* = 20.0, 7.2 Hz, 1H), 7.69 (t, *J* = 1.6 Hz, 1H), 7.53 (d, *J* = 1.7 Hz, 2H), 7.40 (t, *J* = 7.2 Hz, 1H), 4.45 - 4.32 (m, 2H), 4.21 (dd, *J* = 18.9, 1.8 Hz, 1H), 4.09 (dd, *J* = 18.8, 7.2 Hz, 1H), 3.97 - 3.79 (m, 1H), 3.78 - 3.62 (m, 1H), 2.55 - 2.40 (m, 1H), 2.16 - 1.96 (m, 1H). |
| **I-397** | $^1$H NMR (400 MHz, DMSO-$d_6$) δ 8.64 (dd, *J* = 11.0, 7.0 Hz, 1H), 7.69 (t, *J* = 1.6 Hz, 1H), 7.56 - 7.50 (m, 2H), 7.39 (t, *J* = 7.2 Hz, 1H), 4.52 - 4.45 (m, 1H), 4.42 - 4.33 (m, 1H), 4.21 (dd, *J* = 18.8, 2.7 Hz, 1H), 4.09 (dd, *J* = 18.8, 6.1 Hz, 1H), 3.98 - 3.83 (m, 1H), 3.80 - 3.70 (m, 1H), 3.65 (d, *J* = 16.3 Hz, 3H), 2.56 - 2.43 (m, 1H), 2.16 - 2.05 (m, 1H). |
| **I-399** | $^1$H NMR (400 MHz, $CDCl_3$-*d)* δ 7.85 (s, 1H), 7.83 - 7.73 (m, 2H), 7.72 (s, 1H), 4.71 - 4.63 (m, 1H), 4.62 - 4.54 (m, 1H), 4.08 - 3.93 (m, 3H), 3.89 - 3.69 (m, 4H), 2.64 - 2.45 (m, 1H), 2.15 - 2.06 (m, 1H). |
| **I-400** | $^1$H NMR (400 MHz, DMSO-$d_6$) δ 8.65 (dd, *J* = 9.6, 7.2 Hz, 1H), 7.90 - 7.68 (m, 3H), 4.53 -4.44 (m, 1H), 4.41 - 4.31 (m, 1H), 4.25 (dd, *J* = 18.9, 2.7 Hz, 1H), 4.11 (dd, *J* = 18.9, 5.4 Hz, 1H), 3.95 - 3.84 (m, 1H), 3.80 - 3.68 (m, 1H), 3.64 (d, *J* = 17.7 Hz, 3H), 2.58 - 2.42 (m, 1H), 2.15 - 2.05 (m, 1H). |
| **I-403** | $^1$H NMR (400 MHz, DMSO) δ 8.13 (d, *J* = 7.7 Hz, 1H), 7.50 - 7.28 (m, 3H), 6.14 (dd, *J* = 17.2, 10.7 Hz, 1H), 5.34 (dd, *J* = 31.3, 13.9 Hz, 2H), 4.10 (dd, *J* = 14.4, 7.2 Hz, 1H), 3.88 (d, *J* = 17.7 Hz, 1H), 3.60 (d, *J* = 7.1 Hz, 3H), 3.53 (d, *J* = 17.8 Hz, 1H), 2.81 (p, *J* = 7.5 Hz, 1H), 2.08 (ddd, *J* = 20.6, 15.9, 7.6 Hz, 1H), 1.88 - 1.78 (m, 3H), 1.77 - 1.67 (m, 1H), 1.59 (dt, *J* = 12.0, 5.9 Hz, 1H). |
| **I-404** | $^1$H NMR (400 MHz, DMSO) δ 8.15 (d, *J* = 7.7 Hz, 1H), 7.49 - 7.35 (m, 3H), 6.14 (dd, *J* = 17.3, 10.6 Hz, 1H), 5.37 (d, *J* = 17.3 Hz, 1H), 5.30 (d, *J* = 10.6 Hz, 1H), 4.11 (dt, *J* = 14.4, 7.2 Hz, 1H), 3.88 (d, *J* = 17.7 Hz, 1H), 3.59 (s, 3H), 3.53 (d, *J* = 17.8 Hz, 1H), 2.86 - 2.74 (m, 1H), 2.12 - 2.00 (m, 1H), 1.88 - 1.78 (m, 3H), 1.73 (dt, *J* = 12.7, 8.7 Hz, 1H), 1.65 - 1.53 (m, 1H). |
| **I-416** | $^1$H NMR (400 MHz, DMSO) δ 8.16 (d, *J* = 7.8 Hz, 1H), 7.47 - 7.36 (m, 3H), 6.14 (dd, *J* = 17.3, 10.6 Hz, 1H), 5.38 (dd, *J* = 17.3, 0.6 Hz, 1H), 5.34 - 5.26 (m, 1H), 4.17 - 4.04 (m, 1H), 3.88 (d, *J* = 17.7 Hz, 1H), 3.61 (s, 3H), 3.53 (d, *J* = 17.8 Hz, 1H), 2.81 (p, *J* = 8.3 Hz, 1H), 2.10 (dt, *J* = 12.7, 7.3 Hz, 1H), 1.87 - 1.76 (m, 3H), 1.75 - 1.67 (m, 1H), 1.65 - 1.52 (m, 1H). |
| **I-426** | 1H NMR (400 MHz, DMSO) δ 12.46 (s, 1H), 8.09 (d, J = 7.9 Hz, 1H), 7.85 (dd, J = 7.4, 5.5 Hz, 3H), 6.15 (dd, J = 17.3, 10.7 Hz, 1H), 5.91 (dt, J = 5.4, 2.0 Hz, 1H), 5.82 - 5.74 (m, 1H), 5.40 (d, J = 17.3 Hz, 1H), 5.31 (d, J = 10.7 Hz, 1H), 4.87 - 4.74 (m, 1H), 3.97 (d, J = 17.8 Hz, 1H), 3.63 (d, J = 17.8 Hz, 1H), 3.45 (ddd, J = 6.2, 5.2, 2.2 Hz, 1H), 2.38 (dt, J = 13.2, 8.3 Hz, 1H), 1.90 - 1.74 (m, 1H). |
| **I-428** | 1H NMR (400 MHz, $CDCl_3$) δ 7.68 (s, 1H), 7.58 (d, J = 8.9 Hz, 1H), 7.39 (d, J = 8.1 Hz, 1H), 7.22 (d, J = 8.6 Hz, 1H), 6.18 (dd, J = 17.2, 10.7 Hz, 1H), 5.96 (d, J = 5.3 Hz, 1H), 5.91 - 5.83 (m, 1H), 5.54 (d, J = 17.2 Hz, 1H), 5.34 (d, J = 10.7 Hz, 1H), 5.03 (t, J = 8.0 Hz, 1H), 3.98 (d, J = 17.2 Hz, 1H), 3.74 (s, 3H), 3.51 (s, 1H), 3.36 (d, J = 17.2 Hz, 1H), 2.48 (dt, J = 14.1, 8.4 Hz, 1H), 1.89 (dt, J = 14.0, 3.7 Hz, 1H). |

(continued)

| NO. | $^1$H NMR |
|---|---|
| **I-429** | 1H NMR (400 MHz, $CDCl_3$) $\delta$ 7.69 (s, 1H), 7.58 (d, J = 8.9 Hz, 1H), 7.39 (d, J = 8.1 Hz, 1H), 7.22 (d, J = 8.7 Hz, 1H), 6.18 (dd, J = 17.2, 10.7 Hz, 1H), 6.02 - 5.93 (m, 1H), 5.91 - 5.82 (m, 1H), 5.54 (d, J = 17.2 Hz, 1H), 5.34 (d, J = 10.7 Hz, 1H), 5.03 (t, J = 8.0 Hz, 1H), 4.18 (q, J = 7.1 Hz, 2H), 3.98 (d, J = 17.2 Hz, 1H), 3.54 - 3.47 (m, 1H), 3.36 (d, J = 17.2 Hz, 1H), 2.47 (dt, J = 14.0, 8.4 Hz, 1H), 1.89 (dt, J = 14.0, 3.7 Hz, 1H), 1.28 (t, J = 7.1 Hz, 3H). |
| **I-430** | 1H NMR (400 MHz, $CDCl_3$) $\delta$ 7.68 (s, 1H), 7.58 (d, J = 8.9 Hz, 1H), 7.39 (d, J = 8.1 Hz, 1H), 7.22 (d, J = 8.7 Hz, 1H), 6.18 (dd, J = 17.2, 10.7 Hz, 1H), 6.02 - 5.92 (m, 1H), 5.91 - 5.82 (m, 1H), 5.54 (d, J = 17.2 Hz, 1H), 5.34 (d, J = 10.7 Hz, 1H), 5.03 (t, J = 7.9 Hz, 1H), 4.13 (t, J = 6.7 Hz, 2H), 3.98 (d, J = 17.2 Hz, 1H), 3.50 (dd, J = 5.4, 2.9 Hz, 1H), 3.36 (d, J = 17.2 Hz, 1H), 2.47 (dt, J = 14.0, 8.4 Hz, 1H), 1.89 (dt, J = 14.0, 3.8 Hz, 1H), 1.66 - 1.60 (m, 2H), 1.37 (dq, J = 14.7, 7.4 Hz, 2H), 0.93 (t, J = 7.4 Hz, 3H). |
| **I-431** | 1H NMR (400 MHz, $CDCl_3$) $\delta$ 7.61 (s, 1H), 7.51 (d, J = 8.8 Hz, 1H), 7.32 (d, J = 8.0 Hz, 1H), 7.15 (d, J = 8.7 Hz, 1H), 6.10 (dd, J = 17.2, 10.7 Hz, 1H), 5.89 (d, J = 5.1 Hz, 1H), 5.84 - 5.77 (m, 1H), 5.47 (d, J = 17.2 Hz, 1H), 5.27 (d, J = 10.7 Hz, 1H), 4.96 (t, J = 7.9 Hz, 1H), 4.04 (t, J = 6.8 Hz, 2H), 3.91 (d, J = 17.2 Hz, 1H), 3.44 (d, J = 5.8 Hz, 1H), 3.29 (d, J = 17.2 Hz, 1H), 2.40 (dt, J = 14.0, 8.4 Hz, 1H), 1.82 (dt, J = 14.0, 3.7 Hz, 1H), 1.60 - 1.55 (m, 2H), 1.23 (dd, J = 12.0, 4.6 Hz, 8H), 0.81 (t, J = 6.7 Hz, 3H). |
| **I-432** | 1H NMR (400 MHz, $CDCl_3$) $\delta$ 7.69 (s, 1H), 7.59 (d, J = 8.8 Hz, 1H), 7.39 (d, J = 8.1 Hz, 1H), 7.15 (d, J = 8.6 Hz, 1H), 6.18 (dd, J = 17.2, 10.7 Hz, 1H), 5.96 - 5.90 (m, 1H), 5.89 - 5.82 (m, 1H), 5.54 (d, J = 17.2 Hz, 1H), 5.35 (d, J = 10.7 Hz, 1H), 5.02 (dd, J = 8.4, 7.3 Hz, 1H), 4.18 (ddd, J = 9.6, 6.0, 3.6 Hz, 1H), 3.98 (d, J = 17.2 Hz, 1H), 3.50 - 3.44 (m, 1H), 3.36 (d, J = 17.2 Hz, 1H), 2.47 (dt, J = 14.0, 8.5 Hz, 1H), 1.87 (dt, J = 14.0, 3.9 Hz, 1H), 0.76 - 0.70 (m, 4H). |
| **I-433** | 1H NMR (400 MHz, $CDCl_3$) $\delta$ 7.69 (s, 1H), 7.58 (d, J = 8.9 Hz, 1H), 7.39 (d, J = 8.0 Hz, 1H), 7.21 (d, J = 8.6 Hz, 1H), 6.18 (dd, J = 17.2, 10.7 Hz, 1H), 5.96 (d, J = 4.9 Hz, 1H), 5.91 - 5.83 (m, 1H), 5.54 (d, J = 17.2 Hz, 1H), 5.34 (d, J = 10.7 Hz, 1H), 5.02 (t, J = 7.9 Hz, 1H), 4.10 (d, J = 6.9 Hz, 2H), 3.98 (d, J = 17.2 Hz, 1H), 3.52 (d, J = 6.0 Hz, 1H), 3.36 (d, J = 17.2 Hz, 1H), 2.72 - 2.57 (m, 1H), 2.47 (dt, J = 14.0, 8.4 Hz, 1H), 2.13 - 1.97 (m, 2H), 1.97 - 1.82 (m, 3H), 1.82 - 1.69 (m, 2H). |
| **I-434** | 1H NMR (400 MHz, $CDCl_3$) $\delta$ 7.69 (s, 1H), 7.58 (d, J = 8.9 Hz, 1H), 7.40 (d, J = 8.0 Hz, 1H), 7.09 (d, J = 8.6 Hz, 1H), 6.18 (dd, J = 17.2, 10.7 Hz, 1H), 6.02 - 5.96 (m, 1H), 5.95 - 5.90 (m, 1H), 5.54 (d, J = 17.2 Hz, 1H), 5.35 (d, J = 10.7 Hz, 1H), 5.04 (td, J = 8.4, 1.4 Hz, 1H), 4.51 (q, J = 8.4 Hz, 2H), 3.98 (d, J = 17.2 Hz, 1H), 3.69 - 3.58 (m, 1H), 3.37 (d, J = 17.2 Hz, 1H), 2.55 (dt, J = 14.1, 8.4 Hz, 1H), 1.92 (dt, J = 14.1, 4.1 Hz, 1H). |
| **I-435** | 1H NMR (400 MHz, $CDCl_3$) $\delta$ 7.68 (s, 1H), 7.57 (d, J = 8.8 Hz, 1H), 7.40 (d, J = 8.1 Hz, 1H), 7.07 (d, J = 8.6 Hz, 1H), 6.22 - 5.89 (m, 4H), 5.54 (d, J = 17.2 Hz, 1H), 5.35 (d, J = 10.7 Hz, 1H), 5.04 (dd, J = 8.4, 6.9 Hz, 1H), 4.73 - 4.53 (m, 2H), 3.98 (d, J = 17.2 Hz, 1H), 3.67 - 3.57 (m, 1H), 3.37 (d, J = 17.2 Hz, 1H), 2.56 (dt, J = 14.1, 8.5 Hz, 1H), 1.92 (dt, J = 14.1, 4.1 Hz, 1H). |
| **I-436** | 1H NMR (400 MHz, $CDCl_3$) $\delta$ 7.68 (s, 1H), 7.58 (d, J = 8.9 Hz, 1H), 7.39 (d, J = 8.1 Hz, 1H), 7.21 (d, J = 8.7 Hz, 1H), 6.18 (dd, J = 17.2, 10.7 Hz, 1H), 6.00 - 5.93 (m, 1H), 5.91 - 5.83 (m, 1H), 5.54 (d, J = 17.2 Hz, 1H), 5.34 (d, J = 10.7 Hz, 1H), 5.03 (t, J = 7.9 Hz, 1H), 4.22 (t, J = 6.5 Hz, 2H), 3.98 (d, J = 17.2 Hz, 1H), 3.55 - 3.49 (m, 1H), 3.45 (t, J = 6.2 Hz, 2H), 3.36 (d, J = 17.3 Hz, 1H), 3.33 (s, 3H), 2.48 (dt, J = 14.0, 8.4 Hz, 1H), 1.97 - 1.86 (m, 3H). |
| **I-437** | $^1$H NMR (400 MHz, $CDCl_3$) $\delta$ 7.68 (s, 1H), 7.57 (dt, *J* = 8.9, 2.0 Hz, 1H), 7.38 (dt, *J* = 8.2, 2.0 Hz, 1H), 7.21 (d, *J* = 8.9 Hz, 1H), 6.17 (dd, *J* = 17.2, 10.7 Hz, 1H), 6.01 - 5.93 (m, 1H), 5.86 (dt, *J* = 5.5, 2.2 Hz, 1H), 5.53 (d, *J* = 17.2 Hz, 1H), 5.33 (d, *J* = 10.7 Hz, 1H), 5.11 - 4.91 (m, 1H), 4.08 (t, *J* = 6.7 Hz, 2H), 3.97 (d, *J* = 17.2 Hz, 1H), 3.58 - 3.47 (m, 1H), 3.35 (d, *J* = 17.2 Hz, 1H), 2.47 (dt, *J* = 14.0, 8.4 Hz, 1H), 1.89 (dt, *J* = 14.0, 3.8 Hz, 1H), 1.67 - 1.65 (m, 2H), 0.93 (t, *J* = 7.4 Hz, 3H). |
| **I-438** | $^1$H NMR (400 MHz, $CDCl_3$) $\delta$ 7.68 (s, 1H), 7.57 (dt, *J* = 9.0, 2.0 Hz, 1H), 7.38 (dt, *J* = 8.0, 2.0 Hz, 1H), 7.20 (d, *J* = 8.8 Hz, 1H), 6.17 (dd, *J* = 17.2, 10.7 Hz, 1H), 5.96 (ddd, *J* = 5.5, 2.6, 1.5 Hz, 1H), 5.86 (dt, *J* = 5.6, 2.2 Hz, 1H), 5.53 (d, *J* = 17.2 Hz, 1H), 5.33 (d, *J* = 10.7 Hz, 1H), 5.02 (dddt, *J*= 10.6, 8.7, 5.4, 1.6 Hz, 1H), 3.97 (d, *J* = 17.2 Hz, 1H), 3.90 (d, *J* = 6.7 Hz, 2H), 3.57 - 3.46 (m, 1H), 3.35 (d, *J* = 17.2 Hz, 1H), 2.47 (dt, *J* = 13.9, 8.4 Hz, 1H), 2.03 - 1.84 (m, 2H), 0.92 (dd, *J* = 6.7, 1.2 Hz, 6H). |

(continued)

| NO. | $^1H$ NMR |
|---|---|
| **I-439** | $^1H$ NMR (400 MHz, $CDCl_3$) δ 7.68 (s, 1H), 7.57 (dt, *J* = 9.0, 1.9 Hz, 1H), 7.44 - 7.29 (m, 6H), 7.21 (d, *J* = 8.8 Hz, 1H), 6.17 (dd, *J* = 17.2, 10.7 Hz, 1H), 5.97 (ddd, *J* = 5.6, 2.5, 1.5 Hz, 1H), 5.87 (dt, *J* = 5.6, 2.2 Hz, 1H), 5.53 (d, *J* = 17.2 Hz, 1H), 5.33 (d, *J* = 10.7 Hz, 1H), 5.16 (d, *J* = 1.8 Hz, 2H), 5.07 - 4.98 (m, 1H), 3.98 (d, *J* = 17.2 Hz, 1H), 3.56 (ddt, *J* = 10.1, 5.9, 2.4 Hz, 1H), 3.35 (d, *J* = 17.2 Hz, 1H), 2.49 (dt, *J* = 14.0, 8.4 Hz, 1H), 1.92 (dt, *J* = 13.9, 4.0 Hz, 1H). |
| **I-440** | $^1H$ NMR (400 MHz, $CDCl_3$) δ 7.63 (s, 1H), 7.52 (dt, *J* = 8.9, 1.9 Hz, 1H), 7.42 - 7.28 (m, 3H), 7.26 - 7.18 (m, 1H), 7.13 (d, *J* = 8.7 Hz, 1H), 7.09 - 6.99 (m, 2H), 6.24 - 6.05 (m, 2H), 5.94 (dt, *J* = 5.5, 2.3 Hz, 1H), 5.52 (d, *J* = 17.2 Hz, 1H), 5.32 (d, *J* = 10.7 Hz, 1H), 5.08 (tdt, *J* = 7.8, 3.8, 1.9 Hz, 1H), 3.96 (d, *J* = 17.2 Hz, 1H), 3.76 (dtd, *J* = 8.6, 4.1, 2.1 Hz, 1H), 3.34 (d, *J* = 17.2 Hz, 1H), 2.62 (dt, *J* = 14.1, 8.5 Hz, 1H), 2.04 (dt, *J* = 14.0, 4.3 Hz, 1H). |
| **I-441** | $^1H$ NMR (400 MHz, $CDCl_3$) δ 7.68 (s, 1H), 7.57 (dt, *J* = 8.9, 2.0 Hz, 1H), 7.38 (dt, *J* = 8.2, 2.0 Hz, 1H), 7.17 (d, *J* = 8.8 Hz, 1H), 6.17 (dd, *J* = 17.3, 10.7 Hz, 1H), 5.98 (ddd, *J* = 5.6, 2.5, 1.5 Hz, 1H), 5.89 (dt, *J* = 5.5, 2.3 Hz, 1H), 5.53 (d, *J* = 17.2 Hz, 1H), 5.34 (d, *J* = 10.7 Hz, 1H), 5.15 - 4.98 (m, 1H), 4.68 (t, *J* = 4.3 Hz, 1H), 4.56 (t, *J* = 4.2 Hz, 1H), 4.47 - 4.29 (m, 2H), 3.97 (d, *J* = 17.2 Hz, 1H), 3.64 - 3.50 (m, 1H), 3.35 (d, *J* = 17.2 Hz, 1H), 2.50 (dt, *J* = 14.0, 8.4 Hz, 1H), 1.91 (dt, *J* = 14.1, 3.8 Hz, 1H). |
| **I-442** | $^1H$ NMR (400 MHz, $CDCl_3$) δ 7.65 (s, 1H), 7.55 (dt, *J* = 8.8, 1.9 Hz, 1H), 7.36 (dt, *J* = 8.2, 2.0 Hz, 1H), 7.10 (d, *J* = 8.7 Hz, 1H), 6.14 (dd, *J* = 17.3, 10.7 Hz, 1H), 6.00 (ddd, *J* = 5.6, 2.5, 1.6 Hz, 1H), 5.90 (dt, *J* = 5.5, 2.2 Hz, 1H), 5.50 (d, *J* = 17.1 Hz, 1H), 5.30 (d, *J* = 10.7 Hz, 1H), 5.09 - 4.94 (m, 1H), 4.86 - 4.63 (m, 2H), 3.96 (d, *J* = 17.2 Hz, 1H), 3.68 - 3.57 (m, 1H), 3.34 (d, *J* = 17.2 Hz, 1H), 2.55 (dt, *J* = 14.1, 8.5 Hz, 1H), 1.96 (dt, *J* = 14.1, 4.1 Hz, 1H). |
| **I-443** | $^1H$ NMR (400 MHz, $CDCl_3$) δ 7.68 (s, 1H), 7.57 (dt, *J* = 8.9, 2.0 Hz, 1H), 7.38 (dt, *J* = 8.2, 2.0 Hz, 1H), 7.21 (d, *J* = 8.9 Hz, 1H), 6.17 (dd, *J* = 17.2, 10.8 Hz, 1H), 5.97 (ddd, *J* = 5.6, 2.5, 1.5 Hz, 1H), 5.87 (dt, *J* = 5.5, 2.2 Hz, 1H), 5.53 (d, *J* = 17.2 Hz, 1H), 5.33 (d, *J* = 10.7 Hz, 1H), 5.08 - 4.98 (m, 1H), 4.36 - 4.18 (m, 2H), 3.97 (d, *J* = 17.2 Hz, 1H), 3.70 - 3.50 (m, 3H), 3.47 - 3.29 (m, 4H), 2.47 (dt, *J* = 13.9, 8.4 Hz, 1H), 1.90 (dt, *J* = 14.0, 3.8 Hz, 1H). |
| **I-444** | $^1H$ NMR (400 MHz, $CDCl_3$) δ 7.68 (d, *J* = 2.1 Hz, 1H), 7.57 (dt, *J* = 8.9, 2.0 Hz, 1H), 7.39 (dt, *J* = 8.2, 2.0 Hz, 1H), 7.16 (d, *J* = 8.8 Hz, 1H), 6.17 (ddd, *J* = 17.2, 10.8, 1.0 Hz, 1H), 5.96 (ddd, *J* = 5.6, 2.5, 1.5 Hz, 1H), 5.91 - 5.80 (m, 2H), 5.53 (d, *J* = 17.2 Hz, 1H), 5.43 - 5.31 (m, 2H), 5.24 (ddt, *J* = 17.3, 2.8, 1.3 Hz, 1H), 5.14 (ddt, *J* = 10.5, 4.9, 1.2 Hz, 1H), 5.09 - 4.97 (m, 1H), 3.97 (d, *J* = 17.2 Hz, 1H), 3.56 - 3.45 (m, 1H), 3.35 (d, *J* = 17.2 Hz, 1H), 2.47 (dtd, *J* = 14.0, 8.4, 1.4 Hz, 1H), 1.89 (dt, *J* = 14.0, 3.9 Hz, 1H), 1.33 (dd, *J* = 6.5, 4.1 Hz, 3H). |
| **I-445** | $^1H$ NMR (400 MHz, $CDCl_3$) δ 7.68 (s, 1H), 7.57 (dt, *J* = 8.9, 2.0 Hz, 1H), 7.39 (dt, *J* = 8.2, 2.1 Hz, 1H), 7.20 (d, *J* = 8.9 Hz, 1H), 6.17 (dd, *J* = 17.3, 10.7 Hz, 1H), 5.97 (ddd, *J* = 5.6, 2.6, 1.5 Hz, 1H), 5.87 (dt, *J* = 5.5, 2.2 Hz, 1H), 5.53 (dd, *J* = 17.2, 0.7 Hz, 1H), 5.34 (dd, *J* = 10.7, 0.6 Hz, 1H), 5.08 - 4.97 (m, 1H), 4.16 (dd, *J* = 11.6, 3.8 Hz, 1H), 4.07 (dd, *J* = 11.6, 6.0 Hz, 1H), 3.98 (d, *J* = 17.2 Hz, 1H), 3.57 (pd, *J* = 6.4, 3.8 Hz, 2H), 3.36 (s, 4H), 2.48 (dt, *J* = 14.0, 8.4 Hz, 1H), 1.90 (dt, *J* = 14.0, 3.9 Hz, 1H), 1.17 (d, *J* = 6.4 Hz, 3H). |
| **I-446** | 1H NMR (400 MHz, DMSO) δ 8.17 (d, J = 8.4 Hz, 1H), 7.85 (d, J = 10.0 Hz, 3H), 7.54 (s, 1H), 7.02 (s, 1H), 6.13 (dd, J = 17.3, 10.7 Hz, 1H), 5.88 (dd, J = 3.3, 1.4 Hz, 1H), 5.77 (dt, J = 5.2, 2.1 Hz, 1H), 5.39 (d, J = 17.3 Hz, 1H), 5.30 (d, J = 10.6 Hz, 1H), 4.80 (t, J = 7.4 Hz, 1H), 3.95 (d, J = 17.8 Hz, 1H), 3.62 (d, J = 17.8 Hz, 1H), 3.34 - 3.31 (m, 1H), 2.23 (dt, J = 13.3, 8.2 Hz, 1H), 1.71 (dt, J = 13.3, 3.7 Hz, 1H). |
| **I-447** | 1H NMR (400 MHz, DMSO) δ 10.72 (s, 1H), 8.97 (s, 1H), 8.21 (d, J = 8.5 Hz, 1H), 7.86 (dd, J = 12.0, 7.4 Hz, 3H), 6.13 (dd, J = 17.3, 10.7 Hz, 1H), 5.85 - 5.78 (m, 2H), 5.40 (dd, J = 17.3, 0.7 Hz, 1H), 5.31 (dd, J = 10.7, 0.6 Hz, 1H), 4.84 (t, J = 7.8 Hz, 1H), 3.96 (d, J = 17.8 Hz, 1H), 3.63 (d, J = 17.8 Hz, 1H), 3.23 (d, J = 6.9 Hz, 1H), 2.25 (dt, J = 13.3, 8.1 Hz, 1H), 1.68 (dt, J = 13.3, 3.1 Hz, 1H). |
| **I-448** | 1H NMR (400 MHz, DMSO) δ 11.27 (s, 1H), 8.08 (d, J = 8.4 Hz, 1H), 7.91 - 7.82 (m, 3H), 6.14 (dd, J = 17.3, 10.7 Hz, 1H), 5.88 - 5.74 (m, 2H), 5.40 (d, J = 17.3 Hz, 1H), 5.31 (d, J = 10.6 Hz, 1H), 4.89 - 4.78 (m, 1H), 3.96 (d, J = 17.8 Hz, 1H), 3.64 (d, J = 17.8 Hz, 1H), 3.59 (s, 3H), 3.19 (dd, J = 7.3, 2.9 Hz, 1H), 2.28 (dt, J = 13.3, 8.2 Hz, 1H), 1.69 (dt, J = 13.4, 3.8 Hz, 1H). |

(continued)

| NO. | $^1$H NMR |
|---|---|
| **I-449** | 1H NMR (400 MHz, $CDCl_3$) δ 7.74 (d, J = 8.7 Hz, 1H), 7.68 (s, 1H), 7.59 (d, J = 8.9 Hz, 1H), 7.38 (d, J = 8.1 Hz, 1H), 6.18 (dd, J = 17.2, 10.7 Hz, 1H), 5.95 - 5.84 (m, 2H), 5.77 (s, 1H), 5.55 (d, J = 17.2 Hz, 1H), 5.33 (d, J = 10.7 Hz, 1H), 5.00 (t, J = 8.2 Hz, 1H), 3.97 (d, J = 17.2 Hz, 1H), 3.35 (d, J = 17.2 Hz, 1H), 3.32 - 3.26 (m, 1H), 2.85 (d, J = 4.9 Hz, 3H), 2.42 (dt, J = 13.8, 8.3 Hz, 1H), 1.86 (dt, J = 13.8, 3.2 Hz, 1H). |
| **I-450** | $^1$H NMR (400 MHz, $CDCl_3$) δ 7.72 (d, *J* = 8.8 Hz, 1H), 7.67 (s, 1H), 7.58 (dt, *J*= 9.0, 2.0 Hz, 1H), 7.37 (dt, *J* = 8.2, 2.0 Hz, 1H), 6.17 (dd, *J* = 17.2, 10.7 Hz, 1H), 5.94 - 5.83 (m, 2H), 5.71 (s, 1H), 5.54 (d, *J* = 17.3 Hz, 1H), 5.32 (d, *J* = 10.7 Hz, 1H), 5.05 - 4.93 (m, 1H), 3.96 *(d, J*= 17.2 Hz, 1H), 3.39 - 3.21 (m, 4H), 2.41 (dt, *J* = 13.8, 8.3 Hz, 1H), 1.84 (dt, *J*= 13.8, 3.3 Hz, 1H), 1.14 (t, *J* = 7.3 Hz, 3H). |
| **I-451** | 1H NMR (400 MHz, $CDCl_3$) δ 9.96 (s, 1H), 7.66 (s, 1H), 7.57 (d, J = 8.8 Hz, 1H), 7.38 (t, J = 8.2 Hz, 2H), 6.15 (dd, J = 17.2, 10.7 Hz, 1H), 5.91 (dd, J = 17.7, 5.5 Hz, 2H), 5.54 (d, J = 17.2 Hz, 1H), 5.34 (d, J = 10.7 Hz, 1H), 4.85 (d, J = 2.4 Hz, 1H), 3.97 (d, J = 17.4 Hz, 1H), 3.56 (d, J = 1.9 Hz, 1H), 3.36 (d, J = 17.4 Hz, 1H), 3.28 (s, 3H), 2.64 (dt, J = 14.3, 8.9 Hz, 1H), 1.98 (dt, J = 14.2, 4.8 Hz, 1H). |
| **I-452** | $^1$H NMR (400 MHz, $CDCl_3$) δ 7.99 (d, *J* = 8.9 Hz, 1H), 7.67 (s, 1H), 7.57 (dt, *J* = 9.0, 1.9 Hz, 1H), 7.35 (dt, *J* = 8.3, 2.0 Hz, 1H), 6.16 (dd, *J* = 17.3, 10.7 Hz, 1H), 5.95 - 5.84 (m, 2H), 5.53 (d, *J* = 17.2 Hz, 1H), 5.30 (d, *J* = 10.7 Hz, 1H), 5.02 (tt, *J* = 8.5, 2.4 Hz, 1H), 3.96 *(d, J*= 17.1 Hz, 1H), 3.69 - 3.59 (m, 1H), 3.58 - 3.42 (m, 4H), 3.33 (d, *J*= 17.1 Hz, 1H), 2.39 - 2.25 (m, 1H), 2.02 - 1.82 (m, 5H). |
| **I-454** | $^1$H NMR (400 MHz, DMSO) δ 12.45 (s, 1H), 8.08 (d, *J* = 7.9 Hz, 1H), 7.66 - 7.51 (m, 3H), 6.15 (dd, *J* = 17.3, 10.7 Hz, 1H), 5.96 - 5.88 (m, 1H), 5.80 - 5.76 (m, 1H), 5.39 (d, *J* = 17.3 Hz, 1H), 5.30 (d, *J* = 10.6 Hz, 1H), 4.86 - 4.74 (m, 1H), 3.92 (d, *J* = 17.8 Hz, 1H), 3.58 (d, *J* = 17.8 Hz, 1H), 3.49 - 3.40 (m, 1H), 2.44 - 2.32 (m, 1H), 1.90 - 1.77 (m, 1H). |
| **I-456** | $^1$H NMR (400 MHz, $CDCl_3$) δ 7.39 - 7.30 (m, 2H), 7.21 (d, *J* = 8.9 Hz, 1H), 7.03 (d, *J* = 8.5 Hz, 1H), 6.17 (dd, *J* = 17.2, 10.7 Hz, 1H), 6.01 - 5.84 (m, 2H), 5.54 (d, *J* = 17.2 Hz, 1H), 5.34 (d, *J* = 10.7 Hz, 1H), 5.07 - 4.95 (m, 1H), 3.94 (d, *J* = 17.1 Hz, 1H), 3.74 (s, 3H), 3.57 - 3.49 (m, 1H), 3.33 (d, *J* = 17.2 Hz, 1H), 2.55 - 2.43 (m, 1H), 1.95 - 1.84 (m, 1H). |
| **I-457** | $^1$H NMR (400 MHz, $CDCl_3$) δ 7.35 - 7.30 (m, 2H), 7.20 (d, *J* = 8.9 Hz, 1H), 7.05 - 7.00 (m, 1H), 6.22 - 6.12 (m, 1H), 5.98 - 5.94 (m, 1H), 5.89 - 5.84 (m, 1H), 5.53 (d, *J* = 17.2 Hz, 1H), 5.33 (d, *J* = 10.7 Hz, 1H), 5.07 - 4.98 (m, 1H), 4.12 (t, *J*= 6.8 Hz, 2H), 3.93 (d, *J* = 17.2 Hz, 1H), 3.54 - 3.48 (m, 1H), 3.32 (d, J= 17.2 Hz, 1H), 2.53 - 2.43 (m, 1H), 1.94 - 1.85 (m, 1H), 1.70 - 1.63 (m, 2H), 1.37 - 1.29 (m, 4H), 0.94 - 0.86 (m, 3H). |
| **I-458** | $^1$H NMR (400 MHz, $CDCl_3$) δ 7.36 - 7.30 (m, 2H), 7.19 (d, *J* = 8.9 Hz, 1H), 7.06 - 7.00 (m, 1H), 6.21 - 6.13 (m, 1H), 5.99 - 5.94 (m, 1H), 5.89 - 5.85 (m, 1H), 5.85 - 5.77 (m, 1H), 5.65 - 5.56 (m, 1H), 5.33 (d, *J* = 10.7 Hz, 1H), 5.07 - 4.98 (m, 1H), 4.59 - 4.52 (m, 2H), 3.94 (d, *J* = 17.2 Hz, 1H), 3.55 - 3.48 (m, 1H), 3.32 (d, *J* = 17.2 Hz, 1H), 2.53 - 2.43 (m, 1H), 1.95 - 1.85 (m, 1H), 1.77 - 1.69 (m, 3H). |
| **I-459** | $^1$H NMR (400 MHz, $CDCl_3$) δ *7.35* - 7.29 (m, 2H), 7.19 (d, *J* = 8.8 Hz, 1H), 7.05 - 7.00 (m, 1H), 6.22 - 6.12 (m, 1H), 5.98 - 5.93 (m, 1H), 5.89 - 5.84 (m, 1H), 5.53 (dd, *J* = 17.2, 0.8 Hz, 1H), 5.33 (dd, *J* = 10.7, 0.7 Hz, 1H), 5.16 - 5.08 (m, 1H), 5.07 - 4.99 (m, 1H), 3.94 (d, *J* = 17.2 Hz, 1H), 3.55 - 3.50 (m, 1H), 3.50 - 3.38 (m, 2H), 3.35 (s, 3H), 3.30 (d, *J* = 17.2 Hz, 1H), 2.53 - 2.42 (m, 1H), 1.95 - 1.86 (m, 1H), 1.24 (d, *J* = 6.5 Hz, 3H). |
| **I-460** | $^1$H NMR (400 MHz, $CDCl_3$) δ *7.35* - 7.29 (m, 2H), 7.22 (d, *J* = 8.8 Hz, 1H), 7.05 - 6.99 (m, 1H), 6.22 - 6.12 (m, 1H), 6.01 - 5.96 (m, 1H), 5.90 - 5.85 (m, 1H), 5.53 (d, *J* = 17.2 Hz, 1H), 5.33 (d, *J* = 10.7 Hz, 1H), 5.08 -4.96 (m, 1H), 3.99 - 3.90 (m, 3H), 3.56 - 3.50 (m, 1H), 3.32 (d, *J* = 17.2 Hz, 1H), 2.53 - 2.43 (m, 1H), 1.95 - 1.88 (m, 1H), 1.21 - 1.08 (m, 1H), 0.61 - 0.53 (m, 2H), 0.32 - 0.26 (m, 2H). |
| **I-461** | $^1$H NMR (400 MHz, $CDCl_3$) δ 7.37 - 7.30 (m, 2H), 7.20 (d, *J* = 8.8 Hz, 1H), 7.07 - 7.00 (m, 1H), 6.23 - 6.11 (m, 1H), 6.01 - 5.93 (m, 1H), 5.91 - 5.83 (m, 1H), 5.53 (d, *J* = 17.2 Hz, 1H), 5.33 (d, *J* = 10.7 Hz, 1H), 5.07 - 4.97 (m, 1H), 4.09 (t, *J* = 6.8 Hz, 2H), 3.94 (d, *J* = 17.2 Hz, 1H), 3.56 - 3.47 (m, 1H), 3.32 (d, *J* = 17.2 Hz, 1H), 2.55 - 2.41 (m, 1H), 1.95 - 1.87 (m, 1H), 1.71 - 1.64 (m, 2H), 0.94 (t, *J* = 7.4 Hz, 3H). |
| **I-462** | $^1$H NMR (400 MHz, $CDCl_3$) δ 7.36 - 7.30 (m, 2H), 7.20 (d, *J* = 8.9 Hz, 1H), 7.06 - 6.99 (m, 1H), 6.24 - 6.11 (m, 1H), 5.98 - 5.93 (m, 1H), 5.91 - 5.84 (m, 1H), 5.53 (d, *J* = 17.2 Hz, 1H), 5.33 (d, *J* = 10.7 Hz, 1H), 5.07 - 4.97 (m, 1H), 4.12 (t, *J* = 6.8 Hz, 2H), 3.93 (d, *J* = 17.2 Hz, 1H), 3.57 - 3.48 (m, 1H), 3.32 (d, *J* = 17.2 Hz, 1H), 2.54 - 2.43 (m, 1H), 1.97 - 1.84 (m, 1H), 1.69 - 1.62 (m, 2H), 1.36 - 1.23 (m, 6H), 0.95 - 0.84 (m, 3H). |

(continued)

| NO. | $^1$H NMR |
|---|---|
| **I-463** | $^1$H NMR (400 MHz, $CDCl_3$) δ 7.37 - 7.30 (m, 2H), 7.18 (d, *J* = 8.8 Hz, 1H), 7.06 - 6.98 (m, 1H), 6.24 - 6.11 (m, 1H), 5.98 - 5.92 (m, 1H), 5.90 - 5.84 (m, 1H), 5.53 (d, *J* = 17.2 Hz, 1H), 5.33 (d, *J* = 10.7 Hz, 1H), 5.09 - 4.98 (m, 2H), 3.94 (d, *J* = 17.2 Hz, 1H), 3.50 - 3.42 (m, 1H), 3.32 (d, *J* = 17.2 Hz, 1H), 2.52 - 2.41 (m, 1H), 1.92 - 1.82 (m, 1H), 1.25 (dd, *J* = 6.3, 2.4 Hz, 6H). |
| **I-464** | $^1$H NMR (400 MHz, $CDCl_3$) δ 7.36 - 7.29 (m, 2H), 7.26 - 7.14 (m, 5H), 7.05 - 7.00 (m, 1H), 6.22 - 6.12 (m, 1H), 5.99 - 5.93 (m, 1H), 5.90 - 5.83 (m, 1H), 5.53 (d, *J* = 17.2 Hz, 1H), 5.33 (d, *J* = 10.7 Hz, 1H), 5.12 (d, *J* = 3.1 Hz, 2H), 5.06 - 4.99 (m, 1H), 3.94 (d, *J* = 17.2 Hz, 1H), 3.56 - 3.51 (m, 1H), 3.32 (d, *J* = 17.2 Hz, 1H), 2.54 - 2.42 (m, 1H), 2.35 (s, 3H), 1.97 - 1.85 (m, 1H). |
| **I-465** | 1H NMR (400 MHz, $CDCl_3$) δ 7.31 - 7.27 (m, 2H), 7.21 - 6.90 (m, 6H), 6.21 - 6.13 (m, 1H), 6.14 - 6.09 (m, 1H), 5.97 - 5.89 (m, 1H), 5.52 (d, J = 17.3 Hz, 1H), 5.33 (d, J = 10.7 Hz, 1H), 5.12 - 5.03 (m, 1H), 3.92 (d, J = 17.2 Hz, 1H), 3.76 (t, J = 6.0 Hz, 1H), 3.31 (d, J = 17.2 Hz, 1H), 2.70 - 2.57 (m, 1H), 2.34 (s, 3H), 2.08 - 1.98 (m, 1H). |
| **I-466** | $^1$H NMR (400 MHz, DMSO) δ 10.97 (s, 1H), 8.04 (d, *J* = 8.4 Hz, 1H), 7.67 - 7.59 (m, 1H), 7.56 (d, *J = 7.2* Hz, 2H), 6.18 - 6.09 (m, 1H), 5.82 (s, 2H), 5.43 - 5.28 (m, 2H), 4.88 - 4.78 (m, 1H), 4.05 - 3.94 (m, 1H), 3.89 (dd, *J* = 17.8, 4.0 Hz, 1H), 3.59 (dd, *J* = 17.8, 4.0 Hz, 1H), 3.24 (dd, *J* = 8.2, 3.7 Hz, 1H), 2.33 - 2.20 (m, 1H), 1.73 - 1.63 (m, 1H), 1.12 (d, *J* = 6.2 Hz, 6H). |
| **I-467** | $^1$H NMR (400 MHz, DMSO) δ 11.15 (s, 1H), 8.06 (d, *J* = 8.4 Hz, 1H), 7.66 - 7.60 (m, 1H), 7.60 - 7.53 (m, 2H), 6.18 - 6.09 (m, 1H), 5.84 - 5.78 (m, 2H), 5.42 - 5.28 (m, 2H), 4.87 - 4.78 (m, 1H), 3.90 (d, *J* = 17.8 Hz, 1H), 3.85 - 3.76 (m, 2H), 3.59 (d, *J* = 17.8 Hz, 1H), 3.25 - 3.18 (m, 1H), 2.34 - 2.21 (m, 1H), 1.73 - 1.64 (m, 1H), 1.13 (t, *J* = 7.0 Hz, 3H). |
| **I-468** | $^1$H NMR (400 MHz, DMSO) δ 8.13 (d, *J*= 8.4 Hz, 1H), 8.10 - 8.03 (m, 1H), 7.66 - 7.59 (m, 1H), 7.59 - 7.52 (m, 2H), 6.18 - 6.07 (m, 1H), 5.87 - 5.81 (m, 1H), 5.80 - 5.73 (m, 1H), 5.38 (dd, *J* = 17.3, 1.0 Hz, 1H), 5.30 (dd, *J* = 10.6, 1.0 Hz, 1H), 4.85 - 4.77 (m, 1H), 3.90 (d, *J* = 17.8 Hz, 1H), 3.58 (d, *J* = 17.8 Hz, 1H), 3.33 - 3.31 (m, 1H), 3.15 - 3.02 (m, 2H), 2.28 - 2.18 (m, 1H), 1.73 - 1.65 (m, 1H), 1.02 (t, *J* = 7.2 Hz, 3H). |
| **I-469** | $^1$H NMR (400 MHz, DMSO) δ 8.09 (d, *J* = 8.1 Hz, 1H), 7.65 - 7.60 (m, 1H), 7.56 (d, *J*= 7.2 Hz, 2H), 6.17 - 6.07 (m, 1H), 5.88 - 5.82 (m, 1H), 5.81 - 5.73 (m, 1H), 5.37 (dd, *J*= 17.3, 1.0 Hz, 1H), 5.29 (dd, *J* = 10.6, 1.0 Hz, 1H), 4.84 - 4.74 (m, 1H), 3.95 - 3.38 (m, 7H), 2.31 - 2.20 (m, 1H), 1.84 - 1.77 (m, 1H), 1.62 - 1.36 (m, 6H). |
| **I-470** | 1H NMR (400 MHz, DMSO) δ 12.52 (s, 1H), 8.08 (d, J = 7.9 Hz, 1H), 7.80 (t, J = 1.5 Hz, 1H), 7.72 (s, 1H), 7.67 (s, 1H), 6.14 (dd, J = 17.3, 10.7 Hz, 1H), 5.91 (dt, J = 5.4, 2.0 Hz, 1H), 5.83 - 5.74 (m, 1H), 5.39 (d, J = 17.3 Hz, 1H), 5.30 (d, J = 10.7 Hz, 1H), 4.80 (dt, J = 7.9, 6.0 Hz, 1H), 3.92 (d, J = 17.8 Hz, 1H), 3.60 (d, J = 17.8 Hz, 1H), 3.45 (ddd, J = 8.4, 6.3, 2.2 Hz, 1H), 2.38 (dt, J = 13.2, 8.3 Hz, 1H), 1.90 - 1.77 (m, 1H). |
| **I-471** | 1H NMR (400 MHz, $CDCl_3$) δ 7.57 (t, J = 1.4 Hz, 1H), 7.43 (s, 1H), 7.29 (s, 1H), 7.22 (d, J = 8.7 Hz, 1H), 6.17 (dd, J = 17.2, 10.7 Hz, 1H), 6.02 - 5.93 (m, 1H), 5.91 - 5.79 (m, 1H), 5.53 (d, J = 17.2 Hz, 1H), 5.34 (d, J = 10.7 Hz, 1H), 5.03 (t, J = 8.0 Hz, 1H), 3.94 (d, J = 17.2 Hz, 1H), 3.74 (s, 3H), 3.56 - 3.48 (m, 1H), 3.33 (d, J = 17.2 Hz, 1H), 2.48 (dt, J = 14.0, 8.4 Hz, 1H), 1.90 (dt, J = 14.0, 3.8 Hz, 1H). |
| **I-472** | 1H NMR (400 MHz, $CDCl_3$) δ 7.58 (s, 1H), 7.43 (s, 1H), 7.29 (s, 1H), 7.18 (d, J = 8.7 Hz, 1H), 6.17 (dd, J = 17.2, 10.7 Hz, 1H), 5.99 - 5.90 (m, 1H), 5.89 - 5.81 (m, 1H), 5.53 (d, J = 17.2 Hz, 1H), 5.33 (d, J = 10.7 Hz, 1H), 5.19 (dt, J = 9.1, 3.2 Hz, 1H), 5.01 (t, J = 7.8 Hz, 1H), 3.94 (d, J = 17.2 Hz, 1H), 3.46 (dd, J = 6.0, 2.2 Hz, 1H), 3.33 (d, J = 17.2 Hz, 1H), 2.46 (dt, J = 14.0, 8.4 Hz, 1H), 1.86 (ddd, J = 16.1, 8.1, 3.8 Hz, 3H), 1.77 - 1.64 (m, 4H), 1.64 - 1.53 (m, 2H). |
| **I-473** | 1H NMR (400 MHz, $CDCl_3$) δ 7.57 (t, J = 1.5 Hz, 1H), 7.43 (s, 1H), 7.30 (s, 1H), 7.08 (d, J = 8.6 Hz, 1H), 6.17 (dd, J = 17.2, 10.7 Hz, 1H), 6.01 - 5.94 (m, 1H), 5.94 - 5.87 (m, 1H), 5.53 (d, J = 17.2 Hz, 1H), 5.34 (d, J = 10.7 Hz, 1H), 5.04 (td, J = 8.4, 1.6 Hz, 1H), 4.59 (dd, J = 13.9, 12.9 Hz, 2H), 3.93 (d, J = 17.2 Hz, 1H), 3.68 - 3.59 (m, 1H), 3.33 (d, J = 17.2 Hz, 1H), 2.56 (dt, J = 14.1, 8.4 Hz, 1H), 1.92 (dt, J = 14.1, 4.2 Hz, 1H). |
| **I-474** | $^1$H NMR (400 MHz, $CDCl_3$) δ 7.58 (t, *J* = 1.5 Hz, 1H), 7.43 (s, 1H), 7.30 (s, 1H), 7.15 (d, *J* = 8.8 Hz, 1H), 6.17 (dd, *J* = 17.2, 10.7 Hz, 1H), 6.01 - 5.94 (m, 1H), 5.93 - 5.85 (m, 1H), 5.53 (d, *J* = 17.2 Hz, 1H), 5.34 (d, *J* = 10.7 Hz, 1H), 5.04 (td, *J* = 8.5, 1.4 Hz, 1H), 4.73 (d, *J* = 1.9 Hz, 2H), 3.94 (d, *J* = 17.2 Hz, 1H), 3.64 - 3.53 (m, 1H), 3.33 (d, *J* = 17.2 Hz, 1H), 2.57 - 2.47 (m, 2H), 1.91 (dt, *J* = 14.0, 4.0 Hz, 1H). |

(continued)

| NO. | $^1$H NMR |
|---|---|
| **I-475** | 1H NMR (400 MHz, $CDCl_3$) δ 7.57 (t, J = 1.5 Hz, 1H), 7.49 (d, J = 1.7 Hz, 1H), 7.47 (s, 1H), 7.42 (s, 1H), 7.30 (s, 1H), 7.23 (d, J = 8.4 Hz, 2H), 7.19 (d, J = 8.8 Hz, 1H), 6.16 (dd, J = 17.2, 10.7 Hz, 1H), 5.98 - 5.91 (m, 1H), 5.90 - 5.83 (m, 1H), 5.52 (d, J = 17.2 Hz, 1H), 5.33 (d, J = 10.7 Hz, 1H), 5.11 (s, 2H), 5.03 (dd, J = 12.1, 4.8 Hz, 1H), 3.94 (d, J = 17.2 Hz, 1H), 3.55 (ddd, J = 4.5, 3.5, 2.2 Hz, 1H), 3.33 (d, J = 17.2 Hz, 1H), 2.49 (dt, J = 14.0, 8.4 Hz, 1H), 1.91 (dt, J = 14.0, 3.9 Hz, 1H). |
| **I-476** | $^1$H NMR (400 MHz, $CDCl_3$) δ 7.85 - 7.81 (m, 1H), 7.81 - 7.77 (m, 1H), 7.67 - 7.62 (m, 1H), 7.28 (d, *J* = 7.4 Hz, 1H), 6.24 - 6.12 (m, 1H), 6.05 - 5.98 (m, 1H), 5.95 - 5.89 (m, 1H), 5.58 - 5.50 (m, 1H), 5.34 (d, *J* = 10.7 Hz, 1H), 5.10 - 4.98 (m, 1H), 3.98 (d, *J* = 17.3 Hz, 1H), 3.60 - 3.51 (m, 1H), 3.36 (d, *J* = 17.3 Hz, 1H), 2.55 - 2.40 (m, 1H), 1.97 - 1.87 (m, 1H). |
| **I-477** | $^1$H NMR (400 MHz, DMSO) δ 8.13 (d, J = 7.8 Hz, 1H), 8.07-8.03 (m, 1H), 8.01 (d, J = 1.6 Hz, 1H), 7.97 (d, J = 2.0 Hz, 1H), 6.24-6.10 (m, 1H), 5.94-5.86 (m, 1H), 5.85-5.78 (m, 1H), 5.41 (dd, J = 17.2, 1.0 Hz, 1H), 5.31 (dd, J = 10.6, 1.0 Hz, 1H), 4.85-4.75 (m, 1H), 3.97 (d, J = 17.8 Hz, 1H), 3.65 (d, J = 18.3 Hz, 1H), 3.63 (s, 3H), 3.59-3.51 (m, 1H), 2.48-2.35 (m, 1H), 1.93-1.77 (m, 1H). |
| **I-478** | $^1$H NMR (400 MHz, $CDCl_3$) δ 7.83 (d, *J* = 1.8 Hz, 1H), 7.78 (d, *J* = 1.9 Hz, 1H), 7.66 (d, *J* = 1.9 Hz, 1H), 7.17 (d, *J* = 8.8 Hz, 1H), 6.17 (dd, *J* = 17.3, 10.7 Hz, 1H), 6.00 - 5.93 (m, 1H), 5.87 (dt, *J* = 5.6, 2.3 Hz, 1H), 5.54 (d, *J* = 17.2 Hz, 1H), 5.34 (d, *J* = 10.7 Hz, 1H), 5.06 - 4.96 (m, 2H), 3.98 (d, *J* = 17.2 Hz, 1H), 3.51 - 3.44 (m, 1H), 3.35 (d, *J* = 17.2 Hz, 1H), 2.52 - 2.41 (m, 1H), 2.41 - 2.29 (m, 2H), 2.17 - 2.01 (m, 2H), 1.91 - 1.84 (m, 1H), 1.84 - 1.75 (m, 1H), 1.68 - 1.58 (m, 1H). |
| **I-479** | $^1$H NMR (400 MHz, $CDCl_3$) δ 7.86 - 7.81 (m, 1H), 7.80 - 7.76 (m, 1H), 7.69 - 7.63 (m, 1H), 7.36 - 7.27 (m, 4H), 7.19 (d, *J* = 8.8 Hz, 1H), 6.21 - 6.12 (m, 1H), 5.98 - 5.92 (m, 1H), 5.92 - 5.86 (m, 1H), 5.53 (d, *J* = 17.2 Hz, 1H), 5.34 (d, *J* = 10.7 Hz, 1H), 5.12 (s, 2H), 5.07 - 4.98 (m, 1H), 3.98 (d, *J*= 17.2 Hz, 1H), 3.58 - 3.52 (m, 1H), 3.35 (d, *J* = 17.2 Hz, 1H), 2.54 - 2.43 (m, 1H), 1.95 - 1.87 (m, 1H). |
| **I-480** | $^1$H NMR (400 MHz, $CDCl_3$) δ 7.85 - 7.82 (m, 1H), 7.82 - 7.76 (m, 1H), 7.70 - 7.64 (m, 1H), 7.11 (d, *J* = 8.7 Hz, 1H), 6.23 - 6.12 (m, 1H), 6.02 - 5.70 (m, 3H), 5.54 (d, *J* = 17.2 Hz, 1H), 5.35 (d, *J* = 10.7 Hz, 1H), 5.08 -4.99 (m, 1H), 4.60 - 4.44 (m, 2H), 3.98 (d, *J* = 17.2 Hz, 1H), 3.69 - 3.56 (m, 1H), 3.36 (d, *J* = 17.2 Hz, 1H), 2.61 - 2.48 (m, 1H), 1.99 - 1.86 (m, 1H). |
| **I-481** | $^1$H NMR (400 MHz, $CDCl_3$) δ 7.86 - 7.81 (m, 1H), 7.81 - 7.76 (m, 1H), 7.69 - 7.64 (m, 1H), 7.22 (d, *J* = 8.9 Hz, 1H), 6.24 - 6.10 (m, 1H), 6.05 - 5.94 (m, 1H), 5.92 - 5.80 (m, 1H), 5.59 - 5.48 (m, 1H), 5.36 - 5.31 (m, 1H), 5.11 - 4.96 (m, 1H), 4.35 - 4.22 (m, 2H), 3.98 (d, *J* = 17.2 Hz, 1H), 3.67 - 3.61 (m, 2H), 3.61 - 3.49 (m, 3H), 3.35 (d, *J* = 17.2 Hz, 1H), 2.54 - 2.41 (m, 1H), 1.95 - 1.85 (m, 1H), 1.21 (t, *J* = 7.0 Hz, 3H). |
| **I-482** | $^1$H NMR (400 MHz, $CDCl_3$) δ 7.87 - 7.81 (m, 1H), 7.82 - 7.75 (m, 1H), 7.69 - 7.63 (m, 1H), 7.17 (d, *J* = 8.9 Hz, 1H), 6.23 - 6.11 (m, 1H), 6.02 - 5.94 (m, 1H), 5.92 - 5.84 (m, 1H), 5.59 - 5.49 (m, 1H), 5.34 (d, *J* = 10.7 Hz, 1H), 5.09 - 4.98 (m, 1H), 4.69 (p, *J* = 2.3 Hz, 2H), 3.98 (d, *J* = 17.2 Hz, 1H), 3.60 - 3.51 (m, 1H), 3.36 (d, *J* = 17.2 Hz, 1H), 2.50 (dt, *J* = 14.0, 8.4 Hz, 1H), 1.91 (dt, *J* = 14.1, 4.0 Hz, 1H), 1.86 (t, *J* = 2.4 Hz, 3H). |
| **I-483** | $^1$H NMR (400 MHz, $CDCl_3$) δ 7.85 - 7.82 (m, 1H), 7.80 - 7.77 (m, 1H), 7.71 - 7.62 (m, 1H), 7.21 (d, *J* = 8.8 Hz, 1H), 6.23 - 6.11 (m, 1H), 5.98 - 5.92 (m, 1H), 5.91 - 5.83 (m, 1H), 5.54 (d, *J* = 17.1 Hz, 1H), 5.34 (d, *J* = 10.7 Hz, 1H), 5.10 - 4.97 (m, 2H), 3.99 (d, *J* = 17.2 Hz, 1H), 3.51 - 3.42 (m, 1H), 3.36 (d, *J* = 17.2 Hz, 1H), 2.51 - 2.39 (m, 1H), 1.93 - 1.84 (m, 1H), 1.30 - 1.22 (m, 6H). |
| **I-484** | $^1$H NMR (400 MHz, $CDCl_3$) δ 7.79 - 7.76 (m, 1H), 7.73 (s, 1H), 7.71 - 7.62 (m, 1H), 7.60 - 7.55 (m, 1H), 6.15 - 6.06 (m, 1H), 5.85 - 5.77 (m, 2H), 5.47 (d, *J* = 17.2 Hz, 1H), 5.29 - 5.23 (m, 1H), 5.01 - 4.92 (m, 1H), 3.94 - 3.82 (m, 2H), 3.66 (s, 3H), 3.27 (d, *J* = 17.1 Hz, 1H), 3.15 (s, 3H), 2.36 - 2.25 (m, 1H), 1.87 - 1.79 (m, 1H). |
| **I-485** | $^1$H NMR (400 MHz, DMSO) δ 12.44 (s, 1H), 8.17 - 7.94 (m, 1H), 7.77 (d, *J* = 1.3 Hz, 1H), 7.72 (s, 1H), 7.65 (s, 1H), 5.95 - 5.84 (m, 1H), 5.84 - 5.67 (m, 1H), 4.87 - 4.74 (m, 1H), 3.86 - 3.75 (m, 1H), 3.70-3.62 (m, 1H), 3.51-3.43 (m, 1H), 2.46 - 2.31 (m, 1H), 1.97 - 1.73 (m, 1H), 1.55 (d, *J* = 4.8 Hz, 3H). |
| **I-486** | $^1$H NMR (400 MHz, DMSO) δ 8.00 (d, *J* = 7.0 Hz, 1H), 7.77 (d, *J* = 1.3 Hz, 1H), 7.71 (s, 1H), 7.65 (s, 1H), 5.95 - 5.86 (m, 1H), 5.85 - 5.72 (m, 1H), 4.87 - 4.74 (m, 1H), 3.80 (dd, *J* = 17.7, 4.6 Hz, 1H), 3.63 (d, *J* = 11.9 Hz, 3H), 3.59 - 3.51 (m, 1H), 3.41 (d, *J* = 17.8 Hz, 1H), 2.49 - 2.38 (m, 1H), 1.96 - 1.77 (m, 1H), 1.56 (d, *J* = 2.9 Hz, 3H). |

(continued)

| NO. | $^1H$ NMR |
|---|---|
| **I-487** | $^1H$ NMR (400 MHz, DMSO) $\delta$ 8.08 (dd, *J* = 82.2, 7.8 Hz, 1H), 7.77 (d, *J* = 1.4 Hz, 1H), 7.72 (s, 1H), 7.65 (s, 1H), 5.92 - 5.73 (m, 2H), 4.87 (ddd, *J* = 39.5, 9.6, 5.1 Hz, 1H), 4.20 (dt, *J* = 13.5, 6.7 Hz, 2H), 3.90 - 3.71 (m, 1H), 3.60 - 3.37 (m, 2H), 2.71 (ddd, *J* = 9.1, 8.5, 4.6 Hz, 2H), 2.49 - 2.32 (m, 1H), 2.09 (dd, *J* = 6.9, 4.3 Hz, 3H), 2.04 - 1.78 (m, 1H), 1.60 - 1.50 (m, 3H). |
| **I-488** | $^1H$ NMR (400 MHz, DMSO-$d_6$) $\delta$ 12.33 (s, 1H), 7.95 (dd, *J* = 11.7, 8.0 Hz, 1H), 7.67 - 7.44 (m, 3H), 5.99 - 5.84 (m, 1H), 5.83 - 5.68 (m, 1H), 4.80 (ddtq, *J* = 7.9, 5.9, 3.9, 1.9 Hz, 1H), 3.79 (dt, *J* = 17.7, 3.5 Hz, 1H), 3.50 - 3.34 (m, 2H), 2.46 - 2.30 (m, 1H), 1.91 - 1.74 (m, 1H), 1.56 (d, *J* = 4.7 Hz, 3H). |
| **I-489** | $^1H$ NMR (400 MHz, DMSO-$d_6$) $\delta$ 8.00 (d, *J* = 7.9 Hz, 1H), 7.64 - 7.49 (m, 3H), 5.94 - 5.86 (m, 1H), 5.85 - 5.72 (m, 1H), 4.88 - 4.72 (m, 1H), 3.79 (dd, *J* = 17.7, 4.6 Hz, 1H), 3.63 (d, *J* = 12.0 Hz, 3H), 3.60 - 3.51 (m, 1H), 3.40 (dd, *J* = 17.6, 1.7 Hz, 1H), 2.49 - 2.38 (m, 1H), 1.96 - 1.78 (m, 1H), 1.56 (d, *J* = 3.1 Hz, 3H). |
| **I-490** | $^1H$ NMR (400 MHz, DMSO-$d_6$) $\delta$ 7.97 (d, *J* = 7.9 Hz, 1H), 7.64 - 7.49 (m, 3H), 5.94 - 5.86 (m, 1H), 5.85 - 5.72 (m, 1H), 4.86 - 4.76 (m, 1H), 4.21 (dt, *J* = 13.4, 6.6 Hz, 2H), 3.80 (dt, *J* = 17.7, 3.2 Hz, 1H), 3.64 - 3.51 (m, 1H), 3.44 - 3.37 (m, 1H), 2.81 - 2.67 (m, 2H), 2.48 - 2.36 (m, 1H), 2.19 - 2.04 (m, 3H), 1.98 - 1.79 (m, 1H), 1.57 (d, *J* = 2.7 Hz, 3H). |
| **I-491** | $^1H$ NMR (400 MHz, DMSO-$d_6$) $\delta$ 8.07 - 7.96 (m, 1H), 7.65 - 7.48 (m, 3H), 5.93 - 5.85 (m, 1H), 5.85 - 5.74 (m, 1H), 4.87 - 4.76 (m, 1H), 4.50 - 4.32 (m, 2H), 3.80 (dd, *J* = 17.7, 3.5 Hz, 1H), 3.64 - 3.53 (m, 1H), 3.41 (d, *J* = 7.5 Hz, 1H), 3.22 - 3.08 (m, 1H), 3.05 - 2.92 (m, 1H), 2.63 - 2.58 (m, 3H), 2.49 - 2.33 (m, 1H), 1.97 - 1.81 (m, 1H), 1.59 - 1.53 (m, 3H). |
| **I-492** | $^1H$ NMR (400 MHz, DMSO-$d_6$) $\delta$ 8.01 (t, *J* = 8.2 Hz, 1H), 7.60 - 7.53 (m, 3H), 5.93 - 5.87 (m, 1H), 5.84 - 5.74 (m, 1H), 4.85 - 4.75 (m, 1H), 4.45 - 4.39 (m, 2H), 3.83 - 3.77 (m, 1H), 3.58 - 3.51 (m, 3H), 3.42 (d, *J* = 4.4 Hz, 1H), 3.03 (d, *J* = 8.4 Hz, 3H), 2.49 - 2.33 (m, 1H), 1.97 - 1.79 (m, 1H), 1.56 (d, *J* = 3.0 Hz, 3H). |
| **I-493** | $^1H$ NMR (400 MHz, DMSO-$d_6$) $\delta$ 12.43 (s, 1H), 7.98 (dd, *J* = 11.4, 8.0 Hz, 1H), 7.90 - 7.78 (m, 3H), 5.98 - 5.86 (m, 1H), 5.84 - 5.70 (m, 1H), 4.87 - 4.75 (m, 1H), 3.92 - 3.79 (m, 1H), 3.53 - 3.40 (m, 2H), 2.47 - 2.33 (m, 1H), 1.97 - 1.77 (m, 1H), 1.58 (d, *J* = 4.9 Hz, 3H). |
| **I-494** | $^1H$ NMR (400 MHz, DMSO-$d_6$) $\delta$ 12.48 (s, 1H), 7.99 (d, *J* = 7.9 Hz, 1H), 7.88 - 7.76 (m, 3H), 5.90 (dt, *J* = 5.6, 2.1 Hz, 1H), 5.78 (dt, *J* = 5.6, 2.3 Hz, 1H), 4.79 (dtt, *J*= 8.0, 6.0, 2.0 Hz, 1H), 3.83 (d, *J* = 17.7 Hz, 1H), 3.47 - 3.40 (m, 2H), 2.37 (dt, *J* = 13.2, 8.3 Hz, 1H), 1.79 (dt, *J* = 12.8, 6.2 Hz, 1H), 1.55 (s, 3H). |
| **I-495** | $^1H$ NMR (400 MHz, DMSO-$d_6$) $\delta$ 8.01 (d, *J* = 7.9 Hz, 1H), 7.90 - 7.76 (m, 3H), 5.92 - 5.87 (m, 1H), 5.84 - 5.73 (m, 1H), 4.87 - 4.74 (m, 1H), 3.84 (dd, *J* = 17.7, 4.6 Hz, 1H), 3.63 (d, *J* = 12.5 Hz, 3H), 3.60 - 3.51 (m, 1H), 3.44 (dd, *J* = 17.7, 1.9 Hz, 1H), 2.49 - 2.36 (m, 1H), 1.95 - 1.76 (m, 1H), 1.56 (d, *J* = 3.0 Hz, 3H). |
| **I-496** | $^1H$ NMR (400 MHz, $CDCl_3$) $\delta$ 7.66 (s, 1H), 7.56 (dt, *J* = 9.0, 2.0 Hz, 1H), 7.37 (dt, *J* = 8.2, 2.0 Hz, 1H), 7.24 (d, *J* = 8.9 Hz, 1H), 6.07 - 5.73 (m, 2H), 5.07 - 4.92 (m, 1H), 3.83 (d, *J* = 17.3 Hz, 1H), 3.72 (s, 3H), 3.58 - 3.41 (m, 1H), 3.22 (d, *J* = 17.3 Hz, 1H), 2.45 (dt, *J* = 14.0, 8.4 Hz, 1H), 1.86 (dt, *J* = 14.0, 3.8 Hz, 1H), 1.72 (s, 3H). |
| **I-497** | $^1H$ NMR (400 MHz, DMSO-$d_6$) $\delta$ 7.97 (d, *J* = 7.9 Hz, 1H), 7.87 - 7.80 (m, 3H), 5.94 - 5.87 (m, 1H), 5.86 - 5.75 (m, 1H), 4.90 - 4.75 (m, 1H), 4.21 (dt, *J* = 15.1, 6.6 Hz, 2H), 3.85 (dd, *J* = 17.7, 3.7 Hz, 1H), 3.63 - 3.52 (m, 1H), 3.45 (dd, *J* = 17.7, 1.7 Hz, 1H), 2.72 (dt, *J* = 13.6, 6.6 Hz, 2H), 2.50 - 2.39 (m, 1H), 2.09 (d, *J* = 12.3 Hz, 3H), 1.96 - 1.79 (m, 1H), 1.58 (d, *J* = 2.6 Hz, 3H). |
| **I-498** | $^1H$ NMR (400 MHz, $CDCl_3$) $\delta$ 7.67 (s, 1H), 7.56 (dt, *J* = 8.9, 2.0 Hz, 1H), 7.38 (dt, *J* = 8.3, 2.1 Hz, 1H), 7.19 (d, *J* = 8.9 Hz, 1H), 5.96 (ddd, *J* = 5.6, 2.6, 1.5 Hz, 1H), 5.88 (dt, *J* = 5.5, 2.2 Hz, 1H), 5.10 (pd, *J* = 6.4, 3.9 Hz, 1H), 5.05 - 4.92 (m, 1H), 3.84 (d, *J* = 17.3 Hz, 1H), 3.55 - 3.43 (m, 2H), 3.40 (dd, *J* = 10.6, 3.9 Hz, 1H), 3.35 (s, 3H), 3.22 (d, *J* = 17.3 Hz, 1H), 2.46 (dt, *J* = 14.0, 8.5 Hz, 1H), 1.85 (dt, *J* = 14.0, 3.9 Hz, 1H), 1.72 (s, 3H), 1.22 (d, *J* = 6.5 Hz, 3H). |
| **I-499** | $^1H$ NMR (400 MHz, $CDCl_3$) $\delta$ 7.67 (s, 1H), 7.56 (dt, *J* = 9.0, 2.0 Hz, 1H), 7.39 (dt, *J* = 8.3, 2.0 Hz, 1H), 7.16 (d, *J* = 8.9 Hz, 1H), 5.95 (ddt, *J* = 23.0, 5.5, 2.3 Hz, 2H), 5.06 - 4.92 (m, 1H), 4.32 (t, *J* = 6.2 Hz, 2H), 3.84 (d, *J* = 17.3 Hz, 1H), 3.68 - 3.51 (m, 1H), 3.22 (d, *J* = 17.3 Hz, 1H), 2.82 - 2.65 (m, 2H), 2.50 (dt, *J* = 14.1, 8.4 Hz, 1H), 1.89 (dt, *J* = 14.1, 3.7 Hz, 1H), 1.73 (s, 3H). |

(continued)

| NO. | $^1$H NMR |
|---|---|
| **I-500** | $^1$H NMR (400 MHz, $CDCl_3$) $\delta$ 7.66 (s, 1H), 7.49 (d, *J* = 6.6 Hz, 2H), 7.39 (s, 1H), 7.02 - 6.99 (m, 2H), 6.95 (d, *J* = 1.4 Hz, 1H), 6.12 - 6.08 (m, 1H), 6.00 - 5.93 (m, 1H), 4.65 (tq, *J* = 8.0, 4.0 Hz, 1H), 3.86 (t, *J* = 7.7 Hz, 1H), 3.29 - 3.23 (m, 1H), 3.16 - 3.08 (m, 1H), 2.59 - 2.46 (m, 2H), 2.00 (dt, *J* = 14.0, 4.3 Hz, 1H), 1.74 (s, 3H). |
| **I-501** | $^1$H NMR (400 MHz, $CDCl_3$) $\delta$ 7.67 (s, 1H), 7.57 (dt, *J* = 9.1, 2.1 Hz, 1H), 7.39 (dt, *J* = 8.2, 2.0 Hz, 1H), 7.18 (d, *J* = 8.9 Hz, 1H), 6.10 - 5.83 (m, 2H), 5.08 - 4.97 (m, 1H), 4.43 (td, *J* = 5.9, 1.9 Hz, 2H), 3.84 (d, *J* = 17.3 Hz, 1H), 3.60 - 3.50 (m, 3H), 3.23 (d, *J* = 17.3 Hz, 1H), 2.50 (dt, *J*= 14.1, 8.5 Hz, 1H), 1.91 (dt, *J* = 14.0, 3.7 Hz, 1H), 1.73 (s, 3H). |
| **I-502** | $^1$H NMR (400 MHz, $CDCl_3$) $\delta$ 7.67 (s, 1H), 7.57 (dt, *J* = 9.0, 2.0 Hz, 1H), 7.38 (dt, *J* = 8.1, 2.0 Hz, 1H), 7.21 (d, *J* = 8.9 Hz, 1H), 5.95 (ddd, *J* = 5.6, 2.6, 1.5 Hz, 1H), 5.88 (dt, *J* = 5.6, 2.2 Hz, 1H), 5.76 (ddt, *J* = 17.0, 10.2, 6.7 Hz, 1H), 5.14 - 4.95 (m, 3H), 4.17 (td, *J* = 6.7, 0.8 Hz, 2H), 3.83 (d, *J* = 17.3 Hz, 1H), 3.55 - 3.43 (m, 1H), 3.22 (d, *J* = 17.4 Hz, 1H), 2.53 - 2.35 (m, 3H), 1.86 (dt, *J* = 14.0, 3.8 Hz, 1H), 1.72 (s, 3H). |
| **I-503** | $^1$H NMR (400 MHz, $CDCl_3$) $\delta$ 7.66 (s, 1H), 7.56 (dt, *J* = 8.9, 2.0 Hz, 1H), 7.37 (dt, *J* = 8.2, 2.0 Hz, 1H), 7.22 (d, *J* = 8.8 Hz, 1H), 5.97 (ddd, *J* = 5.6, 2.5, 1.5 Hz, 1H), 5.88 (dt, *J* = 5.5, 2.3 Hz, 1H), 5.11 - 4.81 (m, 1H), 4.19 - 4.00 (m, 2H), 3.82 (d, *J*= 17.2 Hz, 1H), 3.63 - 3.50 (m, 2H), 3.36 (s, 3H), 3.22 (d, *J* = 17.3 Hz, 1H), 2.47 (dt, *J* = 14.0, 8.4 Hz, 1H), 1.87 (dt, *J* = 14.0, 3.9 Hz, 1H), 1.72 (s, 3H), 1.14 (d, *J*= 6.4 Hz, 3H). |
| **I-504** | 1H NMR (400 MHz, Chloroform-*d*) $\delta$ 7.29 (d, J = 9.7 Hz, 1H), 7.25 - 7.16 (m, 2H), 6.93 (dt, J = 9.3, 2.4 Hz, 1H), 6.55 (td, J = 72.7, 2.0 Hz, 1H), 6.05 - 5.97 (m, 1H), 5.95 - 5.82 (m, 1H), 5.30 (s, 1H), 5.06 - 4.97 (m, 1H), 3.85 - 3.74 (m, 1H), 3.61 - 3.53 (m, 1H), 3.25 - 3.15 (m, 1H), 2.61 - 2.44 (m, 1H), 2.03 - 1.85 (m, 1H), 1.72 (d, J = 3.1 Hz, 3H), 1.30 - 1.24 (m, 1H). |
| **I-505** | 1H NMR (400 MHz, Chloroform-d) $\delta$ 7.26 - 7.16 (m, 3H), 6.94 (dd, J = 9.1, 2.4 Hz, 1H), 6.75 - 6.33 (m, 1H), 5.99 - 5.91 (m, 1H), 5.92 - 5.78 (m, 1H), 5.05 - 4.97 (m, 1H), 3.83 - 3.71 (m, 4H), 3.57 - 3.48 (m, 1H), 3.19 (dd, J = 17.3, 3.2 Hz, 1H), 2.59 - 2.43 (m, 1H), 1.99 - 1.83 (m, 1H), 1.72 (d, J = 4.9 Hz, 3H). |
| **I-506** | $^1$H NMR (400 MHz, DMSO-$d_6$) $\delta$ 12.10 (s, 1H), 7.60 (dt, *J* = 3.1, 1.6 Hz, 1H),7.57 (t, *J* = 72 Hz, 1H), 7.49 - 7.42 (m, 2H), 5.95 - 5.84 (m, 1H), 5.84 - 5.67 (m, 1H), 4.95 - 4.71 (m, 1H), 3.92 - 3.71 (m, 1H), 3.51 - 3.38 (m, 3H), 2.46 - 2.30 (m, 1H), 1.89 - 1.74 (m, 1H), 1.55 (d, *J* = 4.9 Hz, 3H). |
| **I-507** | $^1$H NMR (400 MHz, DMSO-$d_6$) $\delta$ 8.06 - 7.96 (m, 1H), 7.60 (q, *J*= 1.8 Hz, 1H),7.57 (t, *J*= 72 Hz, 1H), 7.45 (d, *J* = 1.8 Hz, 2H), 5.93 - 5.86 (m, 1H), 5.84 - 5.70 (m, 2H), 4.90 - 4.73 (m, 1H), 3.78 (dd, *J* = 17.7, 4.2 Hz, 1H), 3.63 (d, *J*= 10.9 Hz, 3H), 3.59 - 3.51 (m, 1H), 3.41 (d, *J* = 1.8 Hz, 1H), 2.49 - 2.34 (m, 1H), 1.94 - 1.75 (m, 1H), 1.56 (d, *J* = 3.2 Hz, 3H). |
| **I-508** | $^1$H NMR (400 MHz, DMSO-$d_6$) $\delta$ 8.24 - 7.86 (m, 1H), 7.60 (s, 1H), 7.57 (t, *J* =72 Hz, 1H), 7.45 (s, 2H), 5.85 (dd, *J* = 23.5, 6.3 Hz, 2H), 4.97 - 4.74 (m, 1H), 4.27 - 4.13 (m, 2H), 3.87 - 3.70 (m, 1H), 3.56 (s, 1H), 3.49 - 3.38 (m, 1H), 2.82 - 2.64 (m, 2H), 2.47 - 2.31 (m, 1H), 2.21 - 2.04 (m, 3H), 1.97 - 1.77 (m, 1H), 1.55 (d, *J* = 5.6 Hz, 3H). |
| **I-509** | 1H NMR (400 MHz, Chloroform-d) $\delta$ 7.84 - 7.73 (m, 2H), 7.68 - 7.62 (m, 1H), 7.34 - 7.27 (m, 1H), 6.05 - 5.97 (m, 1H), 5.94 - 5.80 (m, 1H), 5.07 - 4.96 (m, 1H), 3.87 - 3.81 (m, 1H), 3.63 - 3.52 (m, 1H), 3.28 - 3.17 (m, 1H), 2.60 - 2.45 (m, 1H), 2.04 - 1.86 (m, 1H), 1.73 (d, J = 5.2 Hz, 3H) |
| **I-510** | $^1$H NMR (400 MHz, $CDCl_3$) $\delta$ 7.83 - 7.75 (m, 2H), 7.66 - 7.61 (m, 1H), 7.31 (d, *J* = 8.9 Hz, 1H), 6.05 - 5.98 (m, 1H), 5.97 - 5.89 (m, 1H), 5.10 - 4.95 (m, 1H), 3.84 (d, *J* = 17.4 Hz, 1H), 3.63 - 3.50 (m, 1H), 3.23 (d, *J* = 17.4 Hz, 1H), 2.55 - 2.40 (m, 1H), 1.94 - 1.82 (m, 1H), 1.73 (s, 3H). |
| **I-511** | 1H NMR (400 MHz, Chloroform-d) $\delta$ 7.86 - 7.75 (m, 2H), 7.65 (dd, J = 2.2, 1.1 Hz, 1H), 7.26 - 7.18 (m, 1H), 5.99 - 5.92 (m, 1H), 5.91 - 5.79 (m, 1H), 5.05 - 4.96 (m, 1H), 3.84 (dd, J = 17.3, 1.8 Hz, 1H), 3.74 (d, J = 12.4 Hz, 3H), 3.57 - 3.48 (m, 1H), 3.22 (dd, J = 17.3, 3.7 Hz, 1H), 2.58 - 2.41 (m, 1H), 2.00 - 1.83 (m, 1H), 1.74 (d, J = 5.2 Hz, 3H). |
| **I-512** | $^1$H NMR (400 MHz, DMSO) $\delta$ 8.04 - 8.01 (m, 1H), 8.01 - 7.97 (m, 2H), 7.95 (s, 1H), 5.93 - 5.86 (m, 1H), 5.85 - 5.79 (m, 1H), 4.85 - 4.74 (m, 1H), 3.84 (d, *J* = 17.7 Hz, 1H), 3.61 (s, 3H), 3.58 - 3.51 (m, 1H), 3.46 (d, *J* = 17.7 Hz, 1H), 2.48 - 2.37 (m, 1H), 1.88 - 1.76 (m, 1H), 1.56 (s, 3H). |

(continued)

| NO. | $^1H$ NMR |
|---|---|
| **I-513** | $^1H$ NMR (400 MHz, $CDCl_3$) $\delta$ 7.85 - 7.81 (m, 1H), 7.80 - 7.75 (m, 1H), 7.67 - 7.64 (m, 1H), 7.21 (d, *J* = 8.8 Hz, 1H), 6.00 - 5.93 (m, 1H), 5.91 - 5.84 (m, 1H), 5.06 - 4.95 (m, 1H), 4.83 - 4.71 (m, 1H), 3.84 (d, *J* = 17.3 Hz, 1H), 3.54 - 3.44 (m, 1H), 3.22 (d, *J* = 17.3 Hz, 1H), 2.51 - 2.38 (m, 1H), 1.89 - 1.78 (m, 3H), 1.75 - 1.68 (m, 5H), 1.49 - 1.20 (m, 6H). |
| **I-514** | $^1H$ NMR (400 MHz, $CDCl_3$) $\delta$ 7.86 - 7.82 (m, 1H), 7.81 - 7.75 (m, 1H), 7.68 - 7.63 (m, 1H), 7.14 (d, *J* = 8.7 Hz, 1H), 6.01 - 5.94 (m, 1H), 5.93 - 5.87 (m, 1H), 5.34 - 5.29 (m, 1H), 5.08 - 4.96 (m, 1H), 3.88 - 3.77 (m, 1H), 3.61 - 3.50 (m, 1H), 3.29 - 3.18 (m, 1H), 2.57 - 2.46 (m, 1H), 2.46 - 2.42 (m, 1H), 1.86 - 1.78 (m, 3H), 1.73 (d, *J* = 1.5 Hz, 3H), 1.04 - 0.96 (m, 3H). |
| **I-515** | $^1H$ NMR (400 MHz, $CDCl_3$) $\delta$ 8.52 - 8.44 (m, 2H), 7.84 - 7.80 (m, 1H), 7.80 - 7.75 (m, 1H), 7.70 - 7.65 (m, 1H), 7.55 - 7.50 (m, 1H), 7.40 - 7.33 (m, 1H), 7.07 - 7.01 (m, 2H), 4.73 - 4.61 (m, 1H), 3.85 (d, *J* = 17.4 Hz, 1H), 3.25 (d, *J* = 17.4 Hz, 1H), 3.19 - 3.04 (m, 2H), 2.63 - 2.49 (m, 2H), 1.75 (s, 3H). |
| **I-516** | $^1H$ NMR (400 MHz, DMSO) $\delta$ 12.47 (s, 1H), 8.61 (d, *J* = 7.8 Hz, 1H), 8.02 - 7.82 (m, 3H), 5.93 (dt, *J* = 5.6, 2.1 Hz, 1H), 5.77 (dt, *J* = 5.6, 2.3 Hz, 1H), 4.83 (ttd, *J* = 8.3, 4.1, 2.0 Hz, 1H), 4.27 (d, *J* = 18.9 Hz, 1H), 4.16 (d, *J* = 18.9 Hz, 1H), 4.02 (q, *J* = 7.1 Hz, 1H), 3.46 (ddq, *J* = 8.9, 6.8, 2.4 Hz, 1H), 2.39 (dt, *J* = 13.2, 8.3 Hz, 1H), 1.92 (dt, *J* = 13.1, 6.5 Hz, 1H). |
| **I-517** | $^1H$ NMR (400 MHz, $CDCl_3$) $\delta$ 7.70 (s, 1H), 7.61 (d, J = 8.6 Hz, 1H), 7.44 (t, J = 9.9 Hz, 2H), 6.07 - 5.98 (m, 1H), 5.95 - 5.88 (m, 1H), 5.07 (t, J = 8.4 Hz, 1H), 4.04 (d, J = 18.0 Hz, 1H), 3.79 (d, J = 18.0 Hz, 1H), 3.74 (s, 3H), 3.58 - 3.51 (m, 1H), 2.45 (dt, J = 14.1, 8.3 Hz, 1H), 1.96 (dt, J = 14.1, 3.1 Hz, 1H). |
| **I-518** | $^1H$ NMR (400 MHz, $CDCl_3$) $\delta$ 7.70 (s, 1H), 7.65 - 7.58 (m, 1H), 7.49 - 7.43 (m, 1H), 7.28 - 7.25 (m, 1H), 6.13 - 5.80 (m, 3H), 5.13 - 5.00 (m, 1H), 4.41 - 4.24 (m, 2H), 4.04 (d, *J* = 18.0 Hz, 1H), 3.79 (d, *J* = 18.0 Hz, 1H), 3.65 - 3.60 (m, 1H), 2.58 - 2.44 (m, 1H), 2.04 - 1.92 (m, 1H). |
| **I-519** | $^1H$ NMR (400 MHz, $CDCl_3$) $\delta$ 7.70 (s, 1H), 7.65 - 7.57 (m, 1H), 7.49 - 7.37 (m, 2H), 6.04 - 5.88 (m, 2H), 5.14 - 5.01 (m, 1H), 4.10 - 3.94 (m, 3H), 3.79 (d, *J* = 18.0 Hz, 1H), 3.58 - 3.49 (m, 1H), 2.50 - 2.35 (m, 1H), 2.28 - 2.15 (m, 1H), 2.03 - 1.92 (m, 1H), 1.83 - 1.67 (m, 2H), 1.64 - 1.54 (m, 4H), 1.29 - 1.19 (m, 2H). |
| **I-520** | $^1H$ NMR (400 MHz, $CDCl_3$) $\delta$ 7.69 (s, 1H), 7.65 - 7.56 (m, 1H), 7.47 (d, *J* = 8.1 Hz, 1H), 7.39 - 7.30 (m, 2H), 7.06 - 6.95 (m, 2H), 6.83 (d, *J* = 8.3 Hz, 1H), 6.22 - 6.11 (m, 1H), 6.01 - 5.92 (m, 1H), 5.29 - 5.19 (m, 1H), 4.07 (d, *J* = 18.1 Hz, 1H), 4.03 - 3.96 (m, 1H), 3.81 (d, *J* = 18.1 Hz, 1H), 2.88 - 2.74 (m, 1H), 2.05 - 1.92 (m, 1H). |
| **I-521** | $^1H$ NMR (400 MHz, $CDCl_3$) $\delta$ 7.70 (s, 1H), 7.65 - 7.59 (m, 1H), 7.48 - 7.42 (m, 1H), 7.30 (d, *J* = 8.9 Hz, 1H), 6.04 - 5.98 (m, 1H), 5.97 - 5.91 (m, 1H), 5.51 - 5.40 (m, 1H), 5.12 - 5.01 (m, 1H), 4.04 (d, *J* = 18.0 Hz, 1H), 3.79 (d, *J* = 18.0 Hz, 1H), 3.60 - 3.52 (m, 1H), 2.52 - 2.42 (m, 2H), 2.04 - 1.96 (m, 1H), 1.53 (d, *J* = 6.7 Hz, 3H). |
| **I-522** | $^1H$ NMR (400 MHz, $CDCl_3$) $\delta$ 7.70 (s, 1H), 7.64 - 7.57 (m, 1H), 7.51 - 7.40 (m, 1H), 7.29 (d, *J* = 8.7 Hz, 1H), 6.05 - 5.88 (m, 2H), 5.53 - 5.38 (m, 1H), 5.14 - 5.01 (m, 1H), 4.04 (d, *J* = 18.1 Hz, 1H), 3.79 (d, *J* = 18.1 Hz, 1H), 3.58 - 3.49 (m, 1H), 2.52 - 2.39 (m, 2H), 2.01 - 1.91 (m, 1H), 1.53 (d, *J* = 6.7 Hz, 3H). |
| **I-523** | $^1H$ NMR (400 MHz, $CDCl_3$) $\delta$ 7.89 - 7.78 (m, 2H), 7.75 - 7.69 (m, 1H), 7.40 (d, *J* = 8.9 Hz, 1H), 6.09 - 6.03 (m, 1H), 6.00 - 5.93 (m, 1H), 5.08 (t, *J* = 8.7 Hz, 1H), 4.03 (d, *J* = 18.1 Hz, 1H), 3.79 (d, *J* = 18.1 Hz, 1H), 3.62 - 3.54 (m, 1H), 2.53 - 2.40 (m, 1H), 2.02 - 1.92 (m, 1H). |
| **I-524** | $^1H$ NMR (400 MHz, DMSO) $\delta$ 8.63 (d, *J* = 7.7 Hz, 1H), 8.12 (s, 1H), 8.08 (s, 1H), 8.04 (s, 1H), 5.93 (dt, *J* = 5.6, 2.1 Hz, 1H), 5.79 (dt, *J* = 5.6, 2.3 Hz, 1H), 4.88 - 4.80 (m, 1H), 4.34 - 4.11 (m, 2H), 3.62 (s, 3H), 3.60 - 3.53 (m, 1H), 2.48 - 2.40 (m, 1H), 1.98 - 1.87 (m, 1H) |
| **I-525** | $^1H$ NMR (400 MHz, $CDCl_3$) $\delta$ 7.85 (d, *J* = 1.7 Hz, 1H), 7.80 (s, 1H), 7.72 (d, *J* = 1.9 Hz, 1H), 7.40 (d, *J* = 8.8 Hz, 1H), 6.04 - 5.99 (m, 1H), 5.97 - 5.89 (m, 1H), 5.07 (t, *J* = 8.7 Hz, 1H), 4.93 - 4.80 (m, 1H), 4.05 (d, *J* = 18.0 Hz, 1H), 3.79 (d, *J* = 18.0 Hz, 1H), 3.54 - 3.47 (m, 1H), 2.43 (dt, *J* = 14.1, 8.4 Hz, 1H), 1.99 - 1.91 (m, 1H), 1.70 - 1.50 (m, 2H), 1.23 (d, *J* = 6.3 Hz, 3H), 0.88 (t, *J* = 7.4 Hz, 3H). |
| **I-526** | $^1H$ NMR (400 MHz, $CDCl_3$) $\delta$ 7.85 (d, *J* = 1.7 Hz, 1H), 7.83 - 7.78 (m, 1H), 7.72 (d, *J* = 1.8 Hz, 1H), 7.40 (d, *J* = 8.8 Hz, 1H), 6.05 - 5.98 (m, 1H), 5.94 - 5.88 (m, 1H), 5.06 (t, *J* = 8.7 Hz, 1H), 4.93 - 4.79 (m, 1H), 4.04 (d, *J* = 18.0 Hz, 1H), 3.78 (d, *J* = 18.0 Hz, 1H), 3.57 - 3.47 (m, 1H), 2.50 - 2.32 (m, 1H), 2.00 - 1.93 (m, 1H), 1.70 - 1.51 (m, 2H), 1.22 (d, *J* = 6.3 Hz, 3H), 0.90 (t, *J* = 7.4 Hz, 3H). |

(continued)

| NO. | $^1H$ NMR |
|---|---|
| **I-527** | $^1H$ NMR (400 MHz, $CDCl_3$) $\delta$ 7.86 (d, *J* = 1.8 Hz, 1H), 7.80 (d, *J* = 1.9 Hz, 1H), 7.72 (d, *J* = 1.8 Hz, 1H), 7.41 (d, *J* = 8.8 Hz, 1H), 6.06 - 5.98 (m, 1H), 5.97 - 5.87 (m, 1H), 5.06 (t, *J* = 8.5 Hz, 1H), 4.04 (d, *J* = 18.0 Hz, 1H), 4.00 - 3.87 (m, 2H), 3.79 (d, *J* = 18.0 Hz, 1H), 3.60 - 3.49 (m, 1H), 2.50 - 2.37 (m, 1H), 2.00 - 1.92 (m, 1H), 1.78 - 1.65 (m, 6H), 1.31 - 1.13 (m, 3H), 1.03 - 0.89 (m, 2H). |
| **I-528** | $^1H$ NMR (400 MHz, $CDCl_3$) $\delta$ 8.55 - 8.45 (m, 2H), 7.87 - 7.83 (m, 1H), 7.81 - 7.77 (m, 1H), 7.75 - 7.70 (m, 1H), 7.58 - 7.50 (m, 1H), 7.41 - 7.35 (m, 1H), 7.09 (d, *J* = 7.9 Hz, 1H), 7.07 - 7.03 (m, 1H), 4.80 - 4.69 (m, 1H), 4.05 (d, *J* = 18.1 Hz, 1H), 3.82 (d, *J* = 18.1 Hz, 1H), 3.25 - 3.09 (m, 2H), 2.71 - 2.53 (m, 2H). |
| **I-529** | $^1H$ NMR (400 MHz, $CDCl_3$) $\delta$ 7.41 (d, *J* = 8.9 Hz, 1H), 7.37 - 7.31 (m, 2H), 7.13 - 7.06 (m, 1H), 6.03 - 5.98 (m, 1H), 5.95 - 5.90 (m, 1H), 5.07 (t, *J* = 8.6 Hz, 1H), 4.00 (d, *J* = 18.0 Hz, 1H), 3.78 - 3.71 (m, 4H), 3.58 - 3.52 (m, 1H), 2.50 - 2.40 (m, 1H), 2.00 - 1.92 (m, 1H). |
| **I-530** | $^1H$ NMR (400 MHz, $CDCl_3$) $\delta$ 7.68 (s, 1H), 7.58 (dt, *J* = 8.8, 2.0 Hz, 1H), 7.45 - 7.32 (m, 2H), 5.97 (ddd, *J* = 5.6, 2.6, 1.4 Hz, 1H), 5.88 (dt, *J* = 5.5, 2.3 Hz, 1H), 5.08 - 4.95 (m, 1H), 4.91 - 4.58 (m, 2H), 3.76 - 3.68 (m, 4H), 3.56 - 3.47 (m, 2H), 2.44 (dt, *J* = 14.0, 8.4 Hz, 1H), 1.90 (dt, *J* = 14.1, 3.5 Hz, 1H). |
| **I-531** | $^1H$ NMR (400 MHz, $CDCl_3$) $\delta$ 7.41 - 7.30 (m, 3H), 7.09 - 7.01 (m, 1H), 6.01 - 5.95 (m, 1H), 5.93 - 5.86 (m, 1H), 5.08 - 4.98 (m, 1H), 4.88 - 4.60 (m, 2H), 3.72 (s, 3H), 3.69 (dd, *J* = 17.6, 1.9 Hz, 1H), 3.55 - 3.51 (m, 1H), 3.48 (d, *J* = 17.5 Hz, 1H), 2.51 - 2.40 (m, 1H), 1.95 - 1.88 (m, 1H). |
| **I-532** | 1H NMR (400 MHz, $CDCl_3$) $\delta$ 7.68 (s, 1H), 7.60 (dt, J = 8.8, 2.0 Hz, 1H), 7.43 (dt, J = 8.2, 1.9 Hz, 1H), 7.36 (d, J = 8.9 Hz, 1H), 6.17 (dd, J = 54.9, 53.5 Hz, 1H), 5.99 (ddd, J = 5.6, 2.7, 1.3 Hz, 1H), 5.90 (dt, J = 5.5, 2.3 Hz, 1H), 5.04 (t, J = 8.7 Hz, 1H), 3.81 (dd, J = 17.6, 1.5 Hz, 1H), 3.72 (s, 3H), 3.66 (d, J = 17.7 Hz, 1H), 3.53 (dq, J = 8.2, 2.7 Hz, 1H), 2.45 (dt, J = 14.1, 8.3 Hz, 1H), 1.94 (dt, J = 14.1, 3.3 Hz, 1H). |
| **I-533** | $^1H$ NMR (400 MHz, $CDCl_3$) $\delta$ 7.41 - 7.30 (m, 3H), 7.07 (d, *J* = 8.5 Hz, 1H), 6.16 (t, *J* = 54.3 Hz, 1H), 6.00 (d, *J* = 4.3 Hz, 1H), 5.93 - 5.85 (m, 1H), 5.05 (t, *J* = 8.2 Hz, 1H), 3.77 (d, *J* = 17.8 Hz, 1H), 3.73 (s, 3H), 3.63 (d, *J* = 17.7 Hz, 1H), 3.57 - 3.51 (m, 1H), 2.46 (dt, *J* = 14.2, 8.4 Hz, 1H), 1.94 (dt, *J* = 14.1, 3.2 Hz, 1H). |
| **I-534** | $^1H$ NMR (400 MHz, $CDCl_3$) $\delta$ 7.68 (s, 1H), 7.61 - 7.54 (m, 1H), 7.42 - 7.36 (m, 1H), 7.20 (d, *J* = 8.9 Hz, 1H), 6.24 - 6.13 (m, 1H), 5.98 - 5.93 (m, 1H), 5.85 - 5.80 (m, 1H), 5.57 (d, *J* = 17.2 Hz, 1H), 5.36 (d, *J* = 10.7 Hz, 1H), 5.08 - 4.99 (m, 1H), 3.98 (d, *J* = 17.2 Hz, 1H), 3.75 (s, 3H), 3.56 - 3.50 (m, 1H), 3.36 (d, *J* = 17.2 Hz, 1H), 2.58 - 2.46 (m, 1H), 1.97 - 1.89 (m, 1H). |
| **I-535** | $^1H$ NMR (400 MHz, $CDCl_3$) $\delta$ 7.67 (s, 1H), 7.60 -7.54 (m, 1H), 7.41 - 7.36 (m, 1H), 7.21 (d, *J* = 8.9 Hz, 1H), 5.97 - 5.92 (m, 1H), 5.84 - 5.77 (m, 1H), 5.06 - 4.97 (m, 1H), 3.84 (d, *J* = 17.3 Hz, 1H), 3.76 (s, 3H), 3.58 - 3.50 (m, 1H), 3.22 (d, *J* = 17.3 Hz, 1H), 2.59 - 2.48 (m, 1H), 2.00 - 1.92 (m, 1H), 1.75 (s, 3H). |
| **I-536** | $^1H$ NMR (400 MHz, $CDCl_3$) $\delta$ 7.69 (s, 1H), 7.63 -7.57 (m, 1H), 7.48 - 7.37 (m, 2H), 6.04 - 5.96 (m, 1H), 5.92 - 5.83 (m, 1H), 5.09 (t, *J* = 8.7 Hz, 1H), 4.04 (d, *J* = 18.1 Hz, 1H), 3.82 - 3.75 (m, 4H), 3.60 - 3.51 (m, 1H), 2.57 - 2.46 (m, 1H), 2.04 - 1.96 (m, 1H). |
| **I-537** | $^1H$ NMR (400 MHz, $CDCl_3$) $\delta$ 7.32 (dd, *J* = 7.9, 2.0 Hz, 2H), 7.19 (d, *J* = 8.8 Hz, 1H), 7.03 (d, *J* = 8.6 Hz, 1H), 6.18 (dd, *J* = 17.2, 10.7 Hz, 1H), 6.00 - 5.91 (m, 1H), 5.89 - 5.79 (m, 1H), 5.56 (d, *J* = 17.2 Hz, 1H), 5.35 (d, *J* = 10.7 Hz, 1H), 5.09 - 4.99 (m, 1H), 3.94 (d, *J* = 17.2 Hz, 1H), 3.75 (s, 3H), 3.58 - 3.50 (m, 1H), 3.32 (d, *J* = 17.2 Hz, 1H), 2.53 (dt, *J* = 14.0, 8.4 Hz, 1H), 1.93 (dt, *J* = 14.0, 3.8 Hz, 1H). |
| **I-569** | $^1H$ NMR (400 MHz, DMSO-$d_6$) δ 8.42 (d, J = 7.8 Hz, 1H), 7.45 - 7.34 (m, 3H), 4.23 - 4.07 (m, 3H), 3.74 (d, J = 17.7 Hz, 1H), 3.37 - 3.27 (d, J = 17.7 Hz, 1H), 2.87 - 2.76 (m, 1H), 2.69 (t, J = 6.6 Hz, 2H), 2.44 - 2.14 (m, 4H), 2.09 (s, 3H), 1.53 (s, 3H). |
| **I-570** | $^1H$ NMR (400 MHz, Chloroform-d) δ 7.54 - 7.42 (m, 1H), 7.35 - 7.28 (m, 2H), 7.03 (d, J = 8.7 Hz, 1H), 6.20 - 6.07 (m, 1H), 5.60 - 5.49 (m, 1H), 5.40 - 5.32 (m, 1H), 4.66 - 4.53 (m, 2H), 4.09 - 3.97 (m, 1H), 3.97 - 3.86 (m, 2H), 3.85 - 3.75 (m, 3H), 3.37 - 3.26 (m, 1H), 2.63 - 2.45 (m, 1H), 2.13 - 1.97 (m, 1H). |
| **I-571** | $^1H$ NMR (400 MHz, Chloroform-d) δ 7.41 (dd, J = 14.3, 8.3 Hz, 1H), 7.18 - 7.10 (m, 2H), 6.93 - 6.83 (m, 1H), 6.69 - 6.26 (m, 1H), 6.13 - 6.01 (m, 1H), 5.51 - 5.41 (m, 1H), 5.32 - 5.20 (m, 1H), 4.59 - 4.45 (m, 2H), 4.01 - 3.78 (m, 3H), 3.74 (dd, 3H), 3.32 - 3.17 (m, 1H), 2.54 - 2.40 (m, 1H), 2.09 - 1.90 (m, 1H) |

(continued)

| NO. | $^1$H NMR |
|---|---|
| **I-572** | $^1$H NMR (400 MHz, DMSO-$d_6$) δ 8.20 (t, J = 6.3 Hz, 1H), 7.84 - 7.63 (m, 3H), 6.13 (ddd, J = 17.2, 10.6, 4.0 Hz, 1H), 5.35 (dd, J = 29.5, 14.0 Hz, 2H), 4.54 - 4.43 (m, 1H), 4.39 - 4.27 (m, 1H), 3.95 - 3.84 (m, 2H), 3.73 - 3.57 (m, 5H), 2.49 - 2.38 (m, 1H), 2.11 - 1.95 (m, 1H). |
| **I-573** | $^1$H NMR (400 MHz, Chloroform-d) δ 7.54 - 7.42 (m, 2H), 7.33 (s, 1H), 7.21 (d, J = 2.4 Hz, 1H), 6.56 (t, J = 72.7 Hz, 1H), 6.14 (dd, J = 17.2, 10.7 Hz, 1H), 5.53 (dd, J = 17.3, 6.3 Hz, 1H), 5.41 - 5.32 (m, 1H), 4.69 - 4.45 (m, 2H), 4.13 - 3.98 (m, 1H), 3.98 - 3.85 (m, 2H), 3.80 (d, J = 6.3 Hz, 3H), 3.32 (dd, J = 17.3, 3.5 Hz, 1H), 2.72 - 2.39 (m, 1H), 2.25 - 1.97 (m, 1H). |

Table D

| NO. | Analytical separation method | ee% | [α] |
|---|---|---|---|
| **I-5** | Method E | 100.00 | $[\alpha]_D^{20}$=-139.26° (c=0.144, in MeOH) |
| **I-6** | Method E | 100.00 | $[\alpha]_D^{20}$=-130.12° (c=0.106, in MeOH) |
| **I-7** | Method E | 97.01 | $[\alpha]_D^{20}$=-124.57° (c=0.108, in MeOH) |
| **I-11** | Method E | 98.46 | $[\alpha]_D^{20}$=-94.98° (c=0.117, in MeOH) |
| **I-12** | Method E | 97.66 | $[\alpha]_D^{20}$=-230.52° (c=0.101, in DMF) |
| **I-13** | Method E | 100.00 | $[\alpha]_D^{20}$=-140.65° (c=0.1, in MeOH) |
| **I-14** | Method E | 98.97 | $[\alpha]_D^{20}$=-132.92° (c=0.113, in MeOH) |
| **I-15** | Method E | 98.45 | $[\alpha]_D^{20}$=-172.45° (c=0.126, in DMF) |
| **I-16** | Method F | 97.28 | $[\alpha]_D^{20}$=-138.8° (c=0.096, in DMF) |
| **I-17** | Method E | 98.35 | $[\alpha]_D^{20}$=-136.81° (c=0.095, in MeOH) |
| **I-18** | Method E | 99.14 | $[\alpha]_D^{20}$=-121.05° (c=0.091, in MeOH) |
| **I-19** | Method E | 97.43 | $[\alpha]_D^{20}$=-131.43° (c=0.096, in MeOH) |
| **I-20** | Method F | 99.23 | $[\alpha]_D^{20}$=-182.76° (c=0.106, in DMF) |
| **I-21** | Method E | 99.59 | $[\alpha]_D^{20}$=138.67° (c=0.092, in MeOH) |
| **I-22** | Method E | 100.00 | $[\alpha]_D^{20}$=-184.51° (c=0.102, in DMF) |
| **I-23** | Method F | 99.46 | $[\alpha]_D^{20}$=-172.94° (c=0.1, in DMF) |
| **I-24** | Method E | 96.52 | $[\alpha]_D^{20}$=-185.67° (c=0.113, in DMF) |

(continued)

| NO. | Analytical separation method | ee% | [α] |
|---|---|---|---|
| **I-25** | Method F | 98.72 | $[\alpha]_D^{20}$=-153.57° (c=0.098, in DMF) |
| **I-26** | Method E | 100.00 | $[\alpha]_D^{20}$=-175.36° (c=0.105, in DMF) |
| **I-31** | Method E | 98.49 | $[\alpha]_D^{20}$=-173.8° (c=0.115, in DMF) |
| **I-32** | Method F | 98.42 | $[\alpha]_D^{20}$=-181.7° (c=0.096, in DMF) |
| **I-33** | Method E | 97.49 | $[\alpha]_D^{20}$=-177.95° (c=0.106, in DMF) |
| **I-34** | Method E | 98.81 | $[\alpha]_D^{20}$=-167.71° (c=0.121, in DMF) |
| **I-35** | Method F | 97.77 | $[\alpha]_D^{20}$=-176.9° (c=0.114, in DMF) |
| **I-36** | Method F | 97.52 | $[\alpha]_D^{20}$=-164.66° (c=0.092, in DMF) |
| **I-61** | Method E | 98.31 | $[\alpha]_D^{20}$=-160.22° (c=0.101, in DMF) |
| **I-62** | Method E | 98.09 | $[\alpha]_D^{20}$=-156.19° (c=0.106, in DMF) |
| **I-63** | Method E | 100.00 | $[\alpha]_D^{20}$=-138.73° (c=0.104, in DMF) |
| **I-64** | Method E | 99.06 | $[\alpha]_D^{20}$=-137.04° (c=0.111, in DMF) |
| **I-65** | Method E | 98.13 | $[\alpha]_D^{20}$=-148.46° (c=0.129, in DMF) |
| **I-66** | Method E | 99.58 | $[\alpha]_D^{20}$=-154.54° (c=0.105, in DMF) |
| **I-121** | Method E | 100.00 | $[\alpha]_D^{20}$=-214.11° (c=0.106, in DMF) |
| **I-122** | Method F | 98.48 | $[\alpha]_D^{20}$=-188.25° (c=0.107, in DMF) |
| **I-123** | Method E | 97.73 | $[\alpha]_D^{20}$=-138.67° (c=0.11, in MeOH) |
| **I-124** | Method G | Diastereomer 1: 100.00 Diastereomer 2: 100.00 | $[\alpha]_D^{20}$=-221.96° (c=0.109, in MeOH) |
| **I-125** | Method E | 98.88 | $[\alpha]_D^{20}$=-122.38° (c=0.158, in MeOH) |
| **I-126** | Method E | 96.67 | $[\alpha]_D^{20}$=-139.86° (c=0.127, in MeOH) |
| **I-127** | Method E | 100.00 | $[\alpha]_D^{20}$=-218.08° (c=0.104, in DMF) |
| **I-128** | Method E | 99.54 | $[\alpha]_D^{20}$=-221.76° (c=0.107, in DMF) |
| **I-129** | Method E | 100.00 | $[\alpha]_D^{20}$=-196.23° (c=0.106, in DMF) |

(continued)

| NO. | Analytical separation method | ee% | [α] |
|---|---|---|---|
| **I-130** | Method E | 100.00 | $[\alpha]_D^{20}$=-218.02° (c=0.114, in DMF) |
| **I-131** | Method E | 98.69 | $[\alpha]_D^{20}$=-153.87° (c=0.109, in MeOH) |
| **I-135** | Method E | 100.00 | $[\alpha]_D^{20}$=-172.00° (c=0.111, in DMF) |
| **I-136** | Method F | 98.61 | $[\alpha]_D^{20}$=-178.95° (c=0.111, in DMF) |
| **I-161** | Method F | 98.93 | $[\alpha]_D^{20}$=-211.77° (c=0.129, in DMF) |
| **I-210** | Method E | 100.00 | $[\alpha]_D^{20}$=-120.52° (c=0.093, in MeOH) |
| **I-211** | Method E | 95.37 | $[\alpha]_D^{20}$=-122.87° (c=0.091, in MeOH) |
| **I-212** | Method E | 100.00 | $[\alpha]_D^{20}$=-112.91° (c=0.071, in MeOH) |
| **I-213** | Method E | 96.37 | $[\alpha]_D^{20}$=-114.26° (c=0.081, in MeOH) |
| **I-214** | Method E | 97.44 | $[\alpha]_D^{20}$=-110.48° (c=0.161, in MeOH) |
| **I-215** | Method F | 97.51 | $[\alpha]_D^{20}$=-179.36° (c=0.098, in DMF) |
| **I-216** | Method E | 96.65 | $[\alpha]_D^{20}$=-120.23° (c=0.095, in MeOH) |
| **I-217** | Method F | 98.08 | $[\alpha]_D^{20}$=-114.04° (c=0.102, in MeOH) |
| **I-218** | Method E | 99.32 | $[\alpha]_D^{20}$=-111.53° (c=0.109, in MeOH) |
| **I-219** | Method E | 97.88 | $[\alpha]_D^{20}$=-111.11° (c=0.103, in MeOH) |
| **I-220** | Method H | Diastereomer 1: 100.00<br>Diastereomer 2: 100.00 | $[\alpha]_D^{20}$=-183.81° (c=0.112, in DMF) |
| **I-221** | Method E | 95.69 | $[\alpha]_D^{20}$=-130.65° (c=0.095, in MeOH) |
| **I-222** | Method E | 100.00 | $[\alpha]_D^{20}$=-117.9° (c=0.109, in MeOH) |
| **I-227** | Method F | 97.90 | $[\alpha]_D^{20}$=-182.19° (c=0.093, in DMF) |
| **I-228** | Method F | 98.21 | $[\alpha]_D^{20}$=-166.77° (c=0.105, in DMF) |

(continued)

| NO. | Analytical separation method | ee% | [α] |
|---|---|---|---|
| **I-253** | Method F | 97.66 | $[\alpha]_D^{20}$=-153.65° (c=0.107, in DMF) |
| **I-254** | Method F | 96.78 | $[\alpha]_D^{20}$=-137.89° (c=0.097, in DMF) |
| **I-255** | Method F | 98.77 | $[\alpha]_D^{20}$=-165.73° (c=0.151, in DMF) |
| **I-303** | Method F | 98.70 | $[\alpha]_D^{20}$=-162.31° (c=0.105, in DMF) |
| **I-307** | Method F | 100.00 | $[\alpha]_D^{20}$=-157.41° (c=0.105, in DMF) |
| **I-404** | Method F | 98.84 | $[\alpha]_D^{20}$=-185.11° (c=0.101, in DMF) |
| **I-416** | Method F | 99.04 | $[\alpha]_D^{20}$=-164.61° (c=0.111, in DMF) |
| **I-426** | Method F | 96.84 | $[\alpha]_D^{20}$=-184.58° (c=0.106, in DMF) |
| **I-428** | Method F | 97.88 | $[\alpha]_D^{20}$=-173.22° (c=0.115, in DMF) |
| **I-429** | Method F | 98.12 | $[\alpha]_D^{20}$=-170.63° (c=0.157, in DMF) |
| **I-430** | Method F | 99.12 | $[\alpha]_D^{20}$=-168.9° (c=0.111, in DMF) |
| **I-431** | Method F | 99.02 | $[\alpha]_D^{20}$=-163.46° (c=0.104, in DMF) |
| **I-432** | Method F | 98.84 | $[\alpha]_D^{20}$=-171.18° (c=0.11, in DMF) |
| **I-433** | Method F | 99.00 | $[\alpha]_D^{20}$=-166.94° (c=0.131, in DMF) |
| **I-434** | Method F | 98.92 | $[\alpha]_D^{20}$=-147.9° (c=0.156, in DMF) |
| **I-435** | Method F | 98.08 | $[\alpha]_D^{20}$=-119.56° (c=0.122, in DMF) |
| **I-436** | Method F | 100.00 | $[\alpha]_D^{20}$=-170.14° (c=0.109, in DMF) |
| **I-437** | Method F | 97.96 | $[\alpha]_D^{20}$=-171.82° (c=0.109, in DMF) |
| **I-438** | Method F | 99.49 | $[\alpha]_D^{20}$=-171.31° (c=0.108, in DMF) |
| **I-439** | Method F | 98.29 | $[\alpha]_D^{20}$=-159.67° (c=0.115, in DMF) |
| **I-440** | Method F | 100.00 | $[\alpha]_D^{20}$=-207.96° (c=0.109, in DMF) |
| **I-441** | Method F | 99.50 | $[\alpha]_D^{20}$=-164.75° (c=0.126, in DMF) |
| **I-442** | Method F | 97.36 | $[\alpha]_D^{20}$=-136.96° (c=0.128, in DMF) |
| **I-443** | Method F | 99.83 | $[\alpha]_D^{20}$=-158.99° (c=0.155, in DMF) |
| **I-445** | Method F | 95.29 | $[\alpha]_D^{20}$=-170.38° (c=0.093, in DMF) |

(continued)

| NO. | Analytical separation method | ee% | [α] |
|---|---|---|---|
| **I-446** | Method F | 99.51 | $[\alpha]_D^{20}$=-186° (c=0.099, in DMF) |
| **I-447** | Method F | 100.00 | $[\alpha]_D^{20}$=-167.66° (c=0.111, in DMF) |
| **I-448** | Method F | 98.53 | $[\alpha]_D^{20}$=-175.71° (c=0.108, in DMF) |
| **I-449** | Method F | 100.00 | $[\alpha]_D^{20}$=-176.78° (c=0.121, in DMF) |
| **I-450** | Method F | 99.23 | $[\alpha]_D^{20}$=-168.7° (c=0.135, in DMF) |
| **I-452** | Method F | 100.00 | $[\alpha]_D^{20}$=-211.21° (c=0.125, in DMF) |
| **I-454** | Method F | 98.60 | $[\alpha]_D^{20}$=-181.14° (c=0.101, in DMF) |
| **I-456** | Method E | 98.37 | $[\alpha]_D^{20}$=-170.8° (c=0.11, in DMF) |
| **I-457** | Method F | 97.61 | $[\alpha]_D^{20}$=-169.15° (c=0.083, in DMF) |
| **I-458** | Method F | 97.74 | $[\alpha]_D^{20}$=-173.18° (c=0.117, in DMF) |
| **I-459** | Method E | 99.67 | $[\alpha]_D^{20}$=-152.1° (c=0.086, in DMF) |
| **I-460** | Method E | 97.87 | $[\alpha]_D^{20}$=-165.45° (c=0.15, in DMF) |
| **I-461** | Method E | 98.17 | $[\alpha]_D^{20}$=-172.39° (c=0.125, in DMF) |
| **I-462** | Method E | 97.98 | $[\alpha]_D^{20}$=-162.61° (c=0.091, in DMF) |
| **I-463** | Method E | 98.37 | $[\alpha]_D^{20}$=-163.87° (c=0.106, in DMF) |
| **I-464** | Method E | 99.05 | $[\alpha]_D^{20}$=-155.24° (c=0.138, in DMF) |
| **I-465** | Method F | 100.00 | $[\alpha]_D^{20}$=-197.74° (c=0.106, in DMF) |
| **I-467** | Method E | 100.00 | $[\alpha]_D^{20}$=-167.34° (c=0.124, in DMF) |
| **I-468** | Method E | 98.75 | $[\alpha]_D^{20}$=-176.1° (c=0.093, in DMF) |
| **I-469** | Method E | 100.00 | $[\alpha]_D^{20}$=-206.43° (c=0.059, in DMF) |
| **I-470** | Method F | 98.19 | $[\alpha]_D^{20}$=-183.26° (c=0.1, in DMF) |
| **I-471** | Method F | 100.00 | $[\alpha]_D^{20}$=-165.81° (c=0.159, in DMF) |
| **I-472** | Method F | 100.00 | $[\alpha]_D^{20}$=-154.82° (c=0.135, in DMF) |
| **I-473** | Method F | 99.66 | $[\alpha]_D^{20}$=-137.79° (c=0.126, in DMF) |
| **I-474** | Method F | 100.00 | $[\alpha]_D^{20}$=-173.55° (c=0.129, in DMF) |

(continued)

| NO. | Analytical separation method | ee% | [α] |
|---|---|---|---|
| **I-475** | Method F | 100.00 | $[\alpha]_D^{20}$=-146.65° (c=0.145, in DMF) |
| **I-476** | Method F | 98.44 | $[\alpha]_D^{20}$=-167.61° (c=0.091, in DMF) |
| **I-477** | Method E | 95.11 | $[\alpha]_D^{20}$=-181.31° (c=0.097, in DMF) |
| **I-478** | Method F | 100.00 | $[\alpha]_D^{20}$=-170.55° (c=0.102, in DMF) |
| **I-479** | Method F | 99.01 | $[\alpha]_D^{20}$=-155.06° (c=0.121, in DMF) |
| **I-480** | Method F | 100.00 | $[\alpha]_D^{20}$=-134.97° (c=0.16, in DMF) |
| **I-481** | Method F | 99.21 | $[\alpha]_D^{20}$=-153.65° (c=0.174, in DMF) |
| **I-482** | Method F | 97.73 | $[\alpha]_D^{20}$=163.51° (c=0.135, in DMF) |
| **I-483** | Method F | 99.61 | $[\alpha]_D^{20}$=-168.44° (c=0.109, in DMF) |
| **I-484** | Method F | 100.00 | $[\alpha]_D^{20}$=-186.65° (c=0.104, in DMF) |
| **I-496** | Method F | 96.53 | $[\alpha]_D^{20}$=-219.43° (c=0.12, in DMF) |
| **I-498** | Method F | 97.74 | $[\alpha]_D^{20}$=-187.31° (c=0.1, in DMF) |
| **I-499** | Method F | 98.30 | $[\alpha]_D^{20}$=-201.15° (c=0.089, in DMF) |
| **I-501** | Method F | 99.58 | $[\alpha]_D^{20}$=-172.68° (c=0.119, in DMF) |
| **I-502** | Method F | 99.48 | $[\alpha]_D^{20}$=-195.94° (c=0.125, in DMF) |
| **I-503** | Method F | 99.45 | $[\alpha]_D^{20}$=-197.72° (c=0.095, in DMF) |
| **I-510** | Method F | 100.00 | $[\alpha]_D^{20}$=-216.65° (c=0.09, in DMF) |
| **I-512** | Method F | 96.57 | $[\alpha]_D^{20}$=-215.44° (c=0.084, in DMF) |
| **I-513** | Method F | 99.62 | $[\alpha]_D^{20}$=-194.68° (c=0.102, in DMF) |
| **I-514** | Method G | Diastereomer 1: 100.00<br>Diastereomer 2: 100.00 | $[\alpha]_D^{20}$=-208.00° (c=0.105, in DMF) |
| **I-515** | Method F | 96.97 | $[\alpha]_D^{20}$=-156.36° (c=0.096, in DMF) |
| **I-516** | Method F | 91.48 | $[\alpha]_D^{20}$=-185.43° (c=0.089, in DMF) |
| **I-517** | Method F | 96.38 | $[\alpha]_D^{20}$=-176.94° (c=0.094, in DMF) |
| **I-518** | Method F | 99.44 | $[\alpha]_D^{20}$=-167.06° (c=0.108, in DMF) |
| **I-519** | Method F | 97.30 | $[\alpha]_D^{20}$=-172.36° (c=0.155, in DMF) |

(continued)

| NO. | Analytical separation method | ee% | [α] |
|---|---|---|---|
| **I-521** | Method F | 98.50 | $[\alpha]_D^{20}$=-137.07° (c=0.105, in DMF) |
| **I-522** | Method F | 98.69 | $[\alpha]_D^{20}$=-219.48° (c=0.114, in DMF) |
| **I-523** | Method F | 100.00 | $[\alpha]_D^{20}$=-161.09° (c=0.113, in DMF) |
| **I-525** | Method F | 100.00 | $[\alpha]_D^{20}$=-168.98° (c=0.116, in DMF) |
| **I-526** | Method F | 100.00 | $[\alpha]_D^{20}$=-159.21° (c=0.139, in DMF) |
| **I-528** | Method E | 100.00 | $[\alpha]_D^{20}$=-158.38° (c=0.099, in DMF) |
| **I-529** | Method F | 95.78 | $[\alpha]_D^{20}$=-178.1° (c=0.128, in DMF) |
| **I-530** | Method F | 98.91 | $[\alpha]_D^{20}$=-154.98° (c=0.115, in DMF) |
| **I-531** | Method F | 97.86 | $[\alpha]_D^{20}$=-162.16° (c=0.1, in DMF) |
| **I-532** | Method F | 99.25 | $[\alpha]_D^{20}$=-144.48° (c=0.123, in DMF) |
| **I-534** | Method F | 100.00 | $[\alpha]_D^{20}$=-184.00° (c=0.116, in DMF) |
| **I-535** | Method F | 99.73 | $[\alpha]_D^{20}$=-206.09° (c=0.117, in DMF) |
| **I-536** | Method F | 98.65 | $[\alpha]_D^{20}$=-191.8° (c=0.115, in DMF) |
| **I-537** | Method E | 99.46 | $[\alpha]_D^{20}$=-181.65° (c=0.086, in DMF) |
| **I-570** | Method E | Diastereomer 1: 100.00<br>Diastereomer 2: 100.00 | $[\alpha]_D^{20}$=-117.69° (c=0.122, in DMF) |
| **I-571** | Method E | Diastereomer 1: 100.00<br>Diastereomer 2: 100.00 | $[\alpha]_D^{20}$=-116.12° (c=0.157, in DMF) |
| **I-572** | Method E | Diastereomer 1: 100.00<br>Diastereomer 2: 100.00 | $[\alpha]_D^{20}$=-105.64° (c=0.335, in DMF) |
| **I-573** | Method E | Diastereomer 1: 100.00<br>Diastereomer 2: 100.00 | $[\alpha]_D^{20}$=-104.45° (c=0.268, in DMF) |

Taking into account the general details related to the preparation of substituted isooxazoline carboxamide, the compound described below can be obtained using similar preparation examples as described and mentioned above.

Table 2.1: Compounds **2.1-1** to **2.1-574** of the general formula (I.1) of the present invention, wherein Z and G were defined below.

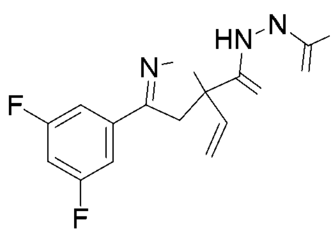

Table 2.1

| Compound NO. | Z | G |
|---|---|---|
| 2.1-1 | Z-1 | 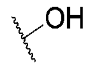 |
| 2.1-2 | Z-1 | 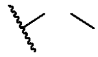 |
| 2.1-3 | Z-1 | 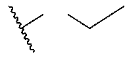 |
| 2.1-4 | Z-1 | 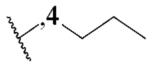 |
| 2.1-5 | Z-1 | 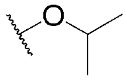 |
| 2.1-6 | Z-1 | 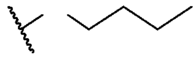 |
| 2.1-7 | Z-1 | 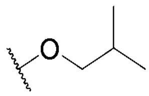 |
| 2.1-8 | Z-1 | 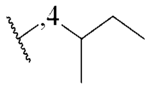 |
| 2.1-9 | Z-1 | 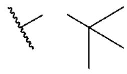 |
| 2.1-10 | Z-1 | 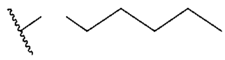 |
| 2.1-11 | Z-1 | 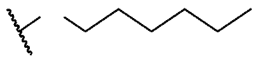 |
| 2.1-12 | Z-1 | 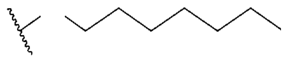 |
| 2.1-13 | Z-1 | 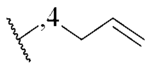 |
| 2.1-14 | Z-1 | 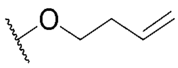 |
| 2.1-15 | Z-1 | 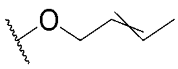 |
| 2.1-16 | Z-1 | 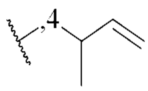 |
| 2.1-17 | Z-1 | 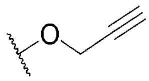 |
| 2.1-18 | Z-1 | 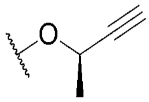 |

(continued)

| Compound NO. | Z | G |
|---|---|---|
| 2.1-19 | Z-1 | 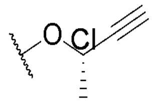 |
| 2.1-20 | Z-1 | 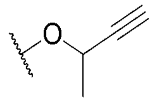 |
| 2.1-21 | Z-1 | 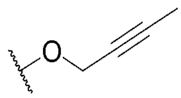 |
| 2.1-22 | Z-1 | 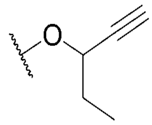 |
| 2.1-23 | Z-1 | 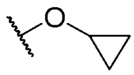 |
| 2.1-24 | Z-1 | 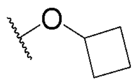 |
| 2.1-25 | Z-1 | 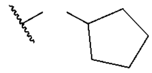 |
| 2.1-26 | Z-1 | 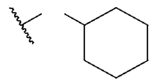 |
| 2.1-27 | Z-1 | 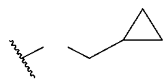 |
| 2.1-28 | Z-1 | 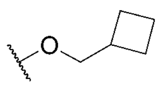 |
| 2.1-29 | Z-1 | 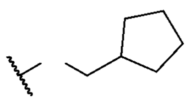 |
| 2.1-30 | Z-1 | 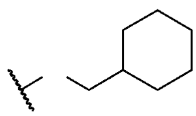 |
| 2.1-31 | Z-1 | 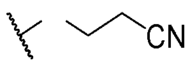 |
| 2.1-32 | Z-1 | 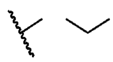 |
| 2.1-33 | Z-1 | 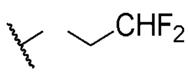 |
| 2.1-34 | Z-1 | 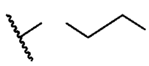 |

(continued)

| Compound NO. | Z | G |
|---|---|---|
| 2.1-35 | Z-1 | 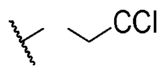 |
| 2.1-36 | Z-1 | 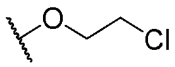 |
| 2.1-37 | Z-1 | 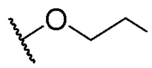 |
| 2.1-38 | Z-1 | 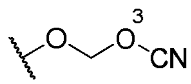 |
| 2.1-39 | Z-1 | 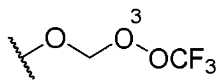 |
| 2.1-40 | Z-1 | 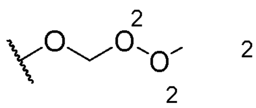 |
| 2.1-41 | Z-1 | 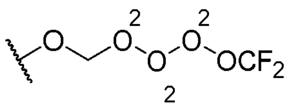 |
| 2.1-42 | Z-1 | 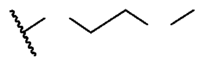 |
| 2.1-43 | Z-1 | 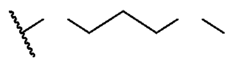 |
| 2.1-44 | Z-1 | 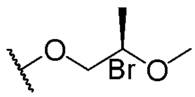 |
| 2.1-45 | Z-1 | 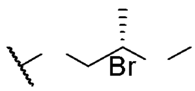 |
| 2.1-46 | Z-1 | 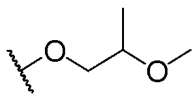 |
| 2.1-47 | Z-1 | 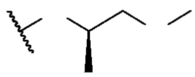 |
| 2.1-48 | Z-1 | 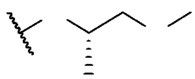 |
| 2.1-49 | Z-1 | 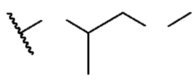 |
| 2.1-50 | Z-1 | 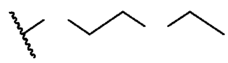 |
| 2.1-51 | Z-1 | 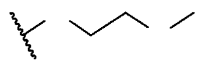 |

(continued)

| Compound NO. | Z | G |
|---|---|---|
| 2.1-52 | Z-1 | 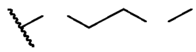 |
| 2.1-53 | Z-1 | 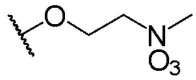 |
| 2.1-54 | Z-1 | 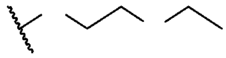 |
| 2.1-55 | Z-1 | 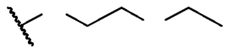 |
| 2.1-56 | Z-1 | 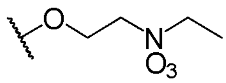 |
| 2.1-57 | Z-1 | 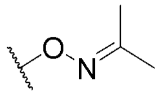 |
| 2.1-58 | Z-1 | 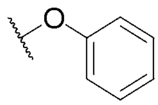 |
| 2.1-59 | Z-1 | 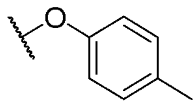 |
| 2.1-60 | Z-1 | 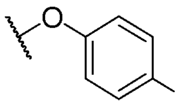 |
| 2.1-61 | Z-1 | 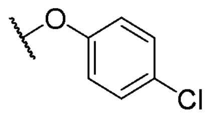 |
| 2.1-62 | Z-1 | 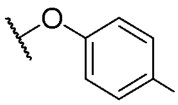 |
| 2.1-63 | Z-1 | 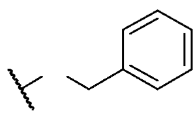 |
| 2.1-64 | Z-1 | 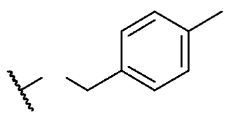 |
| 2.1-65 | Z-1 | 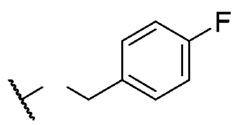 |
| 2.1-66 | Z-1 | 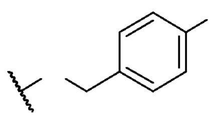 |

(continued)

| Compound NO. | Z | G |
|---|---|---|
| 2.1-67 | Z-1 | 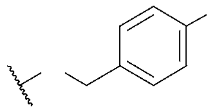 |
| 2.1-68 | Z-1 | 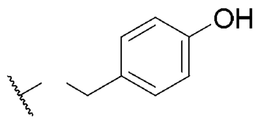 |
| 2.1-69 | Z-1 | 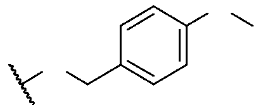 |
| 2.1-70 | Z-1 | 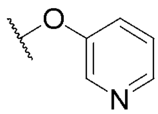 |
| 2.1-71 | Z-1 | 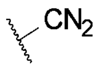 |
| 2.1-72 | Z-1 | 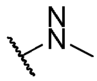 |
| 2.1-73 | Z-1 | 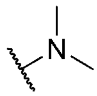 |
| 2.1-74 | Z-1 | 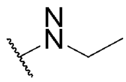 |
| 2.1-75 | Z-1 | 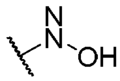 |
| 2.1-76 | Z-1 | 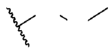 |
| 2.1-77 | Z-1 | 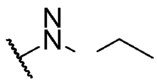 |
| 2.1-78 | Z-1 | 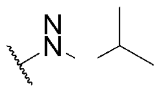 |
| 2.1-79 | Z-1 | 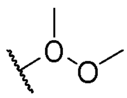 |
| 2.1-80 | Z-1 | 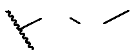 |
| 2.1-81 | Z-1 | 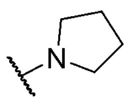 |

(continued)

| Compound NO. | Z | G |
|---|---|---|
| 2.1-82 | Z-1 | 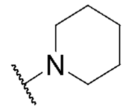 |
| 2.1-83 | Z-2 | 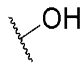 |
| 2.1-84 | Z-2 | 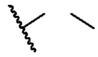 |
| 2.1-85 | Z-2 | 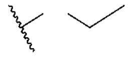 |
| 2.1-86 | Z-2 | 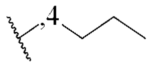 |
| 2.1-87 | Z-2 | 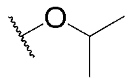 |
| 2.1-88 | Z-2 | 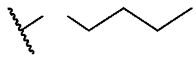 |
| 2.1-89 | Z-2 | 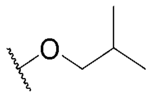 |
| 2.1-90 | Z-2 | 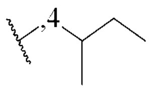 |
| 2.1-91 | Z-2 | 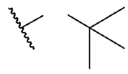 |
| 2.1-92 | Z-2 | 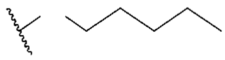 |
| 2.1-93 | Z-2 | 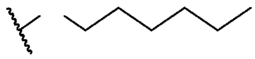 |
| 2.1-94 | Z-2 | 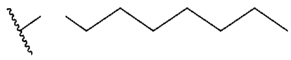 |
| 2.1-95 | Z-2 | 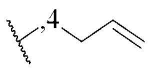 |
| 2.1-96 | Z-2 | 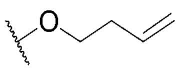 |
| 2.1-97 | Z-2 | 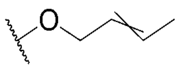 |
| 2.1-98 | Z-2 | 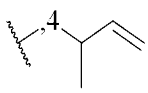 |
| 2.1-99 | Z-2 | 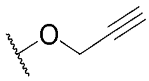 |

(continued)

| Compound NO. | Z | G |
|---|---|---|
| 2.1-100 | Z-2 | 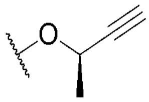 |
| 2.1-101 | Z-2 | 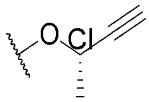 |
| 2.1-102 | Z-2 | 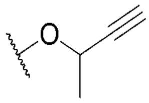 |
| 2.1-103 | Z-2 | 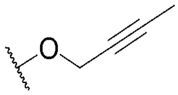 |
| 2.1-104 | Z-2 | 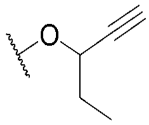 |
| 2.1-105 | Z-2 | 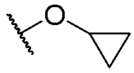 |
| 2.1-106 | Z-2 | 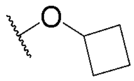 |
| 2.1-107 | Z-2 | 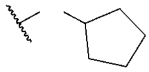 |
| 2.1-108 | Z-2 | 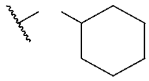 |
| 2.1-109 | Z-2 | 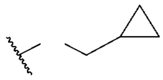 |
| 2.1-110 | Z-2 | 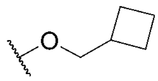 |
| 2.1-111 | Z-2 | 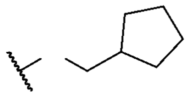 |
| 2.1-112 | Z-2 | 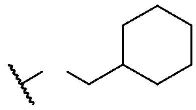 |
| 2.1-113 | Z-2 | 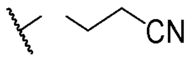 |
| 2.1-114 | Z-2 | 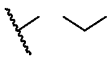 |
| 2.1-115 | Z-2 | 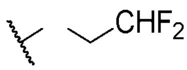 |

(continued)

| Compound NO. | Z | G |
|---|---|---|
| 2.1-116 | Z-2 | 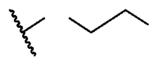 |
| 2.1-117 | Z-2 | 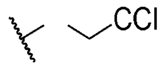 |
| 2.1-118 | Z-2 | 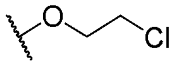 |
| 2.1-119 | Z-2 | 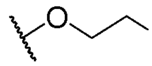 |
| 2.1-120 | Z-2 | 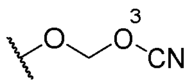 |
| 2.1-121 | Z-2 | 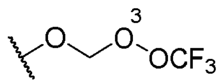 |
| 2.1-122 | Z-2 | 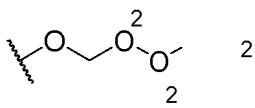 |
| 2.1-123 | Z-2 | 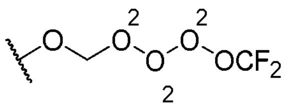 |
| 2.1-124 | Z-2 | 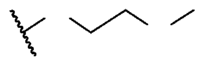 |
| 2.1-125 | Z-2 | 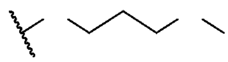 |
| 2.1-126 | Z-2 | 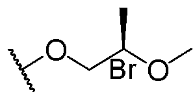 |
| 2.1-127 | Z-2 | 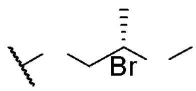 |
| 2.1-128 | Z-2 | 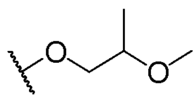 |
| 2.1-129 | Z-2 | 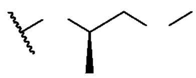 |
| 2.1-130 | Z-2 | 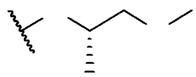 |
| 2.1-131 | Z-2 | 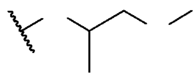 |
| 2.1-132 | Z-2 | 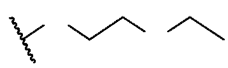 |

(continued)

| Compound NO. | Z | G |
|---|---|---|
| 2.1-133 | Z-2 | 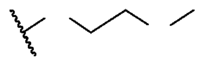 |
| 2.1-134 | Z-2 | 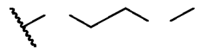 |
| 2.1-135 | Z-2 | 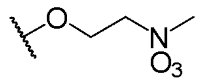 |
| 2.1-136 | Z-2 | 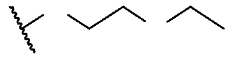 |
| 2.1-137 | Z-2 | 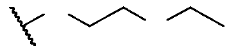 |
| 2.1-138 | Z-2 | 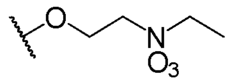 |
| 2.1-139 | Z-2 | 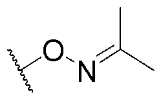 |
| 2.1-140 | Z-2 | 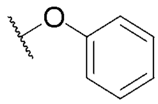 |
| 2.1-141 | Z-2 | 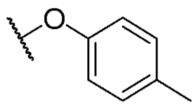 |
| 2.1-142 | Z-2 | 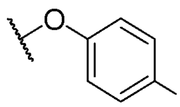 |
| 2.1-143 | Z-2 | 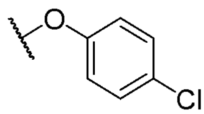 |
| 2.1-144 | Z-2 | 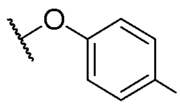 |
| 2.1-145 | Z-2 | 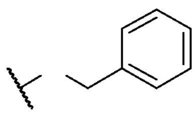 |
| 2.1-146 | Z-2 | 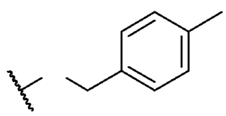 |
| 2.1-147 | Z-2 | 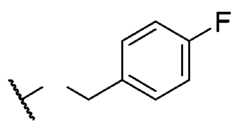 |

(continued)

| Compound NO. | Z | G |
|---|---|---|
| 2.1-148 | Z-2 | 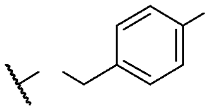 |
| 2.1-149 | Z-2 | 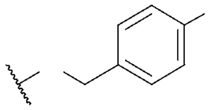 |
| 2.1-150 | Z-2 | 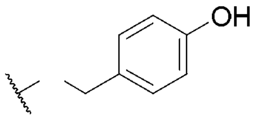 |
| 2.1-151 | Z-2 | 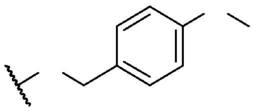 |
| 2.1-152 | Z-2 | 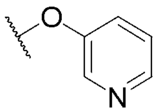 |
| 2.1-153 | Z-2 | 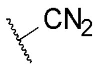 |
| 2.1-154 | Z-2 | 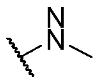 |
| 2.1-155 | Z-2 | 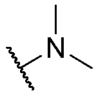 |
| 2.1-156 | Z-2 | 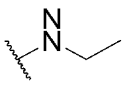 |
| 2.1-157 | Z-2 | 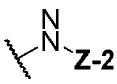 |
| 2.1-158 | Z-2 | 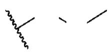 |
| 2.1-159 | Z-2 | 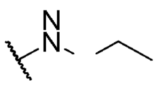 |
| 2.1-160 | Z-2 | 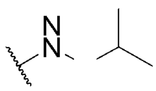 |
| 2.1-161 | Z-2 | 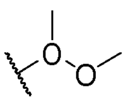 |
| 2.1-162 | Z-2 | 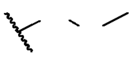 |

(continued)

| Compound NO. | Z | G |
|---|---|---|
| 2.1-163 | Z-2 | 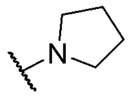 |
| 2.1-164 | Z-2 | 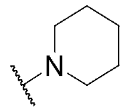 |
| 2.1-165 | Z-3 | 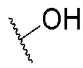 |
| 2.1-166 | Z-3 | 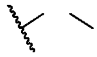 |
| 2.1-167 | Z-3 | 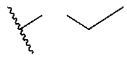 |
| 2.1-168 | Z-3 | 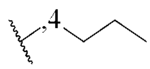 |
| 2.1-169 | Z-3 | 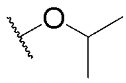 |
| 2.1-170 | Z-3 | 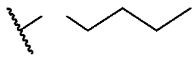 |
| 2.1-171 | Z-3 | 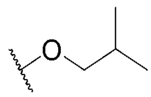 |
| 2.1-172 | Z-3 | 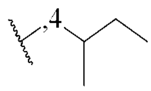 |
| 2.1-173 | Z-3 | 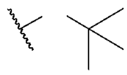 |
| 2.1-174 | Z-3 | 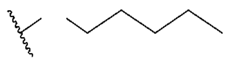 |
| 2.1-175 | Z-3 | 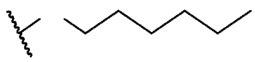 |
| 2.1-176 | Z-3 | 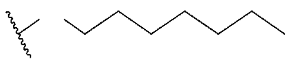 |
| 2.1-177 | Z-3 | 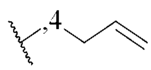 |
| 2.1-178 | Z-3 | 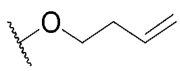 |
| 2.1-179 | Z-3 | 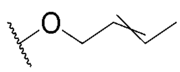 |
| 2.1-180 | Z-3 | 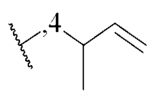 |

(continued)

| Compound NO. | Z | G |
|---|---|---|
| 2.1-181 | Z-3 | 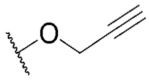 |
| 2.1-182 | Z-3 | 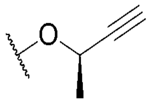 |
| 2.1-183 | Z-3 | 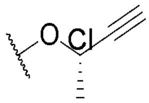 |
| 2.1-184 | Z-3 | 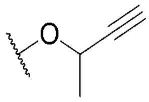 |
| 2.1-185 | Z-3 | 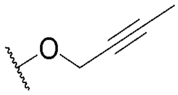 |
| 2.1-186 | Z-3 | 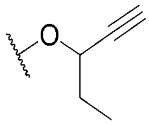 |
| 2.1-187 | Z-3 | 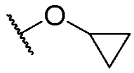 |
| 2.1-188 | Z-3 | 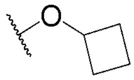 |
| 2.1-189 | Z-3 | 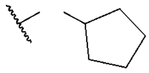 |
| 2.1-190 | Z-3 | 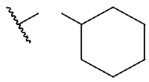 |
| 2.1-191 | Z-3 | 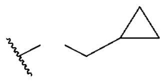 |
| 2.1-192 | Z-3 | 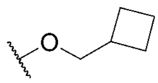 |
| 2.1-193 | Z-3 | 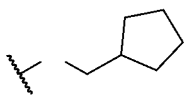 |
| 2.1-194 | Z-3 | 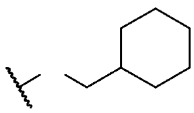 |
| 2.1-195 | Z-3 | 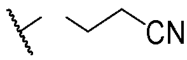 |

(continued)

| Compound NO. | Z | G |
|---|---|---|
| 2.1-196 | Z-3 | 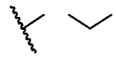 |
| 2.1-197 | Z-3 | 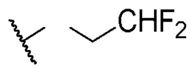 |
| 2.1-198 | Z-3 | 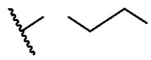 |
| 2.1-199 | Z-3 | 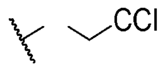 |
| 2.1-200 | Z-3 | 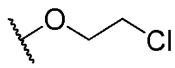 |
| 2.1-201 | Z-3 | 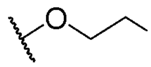 |
| 2.1-202 | Z-3 | 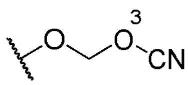 |
| 2.1-203 | Z-3 | 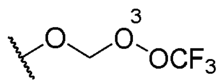 |
| 2.1-204 | Z-3 | 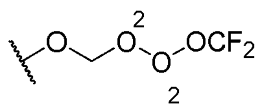 |
| 2.1-205 | Z-3 | 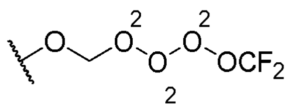 |
| 2.1-206 | Z-3 | 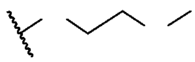 |
| 2.1-207 | Z-3 | 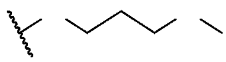 |
| 2.1-208 | Z-3 | 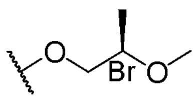 |
| 2.1-209 | Z-3 | 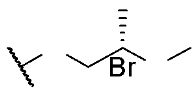 |
| 2.1-210 | Z-3 | 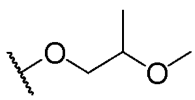 |
| 2.1-211 | Z-3 | 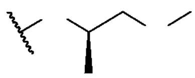 |
| 2.1-212 | Z-3 | 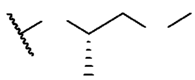 |

(continued)

| Compound NO. | Z | G |
|---|---|---|
| 2.1-213 | Z-3 | 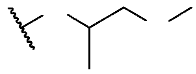 |
| 2.1-214 | Z-3 | 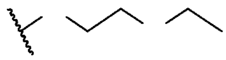 |
| 2.1-215 | Z-3 | 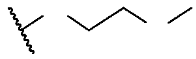 |
| 2.1-216 | Z-3 | 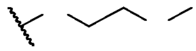 |
| 2.1-217 | Z-3 | 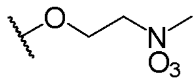 |
| 2.1-218 | Z-3 | 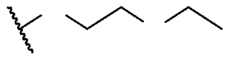 |
| 2.1-219 | Z-3 | 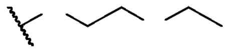 |
| 2.1-220 | Z-3 | 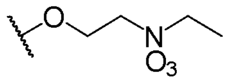 |
| 2.1-221 | Z-3 | 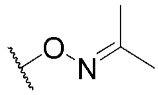 |
| 2.1-222 | Z-3 | 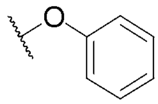 |
| 2.1-223 | Z-3 | 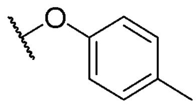 |
| 2.1-224 | Z-3 | 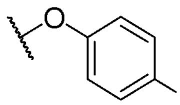 |
| 2.1-225 | Z-3 | 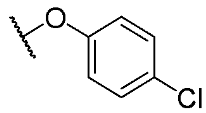 |
| 2.1-226 | Z-3 | 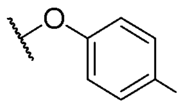 |
| 2.1-227 | Z-3 | 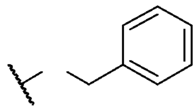 |
| 2.1-228 | Z-3 | 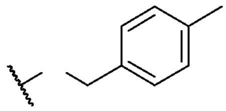 |

(continued)

| Compound NO. | Z | G |
|---|---|---|
| 2.1-229 | Z-3 | 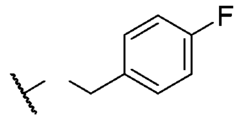 |
| 2.1-230 | Z-3 | 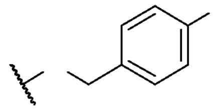 |
| 2.1-231 | Z-3 | 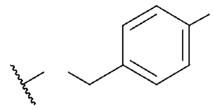 |
| 2.1-232 | Z-3 | 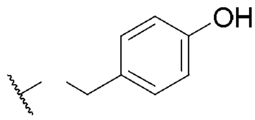 |
| 2.1-233 | Z-3 | 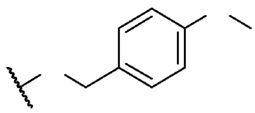 |
| 2.1-234 | Z-3 | 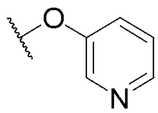 |
| 2.1-235 | Z-3 | 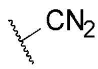 |
| 2.1-236 | Z-3 | 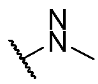 |
| 2.1-237 | Z-3 | 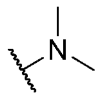 |
| 2.1-238 | Z-3 | 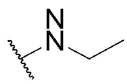 |
| 2.1-239 | Z-3 | 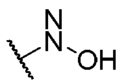 |
| 2.1-240 | Z-3 | 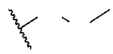 |
| 2.1-241 | Z-3 | 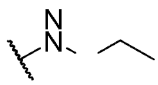 |
| 2.1-242 | Z-3 | 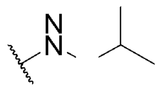 |
| 2.1-243 | Z-3 | 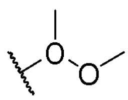 |

(continued)

| Compound NO. | Z | G |
|---|---|---|
| 2.1-244 | Z-3 | 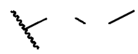 |
| 2.1-245 | Z-3 | 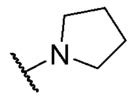 |
| 2.1-246 | Z-3 | 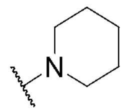 |
| 2.1-247 | Z-4 | 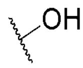 |
| 2.1-248 | Z-4 | 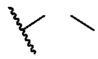 |
| 2.1-249 | Z-4 | 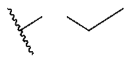 |
| 2.1-250 | Z-4 | 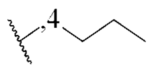 |
| 2.1-251 | Z-4 | 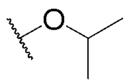 |
| 2.1-252 | Z-4 | 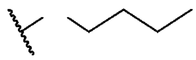 |
| 2.1-253 | Z-4 | 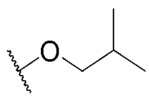 |
| 2.1-254 | Z-4 | 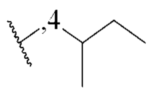 |
| 2.1-255 | Z-4 | 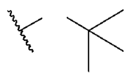 |
| 2.1-256 | Z-4 | 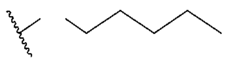 |
| 2.1-257 | Z-4 | 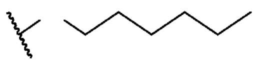 |
| 2.1-258 | Z-4 | 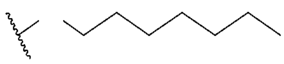 |
| 2.1-259 | Z-4 | 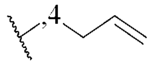 |
| 2.1-260 | Z-4 | 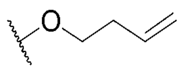 |
| 2.1-261 | Z-4 | 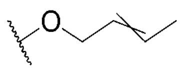 |

(continued)

| Compound NO. | Z | G |
|---|---|---|
| 2.1-262 | Z-4 | 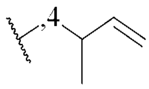 |
| 2.1-263 | Z-4 | 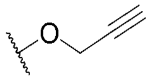 |
| 2.1-264 | Z-4 | 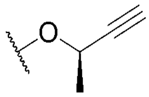 |
| 2.1-265 | Z-4 | 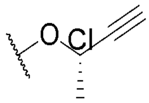 |
| 2.1-266 | Z-4 | 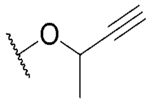 |
| 2.1-267 | Z-4 | 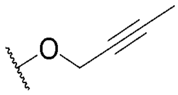 |
| 2.1-268 | Z-4 | 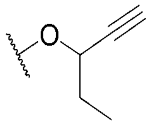 |
| 2.1-269 | Z-4 | 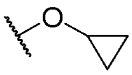 |
| 2.1-270 | Z-4 | 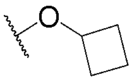 |
| 2.1-271 | Z-4 | 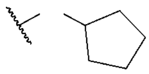 |
| 2.1-272 | Z-4 | 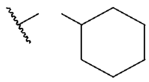 |
| 2.1-273 | Z-4 | 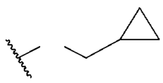 |
| 2.1-274 | Z-4 | 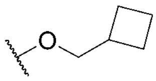 |
| 2.1-275 | Z-4 | 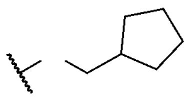 |
| 2.1-276 | Z-4 | 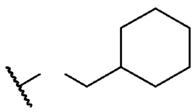 |

(continued)

| Compound NO. | Z | G |
|---|---|---|
| 2.1-277 | Z-4 | 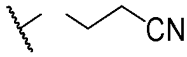 |
| 2.1-278 | Z-4 | 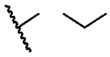 |
| 2.1-279 | Z-4 | 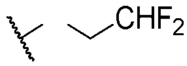 |
| 2.1-280 | Z-4 | 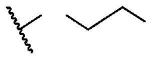 |
| 2.1-281 | Z-4 | 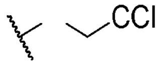 |
| 2.1-282 | Z-4 | 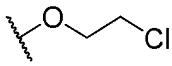 |
| 2.1-283 | Z-4 | 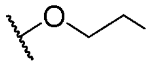 |
| 2.1-284 | Z-4 | 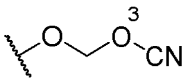 |
| 2.1-285 | Z-4 | 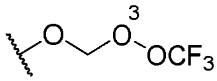 |
| 2.1-286 | Z-4 | 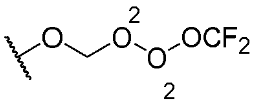 |
| 2.1-287 | Z-4 | 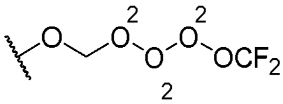 |
| 2.1-288 | Z-4 | 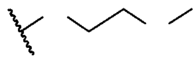 |
| 2.1-289 | Z-4 | 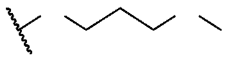 |
| 2.1-290 | Z-4 | 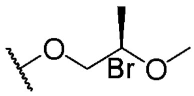 |
| 2.1-291 | Z-4 | 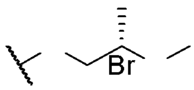 |
| 2.1-292 | Z-4 | 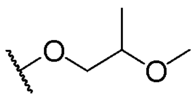 |
| 2.1-293 | Z-4 | 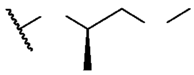 |

(continued)

| Compound NO. | Z | G |
|---|---|---|
| 2.1-294 | Z-4 | 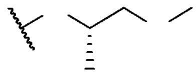 |
| 2.1-295 | Z-4 | 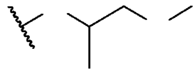 |
| 2.1-296 | Z-4 | 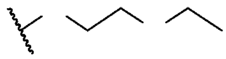 |
| 2.1-297 | Z-4 | 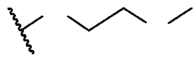 |
| 2.1-298 | Z-4 | 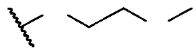 |
| 2.1-299 | Z-4 | 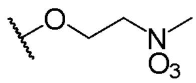 |
| 2.1-300 | Z-4 | 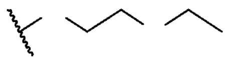 |
| 2.1-301 | Z-4 | 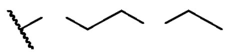 |
| 2.1-302 | Z-4 | 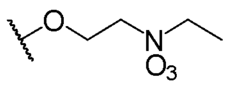 |
| 2.1-303 | Z-4 | 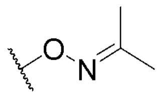 |
| 2.1-304 | Z-4 | 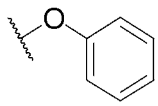 |
| 2.1-305 | Z-4 | 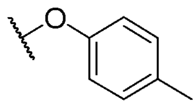 |
| 2.1-306 | Z-4 | 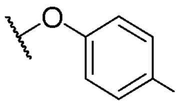 |
| 2.1-307 | Z-4 | 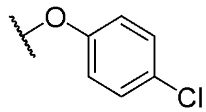 |
| 2.1-308 | Z-4 | 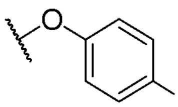 |
| 2.1-309 | Z-4 | 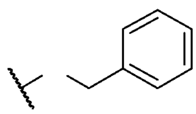 |

(continued)

| Compound NO. | Z | G |
|---|---|---|
| 2.1-310 | Z-4 | 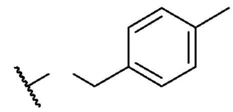 |
| 2.1-311 | Z-4 | 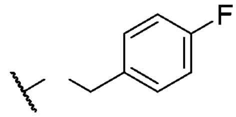 |
| 2.1-312 | Z-4 | 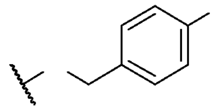 |
| 2.1-313 | Z-4 | 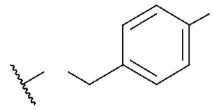 |
| 2.1-314 | Z-4 | 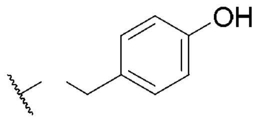 |
| 2.1-315 | Z-4 | 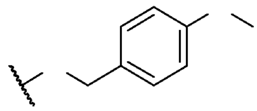 |
| 2.1-316 | Z-4 | 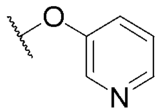 |
| 2.1-317 | Z-4 | 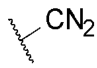 |
| 2.1-318 | Z-4 | 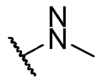 |
| 2.1-319 | Z-4 | 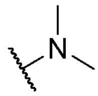 |
| 2.1-320 | Z-4 | 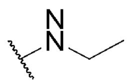 |
| 2.1-321 | Z-4 | 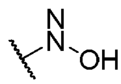 |
| 2.1-322 | Z-4 | 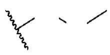 |
| 2.1-323 | Z-4 | 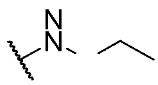 |

(continued)

| Compound NO. | Z | G |
|---|---|---|
| 2.1-324 | Z-4 | 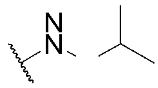 |
| 2.1-325 | Z-4 | 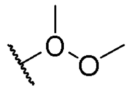 |
| 2.1-326 | Z-4 | 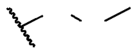 |
| 2.1-327 | Z-4 | 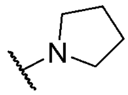 |
| 2.1-328 | Z-4 | 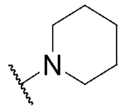 |
| 2.1-329 | Z-5 | 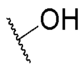 |
| 2.1-330 | Z-5 | 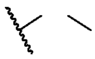 |
| 2.1-331 | Z-5 | 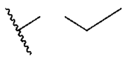 |
| 2.1-332 | Z-5 | 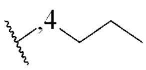 |
| 2.1-333 | Z-5 | 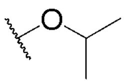 |
| 2.1-334 | Z-5 | 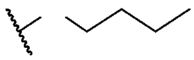 |
| 2.1-335 | Z-5 | 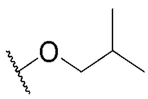 |
| 2.1-336 | Z-5 | 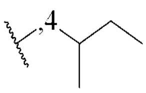 |
| 2.1-337 | Z-5 | 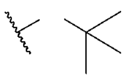 |
| 2.1-338 | Z-5 | 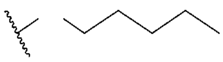 |
| 2.1-339 | Z-5 | 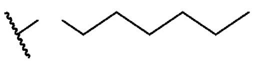 |
| 2.1-340 | Z-5 | 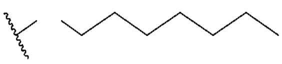 |
| 2.1-341 | Z-5 | 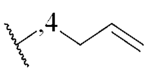 |

(continued)

| Compound NO. | Z | G |
|---|---|---|
| 2.1-342 | Z-5 | 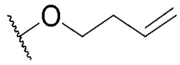 |
| 2.1-343 | Z-5 | 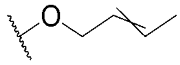 |
| 2.1-344 | Z-5 | 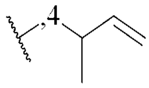 |
| 2.1-345 | Z-5 | 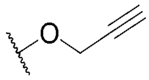 |
| 2.1-346 | Z-5 | 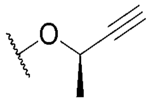 |
| 2.1-347 | Z-5 | 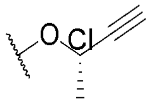 |
| 2.1-348 | Z-5 | 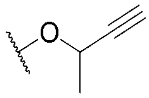 |
| 2.1-349 | Z-5 | 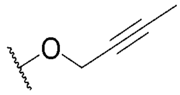 |
| 2.1-350 | Z-5 | 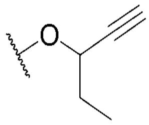 |
| 2.1-351 | Z-5 | 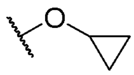 |
| 2.1-352 | Z-5 | 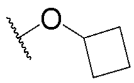 |
| 2.1-353 | Z-5 | 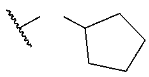 |
| 2.1-354 | Z-5 | 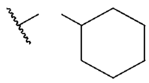 |
| 2.1-355 | Z-5 | 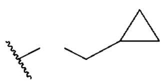 |
| 2.1-356 | Z-5 | 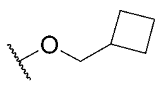 |
| 2.1-357 | Z-5 | 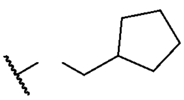 |

(continued)

| Compound NO. | Z | G |
|---|---|---|
| 2.1-358 | Z-5 | 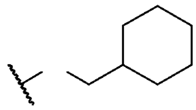 |
| 2.1-359 | Z-5 | 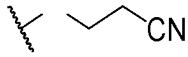 |
| 2.1-360 | Z-5 | 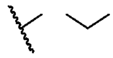 |
| 2.1-361 | Z-5 | 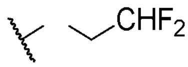 |
| 2.1-362 | Z-5 | 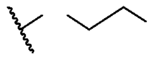 |
| 2.1-363 | Z-5 | 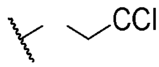 |
| 2.1-364 | Z-5 | 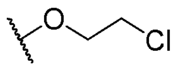 |
| 2.1-365 | Z-5 | 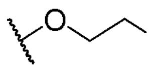 |
| 2.1-366 | Z-5 | 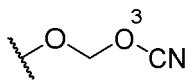 |
| 2.1-367 | Z-5 | 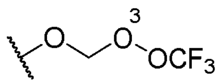 |
| 2.1-368 | Z-5 | 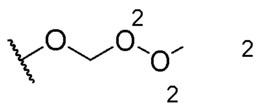 |
| 2.1-369 | Z-5 | 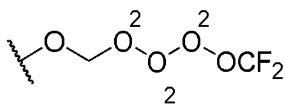 |
| 2.1-370 | Z-5 | 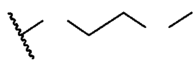 |
| 2.1-371 | Z-5 | 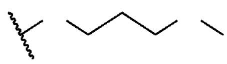 |
| 2.1-372 | Z-5 | 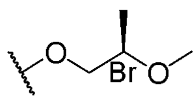 |
| 2.1-373 | Z-5 | 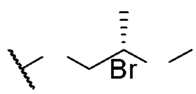 |
| 2.1-374 | Z-5 | 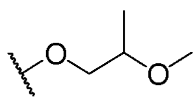 |

(continued)

| Compound NO. | Z | G |
|---|---|---|
| 2.1-375 | Z-5 | 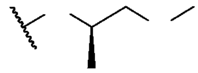 |
| 2.1-376 | Z-5 | 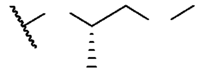 |
| 2.1-377 | Z-5 | 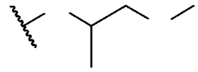 |
| 2.1-378 | Z-5 | 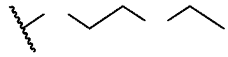 |
| 2.1-379 | Z-5 | 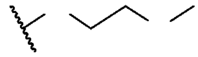 |
| 2.1-380 | Z-5 | 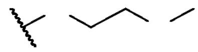 |
| 2.1-381 | Z-5 | 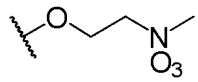 |
| 2.1-382 | Z-5 | 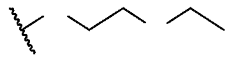 |
| 2.1-383 | Z-5 | 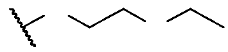 |
| 2.1-384 | Z-5 | 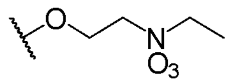 |
| 2.1-385 | Z-5 | 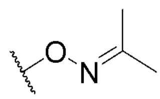 |
| 2.1-386 | Z-5 | 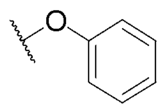 |
| 2.1-387 | Z-5 | 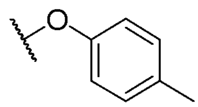 |
| 2.1-388 | Z-5 | 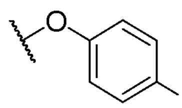 |
| 2.1-389 | Z-5 | 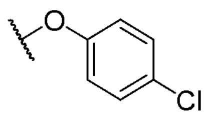 |
| 2.1-390 | Z-5 | 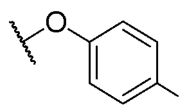 |

(continued)

| Compound NO. | Z | G |
|---|---|---|
| 2.1-391 | Z-5 | 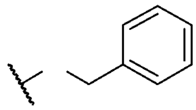 |
| 2.1-392 | Z-5 | 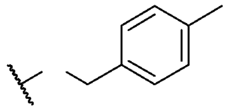 |
| 2.1-393 | Z-5 | 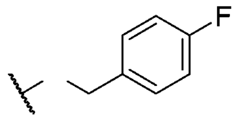 |
| 2.1-394 | Z-5 | 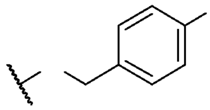 |
| 2.1-395 | Z-5 | 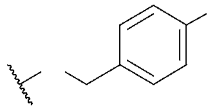 |
| 2.1-396 | Z-5 | 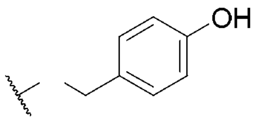 |
| 2.1-397 | Z-5 | 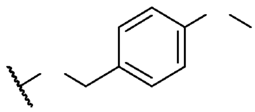 |
| 2.1-398 | Z-5 | 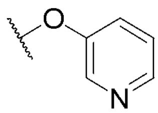 |
| 2.1-399 | Z-5 | 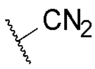 |
| 2.1-400 | Z-5 | 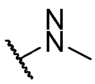 |
| 2.1-401 | Z-5 | 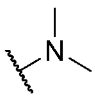 |
| 2.1-402 | Z-5 | 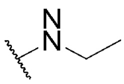 |
| 2.1-403 | Z-5 | 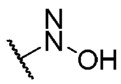 |
| 2.1-404 | Z-5 | 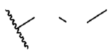 |

(continued)

| Compound NO. | Z | G |
|---|---|---|
| 2.1-405 | Z-5 | 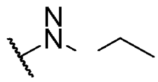 |
| 2.1-406 | Z-5 | 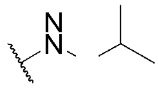 |
| 2.1-407 | Z-5 | 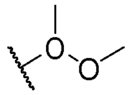 |
| 2.1-408 | Z-5 | 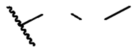 |
| 2.1-409 | Z-5 | 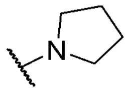 |
| 2.1-410 | Z-5 | 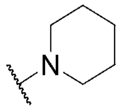 |
| 2.1-411 | Z-6 | 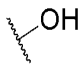 |
| 2.1-412 | Z-6 | 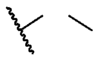 |
| 2.1-413 | Z-6 | 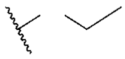 |
| 2.1-414 | Z-6 | 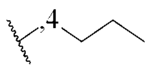 |
| 2.1-415 | Z-6 | 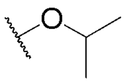 |
| 2.1-416 | Z-6 | 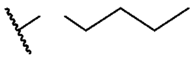 |
| 2.1-417 | Z-6 | 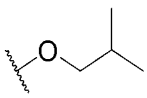 |
| 2.1-418 | Z-6 | 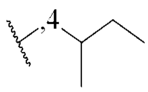 |
| 2.1-419 | Z-6 | 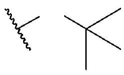 |
| 2.1-420 | Z-6 | 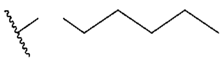 |
| 2.1-421 | Z-6 | 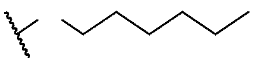 |
| 2.1-422 | Z-6 | 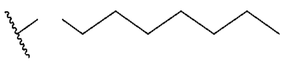 |

(continued)

| Compound NO. | Z | G |
|---|---|---|
| 2.1-423 | Z-6 | 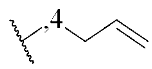 |
| 2.1-424 | Z-6 | 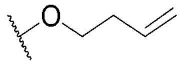 |
| 2.1-425 | Z-6 | 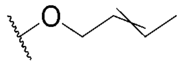 |
| 2.1-426 | Z-6 | 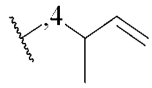 |
| 2.1-427 | Z-6 | 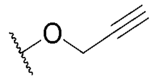 |
| 2.1-428 | Z-6 | 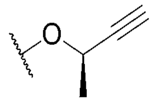 |
| 2.1-429 | Z-6 | 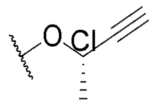 |
| 2.1-430 | Z-6 | 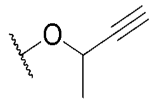 |
| 2.1-431 | Z-6 | 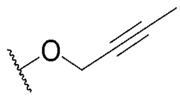 |
| 2.1-432 | Z-6 | 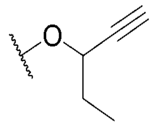 |
| 2.1-433 | Z-6 | 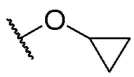 |
| 2.1-434 | Z-6 | 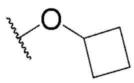 |
| 2.1-435 | Z-6 | 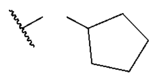 |
| 2.1-436 | Z-6 | 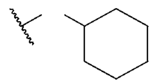 |
| 2.1-437 | Z-6 | 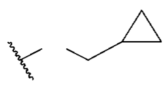 |
| 2.1-438 | Z-6 | 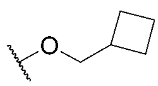 |

(continued)

| Compound NO. | Z | G |
|---|---|---|
| 2.1-439 | Z-6 | 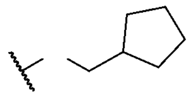 |
| 2.1-440 | Z-6 | 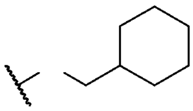 |
| 2.1-441 | Z-6 | 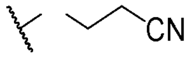 |
| 2.1-442 | Z-6 | 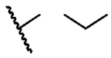 |
| 2.1-443 | Z-6 | 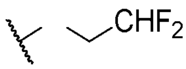 |
| 2.1-444 | Z-6 | 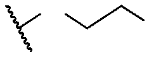 |
| 2.1-445 | Z-6 | 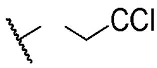 |
| 2.1-446 | Z-6 | 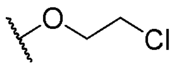 |
| 2.1-447 | Z-6 | 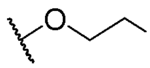 |
| 2.1-448 | Z-6 | 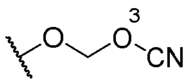 |
| 2.1-449 | Z-6 | 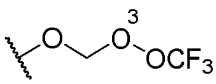 |
| 2.1-450 | Z-6 | 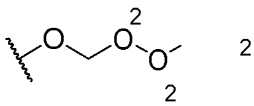 |
| 2.1-451 | Z-6 | 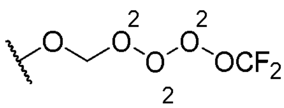 |
| 2.1-452 | Z-6 | 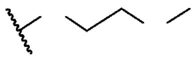 |
| 2.1-453 | Z-6 | 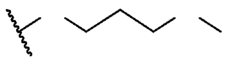 |
| 2.1-454 | Z-6 | 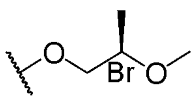 |
| 2.1-455 | Z-6 | 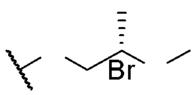 |

(continued)

| Compound NO. | Z | G |
|---|---|---|
| 2.1-456 | Z-6 | 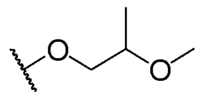 |
| 2.1-457 | Z-6 | 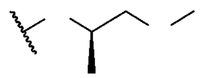 |
| 2.1-458 | Z-6 | 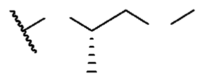 |
| 2.1-459 | Z-6 | 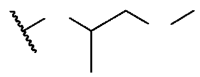 |
| 2.1-460 | Z-6 | 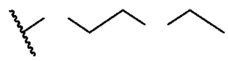 |
| 2.1-461 | Z-6 | 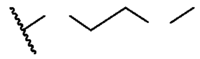 |
| 2.1-462 | Z-6 | 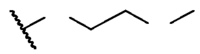 |
| 2.1-463 | Z-6 | 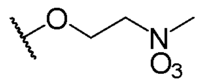 |
| 2.1-464 | Z-6 | 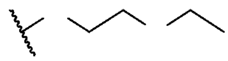 |
| 2.1-465 | Z-6 | 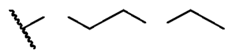 |
| 2.1-466 | Z-6 | 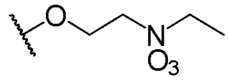 |
| 2.1-467 | Z-6 | 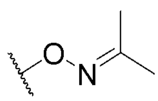 |
| 2.1-468 | Z-6 | 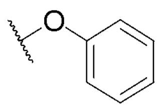 |
| 2.1-469 | Z-6 | 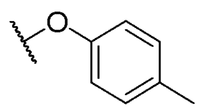 |
| 2.1-470 | Z-6 | 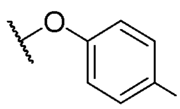 |
| 2.1-471 | Z-6 | 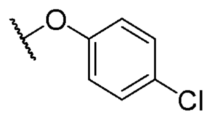 |

(continued)

| Compound NO. | Z | G |
|---|---|---|
| 2.1-472 | Z-6 | 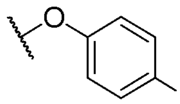 |
| 2.1-473 | Z-6 | 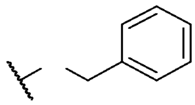 |
| 2.1-474 | Z-6 | 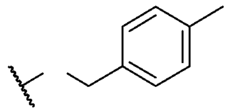 |
| 2.1-475 | Z-6 | 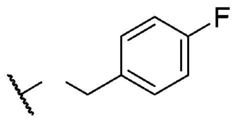 |
| 2.1-476 | Z-6 | 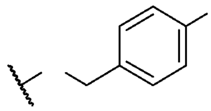 |
| 2.1-477 | Z-6 | 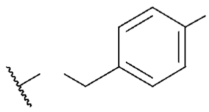 |
| 2.1-478 | Z-6 | 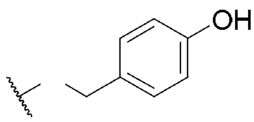 |
| 2.1-479 | Z-6 | 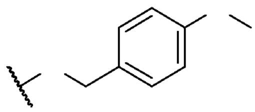 |
| 2.1-480 | Z-6 | 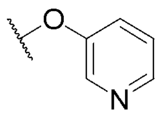 |
| 2.1-481 | Z-6 | 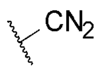 |
| 2.1-482 | Z-6 | 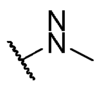 |
| 2.1-483 | Z-6 | 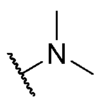 |
| 2.1-484 | Z-6 | 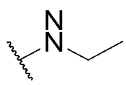 |
| 2.1-485 | Z-6 | 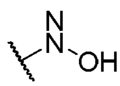 |

(continued)

| Compound NO. | Z | G |
|---|---|---|
| 2.1-486 | Z-6 | 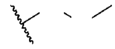 |
| 2.1-487 | Z-6 | 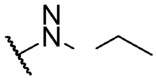 |
| 2.1-488 | Z-6 | 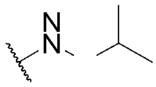 |
| 2.1-489 | Z-6 | 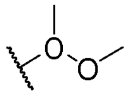 |
| 2.1-490 | Z-6 | 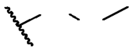 |
| 2.1-491 | Z-6 | 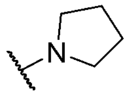 |
| 2.1-492 | Z-6 | 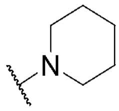 |
| 2.1-493 | Z-7 | 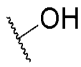 |
| 2.1-494 | Z-7 | 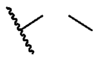 |
| 2.1-495 | Z-7 | 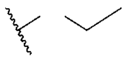 |
| 2.1-496 | Z-7 | 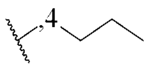 |
| 2.1-497 | Z-7 | 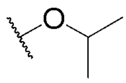 |
| 2.1-498 | Z-7 | 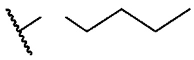 |
| 2.1-499 | Z-7 | 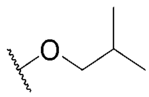 |
| 2.1-500 | Z-7 | 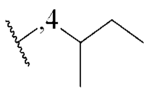 |
| 2.1-501 | Z-7 | 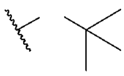 |
| 2.1-502 | Z-7 | 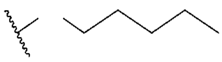 |
| 2.1-503 | Z-7 | 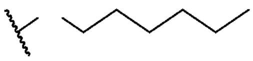 |

(continued)

| Compound NO. | Z | G |
|---|---|---|
| 2.1-504 | Z-7 | 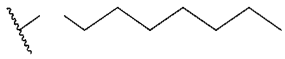 |
| 2.1-505 | Z-7 | 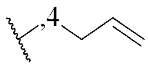 |
| 2.1-506 | Z-7 | 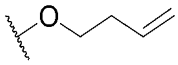 |
| 2.1-507 | Z-7 | 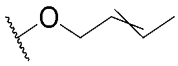 |
| 2.1-508 | Z-7 | 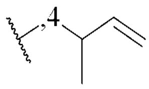 |
| 2.1-509 | Z-7 | 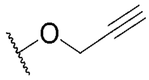 |
| 2.1-510 | Z-7 | 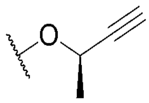 |
| 2.1-511 | Z-7 | 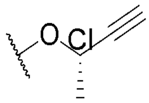 |
| 2.1-512 | Z-7 | 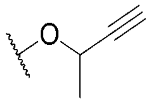 |
| 2.1-513 | Z-7 | 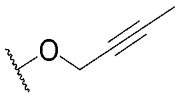 |
| 2.1-514 | Z-7 | 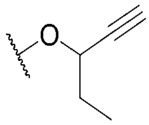 |
| 2.1-515 | Z-7 | 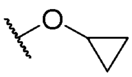 |
| 2.1-516 | Z-7 | 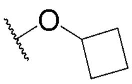 |
| 2.1-517 | Z-7 | 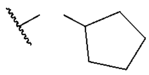 |
| 2.1-518 | Z-7 | 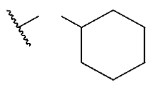 |
| 2.1-519 | Z-7 | 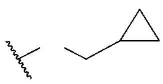 |

(continued)

| Compound NO. | Z | G |
|---|---|---|
| 2.1-520 | Z-7 | 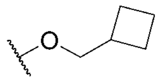 |
| 2.1-521 | Z-7 | 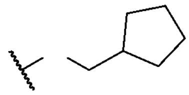 |
| 2.1-522 | Z-7 | 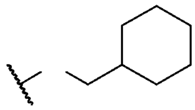 |
| 2.1-523 | Z-7 | 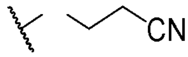 |
| 2.1-524 | Z-7 | 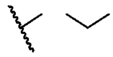 |
| 2.1-525 | Z-7 | 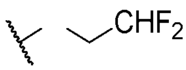 |
| 2.1-526 | Z-7 | 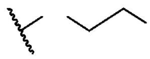 |
| 2.1-527 | Z-7 | 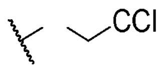 |
| 2.1-528 | Z-7 | 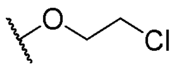 |
| 2.1-529 | Z-7 | 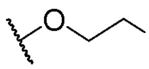 |
| 2.1-530 | Z-7 | 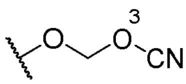 |
| 2.1-531 | Z-7 | 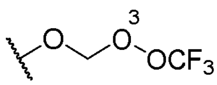 |
| 2.1-532 | Z-7 | 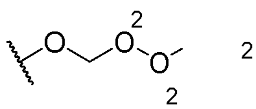 |
| 2.1-533 | Z-7 | 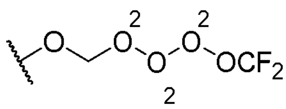 |
| 2.1-534 | Z-7 | 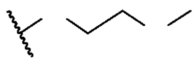 |
| 2.1-535 | Z-7 | 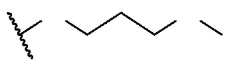 |
| 2.1-536 | Z-7 | 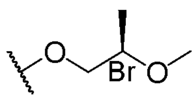 |

(continued)

| Compound NO. | Z | G |
|---|---|---|
| 2.1-537 | Z-7 | 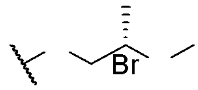 |
| 2.1-538 | Z-7 | 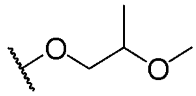 |
| 2.1-539 | Z-7 | 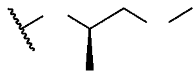 |
| 2.1-540 | Z-7 | 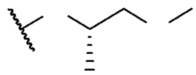 |
| 2.1-541 | Z-7 | 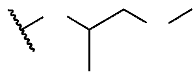 |
| 2.1-542 | Z-7 | 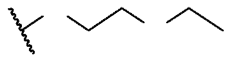 |
| 2.1-543 | Z-7 | 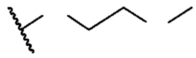 |
| 2.1-544 | Z-7 | 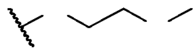 |
| 2.1-545 | Z-7 | 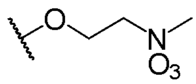 |
| 2.1-546 | Z-7 | 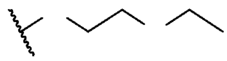 |
| 2.1-547 | Z-7 | 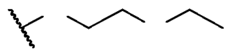 |
| 2.1-548 | Z-7 | 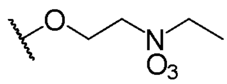 |
| 2.1-549 | Z-7 | 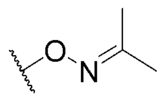 |
| 2.1-550 | Z-7 | 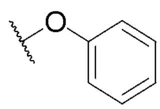 |
| 2.1-551 | Z-7 | 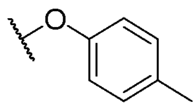 |
| 2.1-552 | Z-7 | 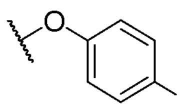 |
| 2.1-553 | Z-7 | 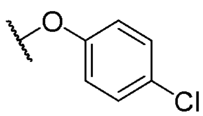 |

(continued)

| Compound NO. | Z | G |
|---|---|---|
| 2.1-554 | Z-7 | 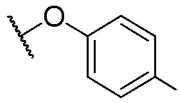 |
| 2.1-555 | Z-7 | 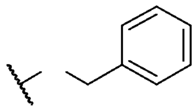 |
| 2.1-556 | Z-7 | 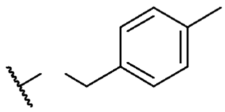 |
| 2.1-557 | Z-7 | 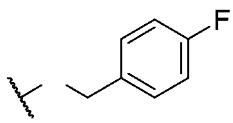 |
| 2.1-558 | Z-7 | 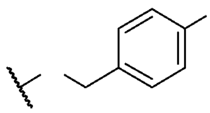 |
| 2.1-559 | Z-7 | 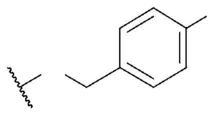 |
| 2.1-560 | Z-7 | 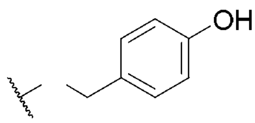 |
| 2.1-561 | Z-7 | 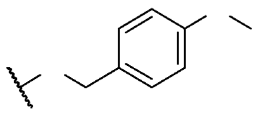 |
| 2.1-562 | Z-7 | 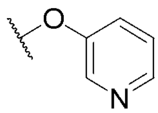 |
| 2.1-563 | Z-7 | 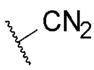 |
| 2.1-564 | Z-7 | 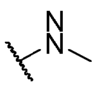 |
| 2.1-565 | Z-7 | 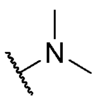 |
| 2.1-566 | Z-7 | 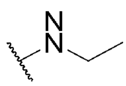 |
| 2.1-567 | Z-7 | 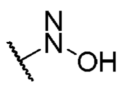 |

(continued)

| Compound NO. | Z | G |
|---|---|---|
| 2.1-568 | Z-7 | 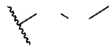 |
| 2.1-569 | Z-7 | 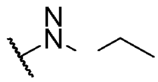 |
| 2.1-570 | Z-7 | 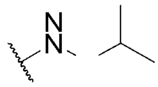 |
| 2.1-571 | Z-7 | 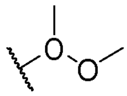 |
| 2.1-572 | Z-7 | 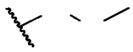 |
| 2.1-573 | Z-7 | 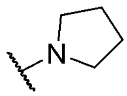 |
| 2.1-574 | Z-7 | 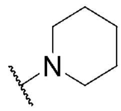 |

Table 2.2: Compounds **2.2-1** to **2.2-574** of the general formula (1.2) of the present invention, with Z and G correspondingly defined in Table 2.1.

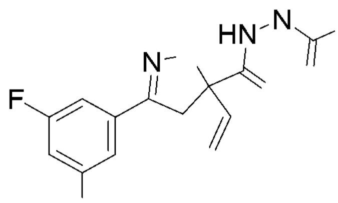

Table 2.3: Compounds **2.3-1** to **2.3-574** of the general formula (1.3) of the present invention, with Z and G correspondingly defined in Table 2.1.

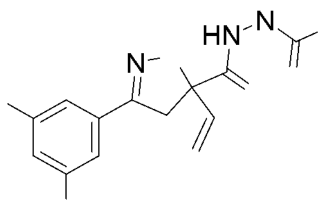

Table 2.4: Compounds **2.4-1** to **2.4-574** of the general formula (I.4) of the present invention, with Z and G correspondingly defined in Table 2.1.

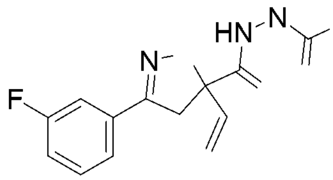

Table 2.5: Compounds **2.5-1** to **2.5-574** of the general formula (1.5) of the present invention, with Z and G correspondingly defined in Table 2.1.

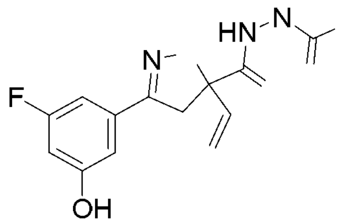

Table 2.6: Compounds **2.6-1** to **2.6-574** of the general formula (1.6) of the present invention, with Z and G correspondingly defined in Table 2.1.

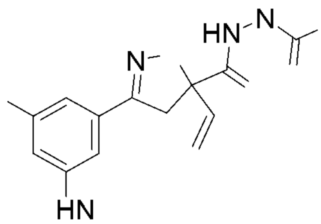

Table 2.7: Compounds **2.7-1** to **2.7-574** of the general formula (1.7) of the present invention, with Z and G correspondingly defined in Table 2.1.

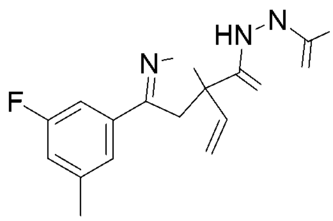

Table 2.8: Compounds **2.8-1** to **2.8-574** of the general formula (1.8) of the present invention, with Z and G correspondingly defined in Table 2.1.

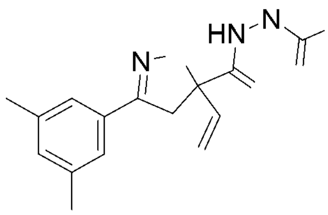

Table 2.9: Compounds **2.9-1** to **2.9-574** of the general formula (1.9) of the present invention, with Z and G correspondingly defined in Table 2.1.

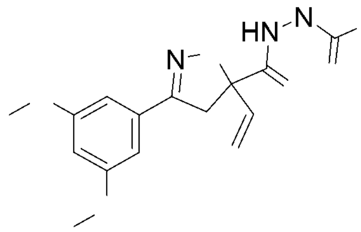

.

Table 2.10: Compounds **2.10-1** to **2.10-574** of the general formula (I.10) of the present invention, with Z and G correspondingly defined in Table 2.1.

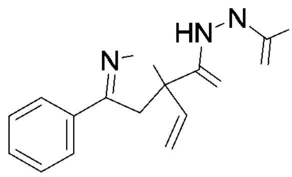

.

Table 2.11: Compounds **2.11-1** to **2.11-574** of the general formula (I.11) of the present invention, with Z and G correspondingly defined in Table 2.1.

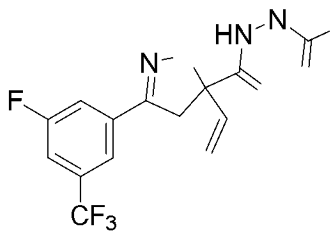

Table 2.12: Compounds **2.12-1** to **2.12-574** of the general formula (1.12) of the present invention, with Z and G correspondingly defined in Table 2.1.

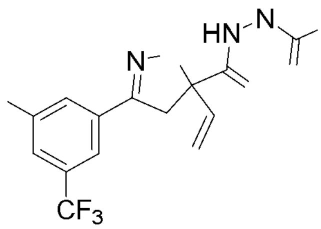

.

Table 2.13: Compounds **2.13-1** to **2.13-574** of the general formula (1.13) of the present invention, with Z and G correspondingly defined in Table 2.1.

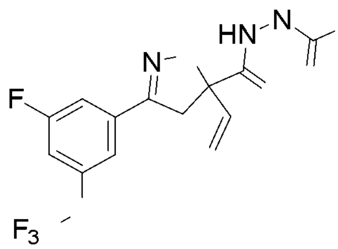

.

Table 2.14: Compounds **2.14-1** to **2.14-574** of the general formula (1.14) of the present invention, with Z and G correspondingly defined in Table 2.1.

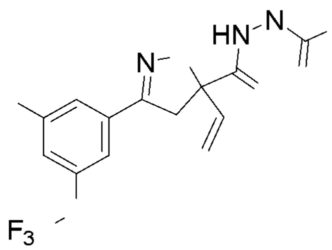

.

Table 2.15: Compounds **2.15-1** to **2.15-574** of the general formula (1.15) of the present invention, with Z and G correspondingly defined in Table 2.1.

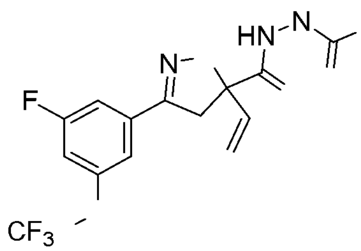

.

Table 2.16: Compounds **2.16-1** to **2.16-574** of the general formula (1.16) of the present invention, with Z and G correspondingly defined in Table 2.1.

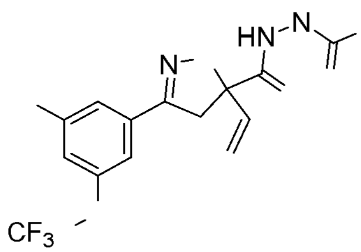

.

Table 2.17: Compounds **2.17-1** to **2.17-574** of the general formula (1.17) of the present invention, with Z and G correspondingly defined in Table 2.1.

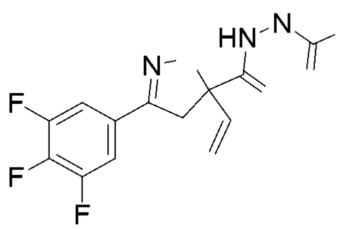

.

Table 2.18: Compounds **2.18-1** to **2.18-574** of the general formula (1.18) of the present invention, with Z and G correspondingly defined in Table 2.1.

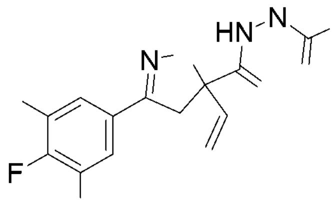

Table 2.19: Compounds **2.19-1** to **2.19-574** of the general formula (1.19) of the present invention, with Z and G correspondingly defined in Table 2.1.

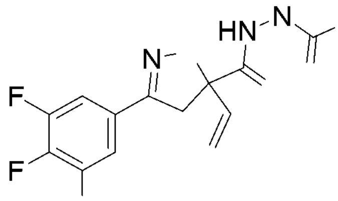

.

Similarly, the structures of general formulas (I.20) to (1.179) were shown in Table 3. In the specific compounds corresponding to each general formula, Z and G were defined in Table 2.1 (corresponding to their numbers "-1" to "-574").

Table 3

| General formula NO. | Structure of general formula | Compound NO. |
|---|---|---|
| I.20 | 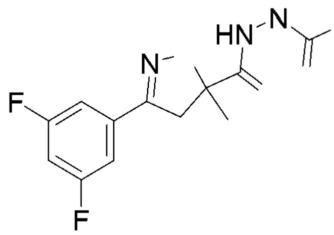 | 2.20-1 to 2.20-574 |
| I.21 | 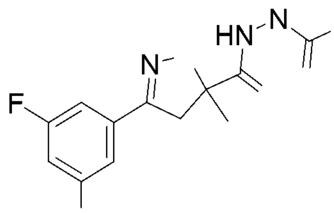 | 2.21-1 to 2.21-574 |
| I.22 | 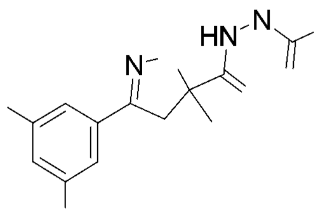 | 2.22-1 to 2.22-574 |
| I.23 | 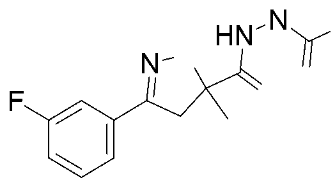 | 2.23-1 to 2.23-574 |

(continued)

| General formula NO. | Structure of general formula | Compound NO. |
|---|---|---|
| I.24 | 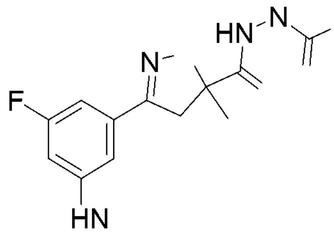 | 2.24-1 to 2.24-574 |
| I.25 | 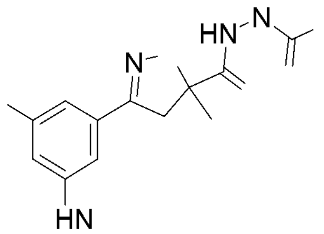 | 2.25-1 to 2.25-574 |
| I.26 | 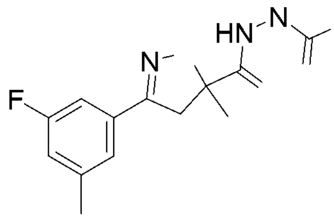 | 2.26-1 to 2.26-574 |
| I.27 | 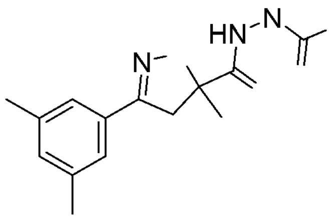 | 2.27-1 to 2.27-574 |
| I.28 | 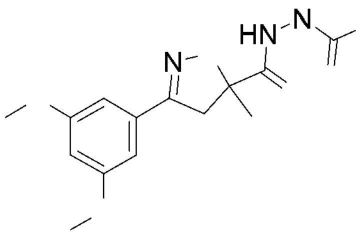 | 2.28-1 to 2.28-574 |
| I.29 | 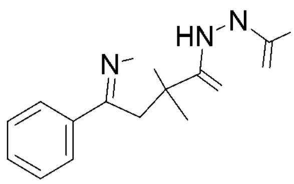 | 2.29-1 to 2.29-574 |
| I.30 | 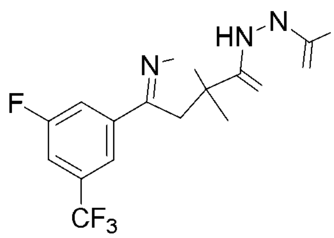 | 2.30-1 to 2.30-574 |

(continued)

| General formula NO. | Structure of general formula | Compound NO. |
|---|---|---|
| I.31 | 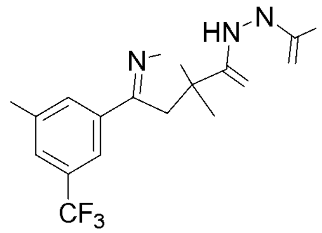 | 2.31-1 to 2.31-574 |
| I.32 | 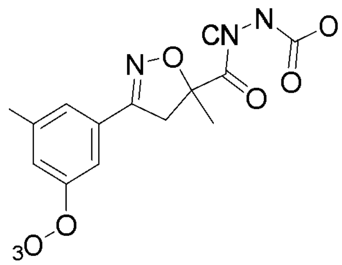 | 2.32-1 to 2.32-574 |
| I.33 | 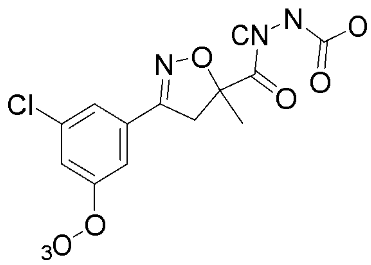 | 2.33-1 to 2.33-574 |
| I.34 | 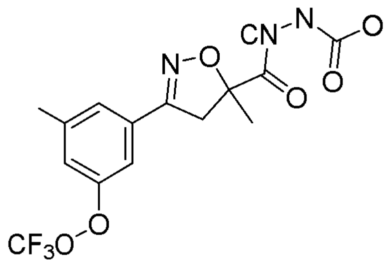 | 2.34-1 to 2.34-574 |
| I.35 | 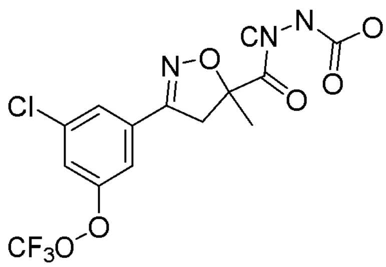 | 2.35-1 to 2.35-574 |
| I.36 | 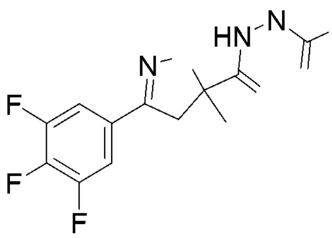 | 2.36-1 to 2.36-574 |

(continued)

| General formula NO. | Structure of general formula | Compound NO. |
|---|---|---|
| I.37 | 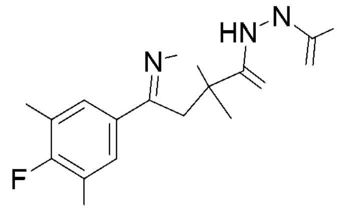 | 2.37-1 to 2.37-574 |
| I.38 | 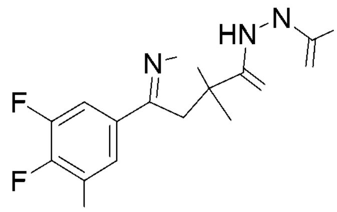 | 2.38-1 to 2.38-574 |
| I.39 | 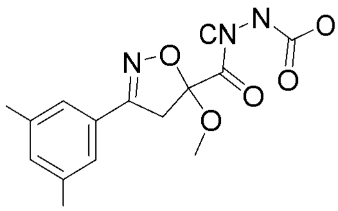 | 2.39-1 to 2.39-574 |
| I.40 | 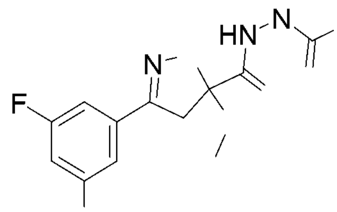 | 2.40-1 to 2.40-574 |
| I.41 | 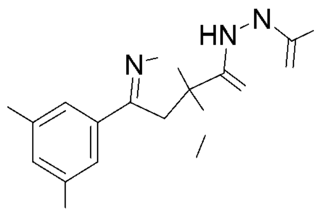 | 2.41-1 to 2.41-574 |
| I.42 | 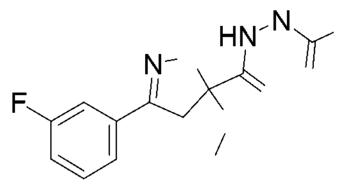 | 2.42-1 to 2.42-574 |
| I.43 | 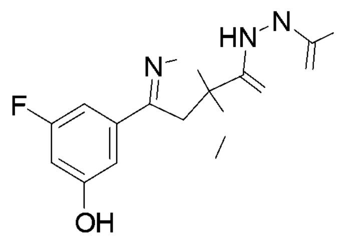 | 2.43-1 to 2.43-574 |

(continued)

| General formula NO. | Structure of general formula | Compound NO. |
|---|---|---|
| I.44 | 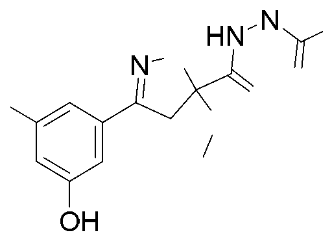 | 2.44-1 to 2.44-574 |
| I.45 | 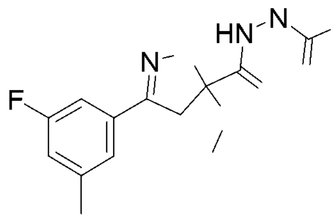 | 2.45-1 to 2.45-574 |
| I.46 | 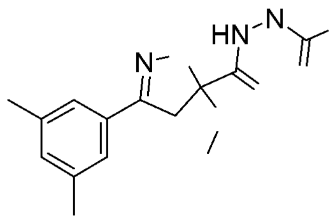 | 2.46-1 to 2.46-574 |
| I.47 | 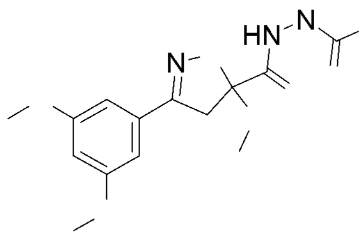 | 2.47-1 to 2.47-574 |
| I.48 | 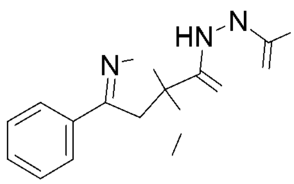 | 2.48-1 to 2.48-574 |
| I.49 | 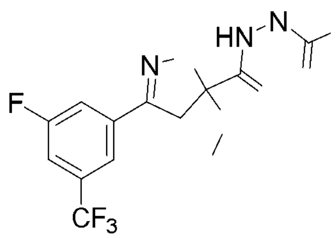 | 2.49-1 to 2.49-574 |
| I.50 | 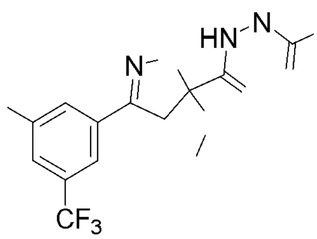 | 2.50-1 to 2.50-574 |

(continued)

| General formula NO. | Structure of general formula | Compound NO. |
|---|---|---|
| I.51 | 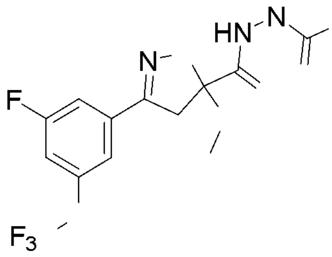 | 2.51-1 to 2.51-574 |
| I.52 | 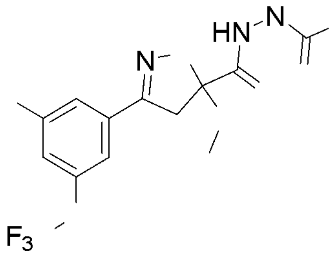 | 2.52-1 to 2.52-574 |
| I.53 | 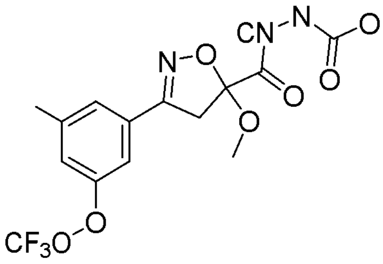 | 2.53-1 to 2.53-574 |
| I.54 | 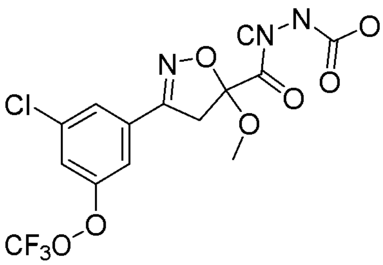 | 2.54-1 to 2.54-574 |
| I.55 | 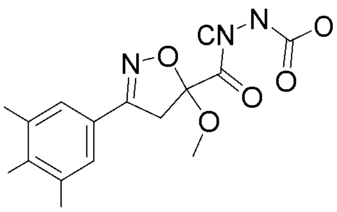 | 2.55-1 to 2.55-574 |
| I.56 | 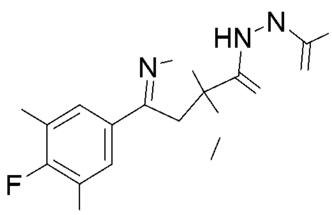 | 2.56-1 to 2.56-574 |

(continued)

| General formula NO. | Structure of general formula | Compound NO. |
|---|---|---|
| I.57 | 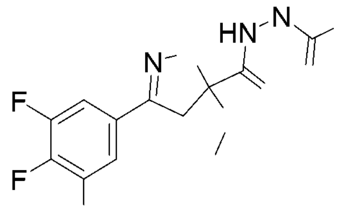 | 2.57-1 to 2.57-574 |
| I.58 | 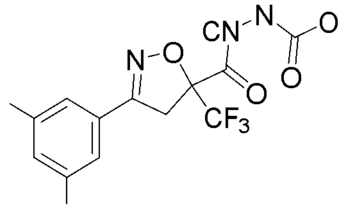 | 2.58-1 to 2.58-574 |
| I.59 | 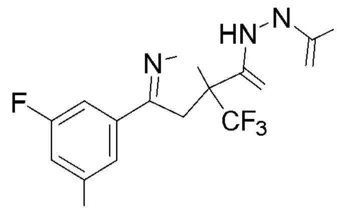 | 2.59-1 to 2.59-574 |
| I.60 | 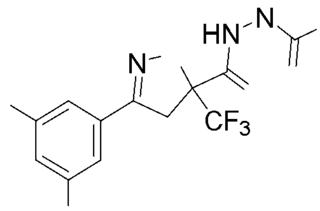 | 2.60-1 to 2.60-574 |
| I.61 | 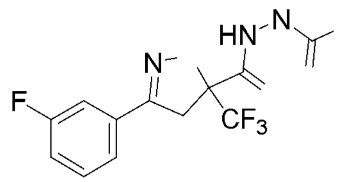 | 2.61-1 to 2.61-574 |
| I.62 | 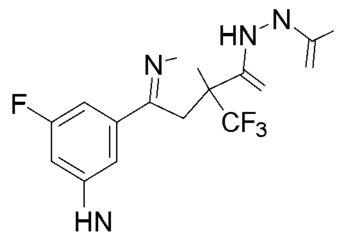 | 2.62-1 to 2.62-574 |
| I.63 | 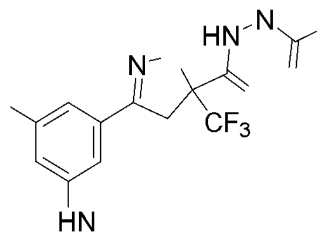 | 2.63-1 to 2.63-574 |

(continued)

| General formula NO. | Structure of general formula | Compound NO. |
|---|---|---|
| I.64 | 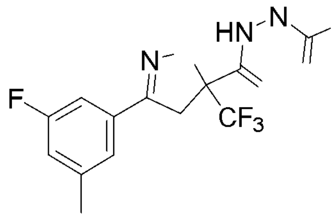 | 2.64-1 to 2.64-574 |
| I.65 | 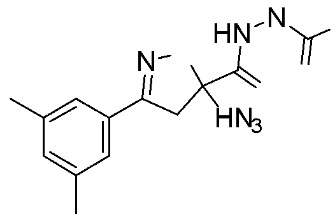 | 2.65-1 to 2.65-574 |
| I.66 | 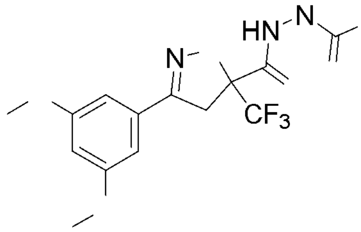 | 2.66-1 to 2.66-574 |
| I.67 | 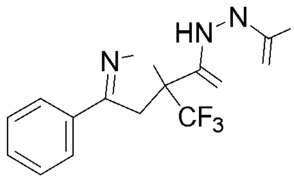 | 2.67-1 to 2.67-574 |
| I.68 | 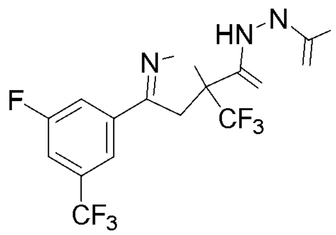 | 2.68-1 to 2.68-574 |
| I.69 | 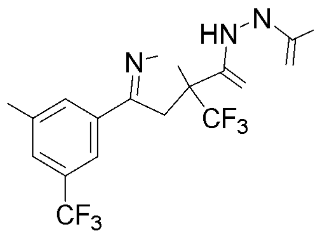 | 2.69-1 to 2.69-574 |
| I.70 | 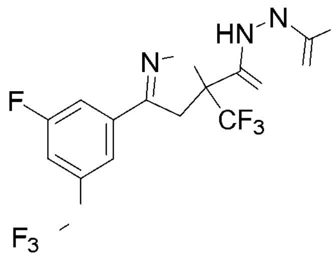 | 2.70-1 to 2.70-574 |

(continued)

| General formula NO. | Structure of general formula | Compound NO. |
|---|---|---|
| I.71 | 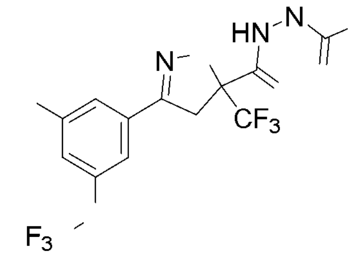 | 2.71-1 to 2.71-574 |
| I.72 | 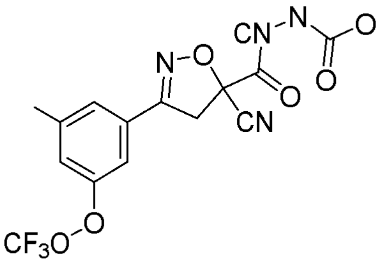 | 2.72-1 to 2.72-574 |
| I.73 | 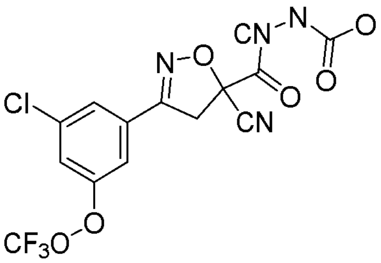 | 2.73-1 to 2.73-574 |
| I.74 | 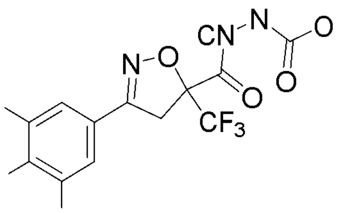 | 2.74-1 to 2.74-574 |
| I.75 | 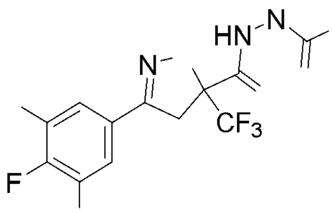 | 2.75-1 to 2.75-574 |
| I.76 | 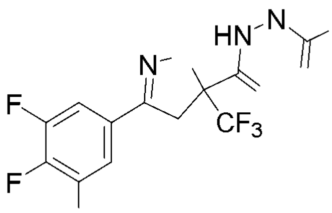 | 2.76-1 to 2.76-574 |

(continued)

| General formula NO. | Structure of general formula | Compound NO. |
|---|---|---|
| I.77 | 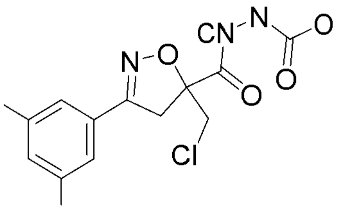 | 2.77-1 to 2.77-574 |
| I.78 | 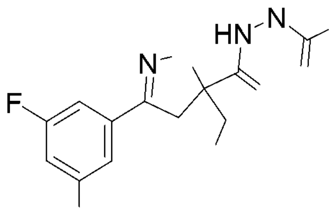 | 2.78-1 to 2.78-574 |
| I.79 | 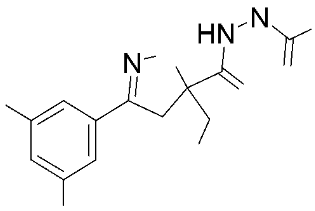 | 2.79-1 to 2.79-574 |
| I.80 | 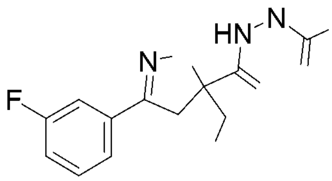 | 2.80-1 to 2.80-574 |
| I.81 | 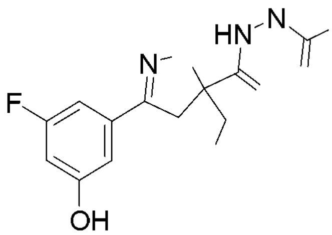 | 2.81-1 to 2.81-574 |
| I.82 | 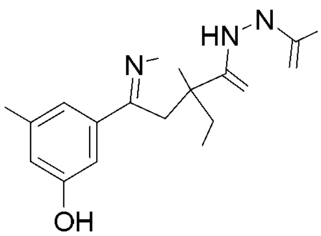 | 2.82-1 to 2.82-574 |
| I.83 | 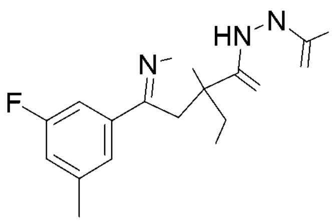 | 2.83-1 to 2.83-574 |

(continued)

| General formula NO. | Structure of general formula | Compound NO. |
|---|---|---|
| I.84 | 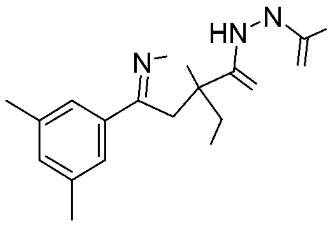 | 2.84-1 to 2.84-574 |
| I.85 | 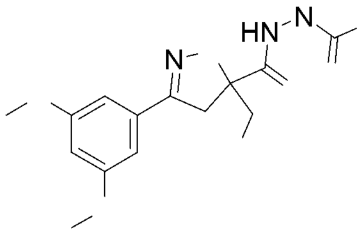 | 2.85-1 to 2.85-574 |
| I.86 | 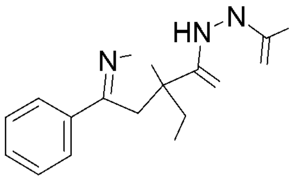 | 2.86-1 to 2.86-574 |
| I.87 | 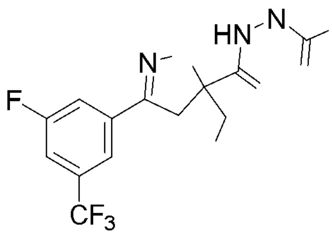 | 2.87-1 to 2.87-574 |
| I.88 | 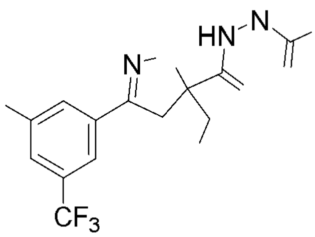 | 2.88-1 to 2.88-574 |
| I.89 | 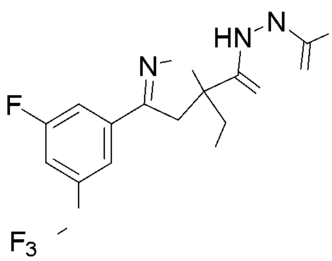 | 2.89-1 to 2.89-574 |

(continued)

| General formula NO. | Structure of general formula | Compound NO. |
|---|---|---|
| I.90 | 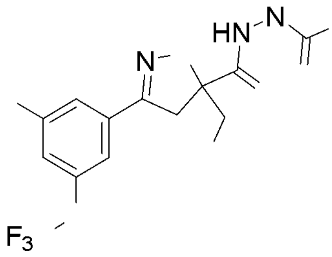 | 2.90-1 to 2.90-574 |
| I.91 | 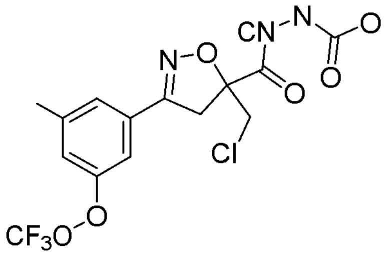 | 2.91-1 to 2.91-574 |
| I.92 | 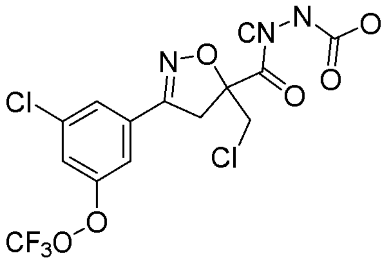 | 2.92-1 to 2.92-574 |
| I.93 | 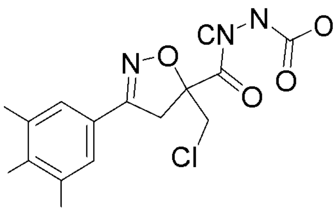 | 2.93-1 to 2.93-574 |
| I.94 | 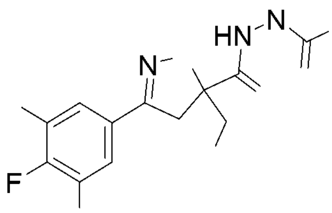 | 2.94-1 to 2.94-574 |
| I.95 | 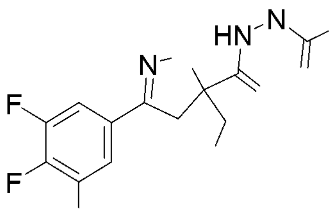 | 2.95-1 to 2.95-574 |

(continued)

| General formula NO. | Structure of general formula | Compound NO. |
|---|---|---|
| I.96 | 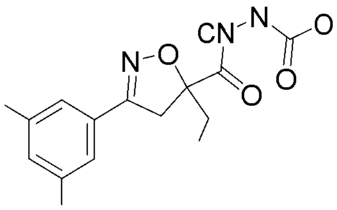 | 2.96-1 to 2.96-574 |
| I.97 | 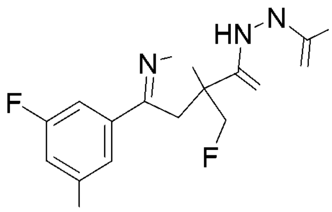 | 2.97-1 to 2.97-574 |
| I.98 | 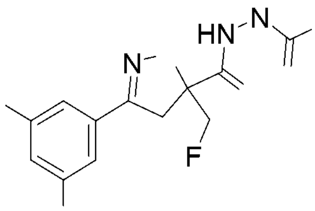 | 2.98-1 to 2.98-574 |
| I.99 | 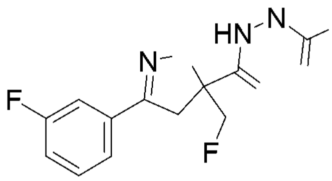 | 2.99-1 to 2.99-574 |
| I.100 | 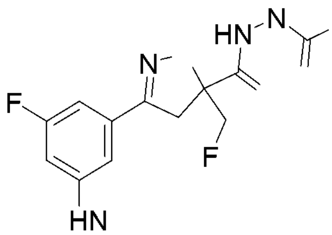 | 2.100-1 to 2.100-574 |
| I.101 | 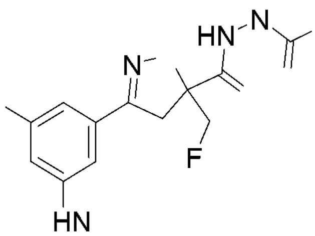 | 2.101-1 to 2.101-574 |
| I.102 | 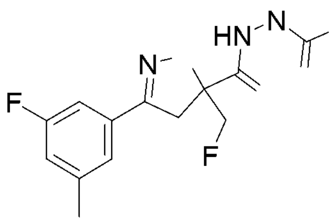 | 2.102-1 to 2.102-574 |

(continued)

| General formula NO. | Structure of general formula | Compound NO. |
|---|---|---|
| I.103 | 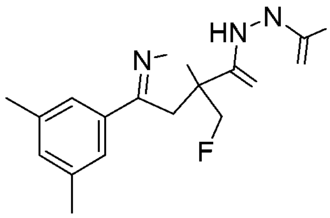 | 2.103-1 to 2.103-574 |
| I.104 | 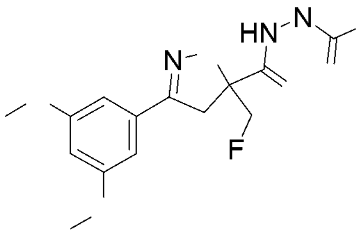 | 2.104-1 to 2.104-574 |
| I.105 | 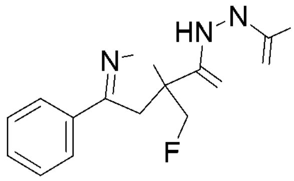 | 2.105-1 to 2.105-574 |
| I.106 | 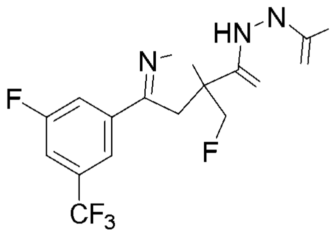 | 2.106-1 to 2.106-574 |
| I.107 | 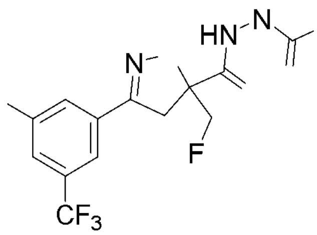 | 2.107-1 to 2.107-574 |
| I.108 | 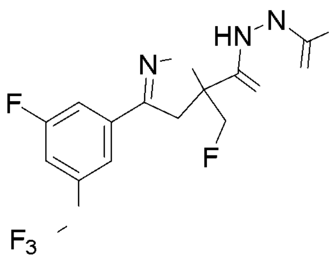 | 2.108-1 to 2.108-574 |

(continued)

| General formula NO. | Structure of general formula | Compound NO. |
|---|---|---|
| I.109 | 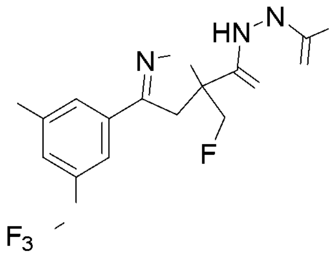 | 2.109-1 to 2.109-574 |
| I.110 | 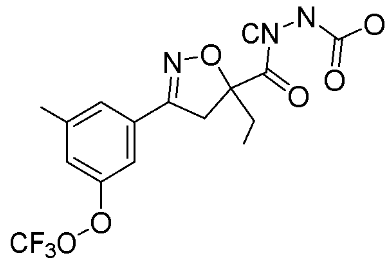 | 2.110-1 to 2.110-574 |
| I.111 | 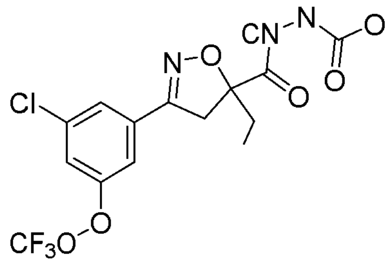 | 2.111-1 to 2.111-574 |
| I.112 | 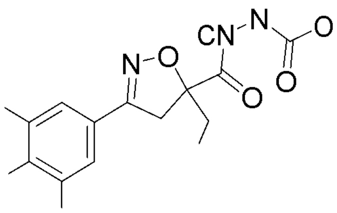 | 2.112-1 to 2.112-574 |
| I.113 | 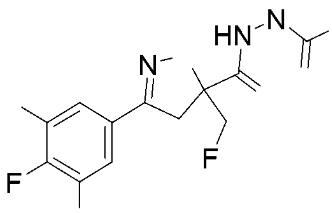 | 2.113-1 to 2.113-574 |
| I.114 | 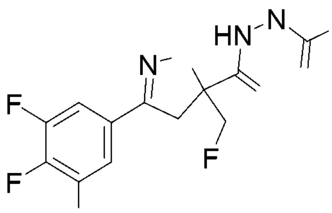 | 2.114-1 to 2.114-574 |

(continued)

| General formula NO. | Structure of general formula | Compound NO. |
|---|---|---|
| I.115 | 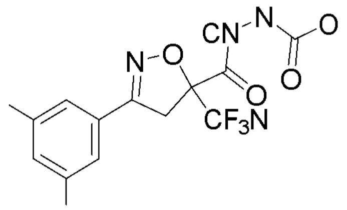 | 2.115-1 to 2.115-574 |
| I.116 | 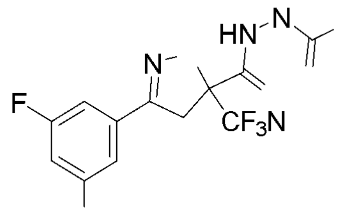 | 2.116-1 to 2.116-574 |
| I.117 | 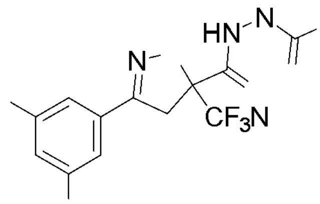 | 2.117-1 to 2.117-574 |
| I.118 | 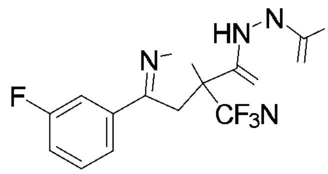 | 2.118-1 to 2.118-574 |
| I.119 | 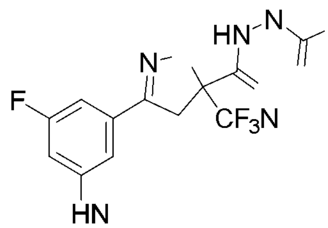 | 2.119-1 to 2.119-574 |
| I.120 | 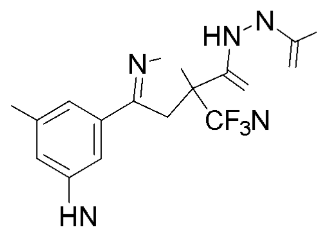 | 2.120-1 to 2.120-574 |
| I.121 | 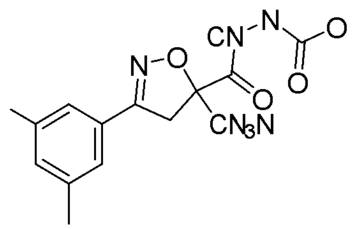 | 2.121-1 to 2.121-574 |

(continued)

| General formula NO. | Structure of general formula | Compound NO. |
|---|---|---|
| I.122 | 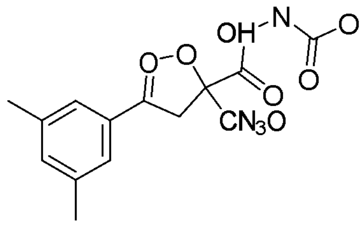 | 2.122-1 to 2.122-574 |
| I.123 | 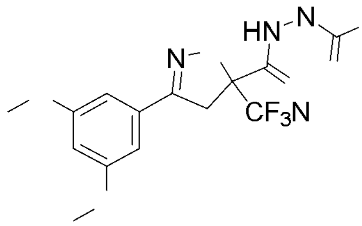 | 2.123-1 to 2.123-574 |
| I.124 | 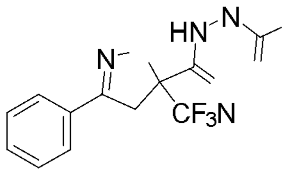 | 2.124-1 to 2.124-574 |
| I.125 | 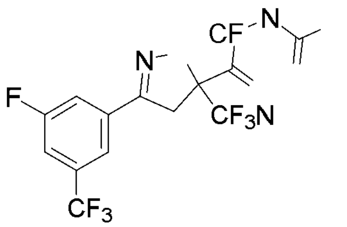 | 2.125-1 to 2.125-574 |
| I.126 | 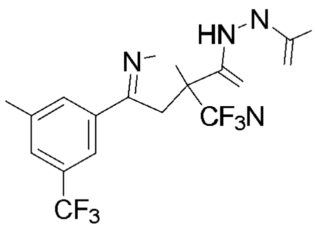 | 2.126-1 to 2.126-574 |
| I.127 | 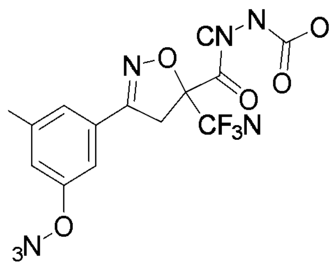 | 2.127-1 to 2.127-574 |
| I.128 | 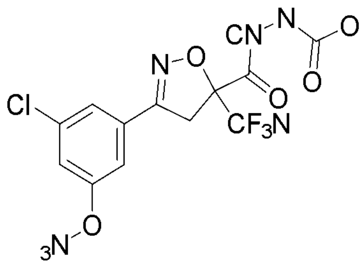 | 2.128-1 to 2.128-574 |

(continued)

| General formula NO. | Structure of general formula | Compound NO. |
|---|---|---|
| I.129 | 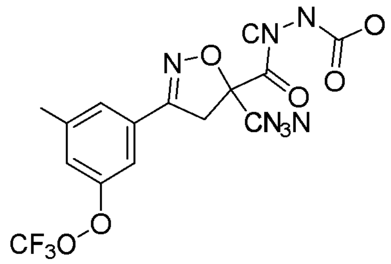 | 2.129-1 to 2.129-574 |
| I.130 | 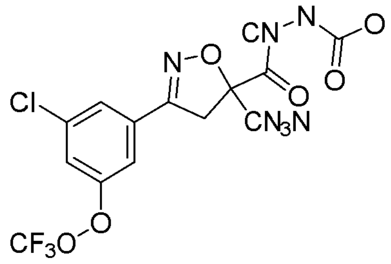 | 2.130-1 to 2.130-574 |
| I.131 | 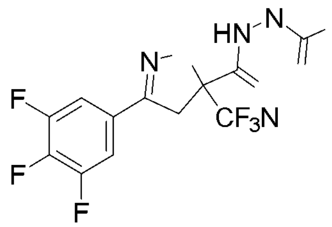 | 2.131-1 to 2.131-574 |
| I.132 | 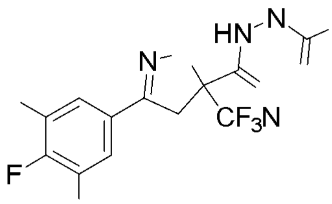 | 2.132-1 to 2.132-574 |
| I.133 | 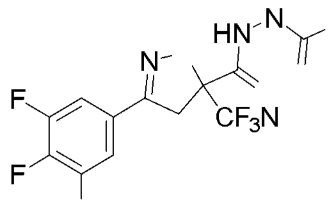 | 2.133-1 to 2.133-574 |
| I.134 | 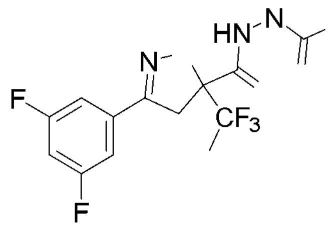 | 2.134-1 to 2.134-574 |
| I.135 | 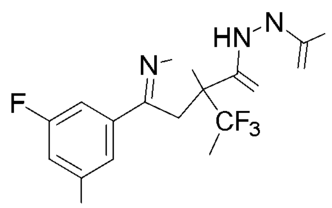 | 2.135-1 to 2.135-574 |

(continued)

| General formula NO. | Structure of general formula | Compound NO. |
|---|---|---|
| I.136 | 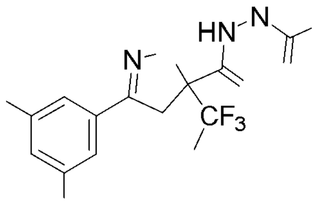 | 2.136-1 to 2.136-574 |
| I.137 | 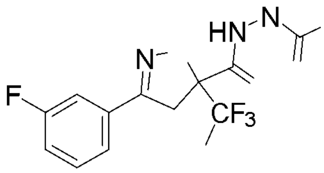 | 2.137-1 to 2.137-574 |
| I.138 | 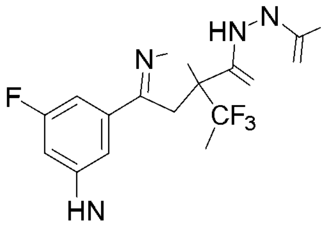 | 2.138-1 to 2.138-574 |
| I.139 | 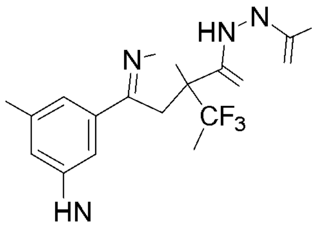 | 2.139-1 to 2.139-574 |
| I.140 | 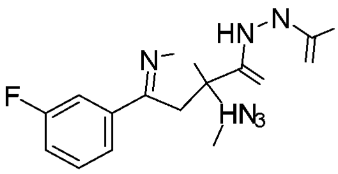 | 2.140-1 to 2.140-574 |
| I.141 | 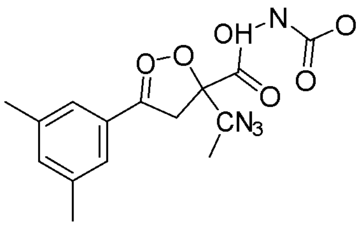 | 2.141-1 to 2.141-574 |
| I.142 | 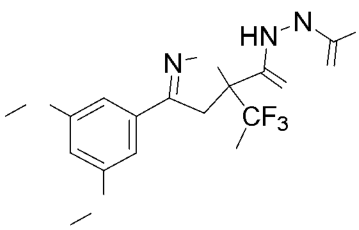 | 2.142-1 to 2.142-574 |

(continued)

| General formula NO. | Structure of general formula | Compound NO. |
|---|---|---|
| I.143 | 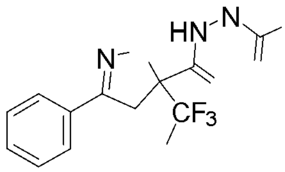 | 2.143-1 to 2.143-574 |
| I.144 | 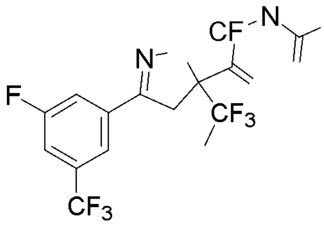 | 2.144-1 to 2.144-574 |
| I.145 | 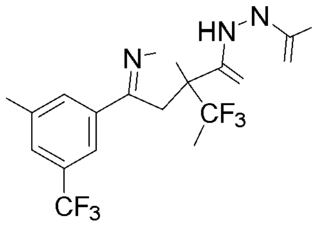 | 2.145-1 to 2.145-574 |
| I.146 | 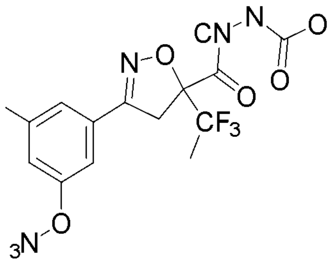 | 2.146-1 to 2.146-574 |
| I.147 | 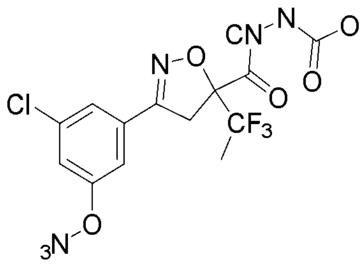 | 2.147-1 to 2.147-574 |
| I.148 | 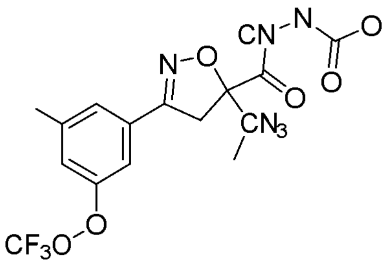 | 2.148-1 to 2.148-574 |

(continued)

| General formula NO. | Structure of general formula | Compound NO. |
|---|---|---|
| I.149 | 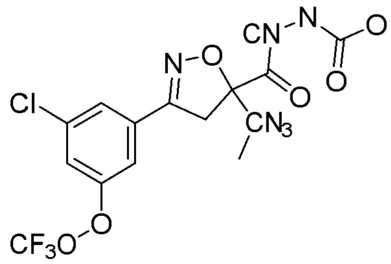 | 2.149-1 to 2.149-574 |
| I.150 | 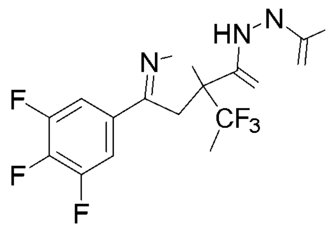 | 2.150-1 to 2.150-574 |
| I.151 | 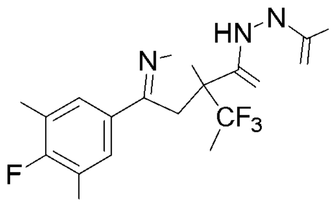 | 2.151-1 to 2.151-574 |
| I.152 | 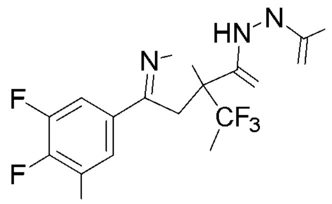 | 2.152-1 to 2.152-574 |
| I.153 | 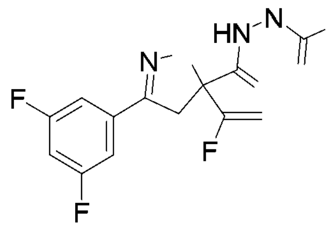 | 2.153-1 to 2.153-574 |
| I.154 | 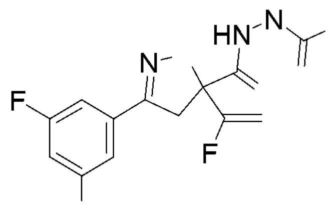 | 2.154-1 to 2.154-574 |
| I.155 | 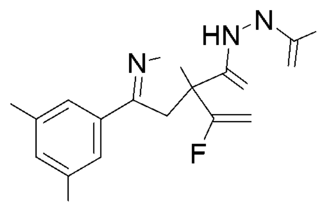 | 2.155-1 to 2.155-574 |

(continued)

| General formula NO. | Structure of general formula | Compound NO. |
|---|---|---|
| I.156 | 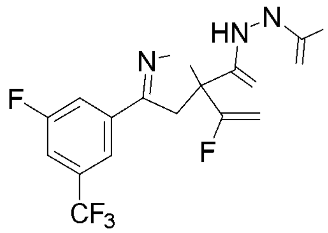 | 2.156-1 to 2.156-574 |
| I.157 | 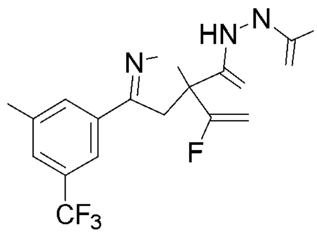 | 2.157-1 to 2.157-574 |
| I.158 | 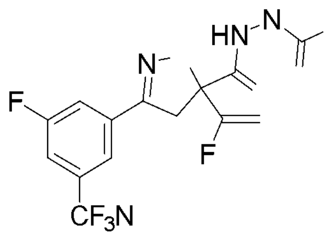 | 2.158-1 to 2.158-574 |
| I.159 | 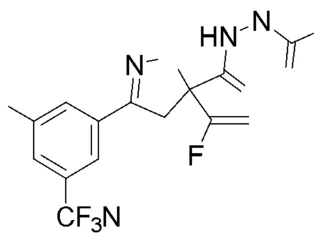 | 2.159-1 to 2.159-574 |
| I.160 | 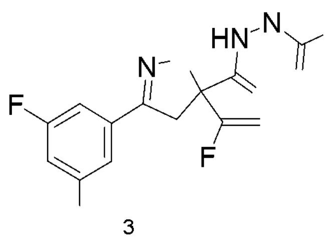 | 2.160-1 to 2.160-574 |
| I.161 | 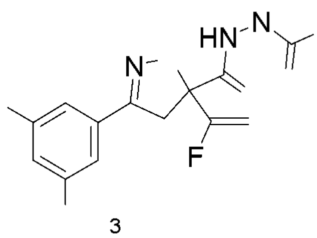 | 2.161-1 to 2.161-574 |

(continued)

| General formula NO. | Structure of general formula | Compound NO. |
| --- | --- | --- |
| I.162 | 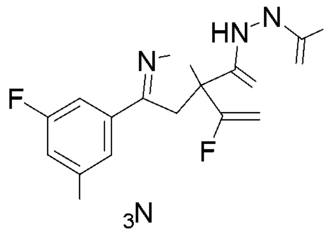 | 2.162-1 to 2.162-574 |
| I.163 | 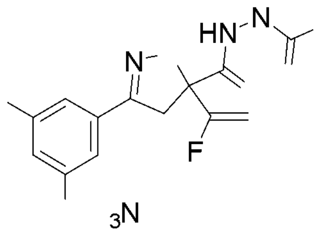 | 2.163-1 to 2.163-574 |
| I.164 | 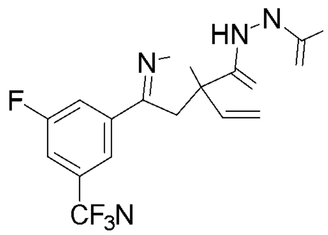 | 2.164-1 to 2.164-574 |
| I.165 | 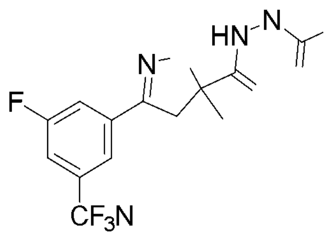 | 2.165-1 to 2.165-574 |
| I.166 | 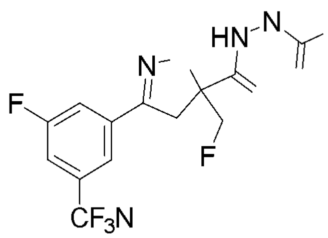 | 2.166-1 to 2.166-574 |
| I.167 | 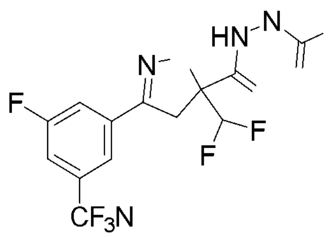 | 2.167-1 to 2.167-574 |

(continued)

| General formula NO. | Structure of general formula | Compound NO. |
|---|---|---|
| I.168 | 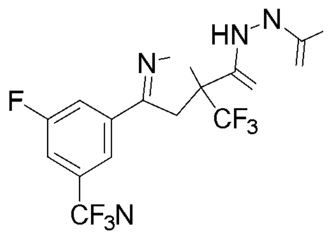 | 2.168-1 to 2.168-574 |
| I.169 | 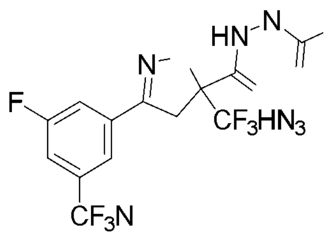 | 2.169-1 to 2.169-574 |
| I.170 | 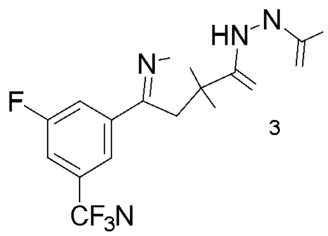 | 2.170-1 to 2.170-574 |
| I.171 | 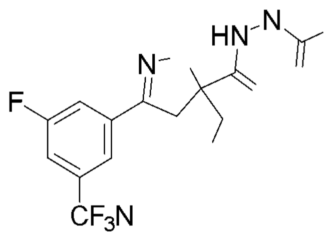 | 2.171-1 to 2.171-574 |
| I.172 | 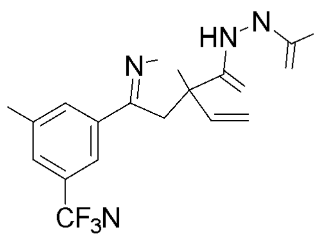 | 2.172-1 to 2.172-574 |
| I.173 | 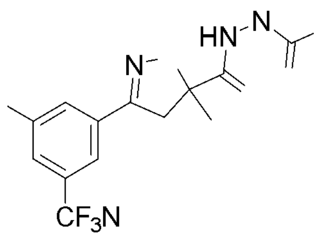 | 2.173-1 to 2.173-574 |

(continued)

| General formula NO. | Structure of general formula | Compound NO. |
| --- | --- | --- |
| I.174 | 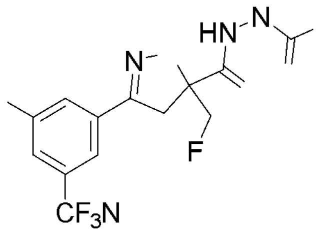 | 2.174-1 to 2.174-574 |
| I.175 | 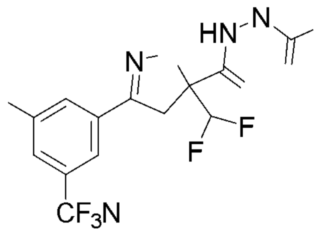 | 2.175-1 to 2.175-574 |
| I.176 | 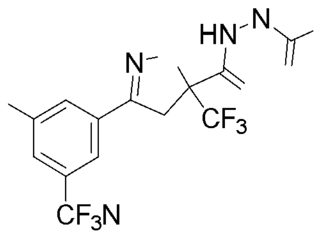 | 2.176-1 to 2.176-574 |
| I.177 | 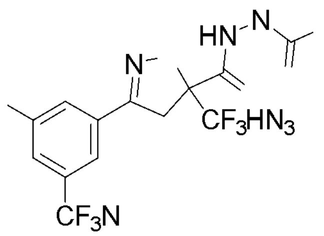 | 2.177-1 to 2.177-574 |
| I.178 | 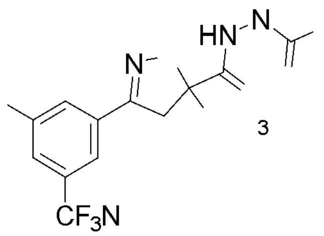 | 2.178-1 to 2.178-574 |
| I.179 | 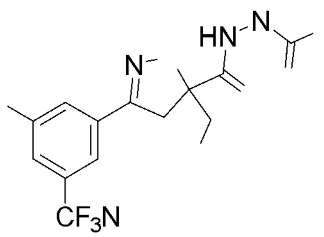 | 2.179-1 to 2.179-574 |

By using preparation examples similar to those provided above or referring to the methods described in WO2018228985A1, WO2019145245A1 and WO2019034602A1, the following control compounds can be prepared.

| NO. | Structure | $^1$H NMR | Analytical separation method | ee% | [α] |
|---|---|---|---|---|---|
| icafo lin | 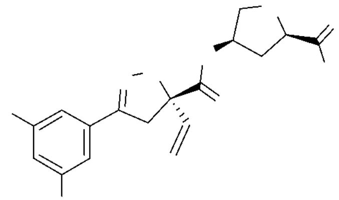 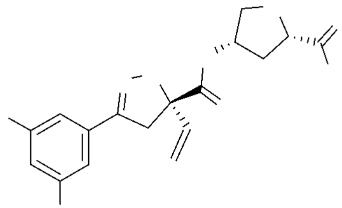 | $^1$H NMR (400 MHz, DMSO) $\delta$ 12.75 (s, 1H), 8.36 - 8.05 (m, 1H), 7.46 - 7.36 (m, 3H), 6.19 - 6.07 (m, 1H), 5.38 (d, J = 17.2 Hz, 1H), 5.31 (d, J = 10.7 Hz, 1H), 4.43 - 4.24 (m, 2H), 3.93 - 3.79 (m, 2H), 3.69 - 3.62 (m, 1H), 3.56 (d, J = 17.8 Hz, 1H), 2.49 - 2.40 (m, 1H), 2.05 - 1.94 (m, 1H). | Method C | Diastereo mer 1: 100.00<br>Diastereo mer 2: 100.00 | $[\alpha]_D^{20}=$ - 154.73 ° (c=0.1 18, in DMF) |
| icafo lin-meth yl | 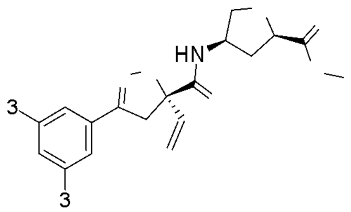 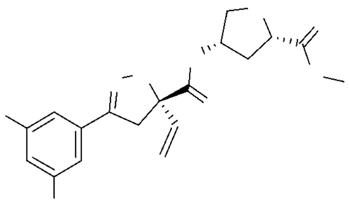 | $^1$H NMR (400 MHz, DMSO) $\delta$ 8.18 (t, $J$ = 7.2 Hz, 1H), 7.41 (d, $J$ = 7.6 Hz, 3H), 6.12 (ddd, $J$ = 17.1, 10.7, 3.8 Hz, 1H), 5.34 (dd, $J$ = 28.0, 13.9 Hz, 2H), 4.53 - 4.41 (m, 1H), 4.37 - 4.26 (m, 1H), 3.94 - 3.80 (m, 2H), 3.65 (d, $J$ = 12.7 Hz, 4H), 3.55 (d, $J$ = 17.8 Hz, 1H), 2.49 - 2.41 (m, 1H), 2.04 (ddd, $J$ = 7.7, 5.9, 3.0 Hz, 1H). | Method F | Diastereo mer 1: 100.00<br>Diastereo mer 2: 100.00 | $[\alpha]_D^{20}=$ - 144.47 ° (c=0.0 96, in DMF) |
| CK1 | 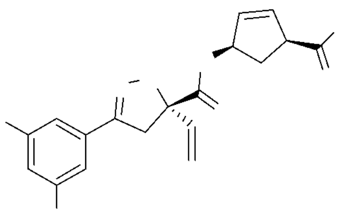 | $^1$H NMR (400 MHz, DMSO) $\delta$ 12.49 (s, 1H), 8.08 (d, $J$ = 7.8 Hz, 1H), 7.51 - 7.33 (m, 3H), 6.14 (dd, $J$ = 17.3, 10.7 Hz, 1H), 5.95 - 5.85 (m, 1H), 5.80 - 5.76 (m, 1H), 5.39 (d, $J$ = 17.3 Hz, 1H), 5.30 (d, $J$ = 10.7 Hz, 1H), 4.85 - 4.74 (m, 1H), 3.89 (d, $J$ = 17.7 Hz, 1H), 3.55 (d, $J$ = 17.8 Hz, 1H), 3.50 - 3.41 (m, 1H), 2.38 (dt, $J$ = 13.2, 8.3 Hz, 1H), 1.90 - 1.77 (m, 1H). | Method E | 100 | $[\alpha]_D^{20}=$ - 211.97 ° (c=0.1 01, in DMF) |

(continued)

| NO. | Structure | $^1$H NMR | Analytical separation method | ee% | [α] |
|---|---|---|---|---|---|
| CK2 | 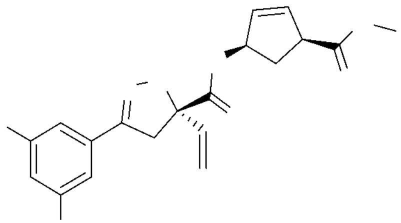 | $^1$H NMR (400 MHz, $CDCl_3$) $\delta$ 7.22 (dd, $J$ = 9.6, 5.6 Hz, 1H), 7.21 - 7.14 (m, 2H), 6.88 (tt, $J$ = 8.7, 2.3 Hz, 1H), 6.17 (dd, $J$ = 17.2, 10.7 Hz, 1H), 6.00 - 5.94 (m, 1H), 5.90 - 5.83 (m, 1H), 5.53 (d, $J$ = 17.2 Hz, 1H), 5.33 (d, $J$ = 17.2 Hz, 1H), 5.07 - 4.97 (m, 1H), 3.92 (d, $J$ = 17.2 Hz, 1H), 3.74 (s, 3H), 3.57 - 3.46 (m, 1H), 3.32 (d, $J$ = 17.2 Hz, 1H), 2.48 (dt, $J$ = 14.0, 8.4 Hz, 1H), 1.89 (dt, $J$ = 14.0, 3.8 Hz, 1H). | Method E | 96.75 | $[\alpha]_D^{20}$= - 198.44 ° (c=0.107, in DMF) |
| CK3 | 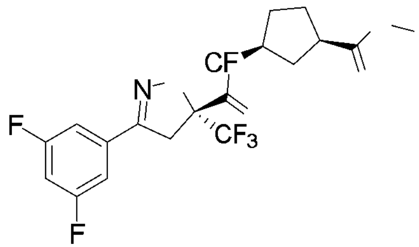 | $^1$H NMR (400 MHz, $CDCl_3$) $\delta$ 7.65 (d, J = 7.8 Hz, 1H), 7.24 - 7.16 (m, 2H), 6.99 - 6.89 (m, 1H), 4.43 - 4.31 (m, 1H), 3.98 (d, J = 18.1 Hz, 1H), 3.76 - 3.69 (m, 4H), 2.99 - 2.89 (m, 1H), 2.18 - 2.05 (m, 2H), 2.01 - 1.88 (m, 3H), 1.84 - 1.75 (m, 1H). | Method F | 99.08 | $[\alpha]_D^{20}$= - 162.55 ° (c=0.1 13, in DMF) |
| CK4 | 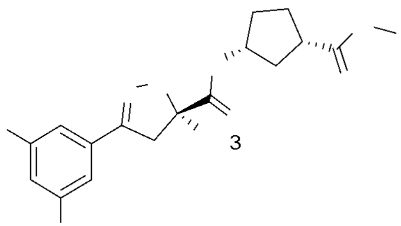 | $^1$H NMR (400 MHz, $CDCl_3$) $\delta$ 7.51 (d, J = 8.1 Hz, 1H), 7.24 - 7.15 (m, 2H), 6.98 - 6.89 (m, 1H), 4.45 - 4.35 (m, 1H), 3.97 (d, J = 18.0 Hz, 1H), 3.79 - 3.68 (m, 4H), 3.00 - 2.87 (m, 1H), 2.30 - 2.20 (m, 1H), 2.06 - 1.93 (m, 3H), 1.91 - 1.80 (m, 1H), 1.75 - 1.64 (m, 1H). | Method F | 98.57 | $[\alpha]_D^{20}$= - 163.43 ° (c=0.1 00, in DMF) |
| CK5 | 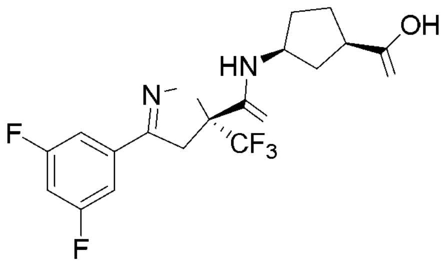 | $^1$H NMR (400 MHz, DMSO) $\delta$ 12.19 (s, 1H), 8.64 (d, J = 7.7 Hz, 1H), 7.68 - 7.40 (m, 3H), 4.23 - 4.01 (m, 3H), 2.82 - 2.66 (m, 1H), 2.12 - 1.96 (m, 1H), 1.90 - 1.72 (m, 4H), 1.70 - 1.57 (m, 1H). | Method E | 98.42 | $[\alpha]_D^{20}$= - 181.00 ° (c=0.0 93, in DMF) |

(continued)

| NO. | Structure | $^1$H NMR | Analytical separation method | ee% | [α] |
|---|---|---|---|---|---|
| CK6 | 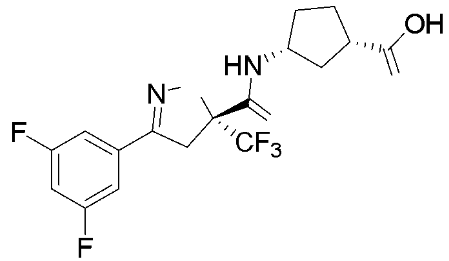 | $^1$H NMR (400 MHz, DMSO) $\delta$ 12.18 (s, 1H), 8.65 (d, J = 7.8 Hz, 1H), 7.60 - 7.39 (m, 3H), 4.22 - 4.00 (m, 3H), 2.81 - 2.65 (m, 1H), 2.16 - 2.03 (m, 1H), 1.87 - 1.69 (m, 4H), 1.69 - 1.57 (m, 1H). | Method E | 100 | $[\alpha]_D^{20}$= - 164.96 ° (c=0.1 08, in DMF) |

**Biological Example 1:** Indoor Herbicide Activity Determination

The testing method for herbicide activity of the compound of the present invention was as follows:

Seed Treatment; pre-emergence: quantitative seeds of gramineous weeds (*Echinochloa crusgalli, Eleusine indica, Digitaria sanguinalis, Alopecurus japonicus, Beckmannia syzigachne, Leptochloa chinensis, Polypogon fugax, Alopecurus aequalis, Lolium multiflorum, Poa annua, Aegilops tauschii, Avena fatua, Alopecurus myosuroides, Alopecurus japonicus* and *Alopecurus aequalis*), broad-leaved weeds (*Eclipta prostrata, Amaranthus retroflexus, Rorippa indica, Malachium aquaticum, Veronica polita, Conyza canadensis, Sesbania cannabina, Sagittariatrifolia* and *Schoenoplectiella juncoides*), and *Cyperus iria* were sown in plastic pots having a diameter of 7 cm and holes at the bottom and filled with nutrient soil (sandy soil, pH 6.1, organic matter 1%) respectively, the seeds were covered with an appropriate amount of soil after being sown, then the soil was wetted with water from the bottom, the seeds were cultured in a constant-temperature illuminated culture room for 24 h, and the soil was sprayed by using a 3WP-2000 traveling spray tower produced by the Nanjing Institute of Agricultural Mechanization of the Ministry of Agriculture, where a rotational speed of a main shaft was 96 mm/r, a spray height was 300 mm, an effective spraying range of a nozzle was 350 mm, a spray area was 0.35 $m^2$, and a flow rate at the nozzle was 390 mL/min.

Post-emergence: an appropriate quantity of seeds of gramineous weeds (*Echinochloa crusgalli, Eleusine indica, Digitaria sanguinalis, Alopecurus japonicus, Beckmannia syzigachne, Leptochloa chinensis, Polypogon fugax, Alopecurus aequalis, Lolium multiflorum, Poa annua, Aegilops tauschii, Avena fatua, Alopecurus myosuroides, Alopecurus japonicus* and *Alopecurus aequalis*), broad-leaved weeds (*Eclipta prostrata, Amaranthus retroflexus, Rorippa indica, Malachium aquaticum, Veronica polita, Conyza canadensis, Sesbania cannabina, Sagittariatrifolia* and *Schoenoplectiella juncoides*), and *Cyperus iria* were sown in plastic pots having a diameter of 7 cm and holes at the bottom and filled with nutrient soil (sandy soil, pH 6.1, organic matter 1%) respectively, the seeds were covered with an appropriate amount of soil after being sown, then the soil was wetted with water from the bottom, the seeds were cultured in a constant-temperature illuminated culture room until a 2-4 leaf stage, and stems and leaves underwent spray treatment. After the treatment, the test materials were placed in a laboratory and cultured in the constant-temperature illuminated culture room after the liquid was naturally dry in the shade, and results were determined 21 days later.

$$\text{Inhibition rate of fresh weight \%} = \left(1 - \frac{\text{fresh weight of treated plants}}{\text{fresh weight of control plants}}\right) \times 100\%$$

Classification standards for prevention and control effects:

A indicates that the inhibition rate was greater than or equal to 80% to 100%;
B indicates that the inhibition rate was greater than or equal to 60% to less than 80%;
C indicates that the inhibition rate was greater than or equal to 40% to less than 60%;
D indicates that the inhibition rate was greater than or equal to 20% to less than 40%;
E indicates that the inhibition rate was less than 20%.

According to the above testing method, some compounds of formula (I) and were selected with the control compounds for parallel testing of herbicide activity. The results were shown in Tables 4-1 to 4-5.

Table 4-1: Herbicidal activities of some compounds of the general formula (I) (Seed Treatment; pre-emergence)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-1** | 240 | A | A | A | A | A |
| **I-3** | 240 | A | A | A | A | A |
| **I-4** | 120 | A | A | A | A | A |
| **I-5** | 120 | A | A | A | A | A |
| **I-6** | 120 | A | B | A | A | A |
| **I-7** | 120 | A | B | A | A | A |
| **I-8** | 240 | A | A | A | A | A |
| **I-9** | 240 | A | A | A | A | A |
| **I-10** | 240 | A | A | A | A | A |

(continued)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-11** | 120 | A | A | A | A | A |
| **I-12** | 120 | B | B | A | A | A |
| **I-13** | 120 | A | B | A | A | A |
| **I-14** | 120 | A | B | A | A | A |
| **I-15** | 120 | A | A | A | A | A |
| **I-16** | 120 | A | A | A | A | A |
| **I-17** | 120 | A | A | A | A | A |
| **I-18** | 120 | A | A | A | A | A |
| **I-19** | 120 | A | A | A | A | A |
| **I-20** | 120 | A | A | A | A | A |
| **I-21** | 120 | A | A | A | A | A |
| **I-22** | 120 | A | B | A | A | A |
| **I-23** | 120 | A | A | A | A | A |
| **I-24** | 120 | A | - | - | - | - |
| **I-25** | 120 | A | A | A | A | A |
| **I-26** | 120 | A | - | - | - | - |
| **I-27** | 240 | A | A | A | A | A |
| **I-29** | 240 | A | A | A | A | A |
| **I-31** | 120 | A | A | - | A | - |
| **I-32** | 120 | A | A | B | A | B |
| **I-33** | 120 | A | A | A | A | A |
| **I-34** | 120 | A | A | B | A | B |
| **I-35** | 120 | A | A | A | A | A |
| **I-36** | 120 | A | A | B | A | A |
| **I-37** | 240 | A | A | A | A | A |
| **I-39** | 240 | A | A | A | A | A |
| **I-41** | 240 | A | - | A | A | A |
| **I-43** | 240 | A | - | A | A | A |
| **I-47** | 240 | A | - | A | A | A |
| **I-49** | 240 | A | A | A | A | A |
| **I-51** | 240 | A | A | A | A | A |
| **I-55** | 240 | A | - | A | A | A |
| **I-57** | 240 | A | A | A | A | A |
| **I-59** | 240 | A | A | A | A | A |
| **I-61** | 120 | A | A | A | A | A |
| **I-62** | 120 | A | B | A | A | A |
| **I-63** | 120 | A | B | A | A | A |
| **I-64** | 120 | A | B | A | A | A |
| **I-65** | 120 | A | B | A | A | A |

(continued)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-66** | 120 | A | - | - | A | - |
| **I-69** | 240 | A | A | A | A | A |
| **I-71** | 240 | A | - | A | A | A |
| **I-73** | 240 | A | - | A | A | A |
| **I-75** | 240 | A | - | - | A | A |
| **I-77** | 240 | A | - | - | A | A |
| **I-79** | 240 | A | A | A | A | - |
| **I-81** | 240 | A | A | A | A | - |
| **I-85** | 120 | - | - | A | A | A |
| **I-89** | 240 | A | - | A | A | A |
| **I-93** | 240 | A | - | A | A | A |
| **I-97** | 240 | A | - | - | - | - |
| **I-101** | 240 | - | - | - | A | - |
| **I-105** | 240 | A | - | - | A | A |
| **I-109** | 240 | A | - | - | A | A |
| **I-111** | 240 | A | A | A | A | A |
| **I-113** | 240 | A | A | A | A | A |
| **I-114** | 120 | A | A | A | A | A |
| **I-115** | 240 | A | - | A | A | A |
| **I-116** | 240 | A | - | A | A | A |
| **I-117** | 240 | A | - | A | A | A |
| **I-118** | 240 | A | - | A | A | A |
| **I-119** | 240 | A | - | A | A | A |
| **I-120** | 240 | A | - | A | A | A |
| **I-121** | 120 | A | A | A | A | A |
| **I-122** | 120 | A | A | A | A | A |
| **I-123** | 120 | B | A | A | A | A |
| **I-124** | 120 | A | A | A | A | A |
| **I-125** | 120 | B | A | A | A | A |
| **I-126** | 120 | A | A | A | A | A |
| **I-127** | 120 | B | A | A | A | A |
| **I-128** | 120 | A | A | A | A | A |
| **I-129** | 120 | B | A | A | A | A |
| **I-130** | 120 | A | A | A | A | A |
| **I-131** | 120 | A | - | A | A | A |
| **I-132** | 240 | A | A | A | A | A |
| **I-134** | 240 | A | A | A | A | A |
| **I-135** | 120 | A | A | A | A | A |
| **I-136** | 120 | A | A | A | A | A |

(continued)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-137** | 240 | A | A | A | A | A |
| **I-139** | 240 | A | A | A | A | A |
| **I-141** | 240 | A | - | A | A | A |
| **I-143** | 240 | A | - | A | A | A |
| **I-147** | 240 | A | - | A | A | A |
| **I-149** | 240 | A | A | A | A | A |
| **I-151** | 240 | A | - | A | A | A |
| **I-153** | 240 | A | - | A | A | A |
| **I-155** | 240 | A | - | A | A | A |
| **I-157** | 240 | A | A | A | A | A |
| **I-159** | 240 | A | A | A | A | A |
| **I-161** | 120 | A | A | A | A | A |
| **I-164** | 240 | A | - | A | A | A |
| **I-166** | 240 | A | - | A | A | A |
| **I-168** | 240 | A | - | A | A | A |
| **I-170** | 240 | A | - | A | A | A |
| **I-172** | 240 | A | - | A | A | A |
| **I-174** | 240 | A | - | A | A | - |
| **I-176** | 240 | A | - | A | A | - |
| **I-180** | 240 | A | - | A | A | A |
| **I-184** | 240 | A | - | A | A | A |
| **I-188** | 240 | A | - | A | A | A |
| **I-192** | 240 | A | - | A | A | - |
| **I-196** | 240 | - | - | - | A | - |
| **I-200** | 240 | A | - | A | A | A |
| **I-204** | 240 | - | A | - | - | - |
| **I-206** | 240 | A | A | A | A | A |
| **I-208** | 240 | A | A | A | A | A |
| **I-210** | 120 | A | A | A | A | A |
| **I-211** | 120 | A | A | A | A | A |
| **I-212** | 120 | A | A | A | A | A |
| **I-213** | 120 | A | A | A | A | A |
| **I-214** | 120 | A | A | A | A | A |
| **I-215** | 120 | A | A | A | A | A |
| **I-216** | 120 | A | A | A | A | A |
| **I-217** | 120 | A | A | A | A | A |
| **I-218** | 120 | A | A | A | A | A |
| **I-219** | 120 | A | A | A | A | A |
| **I-220** | 120 | A | A | A | A | A |

(continued)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-221** | 120 | A | A | A | A | A |
| **I-222** | 120 | A | - | - | - | - |
| **I-223** | 240 | A | A | A | A | A |
| **I-225** | 240 | A | A | A | A | A |
| **I-227** | 120 | A | A | A | A | A |
| **I-228** | 120 | A | A | A | A | A |
| **I-229** | 240 | A | A | A | A | A |
| **I-231** | 240 | A | A | A | A | A |
| **I-233** | 240 | A | A | A | A | A |
| **I-235** | 240 | A | - | A | A | A |
| **I-239** | 240 | A | - | A | A | A |
| **I-241** | 240 | A | A | A | A | A |
| **I-243** | 240 | A | A | A | A | A |
| **I-245** | 240 | A | A | A | A | A |
| **I-247** | 240 | A | - | A | A | A |
| **I-249** | 240 | A | A | A | A | A |
| **I-251** | 240 | A | A | A | A | A |
| **I-253** | 120 | A | A | - | A | A |
| **I-254** | 120 | A | A | - | A | A |
| **I-255** | 120 | A | A | - | A | A |
| **I-258** | 240 | A | - | A | A | A |
| **I-260** | 240 | A | - | A | A | A |
| **I-262** | 240 | A | - | A | A | A |
| **I-264** | 240 | A | - | A | A | A |
| **I-266** | 240 | A | - | A | A | A |
| **I-268** | 240 | A | A | A | A | A |
| **I-270** | 240 | A | A | A | A | A |
| **I-274** | 240 | A | A | A | A | B |
| **I-278** | 240 | A | A | A | A | A |
| **I-282** | 240 | A | - | - | - | - |
| **I-286** | 240 | A | - | - | - | - |
| **I-290** | 240 | A | - | A | A | A |
| **I-294** | 240 | A | A | A | A | A |
| **I-298** | 240 | A | - | A | A | A |
| **I-303** | 120 | A | B | A | A | A |
| **I-307** | 120 | - | - | - | - | A |
| **I-310** | 240 | A | - | A | A | A |
| **I-312** | 240 | A | A | A | A | A |
| **I-314** | 240 | A | A | A | A | A |

(continued)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-316** | 240 | A | - | A | A | A |
| **I-318** | 240 | A | A | A | A | A |
| **I-322** | 240 | A | - | B | A | A |
| **I-326** | 240 | A | - | A | A | A |
| **I-328** | 240 | A | - | A | A | A |
| **I-329** | 240 | A | - | A | A | A |
| **I-330** | 240 | A | - | A | A | A |
| **I-331** | 240 | A | A | A | A | A |
| **I-332** | 240 | A | - | A | A | A |
| **I-333** | 240 | A | A | A | A | A |
| **I-335** | 240 | A | A | A | A | A |
| **I-337** | 240 | A | - | A | A | A |
| **I-339** | 240 | A | - | A | A | A |
| **I-341** | 240 | A | - | A | A | A |
| **I-343** | 240 | A | - | A | A | A |
| **I-347** | 240 | A | A | A | A | A |
| **I-349** | 240 | A | - | A | A | A |
| **I-350** | 240 | A | - | A | A | A |
| **I-352** | 240 | A | - | A | A | A |
| **I-356** | 240 | A | A | A | A | A |
| **I-357** | 240 | A | A | A | A | A |
| **I-358** | 240 | A | - | A | A | B |
| **I-360** | 240 | A | - | A | A | B |
| **I-362** | 240 | A | A | A | A | A |
| **I-363** | 240 | A | - | A | A | A |
| **I-364** | 240 | A | - | A | A | A |
| **I-365** | 240 | A | - | A | A | A |
| **I-366** | 240 | A | A | A | A | A |
| **I-367** | 240 | - | - | - | - | B |
| **I-368** | 240 | A | A | A | A | A |
| **I-369** | 240 | A | A | A | A | A |
| **I-370** | 240 | A | A | A | A | A |
| **I-371** | 240 | A | A | A | A | A |
| **I-372** | 240 | A | A | A | A | A |
| **I-373** | 240 | A | B | A | A | A |
| **I-374** | 240 | A | A | A | A | A |
| **I-375** | 240 | A | B | A | A | A |
| **I-376** | 240 | A | - | B | B | - |
| **I-377** | 240 | A | - | A | A | A |

(continued)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-378** | 240 | A | - | A | A | A |
| **I-380** | 240 | A | - | A | A | A |
| **I-382** | 240 | A | - | A | A | A |
| **I-383** | 240 | A | - | A | A | A |
| **I-384** | 240 | A | - | A | A | A |
| **I-385** | 240 | A | - | B | A | A |
| **I-386** | 240 | A | - | A | A | A |
| **I-387** | 240 | A | - | A | A | A |
| **I-388** | 240 | A | - | B | A | A |
| **I-390** | 240 | A | - | B | A | A |
| **I-392** | 240 | A | - | A | A | A |
| **I-393** | 240 | A | - | A | A | A |
| **I-394** | 240 | A | - | A | A | A |
| **I-395** | 240 | A | - | A | A | - |
| **I-397** | 240 | A | - | A | A | - |
| **I-399** | 240 | A | - | B | A | B |
| **I-400** | 240 | A | - | - | A | - |
| **I-403** | 240 | A | A | A | A | A |
| **I-404** | 120 | A | B | A | A | A |
| **I-416** | 120 | B | C | D | E | E |
| **I-426** | 120 | A | A | A | A | A |
| **I-428** | 120 | A | A | A | A | A |
| **I-429** | 120 | A | A | A | A | A |
| **I-430** | 120 | A | A | A | A | A |
| **I-431** | 120 | A | A | A | A | A |
| **I-432** | 120 | A | A | A | A | A |
| **I-433** | 120 | A | A | B | A | B |
| **I-434** | 120 | A | A | A | A | A |
| **I-435** | 120 | A | A | B | A | A |
| **I-436** | 120 | A | A | B | A | A |
| **I-437** | 120 | A | A | A | A | A |
| **I-438** | 120 | A | A | - | - | - |
| **I-439** | 120 | A | A | - | - | - |
| **I-440** | 120 | A | A | B | A | A |
| **I-441** | 120 | A | A | A | A | A |
| **I-442** | 120 | A | A | B | A | B |
| **I-443** | 120 | A | A | - | - | - |
| **I-444** | 120 | A | A | - | - | - |
| **I-445** | 120 | A | A | A | A | A |

(continued)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-446** | 120 | A | B | - | B | - |
| **I-447** | 120 | A | A | B | B | B |
| **I-448** | 120 | A | A | B | B | A |
| **I-449** | 120 | A | - | - | - | - |
| **I-450** | 120 | A | - | - | - | - |
| **I-451** | 120 | A | - | A | A | A |
| **I-452** | 120 | A | - | - | - | - |
| **I-454** | 120 | A | A | A | A | A |
| **I-456** | 120 | A | A | A | A | A |
| **I-457** | 120 | A | A | B | A | - |
| **I-458** | 120 | A | A | A | A | A |
| **I-459** | 120 | A | A | A | A | A |
| **I-460** | 120 | A | A | A | A | A |
| **I-461** | 120 | A | A | A | A | A |
| **I-462** | 120 | A | B | A | A | A |
| **I-463** | 120 | A | A | A | A | A |
| **I-464** | 120 | A | A | A | A | A |
| **I-465** | 120 | A | B | A | A | A |
| **I-466** | 120 | A | B | A | A | A |
| **I-467** | 120 | A | A | A | A | A |
| **I-468** | 120 | A | - | A | B | B |
| **I-469** | 120 | A | - | B | B | - |
| **I-470** | 120 | A | A | - | - | - |
| **I-471** | 120 | A | A | - | - | - |
| **I-472** | 120 | A | A | - | - | B |
| **I-473** | 120 | A | A | B | B | B |
| **I-474** | 120 | A | A | A | A | A |
| **I-475** | 120 | A | A | - | - | - |
| **I-476** | 120 | A | A | - | B | B |
| **I-477** | 120 | A | A | - | - | B |
| **I-478** | 120 | A | A | B | A | A |
| **I-479** | 120 | A | A | B | A | A |
| **I-480** | 120 | A | A | B | A | A |
| **I-481** | 120 | A | A | B | A | A |
| **I-482** | 120 | A | A | B | A | A |
| **I-483** | 120 | A | A | - | - | - |
| **I-484** | 120 | A | A | - | - | - |
| **I-485** | 240 | A | A | A | A | A |
| **I-486** | 240 | A | A | A | A | A |

(continued)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-487** | 240 | A | A | A | A | A |
| **I-488** | 240 | A | A | A | A | A |
| **I-489** | 240 | A | A | A | A | A |
| **I-490** | 240 | A | A | A | A | A |
| **I-491** | 240 | A | A | A | A | A |
| **I-492** | 240 | A | A | A | A | A |
| **I-493** | 240 | A | A | A | A | A |
| **I-494** | 120 | A | A | A | A | A |
| **I-495** | 240 | A | A | A | A | A |
| **I-496** | 120 | A | A | A | A | A |
| **I-497** | 240 | A | A | A | A | A |
| **I-498** | 120 | A | A | A | A | A |
| **I-499** | 120 | A | A | A | A | A |
| **I-500** | 120 | A | A | A | A | A |
| **I-501** | 120 | A | A | - | - | - |
| **I-502** | 120 | A | A | - | - | - |
| **I-503** | 120 | A | A | - | - | - |
| **I-504** | 240 | A | B | A | A | A |
| **I-505** | 240 | A | B | A | A | A |
| **I-506** | 240 | A | A | A | A | A |
| **I-507** | 240 | A | A | A | A | A |
| **I-508** | 240 | A | A | A | A | A |
| **I-509** | 240 | A | A | A | A | A |
| **I-510** | 120 | A | A | A | A | A |
| **I-511** | 240 | A | A | A | A | A |
| **I-512** | 120 | A | A | A | A | A |
| **I-513** | 120 | A | A | A | A | A |
| **I-514** | 120 | A | A | A | A | A |
| **I-515** | 120 | A | A | - | - | - |
| **I-516** | 120 | A | A | A | A | A |
| **I-517** | 120 | A | A | A | A | A |
| **I-518** | 120 | A | A | A | A | A |
| **I-519** | 120 | A | A | A | A | A |
| **I-520** | 120 | - | - | A | A | B |
| **I-521** | 120 | A | A | A | A | A |
| **I-522** | 120 | A | A | A | A | A |
| **I-523** | 120 | A | A | - | - | - |
| **I-524** | 120 | A | A | - | B | A |
| **I-525** | 120 | A | A | - | - | - |

(continued)

| NO. | Dosage [g a.i./ha] | *Leptochloa chinensis* | *Eleusine indica* | *Lolium multiflorum* | *Alopecurus myosuroides* | *Alopecurus japonicus* |
|---|---|---|---|---|---|---|
| **I-526** | 120 | A | A | - | - | - |
| **I-527** | 120 | A | A | - | - | - |
| **I-528** | 120 | A | A | - | - | - |
| **I-529** | 120 | A | A | A | A | A |
| **I-530** | 120 | A | A | A | A | A |
| **I-531** | 120 | A | A | A | A | A |
| **I-532** | 120 | A | A | - | - | - |
| **I-533** | 120 | A | A | - | - | - |
| **I-569** | 120 | A | - | A | A | A |
| **I-570** | 120 | A | A | A | A | A |
| **I-571** | 120 | A | A | A | A | A |
| **I-572** | 120 | A | A | A | A | A |
| **I-573** | 120 | A | B | A | A | A |
| "-" = Not tested | | | | | | |

Table 4-2: Herbicidal activities of some compounds of the general formula (I) and control compounds (30 g a.i./ha, post-emergence)

| NO. | *Digitaria sanguinalis* | *Eclipta prostrata* | *Cyperus iria* |
|---|---|---|---|
| **I-4** | A | B | A |
| **I-5** | A | C | A |
| **I-210** | A | - | A |
| **I-211** | A | - | A |
| **I-212** | A | - | A |
| **I-213** | A | - | A |
| **I-214** | A | - | A |
| **I-215** | A | - | A |
| **I-216** | A | - | A |
| **I-217** | A | - | A |
| **I-219** | A | - | A |
| **I-221** | A | - | A |
| **I-220** | A | - | B |
| **I-218** | A | - | A |
| **I-432** | B | - | B |
| **I-438** | B | B | B |
| **I-440** | B | - | B |
| **I-441** | B | - | A |
| **I-517** | A | - | A |
| **I-518** | A | - | A |
| **I-476** | - | - | A |

(continued)

| NO. | *Digitaria sanguinalis* | *Eclipta prostrata* | *Cyperus iria* |
|---|---|---|---|
| **I-477** | B | - | A |
| **I-454** | A | - | A |
| **I-456** | A | - | - |
| **I-457** | A | - | A |
| **I-529** | - | - | A |
| **I-531** | A | - | A |
| icafolin | C | E | C |
| icafolin-methyl | C | C | C |
| CK1 | B | E | B |
| CK2 | B | D | B |
| CK3 | C | E | C |
| CK4 | D | E | D |
| CK5 | C | E | C |
| CK6 | D | E | D |
| "-" = Not tested | | | |

Table 4-3: Herbicidal activities of some compounds of the general formula (I) and control compounds (60 g a.i./ha, post-emergence)

| NO. | *Digitaria sanguinalis* | *Eclipta prostrata* | *Cyperus iria* |
|---|---|---|---|
| **I-1** | A | B | A |
| **I-111** | A | B | A |
| **I-113** | A | B | A |
| **I-206** | A | A | A |
| **I-208** | A | A | A |
| **I-303** | A | - | A |
| **I-310** | A | B | A |
| **I-347** | B | C | A |
| **I-356** | B | C | A |
| **I-27** | A | C | A |
| **I-29** | A | C | A |
| **I-132** | A | B | A |
| **I-134** | A | B | A |
| **I-223** | A | B | A |
| **I-225** | A | B | A |
| **I-37** | A | B | A |
| **I-39** | A | B | A |
| **I-137** | A | C | A |
| **I-139** | A | C | A |
| **I-229** | A | B | A |
| **I-231** | A | B | A |

(continued)

| NO. | *Digitaria sanguinalis* | *Eclipta prostrata* | *Cyperus iria* |
|---|---|---|---|
| **I-268** | A | C | A |
| **I-270** | A | C | A |
| **I-49** | B | B | A |
| **I-51** | B | B | A |
| **I-241** | A | B | A |
| **I-243** | A | B | A |
| **I-55** | B | B | A |
| **I-245** | A | B | A |
| **I-247** | A | B | A |
| **I-57** | A | B | A |
| **I-59** | A | B | A |
| **I-157** | A | B | B |
| **I-159** | A | B | B |
| **I-249** | A | B | A |
| **I-251** | A | B | A |
| 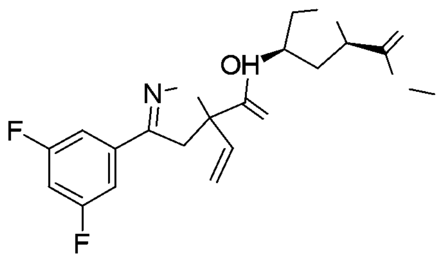 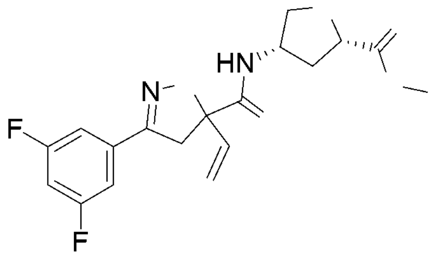 N N)-isomer (WO2018228986 A1) | C | C | C |
| "-" = Not tested | | | |

Table 4-4: Herbicidal activities of some compounds of the general formula (I) and control compounds (60 g a.i./ha, Seed Treatment; pre-emergence)

| NO. | *Digitaria sanguinalis* | *Eclipta prostrata* | *Cyperus iria* | *Aegilops tauschii* |
|---|---|---|---|---|
| **I-4** | A | A | A | - |
| **I-5** | A | - | B | A |
| **I-15** | B | - | - | A |
| **I-19** | A | - | B | A |
| **I-23** | - | B | A | A |
| **I-128** | B | - | - | A |
| **I-129** | B | - | - | A |

(continued)

| NO. | *Digitaria sanguinalis* | *Eclipta prostrata* | *Cyperus iria* | *Aegilops tauschii* |
|---|---|---|---|---|
| **I-210** | A | - | A | A |
| **I-211** | A | - | A | A |
| **I-212** | A | - | A | A |
| **I-213** | A | - | A | A |
| **I-214** | A | - | A | A |
| **I-215** | A | - | A | A |
| **I-216** | A | - | A | A |
| **I-217** | A | - | A | A |
| **I-218** | A | - | A | A |
| **I-219** | A | - | A | A |
| **I-220** | A | - | A | A |
| **I-221** | A | - | A | A |
| **I-31** | - | A | B | B |
| **I-32** | - | A | B | B |
| **I-33** | - | A | B | B |
| **I-34** | - | A | B | B |
| **I-36** | - | B | B | B |
| **I-35** | - | B | B | B |
| **I-64** | - | A | - | B |
| **I-65** | - | B | - | A |
| **I-253** | - | B | A | - |
| **I-254** | - | A | A | - |
| **I-255** | B | B | A | A |
| **I-426** | A | B | A | - |
| **I-428** | A | B | A | - |
| **I-429** | B | B | B | B |
| **I-431** | - | A | A | - |
| **I-436** | A | B | B | - |
| **I-437** | A | B | A | - |
| **I-438** | - | A | A | - |
| **I-439** | - | A | A | - |
| **I-440** | B | B | A | - |
| **I-443** | A | A | A | - |
| **I-444** | - | A | A | - |
| **I-501** | A | B | B | - |
| **I-516** | A | B | A | A |
| **I-517** | A | - | A | - |
| **I-518** | B | A | A | - |
| **I-519** | A | A | A | - |

(continued)

| NO. | *Digitaria sanguinalis* | *Eclipta prostrata* | *Cyperus iria* | *Aegilops tauschii* |
|---|---|---|---|---|
| **I-521** | A | A | A | A |
| **I-532** | - | A | A | - |
| **I-477** | B | C | B | A |
| **I-478** | - | A | A | - |
| **I-483** | - | A | A | - |
| **I-514** | - | A | A | - |
| **I-524** | A | B | A | - |
| **I-463** | B | B | B | B |
| **I-459** | B | B | B | - |
| **I-531** | B | A | A | - |
| **I-471** | - | A | A | - |
| icafolin | D | E | D | C |
| icafolin-methyl | D | C | D | C |
| CK3 | B | B | C | C |
| CK4 | E | E | C | E |
| CK5 | B | C | B | D |
| CK6 | E | E | C | E |
| "-" = Not tested | | | | |

Table 4-5: Herbicidal activities of some compounds of the general formula (I) and control compounds 120 g a.i./ha, Seed Treatment; pre-emergence)

| NO. | *Digitaria sanguinalis* | *Eclipta prostrata* | *Cyperus iria* | *Aegilops tauschii* |
|---|---|---|---|---|
| **I-1** | - | - | A | A |
| **I-3** | A | A | A | - |
| **I-8** | - | - | A | A |
| **I-9** | - | B | A | A |
| **I-10** | - | B | A | A |
| **I-206** | A | B | A | A |
| **I-208** | A | A | A | A |
| **I-368** | B | C | B | A |
| **I-377** | A | C | B | A |
| **I-392** | A | B | B | A |
| **I-371** | B | C | B | A |
| **I-386** | A | C | B | A |
| **I-399** | A | B | B | A |
| **I-369** | B | C | B | A |
| **I-378** | A | C | B | A |
| **I-380** | A | - | A | A |
| **I-382** | A | - | A | A |

(continued)

| NO. | *Digitaria sanguinalis* | *Eclipta prostrata* | *Cyperus iria* | *Aegilops tauschii* |
|---|---|---|---|---|
| **I-383** | A | - | B | A |
| **I-384** | A | - | B | A |
| **I-393** | B | B | B | A |
| **I-372** | A | A | A | A |
| **I-387** | A | B | B | A |
| **I-400** | A | A | B | A |
| **I-370** | B | B | A | A |
| **I-385** | B | - | - | A |
| **I-394** | B | B | - | A |
| **I-373** | A | A | B | B |
| **I-388** | B | B | B | A |
| **I-390** | B | B | B | A |
| **I-395** | B | B | B | B |
| **I-493** | B | B | A | A |
| **I-495** | - | B | A | A |
| **I-497** | - | - | A | A |
| **I-511** | A | A | A | A |
| **I-488** | B | A | A | A |
| **I-489** | B | - | A | A |
| **I-490** | A | A | A | A |
| **I-492** | A | A | B | A |
| **I-485** | B | A | A | A |
| **I-486** | B | A | A | A |
| **I-487** | B | A | A | A |
| **I-505** | A | A | A | A |

(continued)

| NO. | *Digitaria sanguinalis* | *Eclipta prostrata* | *Cyperus iria* | *Aegilops tauschii* |
|---|---|---|---|---|
| 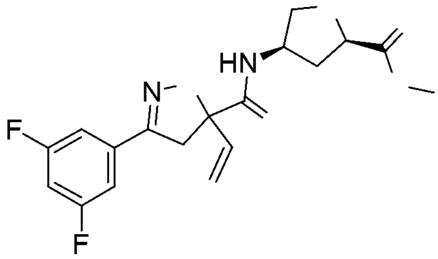 ,4 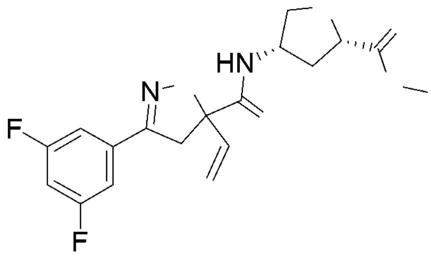 N N)-isomer (WO2018228986 A1) | D | C | D | C |
| "-" = Not tested | | | | |

**Biological Example 2:** Field Herbicide Activity Determination

Apply the herbicide of the present invention to the vacant land in the vineyard for controlling unwanted vegetation.

The experimental plot has a flat terrain and medium loam soil. The main weeds in the field include *Eleusine indica, Echinochloa crus-galli, Digitaria sanguinalis, Alternanthera philoxeroides,* and *Erigeron canadensis.* Spray before weeds emerge, the spray adopts a fan-shaped nozzle dedicated to herbicides, Water consumption of spray is 450 L/ha. The area of each treated experimental community is 20 m$^2$, weigh the required herbicide dosage for each experimental community according to the set dosage, prepare herbicides into corresponding volumes of solution using secondary dilution method.

Investigate the plant control effect of herbicides on weeds 15 days after treatment, investigate the final plant control and fresh weight control effects of herbicides on weeds 30 days after treatment.

The control ratio with quantity (fresh weight) of weed = (Quantity(fresh weight)of weed in the control area- Quantity (fresh weight)of weed in the treatment area)/ Quantity(fresh weight)of weed in the control area$\times$ 100

The results indicated that the compounds of the present invention generally exhibited good weed control efficacy, and can produce good weed control efficacy even at lower application doses.

The above-mentioned examples are only preferred examples of the present invention. It should be pointed out that for one skilled in the art, some changes and improvements can be made without deviating from the idea of the present invention, which should be within the protection scope of the present invention.

## Claims

1. A 3-phenylisoxazoline-5-carboxamide compound of formula (I), stereoisomer thereof and agriculturally acceptable salt thereof

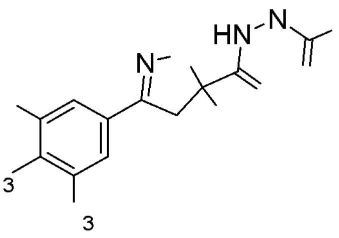

wherein

$R_1$ represents -CN or F,
or
represents $C_1$-$C_5$-alkyl, $C_3$-$C_6$-cycloalkyl, $C_2$-$C_5$-alkenyl, $C_2$-$C_5$-alkynyl or $C_1$-$C_5$-alkoxy, each of which is substituted by $m_1$ radicals selected from halogen, -CN, -OH and $C_1$-$C_5$-alkoxy;
G represents $-OR_3$ or $-NR_4R_5$;
$R_3$ represents H,
or
represents $C_1$-$C_{12}$-alkyl, $C_3$-$C_7$-cycloalkyl, $C_3$-$C_7$-cycloalkyl-$C_1$-$C_8$-alkyl, $C_2$-$C_8$-alkenyl, $C_2$-$C_8$-alkynyl, $C_5$-$C_6$-cycloalkenyl, -N=($C_1$-$C_6$-cycloalkyl), -N=C($C_1$-$C_5$-alkyl)$_2$, phenyl, $C_1$-$C_4$-alkyl-phenyl, aromatic heterocyclic group or $C_1$-$C_4$-alkyl-aromatic heterocyclic group, each of which is optionally substituted by $m_3$ radicals selected from halogen, -CN, -OH, $C_1$-$C_6$-alkoxy, $C_1$-$C_6$-alkoxycarbonyl, aromatic heterocyclic group, aryl group and $-S(O)_nR_2$;
$R_4$ and $R_5$ independently of one another each represent H, -OH, $C_1$-$C_{12}$-alkyl, $C_1$-$C_3$-alkoxy, $C_1$-$C_6$-alkoxy-$C_1$-$C_3$-alkyl, $C_1$-$C_6$-alkoxycarbonyl-$C_1$-$C_6$-alkyl, N($C_1$-$C_3$-alkyl)$_2$ or $-S(O)_nR_2$,
or
$R_4$ and $R_5$ together with the nitrogen atom to which they are attached form a saturated, partially or fully unsaturated five-, six- or seven-membered ring which, in addition to the nitrogen atom, contains r carbon atoms and o oxygen atoms, and which is optionally substituted by $m_4$ radicals selected from halogen, $C_1$-$C_6$-alkyl, halogenated $C_1$-$C_6$-alkyl, oxo and $-CO_2R_6$;
$R_6$ represents H,
or
represents $C_1$-$C_8$-alkyl, $C_3$-$C_6$-cycloalkyl, $C_3$-$C_8$-alkenyl or $C_3$-$C_8$-alkynyl, each of which is optionally substituted by $m_5$ radicals selected from halogen, -CN and $C_1$-$C_2$-alkoxy;
when Z is selected from Z-1 to Z-4, where Z-1 to Z-4 have the following meaning:

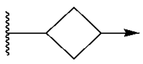 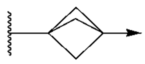 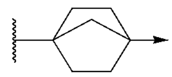 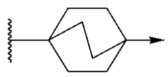

OH **Z-2** **Z-2**

$X_3$ represents H, F, Cl, Br or I;
$X_1$ and $X_2$ independently of one another each represent H, F, Cl, Br, I, -OH, -CN, $-NO_2$, - $S(O)_nR_2$ or $-CO_2R_6$,
or
represent $C_1$-$C_3$-alkyl, $C_1$-$C_3$-alkoxy, $C_3$-$C_4$-cycloalkyl, $C_2$-$C_3$-alkenyl or $C_2$-$C_3$-alkynyl, each of which is substituted by $m_6$ radicals selected from F, Cl, Br and I;
when Z is selected from Z-5 to Z-7, where Z-5 to Z-7 have the following meaning:

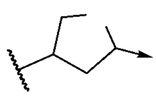 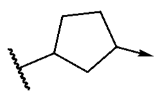 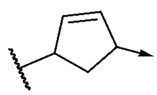

$X_3$ represents H or F;
$X_1$ represents F, Cl, Br, I, $-CH_3$, -CN, $-NO_2$, $-S(O)_nR_2$ or $-OS(O)_nR_2$;
$X_2$ represents $-CF_3$, $-CF_2H$, $-OCF_3$, $-OCF_2H$ or $-OCFH_2$;
$R_2$ represents $C_1$-$C_4$-alkyl or $C_3$-$C_4$-cycloalkyl, each of which is substituted by $m_2$ radicals selected from F and Cl;
where the arrow in each case denotes a bond to the group CO-G of formula (I);
$m_1$ is 0, 1, 2 or 3;

$m_2$ is 0, 1, 2 or 3;
$m_3$ is 0, 1, 2, 3, 4, 5, 6, 7 or 8;
$m_4$ is 0, 1, 2, 3, 4 or 5;
$m_5$ is 0, 1, 2, 3, 4 or 5;
$m_6$ is 0, 1, 2 or 3;
n is 0, 1 or 2;
o is 0, 1 or 2; and
r is 3, 4, 5 or 6.

**2.** The compound of formula (I), stereoisomer thereof and agriculturally acceptable salt thereof according to claim 1, wherein

$R_1$ represents $C_1$-$C_3$-alkyl, $C_3$-$C_4$-cycloalkyl, $C_2$-$C_3$-alkenyl, $C_2$-$C_3$-alkynyl or $C_1$-$C_3$-alkoxy, each of which is substituted by $m_1$ radicals selected from halogen, -CN, -OH and $C_1$-$C_2$-alkoxy;
G represents $-OR_3$ or $-NR_4R_5$;
$R_3$ represents H,
or
represents $C_1$-$C_{10}$-alkyl, $C_3$-$C_6$-cycloalkyl, $C_3$-$C_6$-cycloalkyl-$C_1$-$C_6$-alkyl, $C_2$-$C_6$-alkenyl, $C_5$-$C_6$-cycloalkenyl, -N=($C_1$-$C_5$-cycloalkyl), -N=C($C_1$-$C_3$-alkyl)$_2$, phenyl, $C_1$-$C_3$-alkylphenyl, aromatic heterocyclic group, $C_1$-$C_3$-alkyl aromatic heterocyclic group or $C_2$-$C_6$-alkynyl, each of which is substituted by $m_3$ radicals selected from F, Cl, Br, I, -CN, -OH, $-S(O)_nR_2$, $C_1$-$C_4$-alkoxy, aryl group and aromatic heterocyclic group;
$R_4$ and $R_5$ independently of one another each represent H, -OH, $C_1$-$C_6$-alkyl, $C_1$-$C_3$-alkoxy, $C_1$-$C_3$-alkoxy $C_1$-$C_3$-alkyl or $-S(O)_nR_2$,
or
$R_4$ and $R_5$ together with the nitrogen atom to which they are attached form a saturated, partially or fully unsaturated five- or six-membered ring which, in addition to the nitrogen atom, contains r carbon atoms and o oxygen atoms, and which is optionally substituted by $m_4$ radicals selected from halogen, $C_1$-$C_6$-alkyl, halogenated $C_1$-$C_6$-alkyl, oxo and $-CO_2R_6$;
$R_2$ represents $C_1$-$C_4$-alkyl;
$R_6$ represents H,
or
represents $C_1$-$C_6$-alkyl, $C_3$-$C_6$-cycloalkyl, $C_3$-$C_8$-alkenyl or $C_3$-$C_8$-alkynyl, each of which is optionally substituted by $m_5$ radicals selected from halogen, -CN and $C_1$-$C_2$-alkoxy;
$m_1$ is 0, 1, 2 or 3;
$m_3$ is 0, 1, 2, 3, 4, 5, 6, 7 or 8;
$m_4$ is 0, 1, 2, 3 or 4;
$m_5$ is 0, 1, 2 or 3;
n is 0, 1 or 2;
o is 0, 1 or 2; and
r is 3, 4 or 5.

**3.** The compound of formula (I), stereoisomer thereof and agriculturally acceptable salt thereof according to claim 2, wherein

$R_1$ represents $C_1$-$C_3$-alkyl, $C_2$-$C_3$-alkenyl, $C_2$-$C_3$-alkynyl or $C_1$-$C_3$-alkoxy, each of which is substituted by $m_1$ radicals selected from F, Cl and Br;
G represents $-OR_3$ or $-NR_4R_5$;
$R_3$ represents H,
or
represents $C_1$-$C_7$-alkyl, $C_3$-$C_6$-cycloalkyl, $C_3$-$C_6$-cycloalkyl-$C_1$-$C_6$-alkyl, $C_2$-$C_6$-alkenyl, $C_5$-$C_6$-cycloalkenyl, $C_2$-$C_6$-alkynyl, $C_1$-$C_3$-alkoxy-$C_1$-$C_3$-alkyl, -N=C($C_1$-$C_3$-alkyl)$_2$, phenyl, $C_1$-$C_3$-alkyl-phenyl, aromatic heterocyclic group, $C_1$-$C_3$-alkyl-aromatic heterocyclic group, phenyl-$C_1$-$C_3$-alkyl or aromatic heterocyclic group-$C_1$-$C_3$-alkyl, each of which is substituted by $m_3$ radicals selected from F, Cl, Br, I, -CN, -OH, $-OCH_3$ or $-S(O)_nR_2$;
$R_4$ and $R_5$ independently of one another each represent H, -OH, $C_1$-$C_6$-alkyl, $C_1$-$C_3$-alkoxy or $-S(O)_nR_2$,
or
$R_4$ and $R_5$ together with the nitrogen atom to which they are attached form a saturated five- or six-membered ring which, in addition to the nitrogen atom, contains four or five carbon atoms;
$R_2$ represents $C_1$-$C_3$-alkyl;

$m_1$ is 0, 1, 2 or 3;
$m_3$ is 0, 1, 2, 3, 4, 5, 6, 7 or 8; and
n is 0, 1 or 2.

**4.** The compound of formula (I), stereoisomer thereof and agriculturally acceptable salt thereof according to claim 3, wherein

$R_1$ represents $-CH_3$, $-CH=CH_2$, $-CF=CH_2$, $-CF_3$, $-CF_2H$, $-CH_2F$, $-CH_2Cl$, $-CF_2CH_3$ or $-OCH_3$;
G represents $-OR_3$ or $-NR_4R_5$;
$R_3$ represents H,
or
represents $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$, $-(CH_2)_4CH_3$, $-(CH_2)_5CH_3$, $-(CH_2)_6CH_3$, $-CH_2CH=CH_2$, $-CH_2CH_2CH=CH_2$, $-CH_2CH=CHCH_3$, $-CH(CH_3)CH=CH_2$, $-CH_2C{\equiv}CH$, (S)-$CH(CH_3)C{\equiv}CH$, (R)-$CH(CH_3)C{\equiv}CH$, $-CH_2C{\equiv}CCH_3$, $-CH(CH_2CH_3)C{\equiv}CH$, $-CH_2CH_2S(O)_nCH_3$, $-CH_2CH_2S(O)_nCH_2CH_3$, cyclopropyl, cyclobutyl, cyclopentyl, cyclohexyl, $-CH_2$-cyclopropyl, $-CH_2$-cyclobutyl, $-CH_2$-cyclopentyl, $-CH_2$-cyclohexyl, $-CH_2CH_2CN$, $-CH_2CF_3$, $-CH_2CF_2H$, $-CH_2CH_2F$, $-CH_2CH_2Cl$, $-CH_2CH_2Br$, -CH2CCl3, $-CH_2CF_2CF_3$, $-CH_2(CF_2)_2H$, $-CH_2(CF_2)_3H$, $-CH_2(CF_2)_4H$, $-CH_2CH_2OCH_3$, $-(CH_2)_3OCH_3$, (S)-$CH_2CH(CH_3)OCH_3$, (S)-$CH(CH_3)CH_2OCH_3$, (*R*)-$CH_2CH(CH_3)OCH_3$, (*R*)-$CH(CH_3)CH_2OCH_3$, $-CH_2CH_2OCH_2CH_3$, $-N=C(CH_3)_2$, $-C_6H_5$, *p*-$CH_3$-$C_6H_4$-, *p*-F-$C_6H_4$-, *p*-Cl-$C_6H_4$-, *p*-Br-$C_6H_4$-, $-CH_2$-$C_6H_5$, *p*-$CH_3$-$C_6H_4$-$CH_2$-, *p*-F-$C_6H_4$-$CH_2$-, *p*-Cl-$C_6H_4$-$CH_2$-, *p*-Br-$C_6H_4$-$CH_2$-, *p*-CN-$C_6H_4$-$CH_2$-, *p*-$CH_3O$-$C_6H_4$-$CH_2$- or 3-pyridyl;
n is 0, 1, or 2;
or
$R_4$ and $R_5$ independently of one another each represent H, $-CH_3$, $-CH_2CH_3$, -OH, $-OCH_3$, $-OCH_2CH_3$, $-OCH(CH_3)_2$ or $-SO_2CH_3$,
or
$R_4$ and $R_5$ together with the nitrogen atom to which they are attached form a saturated five- or six-membered ring which, in addition to the nitrogen atom, contains four or five carbon atoms.

**5.** The compound of formula (I), stereoisomer thereof and agriculturally acceptable salt thereof according to claim 1, wherein

Z represents Z-1;
$X_3$ represents H or F;
$X_1$ and $X_2$ independently of one another each represent H, F, Cl, Br or -CN,
or
represent $C_1$-$C_3$-alkyl or $C_1$-$C_3$-alkoxy, each of which is substituted by $m_6$ radicals selected from F, Cl and Br;
$m_6$ is 0, 1, 2, or 3.

**6.** The compound of formula (I), stereoisomer thereof and agriculturally acceptable salt thereof according to claim 5, wherein

Z represents Z-1;
$X_3$ represents H or F;
$X_1$ and $X_2$ independently of one another each represent H, F, Cl, Br, $-CH_3$, -CN, $-OCH_3$, $-CF_3$, $-CF_2H$, $-OCF_3$ or $-OCF_2H$.

**7.** The compound of formula (I), stereoisomer thereof and agriculturally acceptable salt thereof according to claim 1, wherein

Z represents Z-5 or Z-7;
$X_3$ represents H;
$X_1$ represents F, Cl or Br; and
$X_2$ represents $-CF_3$, $-CF_2H$, $-OCF_3$ or $-OCF_2H$.

**8.** A method for preparing the compound of formula (I), stereoisomer thereof and agriculturally acceptable salt thereof according to anyone of claims 1-7, comprising the following steps:

(1) condensing a compound of general formula (V) with hydroxylamine or hydroxylamine salt to produce a compound of general formula (IV);

(2) producing a compound of formula (III) from the compound of formula (IV) under the action of a chlorinating agent;

(3) performing cyclization under the action of a base, or chiral selective cyclization under the action of a chiral catalyst of the compound of general formula (III) with a compound of general formula (VII), to directly give a compound of general formula (II) or to give the compound of general formula (II) upon hydrolysis; and

(4) reacting the compound of general formula (II) with a compound of general formula (VI) to give the 3-phenylisoxazoline-5-carboxamide compound of formula (I);

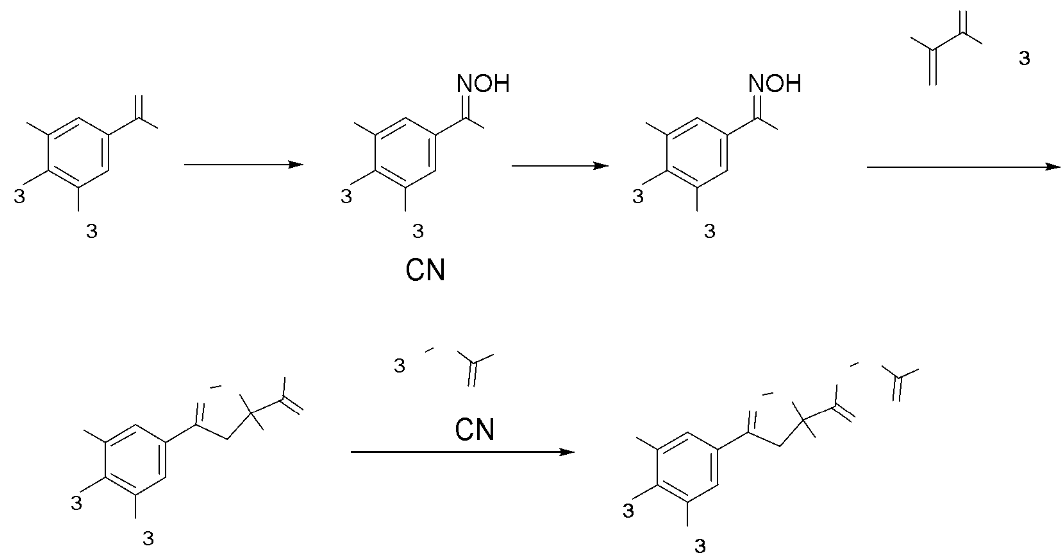

(II)

wherein

$R_7$ represents H,
or
represents $C_1$-$C_5$-alkyl, $C_3$-$C_5$-cycloalkyl, $C_2$-$C_5$-alkenyl, $C_2$-$C_5$-alkynyl or benzyl, each of which is substituted by m radicals selected from F, Cl, Br, -CN, -OH and $C_1$-$C_2$-alkoxy, where $X_1$, $X_2$, $X_3$, $R_1$, G and Z have the meaning according to claims 1-7.

**9.** A herbicidal composition, comprising at least one of the compound of formula (I), stereoisomer thereof or salt thereof according to any of claims 1-7 as an active ingredient, wherein the weight percentage of the active ingredient in the composition is 0.1% to 99.9%.

**10.** The herbicidal composition according to claim 9, further comprising a formulation adjuvant.

**11.** The herbicidal composition according to claim 9, comprising at least one further active compound selected from insecticide, acaricide, herbicide, fungicide, safener, and/or growth regulator.

**12.** The herbicidal composition according to claim 9, comprising a safener.

**13.** The herbicidal composition according to claim 12, wherein the safener is selected from mefenpyr-diethyl, cyprosulfamide, isoxadifen-ethyl, cloquintocet-mexyl, benoxacor, furilazole, dichlormid or Metcamifen.

**14.** A method for controlling harmful plants, comprising applying an effective amount of at least one compound of formula (I) according to anyone of claims 1-7 or the herbicidal composition according to anyone of claims 9-13, to a plant or a site of harmful vegetation.

**15.** Use of the compound of formula (I) according to anyone of claims 1-7 or the herbicidal composition according to anyone of claims 9-13 for controlling harmful plants.

**16.** The use according to claim 15, wherein the compound of formula (I) or herbicidal composition containing the compound of formula (I) is used for controlling harmful plants in crops of useful plants.

**17.** The use according to claim 16, wherein the useful plants are transgenic plants or plants processed by genome editing technology.

## INTERNATIONAL SEARCH REPORT

International application No.
**PCT/CN2024/096921**

**A. CLASSIFICATION OF SUBJECT MATTER**

C07D261/04(2006.01)i； C07D413/12(2006.01)i； A01N43/80(2006.01)i

According to International Patent Classification (IPC) or to both national classification and IPC

**B. FIELDS SEARCHED**

Minimum documentation searched (classification system followed by classification symbols)

IPC： C07D A01N

Documentation searched other than minimum documentation to the extent that such documents are included in the fields searched

Electronic data base consulted during the international search (name of data base and, where practicable, search terms used)

CNABS; CNTXT; DWPI; CNKI; ENTXT; ENTXTC; 超星读秀; DUXIU; ISI_Web of Science; STN-REG; STN-CAPLUS: 江苏中旗科技股份有限公司, 张璞, 张烽, 叶如意, 黄健楠, 白从强, 卜龙, 姚凯诚, 徐丹, 吴耀军, 苯基异噁唑啉, 除草, 结构式(I), herbicid+, phenylisoxazoline

**C. DOCUMENTS CONSIDERED TO BE RELEVANT**

| Category* | Citation of document, with indication, where appropriate, of the relevant passages | Relevant to claim No. |
|---|---|---|
| X | CN 111164077 A (BAYER AG) 15 May 2020 (2020-05-15)<br>claims 1 and 8-16, and description, paragraphs 0157-0159, 0172-0175 and 0429 | 1-4, 8-17 |
| Y | CN 111164077 A (BAYER AG) 15 May 2020 (2020-05-15)<br>claims 1 and 8-16, and description, paragraphs 0157-0159, 0172-0175 and 0429 | 1-17 |
| Y | CN 110770232 A (BAYER AG et al.) 07 February 2020 (2020-02-07)<br>claim 1, and description, paragraph 0164, and embodiment I-1 | 1-17 |
| Y | CN 111868042 A (BAYER AG) 30 October 2020 (2020-10-30)<br>claim 1, and description, paragraph 0221, and embodiment I-007 | 1-17 |
| A | CN 103596936 A (BAYER INTELLECTUAL PROPERTY GMBH) 19 February 2014 (2014-02-19)<br>entire document | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 August 2024** | **24 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**
**Information on patent family members**

International application No.
**PCT/CN2024/096921**

| Patent document cited in search report | Publication date (day/month/year) | Patent family member(s) | Publication date (day/month/year) |
|---|---|---|---|
| CN 111164077 A | 15 May 2020 | JP 2020531419 A | 05 November 2020 |
| | | JP 7198519 B2 | 04 January 2023 |
| | | AR 112693 A1 | 27 November 2019 |
| | | US 2020369630 A1 | 26 November 2020 |
| | | BR 112020003266 A2 | 13 October 2020 |
| | | EP 3668845 A1 | 24 June 2020 |
| | | EP 3668845 B1 | 26 June 2024 |
| | | WO 2019034602 A1 | 21 February 2019 |
| | | CN 111164077 B | 19 December 2023 |
| CN 110770232 A | 07 February 2020 | ES 2894277 T3 | 14 February 2022 |
| | | EA 039486 B1 | 01 February 2022 |
| | | BR 112019026261 A2 | 30 June 2020 |
| | | BR 112019026261 B1 | 19 December 2023 |
| | | AR 112142 A1 | 25 September 2019 |
| | | US 2021292312 A1 | 23 September 2021 |
| | | US 11597724 B2 | 07 March 2023 |
| | | JP 2020523346 A | 06 August 2020 |
| | | JP 7083851 B2 | 13 June 2022 |
| | | MX 2019014980 A | 24 February 2020 |
| | | AU 2018285212 A1 | 16 January 2020 |
| | | AU 2018285212 B2 | 30 June 2022 |
| | | WO 2018228985 A1 | 20 December 2018 |
| | | CA 3066852 A1 | 20 December 2018 |
| | | EP 3638665 A1 | 22 April 2020 |
| | | EP 3638665 B1 | 21 July 2021 |
| | | IN 201917046419 A | 17 January 2020 |
| | | HK 40017722 A0 | 25 September 2020 |
| | | ZA 201907558 A | 26 May 2021 |
| | | IN 411240 B | 18 November 2022 |
| | | MX 398993 B | 06 January 2023 |
| | | CN 110770232 B | 15 August 2023 |
| CN 111868042 A | 30 October 2020 | BR 112020015096 A2 | 08 December 2020 |
| | | WO 2019145245 A1 | 01 August 2019 |
| | | CA 3089286 A1 | 01 August 2019 |
| | | AR 114233 A1 | 05 August 2020 |
| | | ES 2937924 T3 | 03 April 2023 |
| | | EP 3743411 A1 | 02 December 2020 |
| | | EP 3743411 B1 | 21 December 2022 |
| | | JP 2021511340 A | 06 May 2021 |
| | | JP 7217751 B2 | 03 February 2023 |
| | | US 2022306591 A1 | 29 September 2022 |
| | | AU 2019210916 A1 | 13 August 2020 |
| | | AU 2019210916 B2 | 02 March 2023 |
| | | IN 202017031756 A | 18 September 2020 |
| | | ZA 202005252 A | 30 March 2022 |
| | | CN 111868042 B | 05 December 2023 |
| | | IN 488229 B | 29 December 2023 |
| CN 103596936 A | 19 February 2014 | MX 2013011217 A | 17 October 2013 |
| | | MX 350825 B | 22 September 2017 |
| | | CA 2831704 A1 | 04 October 2012 |

Form PCT/ISA/210 (patent family annex) (July 2022)

**INTERNATIONAL SEARCH REPORT**
**Information on patent family members**

International application No.
**PCT/CN2024/096921**

| Patent document cited in search report | Publication date (day/month/year) | Patent family member(s) | Publication date (day/month/year) |
|---|---|---|---|
| | | CA 2831704 C | 01 October 2019 |
| | | US 2014100108 A1 | 10 April 2014 |
| | | US 9078442 B2 | 14 July 2015 |
| | | AU 2012234393 A1 | 17 October 2013 |
| | | AU 2012234393 B2 | 02 February 2017 |
| | | ES 2612694 T3 | 18 May 2017 |
| | | BR 112013024629 A2 | 21 March 2017 |
| | | BR 112013024629 B1 | 16 April 2019 |
| | | EP 2691379 A1 | 05 February 2014 |
| | | EP 2691379 B1 | 02 November 2016 |
| | | JP 2014515015 A | 26 June 2014 |
| | | JP 5968999 B2 | 10 August 2016 |
| | | WO 2012130798 A1 | 04 October 2012 |
| | | WO 2012130798 A8 | 29 August 2013 |
| | | ZA 201306338 A | 23 December 2014 |
| | | CN 103596936 B | 09 November 2016 |
| | | IN 315751 B | 12 July 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012130798 A8
- WO 2014048827 A1
- WO 2019145245 A1
- WO 2020182723 A1
- WO 2018228985 A1
- WO 2019034602 A1
- US 2521902 A
- JP 60112746 B
- US 4272417 A
- US 4245432 A
- US 4808430 A
- US 5876739 A
- US 20030176428 A1
- WO 2002080675 A1
- WO 2002028186 A2
- US 765491
- US 11765494 B
- US 10926819 B
- US 10782020 B
- US 12032479 B
- US 10783417 B
- US 10782096 B
- US 11657964 B
- US 12192904 B
- US 11396808 B
- US 12166253 B
- US 12166239 B
- US 12166124 B
- US 12166209 B
- US 11762886 B
- US 12364335 B
- US 11763947 B
- US 12252453 B
- US 12209354 B
- US 12491396 B
- US 12497221 B
- WO 8910396 A
- WO 0166704 A
- WO 9638567 A
- WO 9924585 A
- WO 9924586 A
- WO 2009144079 A
- WO 2002046387 A
- US 6768044 B
- WO 9934008 A
- WO 0236787 A
- WO 2004024928 A
- WO 2007103567 A
- WO 2008150473 A
- US 5084082 A
- WO 9741218 A
- US 5773702 A
- WO 99057965 A
- US 5198599 A
- WO 01065922 A
- WO 2018228986 A1


### Non-patent literature cited in the description


- Reviews: 1,3 dipolar Cycloaddition Chemistry. Wiley, 1984
- **KANEMASA** ; **TSUGE**. *Heterocycles*, 1990, vol. 30, 719
- **KIM, JAE N.** ; **RYU, EUNG K.** *J. Org. Chem.*, 1992, vol. 57, 6649
- **OLSSEN**. *J. Org. Chem.*, 1988, vol. 53, 2468
- **CHEN, F. M. F.** ; **BENOITON, N. L.** *Synthesis*, 1979, 709
- *J. of Polymer Science*, 1979, vol. 17 (6), 1655
- **D. TIEBES**. Combinatorial Chemistry-Synthesis, Analysis, Screening. Wiley, 1999, 1-34
- **WINNACKER-KÜCHLER**. Chemische Technologie'' [Chemical Technology. C. Hauser Verlag, 1986, vol. 7
- **WADE VAN VALKENBURG**. Pesticide Formulations. Marcel Dekker, 1973
- Spray Drying. **K. MARTENS**. Handbook. G. Goodwin Ltd, 1979
- **WATKINS**. Handbook of Insecticide Dust Diluents and Carriers. Darland Books
- **H. V. OLPHEN**. Introduction to Clay Colloid Chemistry. J. Wiley & Sons
- **C. MARSDEN**. Solvents Guide. Interscience, 1963
- **MCCUTCHEON**. Detergents and Emulsifiers Annual. MC Publ. Corp.
- **SISLEY** ; **WOOD**. Encyclopedia of Surface Active Agents. Chem. Publ. Co. Inc., 1964
- **SCHÖNFELDT**. Grenzflüchenaktive Äthylenoxidaddkte'' [Surface-active ethylene oxide adducts. Wiss. Verlagagesell, 1976
- Spray-Drying Handbook. G. Goodwin Ltd., 1979

- **J. E. BROWNING**. Agglomeration. *Chemical and Engineering*, 1967, 147
- Perry's Chemical Engineer's Handbook. McGraw-Hill, 1973, 8-57
- **G. C. KLINGMAN**. Weed Control as a Science. John Wiley and Sons Inc., 1961, 81-96
- **J. D. FREYER** ; **S. A. EVANS**. Weed Control Handbook. Blackwell Scientific Publications, 1968, 101-103
- World Herbicide New Product Technology Handbook. China Agricultural Science and Farming Techniques Press, 2010
- **COMAI et al.** *Science*, 1983, vol. 221, 370-371
- **BARRY et al.** *Curr. Topics Plant Physiol.*, 1992, vol. 7, 139-145
- **SHAH et al.** *Science*, 1986, vol. 233, 478-481
- **GASSER et al.** *J. Biol. Chem.*, 1988, vol. 263, 4280-4289
- *Weed Science*, 2002, vol. 50, 700-712
- **MARCO D. MIGLIORE et al.** *J Med. Chem.*, 2007, vol. 50, 6485-6492